# EUROPEAN PATENT SPECIFICATION

(11) **EP 2 023 940 B1**
(45) Date of publication and mention of the grant of the patent: **22.06.2011**
(21) Application number: 07811875.9
(22) Date of filing: 07.05.2007
(51) Int. Cl.: C12N 15/113

(54) **COMPOUNDS AND METHODS FOR MODULATING EXPRESSION OF SGLT2**
VERBINDUNGEN UND VERFAHREN ZUR MODULATION DER EXPRESSION VON SGLT2
COMPOSÉS ET PROCÉDÉS DE MODULATION DE L'EXPRESSION DE SGLT2

(30) Priority: 05.05.2006 US 746631 P; 11.05.2006 US 747059 P; 23.06.2006 US 805660 P; 06.11.2006 US 864554 P; 27.01.2007 WO PCT/US2007/061183
(43) Date of publication of application: 18.02.2009
(73) Proprietor: Isis Pharmaceuticals, Inc., Carlsbad, CA 92008 (US)
(72) Inventor: BHANOT, Sanjay, Carlsbad, CA 92009 (US); GEARY, Richard, S., Carlsbad, CA 92009 (US); MCKAY, Robert, Poway, CA 92064 (US); MONIA, Brett, P., Encinitas, CA 92024 (US); SETH, Punit, P., San Marcos, CA 92078 (US); SIWKOWSKI, Andrew, M., Carlsbad, CA 92009 (US); SWAYZE, Eric, E., Carlsbad, CA 92009 (US); WANCEWICZ, Edward, Poway, CA 92064 (US)
(74) Representative: Hallybone, Huw George
(86) International application number: PCT/US2007/068406
(87) International publication number: WO 2007/143316

(56) References cited:
- WO-A-2005/038013
- WO-A-2005/042552
- US-A1- 2003 083 280
- US-A1- 2006 063 722
- US-A1- 2007 299 028
- YOU G ET AL: "Molecular characteristics of Na+-coupled glucose transporters in adult and embryonic rat kidney" JOURNAL OF BIOLOGICAL CHEMISTRY, AMERICAN SOCIETY OF BIOLOCHEMICAL BIOLOGISTS, BIRMINGHAM, US, vol. 270, no. 49, 1 January 1995 (1995-01-01), pages 29365-29371, XP002139557 ISSN: 0021-9258
- ZHOU ET AL.: 'Human Cardiomyocytes Express High Level of Na+/Glucose Cotransporter 1 (SGLT1)' JOURNAL OF CELLULAR BIOCHEMISTRY vol. 90, 2003, pages 339 - 346, XP008102346

### SEQUENCE LISTING

<110> Isis Pharmaceuticals, Inc.
   Sanjay Bhanot
   Richard S. Geary
   Robert Mc Kay
   Brett P. Monia
   Punit P. Seth
   Andrew M Siwkowski
   Eric E. Swayze
   Edward Wancewitz
<120> COMPOUNDS AND METHODS FOR MODULATING EXPRESSION CF SGLT2
<130> CORE0061WO9
<150> PCT/ US2007/ 061183
   <151 > 2007-01-27
<150> 60/ 746, 631
   < 151 > 2006- 05- 05
<150> 60/ 747, 059 <151 > 2006- 05- 11
<150> 60/ 805, 660 <151 > 2006-06-23
<150> 60/ 864, 554 <151 > 2006-11-06
<160> 1576
<170> Fast SEQ for Windows Version 4. 0
<210> 1
   <211> 14121
   <212> DNA
   <213> H. sapiens
<400> 1
   attcccaccg ggacctgcgg ggctgagtgc ccttctcggt tgctgccgct gaggagcccg 60
   cccagccagc cagggccgcg aggccgaggc caggccgcag cccaggagcc gccccaccgc 120
   agctggcgat ggacccgccg aggcccgcgc tgctggcgct gctggcgctg cctgcgctgc 180
   tgctgctgct gctggcgggc gccagggccg aagaggaaat gctggaaaat gtcagcctgg 240
   t ct gt ccaaa agat gcgacc cgat t caagc acct ccggaa gt acacat ac aact at gagg 300
   ct gagagt t c cagt ggagt c cct gggact g ct gat t caag aagt gccacc aggat caact 360
   gcaaggt t ga gct ggaggt t ccccagct ct gcagct t cat cct gaagacc agccagt gca 420
   ccctgaaaga ggtgtatggc ttcaaccctg agggcaaagc cttgctgaag aaaaccaaga 480
   act ct gagga gt t t gct gca gccat gt cca ggt at gagct caagct ggcc at t ccagaag 540
   ggaagcaggt tttcctttac ccggagaaag atgaacctac ttacatcctg aacatcaaga 600
   ggggcatcat ttctgccctc ctggttcccc cagagacaga agaagccaag caagtgttgt 660
   ttctggatac cgtgtatgga aactgctcca ctcactttac cgtcaagacg aggaagggca 720
   at gt ggcaac agaaat at cc act gaaagag acct ggggca gt gt gat cgc t t caagccca 780
   tccgcacagg catcagccca cttgctctca tcaaaggcat gacccgcccc ttgtcaactc 840
   t gat cagcag cagccagt cc t gt cagt aca cact ggacgc t aagaggaag cat gt ggcag 900
   aagccatctg caaggagcaa cacctcttcc tgcctttctc ctacaacaat aagtatggga 960
   tggtagcaca agtgacacag actttgaaac ttgaagacac accaaagatc aacagccgct 1020
   tctttggtga aggtactaag aagatgggcc tcgcatttga gagcaccaaa tccacatcac 1080
   ctccaaagca ggccgaagct gttttgaaga ctctccagga actgaaaaaa ctaaccatct 1140
   ct gagcaaaa t at ccagaga gct aat ct ct t caat aagct ggt t act gag ct gagaggcc 1200
   t cagt gat ga agcagt caca t ct ct ct t gc cacagct gat t gaggt gt cc agccccat ca 1260
   ct t t acaagc ct t ggt t cag t gt ggacagc ct cagt gct c cact cacat c ct ccagt ggc 1320
   t gaaacgt gt gcat gccaac cccct t ct ga t agat gt ggt cacct acct g gt ggccct ga 1380
   t ccccgagcc ct cagcacag cagct gcgag agat ct t caa cat ggcgagg gat cagcgca 1440
   gccgagccac cttgtatgcg ctgagccacg cggtcaacaa ctatcataag acaaacccta 1500
   cagggaccca ggagctgctg gacattgcta attacctgat ggaacagatt caagatgact 1560
   gcactgggga tgaagattac acctatttga ttctgcgggt cattggaaat atgggccaaa 1620
   ccatggagca gttaactcca gaactcaagt cttcaatcct caaatgtgtc caaagtacaa 1680
   agccatcact gatgatccag aaagctgcca tccaggctct gcggaaaatg gagcctaaag 1740
   acaaggacca ggaggttctt cttcagactt tccttgatga tgcttctccg ggagataagc 1800
   gactggctgc ctatcttatg ttgatgagga gtccttcaca ggcagatatt aacaaaattg 1860
   tccaaattct accatgggaa cagaatgagc aagtgaagaa ctttgtggct tcccatattg 1920
   ccaatatctt gaactcagaa gaattggata tccaagatct gaaaaagtta gtgaaagaag 1980
   ctctgaaaga atctcaactt ccaactgtca tggacttcag aaaattctct cggaactatc 2040
   aactctacaa atctgtttct cttccatcac ttgacccagc ctcagccaaa atagaaggga 2100
   atcttatatt tgatccaaat aactaccttc ctaaagaaag catgctgaaa actaccctca 2160
   ctgcctttgg atttgcttca gctgacctca tcgagattgg cttggaagga aaaggctttg 2220
   agccaacatt ggaagctctt tttgggaagc aaggattttt cccagacagt gtcaacaaag 2280
   ctttgtactg ggttaatggt caagttcctg atggtgtctc taaggtctta gtggaccact 2340
   ttggctatac caaagatgat aaacatgagc aggatatggt aaatggaata atgctcagtg 2400
   ttgagaagct gattaaagat ttgaaatcca aagaagtccc ggaagccaga gcctacctcc 2460
   gcatcttggg agaggagctt ggttttgcca gtctccatga cctccagctc ctgggaaagc 2520
   tgcttctgat gggtgcccgc actctgcagg ggatccccca gatgattgga gaggtcatca 2580
   ggaagggctc aaagaatgac ttttttcttc actacatctt catggagaat gcctttgaac 2640
   tccccactgg agctggatta cagttgcaaa tatcttcatc tggagtcatt gctcccggag 2700
   ccaaggctgg agtaaaactg gaagtagcca acatgcaggc tgaactggtg gcaaaaccct 2760
   ccgtgtctgt ggagtttgtg acaaatatgg gcatcatcat tccggacttc gctaggagtg 2820
   gggtccagat gaacaccaac ttcttccacg agtcgggtct ggaggctcat gttgccctaa 2880
   aagctgggaa gctgaagttt atcattcctt ccccaaagag accagtcaag ctgctcagtg 2940
   gaggcaacac attacatttg gtctctacca ccaaaacgga ggtgatccca cctctcattg 3000
   agaacaggca gtcctggtca gtttgcaagc aagtctttcc tggcctgaat tactgcacct 3060
   caggcgctta ctccaacgcc agctccacag actccgcctc ctactatccg ctgaccgggg 3120
   acaccagatt agagctggaa ctgaggccta caggagagat tgagcagtat tctgtcagcg 3180
   caacctatga gctccagaga gaggacagag ccttggtgga taccctgaag tttgtaactc 3240
   aagcagaagg tgcgaagcag actgaggcta ccatgacatt caaatataat cggcagagta 3300
   tgaccttgtc cagtgaagtc caaattccgg attttgatgt tgacctcgga acaatcctca 3360
   gagttaatga tgaatctact gagggcaaaa cgtcttacag actcaccctg gacattcaga 3420
   acaagaaaat tactgaggtc gccctcatgg gccacctaag ttgtgacaca aaggaagaaa 3480
   gaaaaatcaa gggtgttatt tccatacccc gtttgcaagc agaagccaga agtgagatcc 3540
   tcgcccactg gtcgcctgcc aaactgcttc tccaaatgga ctcatctgct acagcttatg 3600
   gctccacagt ttccaagagg gtggcatggc attatgatga agagaagatt gaatttgaat 3660
   ggaacacagg caccaatgta gataccaaaa aaatgacttc caatttccct gtggatctct 3720
   ccgattatcc taagagcttg catatgtatg ctaatagact cctggatcac agagtccctg 3780
   aaacagacat gactttccgg cacgtgggtt ccaaattaat agttgcaatg agctcatggc 3840
   ttcagaaggc atctgggagt cttccttata cccagacttt gcaagaccac ctcaatagcc 3900
   tgaaggagtt caacctccag aacatgggat tgccagactt ccacatccca gaaaacctct 3960
   tcttaaaaag cgatggccgg gtcaaatata ccttgaacaa gaacagtttg aaaattgaga 4020
   ttcctttgcc ttttggtggc aaatcctcca gagatctaaa gatgttagag actgttagga 4080
   caccagccct ccacttcaag tctgtgggat tccatctgcc atctcgagag ttccaagtcc 4140
   ctacttttac cattcccaag ttgtatcaac tgcaagtgcc tctcctgggt gttctagacc 4200
   tctccacgaa tgtctacagc aacttgtaca actggtccgc ctcctacagt ggtggcaaca 4260
   ccagcacaga ccatttcagc cttcgggctc gttaccacat gaaggctgac tctgtggttg 4320
   acctgctttc ctacaatgtg caaggatctg gagaaacaac atatgaccac aagaatacgt 4380
   tcacactatc atgtgatggg tctctacgcc acaaatttct agattcgaat atcaaattca 4440
   gtcatgtaga aaaacttgga aacaacccag tctcaaaagg tttactaata ttcgatgcat 4500
   ctagttcctg gggaccacag atgtctgctt cagttcattt ggactccaaa aagaaacagc 4560
   atttgtttgt caaagaagtc aagattgatg ggcagttcag agtctcttcg ttctatgcta 4620
   aaggcacata tggcctgtct tgtcagaggg atcctaacac tggccggctc aatggagagt 4680
   ccaacctgag gtttaactcc tcctacctcc aaggcaccaa ccagataaca ggaagatatg 4740
   aagatggaac cctctccctc acctccacct ctgatctgca aagtggcatc attaaaaata 4800
   ctgcttccct aaagtatgag aactacgagc tgactttaaa atctgacacc aatgggaagt 4860
   ataagaactt tgccacttct aacaagatgg atatgacctt ctctaagcaa aatgcactgc 4920
   tgcgttctga atatcaggct gattacgagt cattgaggtt cttcagcctg ctttctggat 4980
   cactaaattc ccatggtctt gagttaaatg ctgacatctt aggcactgac aaaattaata 5040
   gtggtgctca caaggcgaca ctaaggattg gccaagatgg aatatctacc agtgcaacga 5100
   ccaacttgaa gtgtagtctc ctggtgctgg agaatgagct gaatgcagag cttggccctc 5160
   ctggggcatc tatgaaatta acaacaaatg gccgcttcag ggaacacaat gcaaaattca 5220
   gtctggatgg gaaagccgcc ctcacagagc tatcactggg aagtgcttat caggccatga 5280
   ttctgggtgt cgacagcaaa aacattttca acttcaaggt cagtcaagaa ggacttaagc 5340
   tctcaaatga catgatgggc tcatatgctg aaatgaaatt tgaccacaca aacagtctga 5400
   acattgcagg cttatcactg gactttcttc caaaacttga caacatttac agctctgaca 5460
   agttttataa gcaaactgtt aatttacagc tacagcccta ttctctggta actactttaa 5520
   acagtgacct gaaatacaat gctctggatc tcaccaacaa tgggaaacta cggctagaac 5580
   ccctgaagct gcatgtggct ggtaacctaa aaggagccta ccaaaataat gaaataaaac 5640
   acatctatgc catctcttct gctgccttat cagcaagcta taaagcagac actgttgcta 5700
   aggttcaggg tgtggagttt agccatcggc tcaacacaga catcgctggg ctggcttcag 5760
   ccattgacat gagcacaaac tataattcag actcactgca tttcagcaat gtcttccgtt 5820
   ctgtaatggc cccgtttacc atgaccatcg atgcacatac aaatggcaat gggaaactcg 5880
   ctctctgggg agaacatact gggcagctgt atagcaaatt cctgttgaaa gcagaacctc 5940
   tggcatttac tttctctcat gattacaaag gctccacaag tcatcatctc gtgtctagga 6000
   aaagcatcag tgcagctctt gaacacaaag tcagtgccct gcttactcca gctgagcaga 6060
   caggcacctg gaaactcaag acccaattta acaacaatga atacagccag gacttggatg 6120
   cttacaacac taaagataaa attggcgtgg agcttactgg acgaactctg gctgacctaa 6180
   ctctactaga ctccccaatt aaagtgccac ttttactcag tgagcccatc aatatcattg 6240
   atgctttaga gatgagagat gccgttgaga agccccaaga atttacaatt gttgcttttg 6300
   taaagtatga taaaaaccaa gatgttcact ccattaacct cccatttttt gagaccttgc 6360
   aagaatattt tgagaggaat cgacaaacca ttatagttgt agtggaaaac gtacagagaa 6420
   acctgaagca catcaatatt gatcaatttg taagaaaata cagagcagcc ctgggaaaac 6480
   tcccacagca agctaatgat tatctgaatt cattcaattg ggagagacaa gtttcacatg 6540
   ccaaggagaa actgactgct ctcacaaaaa agtatagaat tacagaaaat gatatacaaa 6600
   ttgcattaga tgatgccaaa atcaacttta atgaaaaact atctcaactg cagacatata 6660
   tgatacaatt tgatcagtat attaaagata gttatgattt acatgatttg aaaatagcta 6720
   ttgctaatat tattgatgaa atcattgaaa aattaaaaag tcttgatgag cactatcata 6780
   tccgtgtaaa tttagtaaaa acaatccatg atctacattt gtttattgaa aatattgatt 6840
   ttaacaaaag tggaagtagt actgcatcct ggattcaaaa tgtggatact aagtaccaaa 6900
   tcagaatcca gatacaagaa aaactgcagc agcttaagag acacatacag aatatagaca 6960
   tccagcacct agctggaaag ttaaaacaac acattgaggc tattgatgtt agagtgcttt 7020
   tagatcaatt gggaactaca atttcatttg aaagaataaa tgatgttctt gagcatgtca 7080
   aacactttgt tataaatctt attggggatt ttgaagtagc tgagaaaatc aatgccttca 7140
   gagccaaagt ccatgagtta atcgagaggt atgaagtaga ccaacaaatc caggttttaa 7200
   tggataaatt agtagagttg acccaccaat acaagttgaa ggagactatt cagaagctaa 7260
   gcaatgtcct acaacaagtt aagataaaag attactttga gaaattggtt ggatttattg 7320
   atgatgctgt gaagaagctt aatgaattat cttttaaaac attcattgaa gatgttaaca 7380
   aattccttga catgttgata aagaaattaa agtcatttga ttaccaccag tttgtagatg 7440
   aaaccaatga caaaatccgt gaggtgactc agagactcaa tggtgaaatt caggctctgg 7500
   aactaccaca aaaagctgaa gcattaaaac tgtttttaga ggaaaccaag gccacagttg 7560
   cagtgtatct ggaaagccta caggacacca aaataacctt aatcatcaat tggttacagg 7620
   aggctttaag ttcagcatct ttggctcaca tgaaggccaa attccgagag actctagaag 7680
   atacacgaga ccgaatgtat caaatggaca ttcagcagga acttcaacga tacctgtctc 7740
   tggtaggcca ggtttatagc acacttgtca cctacatttc tgattggtgg actcttgctg 7800
   ctaagaacct tactgacttt gcagagcaat attctatcca agattgggct aaacgtatga 7860
   aagcattggt agagcaaggg ttcactgttc ctgaaatcaa gaccatcctt gggaccatgc 7920
   ctgcctttga agtcagtctt caggctcttc agaaagctac cttccagaca cctgatttta 7980
   tagtccccct aacagatttg aggattccat cagttcagat aaacttcaaa gacttaaaaa 8040
   atataaaaat cccatccagg ttttccacac cagaatttac catccttaac accttccaca 8100
   ttccttcctt tacaattgac tttgtcgaaa tgaaagtaaa gatcatcaga accattgacc 8160
   agatgcagaa cagtgagctg cagtggcccg ttccagatat atatctcagg gatctgaagg 8220
   tggaggacat tcctctagcg agaatcaccc tgccagactt ccgtttacca gaaatcgcaa 8280
   ttccagaatt cataatccca actctcaacc ttaatgattt tcaagttcct gaccttcaca 8340
   taccagaatt ccagcttccc cacatctcac acacaattga agtacctact tttggcaagc 8400
   tatacagtat tctgaaaatc caatctcctc ttttcacatt agatgcaaat gctgacatag 8460
   ggaatggaac cacctcagca aacgaagcag gtatcgcagc ttccatcact gccaaaggag 8520
   agtccaaatt agaagttctc aattttgatt ttcaagcaaa tgcacaactc tcaaccccta 8580
   agattaatcc gctggctctg aaggagtcag tgaagttctc cagcaagtac ctgagaacgg 8640
   agcatgggag tgaaatgctg ttttttggaa atgctattga gggaaaatca aacacagtgg 8700
   caagtttaca cacagaaaaa aatacactgg agcttagtaa tggagtgatt gtcaagataa 8760
   acaatcagct taccctggat agcaacacta aatacttcca caaattgaac atccccaaac 8820
   tggacttctc tagtcaggct gacctgcgca acgagatcaa gacactgttg aaagctggcc 8880
   acatagcatg gacttcttct ggaaaagggt catggaaatg ggcctgcccc agattctcag 8940
   atgagggaac acatgaatca caaattagtt tcaccataga aggacccctc acttcctttg 9000
   gactgtccaa taagatcaat agcaaacacc taagagtaaa ccaaaacttg gtttatgaat 9060
   ctggctccct caacttttct aaacttgaaa ttcaatcaca agtcgattcc cagcatgtgg 9120
   gccacagtgt tctaactgct aaaggcatgg cactgtttgg agaagggaag gcagagttta 9180
   ctgggaggca tgatgctcat ttaaatggaa aggttattgg aactttgaaa aattctcttt 9240
   tcttttcagc ccagccattt gagatcacgg catccacaaa caatgaaggg aatttgaaag 9300
   ttcgttttcc attaaggtta acagggaaga tagacttcct gaataactat gcactgtttc 9360
   tgagtcccag tgcccagcaa gcaagttggc aagtaagtgc taggttcaat cagtataagt 9420
   acaaccaaaa tttctctgct ggaaacaacg agaacattat ggaggcccat gtaggaataa 9480
   atggagaagc aaatctggat ttcttaaaca ttcctttaac aattcctgaa atgcgtctac 9540
   cttacacaat aatcacaact cctccactga aagatttctc tctatgggaa aaaacaggct 9600
   tgaaggaatt cttgaaaacg acaaagcaat catttgattt aagtgtaaaa gctcagtata 9660
   agaaaaacaa acacaggcat tccatcacaa atcctttggc tgtgctttgt gagtttatca 9720
   gtcagagcat caaatccttt gacaggcatt ttgaaaaaaa cagaaacaat gcattagatt 9780
   ttgtcaccaa atcctataat gaaacaaaaa ttaagtttga taagtacaaa gctgaaaaat 9840
   ctcacgacga gctccccagg acctttcaaa ttcctggata cactgttcca gttgtcaatg 9900
   ttgaagtgtc tccattcacc atagagatgt cggcattcgg ctatgtgttc ccaaaagcag 9960
   tcagcatgcc tagtttctcc atcctaggtt ctgacgtccg tgtgccttca tacacattaa 10020
   tcctgccatc attagagctg ccagtccttc atgtccctag aaatctcaag ctttctcttc 10080
   cacatttcaa ggaattgtgt accataagcc atatttttat tcctgccatg ggcaatatta 10140
   cctatgattt ctcctttaaa tcaagtgtca tcacactgaa taccaatgct gaacttttta 10200
   accagtcaga tattgttgct catctccttt cttcatcttc atctgtcatt gatgcactgc 10260
   agtacaaatt agagggcacc acaagattga caagaaaaag gggattgaag ttagccacag 10320
   ctctgtctct gagcaacaaa tttgtggagg gtagtcataa cagtactgtg agcttaacca 10380
   cgaaaaatat ggaagtgtca gtggcaaaaa ccacaaaagc cgaaattcca attttgagaa 10440
   tgaatttcaa gcaagaactt aatggaaata ccaagtcaaa acctactgtc tcttcctcca 10500
   tggaatttaa gtatgatttc aattcttcaa tgctgtactc taccgctaaa ggagcagttg 10560
   accacaagct tagcttggaa agcctcacct cttacttttc cattgagtca tctaccaaag 10620
   gagatgtcaa gggttcggtt ctttctcggg aatattcagg aactattgct agtgaggcca 10680
   acacttactt gaattccaag agcacacggt cttcagtgaa gctgcagggc acttccaaaa 10740
   ttgatgatat ctggaacctt gaagtaaaag aaaattttgc tggagaagcc acactccaac 10800
   gcatatattc cctctgggag cacagtacga aaaaccactt acagctagag ggcctctttt 10860
   tcaccaacgg agaacataca agcaaagcca ccctggaact ctctccatgg caaatgtcag 10920
   ctcttgttca ggtccatgca agtcagccca gttccttcca tgatttccct gaccttggcc 10980
   aggaagtggc cctgaatgct aacactaaga accagaagat cagatggaaa aatgaagtcc 11040
   ggattcattc tgggtctttc cagagccagg tcgagctttc caatgaccaa gaaaaggcac 11100
   accttgacat tgcaggatcc ttagaaggac acctaaggtt cctcaaaaat atcatcctac 11160
   cagtctatga caagagctta tgggatttcc taaagctgga tgtaaccacc agcattggta 11220
   ggagacagca tcttcgtgtt tcaactgcct ttgtgtacac caaaaacccc aatggctatt 11280
   cattctccat ccctgtaaaa gttttggctg ataaattcat tactcctggg ctgaaactaa 11340
   atgatctaaa ttcagttctt gtcatgccta cgttccatgt cccatttaca gatcttcagg 11400
   ttccatcgtg caaacttgac ttcagagaaa tacaaatcta taagaagctg agaacttcat 11460
   catttgccct caacctacca acactccccg aggtaaaatt ccctgaagtt gatgtgttaa 11520
   caaaatattc tcaaccagaa gactccttga ttcccttttt tgagataacc gtgcctgaat 11580
   ctcagttaac tgtgtcccag ttcacgcttc caaaaagtgt ttcagatggc attgctgctt 11640
   tggatctaaa tgcagtagcc aacaagatcg cagactttga gttgcccacc atcatcgtgc 11700
   ctgagcagac cattgagatt ccctccatta agttctctgt acctgctgga attgtcattc 11760
   cttcctttca agcactgact gcacgctttg aggtagactc tcccgtgtat aatgccactt 11820
   ggagtgccag tttgaaaaac aaagcagatt atgttgaaac agtcctggat tccacatgca 11880
   gctcaaccgt acagttccta gaatatgaac taaatgtttt gggaacacac aaaatcgaag 11940
   atggtacgtt agcctctaag actaaaggaa cacttgcaca ccgtgacttc agtgcagaat 12000
   atgaagaaga tggcaaattt gaaggacttc aggaatggga aggaaaagcg cacctcaata 12060
   tcaaaagccc agcgttcacc gatctccatc tgcgctacca gaaagacaag aaaggcatct 12120
   ccacctcagc agcctcccca gccgtaggca ccgtgggcat ggatatggat gaagatgacg 12180
   acttttctaa atggaacttc tactacagcc ctcagtcctc tccagataaa aaactcacca 12240
   tattcaaaac tgagttgagg gtccgggaat ctgatgagga aactcagatc aaagttaatt 12300
   gggaagaaga ggcagcttct ggcttgctaa cctctctgaa agacaacgtg cccaaggcca 12360
   caggggtcct ttatgattat gtcaacaagt accactggga acacacaggg ctcaccctga 12420
   gagaagtgtc ttcaaagctg agaagaaatc tgcagaacaa tgctgagtgg gtttatcaag 12480
   gggccattag gcaaattgat gatatcgacg tgaggttcca gaaagcagcc agtggcacca 12540
   ctgggaccta ccaagagtgg aaggacaagg cccagaatct gtaccaggaa ctgttgactc 12600
   aggaaggcca agccagtttc cagggactca aggataacgt gtttgatggc ttggtacgag 12660
   ttactcaaaa attccatatg aaagtcaagc atctgattga ctcactcatt gattttctga 12720
   acttccccag attccagttt ccggggaaac ctgggatata cactagggag gaactttgca 12780
   ctatgttcat aagggaggta gggacggtac tgtcccaggt atattcgaaa gtccataatg 12840
   gttcagaaat actgttttcc tatttccaag acctagtgat tacacttcct ttcgagttaa 12900
   ggaaacataa actaatagat gtaatctcga tgtataggga actgttgaaa gatttatcaa 12960
   aagaagccca agaggtattt aaagccattc agtctctcaa gaccacagag gtgctacgta 13020
   atcttcagga ccttttacaa ttcattttcc aactaataga agataacatt aaacagctga 13080
   aagagatgaa atttacttat cttattaatt atatccaaga tgagatcaac acaatcttca 13140
   atgattatat cccatatgtt tttaaattgt tgaaagaaaa cctatgcctt aatcttcata 13200
   agttcaatga atttattcaa aacgagcttc aggaagcttc tcaagagtta cagcagatcc 13260
   atcaatacat tatggccctt cgtgaagaat attttgatcc aagtatagtt ggctggacag 13320
   tgaaatatta tgaacttgaa gaaaagatag tcagtctgat caagaacctg ttagttgctc 13380
   ttaaggactt ccattctgaa tatattgtca gtgcctctaa ctttacttcc caactctcaa 13440
   gtcaagttga gcaatttctg cacagaaata ttcaggaata tcttagcatc cttaccgatc 13500
   cagatggaaa agggaaagag aagattgcag agctttctgc cactgctcag gaaataatta 13560
   aaagccaggc cattgcgacg aagaaaataa tttctgatta ccaccagcag tttagatata 13620
   aactgcaaga tttttcagac caactctctg attactatga aaaatttatt gctgaatcca 13680
   aaagattgat tgacctgtcc attcaaaact accacacatt tctgatatac atcacggagt 13740
   tactgaaaaa gctgcaatca accacagtca tgaaccccta catgaagctt gctccaggag 13800
   aacttactat catcctctaa ttttttaaaa gaaatcttca tttattcttc ttttccaatt 13860
   gaactttcac atagcacaga aaaaattcaa actgcctata ttgataaaac catacagtga 13920
   gccagccttg cagtaggcag tagactataa gcagaagcac atatgaactg gacctgcacc 13980
   aaagct ggca ccagggct cg gaaggt ct ct gaact cagaa ggat ggcat t t t t t gcaagt 14040
   taaagaaaat caggatctga gttattttgc taaacttggg ggaggaggaa caaataaatg 14100
   gagt ct t t at t gt gt at cat a 14121
<210> 2
   <211> 13928
   <212> DNA
   <213> Musmusculus
<400> 2
   atcagtgcct gcagtggatc aagtacctgc ctgagctccg cctccgaaga ccctgtagag 60
   caagcagcag gggctaggcc cgtggccagg ccacagccag gaagccaccc caccatccat 120
   ccgccatggg cccacgaaag cctgccctgc ggacgccgtt actgctgctg ttcctgctac 180
   tgttcttgga caccagcgtc tgggctcaag atgaagtcct ggaaaactta agcttcagct 240
   gtccaaaaga tgcaactcga ttcaagcacc tccgaaagta cgtgtacaac tatgaagctg 300
   aaagttccag cggtgtccag ggcacagctg actccagaag cgccaccaag atcaactgta 360
   aggtagagct ggaggtcccc caaatctgtg gtttcatcat gaggaccaac cagtgtaccc 420
   ttaaagaggt gtatggcttc aaccctgagg gcaaggcctt gatgaagaaa accaagaact 480
   ctgaagagtt tgcagctgcc atgtccaggt acgaactcaa gctggccatt cctgaaggga 540
   aacaaattgt tctttaccct gacaaggatg aacctaaata tatcctgaac atcaagaggg 600
   gcatcatctc tgctcttctg gttcccccag agacagaaga ggaccaacaa gagttgttcc 660
   tggataccgt gtatggaaac tgctcaactc aggttaccgt gaattcaaga aagggaaccg 720
   taccaacaga aatgtccaca gagagaaacc tgcagcaatg tgacggcttc cagcccatca 780
   gtacaagtgt cagccctctc gctctcatca aaggcctggt ccaccccttg tcaactctta 840
   tcagcagcag ccaaacttgc cagtacaccc tggatcctaa gaggaagcat gtgtctgaag 900
   ctgtctgtga tgagcagcat cttttcctgc ctttctccta caagaataag tatgggatca 960
   tgacacgtgt tacacagaaa ctgagtcttg aagacacacc taagatcaac agtcgcttct 1020
   tcagtgaagg taccaaccgg atgggtctgg cctttgagag caccaagtcc acgtcatccc 1080
   caaagcaggc tgatgctgtt ttgaagaccc ttcaagaact gaaaaaattg tccatctcag 1140
   agcagaatgc tcagagagca aatctcttca ataaactggt tactgagctg agaggcctca 1200
   ctggtgaagc aatcacatcc ctcttgccac agctgattga agtgtccagc cccatcactt 1260
   tacaagcctt ggttcagtgt ggacagccac agtgctatac tcacatcctc cagtggctga 1320
   aaactgagaa ggctcacccc ctcctggttg acattgtcac ctacctgatg gctctgatcc 1380
   caaatccctc aacacagagg ctgcaggaaa tctttaatac tgccaaggag cagcagagcc 1440
   gagccactct gtatgcactg agccacgcag ttaacagcta ttttgatgtg gaccattcaa 1500
   ggagcccagt tctgcaggat atcgctggtt acctgttgaa acagatcgac aatgaatgca 1560
   cgggcaatga agaccacacc ttcttgattc tgagggtcat tggaaatatg ggaagaacca 1620
   tggaacaagt aatgccagcc ctcaagtcct cagtcctgag ctgtgtacga agtacaaaac 1680
   catctctgct gattcagaaa gctgctctcc aggccctgag gaagatggaa ctggaagatg 1740
   aggtccggac gatccttttt gatacatttg taaatggtgt cgctcccgtg gagaagagac 1800
   tggctgccta tctcttgctg atgaagaacc cttcctcatc agatattaac aaaattgccc 1860
   aacttctcca atgggaacag agtgagcagg tgaagaactt cgtggcatct cacattgcca 1920
   acatcttgaa ctcggaagaa ctgtatgtcc aagatctgaa agttttgatc aaaaatgctc 1980
   tggagaattc tcaatttcca acgatcatgg acttcagaaa attttcccga aactatcaga 2040
   tttccaaatc tgcttctctc ccaatgttcg acccagtctc agtcaaaata gaagggaatc 2100
   ttatatttga tccaagcagt tatcttccca gagaaagctt gctgaaaaca accctcacag 2160
   tctttggact tgcttcactt gatctctttg agattggttt agaaggaaaa gggtttgagc 2220
   caacactaga agctcttttt ggtaagcaag gattcttccc agacagtgtc aacaaggctt 2280
   tgtattgggt caatggccga gttccagatg gtgtctccaa ggtcttggtg gaccactttg 2340
   gctatactac agatggcaag catgaacagg acatggtgaa tggaatcatg cccattgtgg 2400
   acaagttgat caaagatctg aaatctaaag aaattcctga agccagggcc tatctccgca 2460
   tcctaggaaa agagctaagc tttgtcagac tccaagacct ccaagtcctg gggaagctgt 2520
   tgctgagtgg tgcacaaact ttgcagggaa tcccccagat ggttgtacag gccatcagag 2580
   aagggtcaaa gaatgacttg tttctccact acatcttcat ggacaatgcc tttgagctcc 2640
   ccactggagc agggttacag ctgcaagtgt cctcgtctgg agtcttcacc cccgggatca 2700
   aggctggtgt aagactggaa ttagccaaca tacaggcaga gctagtggca aagccctctg 2760
   tgtccttgga gtttgtgaca aatatgggca tcatcatccc agacttcgct aagagcagtg 2820
   tccagatgaa caccaacttc ttccacgagt caggcctgga ggcgcgagtg gccctgaagg 2880
   ctgggcagct gaaggtcatc attccttctc caaagaggcc agtcaagctg ttcagtggca 2940
   gcaacacact gcatctggtc tctaccacca aaacagaagt gatcccacct ctggttgaga 3000
   acaggcagtc ctggtcaact tgcaagcctc tcttcactgg aatgaactac tgtaccacag 3060
   gagcttactc caacgccagc tccacggagt ctgcctctta ctacccactg acaggggaca 3120
   caaggtatga gctggagctg aggcccacgg gagaagtgga gcagtattct gccactgcaa 3180
   cctatgaact cctaaaagag gacaagtctt tggttgacac attgaagttc ctagttcaag 3240
   cagaaggagt gcagcagtct gaagctactg tactgttcaa atataatcgg agaagcagga 3300
   ccttatctag tgaagtccta attccagggt ttgatgtcaa cttcgggaca atactaagag 3360
   ttaatgatga atctgctaag gacaaaaaca cttacaaact catcctggac attcagaaca 3420
   agaaaat cac t gaggt ct ct ct cgt gggcc act t gagt t a t gat aaaaag ggagat ggca 3480
   agatcaaagg tgttgtttcc ataccacgtt tgcaagcaga agccaggagt gaggtccaca 3540
   cccact ggt c ct ccaccaaa ct gct ct t cc aaat ggact c at ct gct aca gct t acggct 3600
   caacaat t t c caagagagt g acat ggcgt t acgat aat ga gat aat agaa t t t gat t gga 3660
   acacgggaac caatgtggat accaaaaaag tggcctccaa tttccctgtg gatctttccc 3720
   at t at cct ag aat gt t gcat gagt at gcca at ggt ct cct ggat cacaga gt ccct caaa 3780
   cagat gt gac t t t t cgggac at gggt t cca aat t aat t gt t gcaacaaac acat ggct t c 3840
   agat ggcaac caggggt ct t cct t accccc aaact ct aca ggat cacct c aat agcct ct 3900
   cagagt t gaa cct cct gaaa at gggact gt ct gact t cca t at t ccagac aacct ct t cc 3960
   t aaagact ga t ggcagagt c aaat acacaa t gaacaggaa caaaat aaac at t gacat cc 4020
   ctttgccttt gggtggcaag tcttcaaaag acctcaagat gccagagagt gtgaggacac 4080
   cagccct caa ct t caagt ct gt gggat t cc at ct gccat c t cgagaggt c caggt cccca 4140
   cttttacaat ccccaagaca catcagcttc aagtgcctct cttgggtgtt ctagaccttt 4200
   ccacaaatgt ctacagcaat ttgtacaact ggtcagcctc ctacactggt ggcaacacca 4260
   gcagagacca cttcagcctt caggctcagt accgcatgaa gactgactct gtggttgacc 4320
   t gt t t t cct a cagt gt gcaa ggat ct ggag aaacaacat a t gacagcaag aacacat t t a 4380
   cat t gt cct g t gat ggat ct ct acaccat a aat t t ct aga ct caaaat t c aaagt cagcc 4440
   acgt agaaaa at t t ggaaac agcccagt ct caaaaggt t t act aacat t t gaaacat ct a 4500
   gtgccttggg accacagatg tctgctactg ttcacctaga ctcaaaaaag aaacaacatc 4560
   tatacgtcaa agatatcaag gttgatggac agttcagagc ttcttcattt tatgctcaag 4620
   gcaaat at gg cct gt ct t gt gagagagat g t t acaact gg ccagct gagc ggcgaat cca 4680
   acatgagatt taactccacc tacttccagg gcaccaacca gatcgtggga atgtaccagg 4740
   atggagccct gtccatcacc tccacttctg acctgcaaga tggcatattc aagaacacag 4800
   ct t cct t gaa at at gaaaac t at gagct ga ct ct gaaat c t gat agcagt gggcagt at g 4860
   agaact t cgc t gct t ccaac aagct ggat g t gacct t ct c t acgcaaagt gcact gct gc 4920
   gttctgaaca ccaggccaat tacaagtccc tgaggcttgt cacccttctt tcaggatccc 4980
   tcacttccca gggtgtagaa ttaaatgctg acatcttggg cacagacaaa attaatactg 5040
   gtgctcacaa ggcaacacta aagattgcac gtgatggact atcaaccagt gcgaccacca 5100
   act t gaagt a cagccccct g ct gct ggaga at gagt t gaa t gcagagct t gggct ct ct g 5160
   gggcatccat gaaattatca acaaacggcc gcttcaaaga acaccatgca aaattcagtc 5220
   t t gat gggag agct gccct c acagaggt gt cact ggggag cat t t accag gccat gat t c 5280
   tgggtgcaga cagcaaaaac atcttcaact tcaaactcag ccgagaaggg ctgaggctgt 5340
   ccaatgattt gatgggctcc tatgctgaga tgaaacttga ccacacacac agtctgaaca 5400
   ttgcaggtct ctcactggac ttcttctcaa aaatggacaa tatttacagt ggagacaagt 5460
   t ct at aagca gaat t t t aac t t acagct ac agccct at t c t t t cat aact act t t aagca 5520
   acgacct gag at at ggt gct ct agat t t ga ccaacaat gg aaggt t t cgg ct ggagccac 5580
   t gaagct gaa t gt gggt ggc aact t t aaag gaacct at ca aaat aat gag ct gaaacat a 5640
   tctataccat atcttatact gacctggtag tagcaagtta cagagcagac actgtggcta 5700
   aggt t caggg t gt cgaat t c agccat aggc t aaat gcaga cat t gaagga ct gact t cct 5760
   ct gt t gat gt cact accagc t acaat t cag at ccact gca t t t t aacaat gt t t t ccact 5820
   t t t ct ct ggc acct t t t acc t t gggcat cg acacacat ac aagt ggt gat gggaaact gt 5880
   ccttctgggg agaacacact gggcagctat atagtaagtt tctgttgaaa gcagaacctc 5940
   t ggcact t at t gt ct ct cat gact acaaag gat ccacaag ccacagt ct c ccgt acgaga 6000
   gcagcatcag cacggctctt gaacacacag tcagtgcctt gctgacgcca gctgagcaga 6060
   caagcacct g gaaat t caag accaaact ga at gacaaagt at acagccag gact t t gaag 6120
   cct acaacac t aaagacaaa at cggt gt t g agct t agt gg acgggct gac ct ct ct gggc 6180
   t gt at t ct cc aat t aaact a ccgt t t t t ct acagt gagcc t gt caat gt c ct t aat ggct 6240
   tagaggtaaa tgatgctgtt gacaagcccc aagaattcac aattattgct gtggtgaagt 6300
   acgat aagaa ccaggat gt t cacaccat ca acct cccat t ct t caaaagc ct gccagact 6360
   at t t ggagag aaat cgaaga ggaat gat aa gt ct act gga agccat gcga ggggaat t gc 6420
   aacgcct cag t gt t gat cag t t t gt gagga aat acagagc ggccct gagc agact t cct c 6480
   agcagat t ca t cat t at ct g aat gcat ct g act gggagag acaagt agct ggt gccaagg 6540
   aaaaaat aac t t ct t t cat g gaaaat t at a gaat t acaga t aat gat gt a ct aat t gcca 6600
   t agat agt gc caaaat caac t t caat gaaa aact ct ct ca act t gagaca t acgcgat ac 6660
   aat t t gat ca gt at at t aaa gat aat t at g at ccacat ga ct t aaaaaga act at t gct g 6720
   agat t at t ga t cgaat cat t gaaaagt t aa aaat t ct t ga t gaacagt at cat at ccgt g 6780
   t aaat ct agc aaaat caat c cat aat ct ct at t t at t t gt t gaaaacgt t gat ct t aacc 6840
   aagt cagt ag t agt aacacc t ct t ggat cc aaaat gt gga t t ccaat t at caagt cagaa 6900
   tccaaattca agaaaaacta cagcagctca ggacacaaat tcagaatata gacattcagc 6960
   agct t gct gc agaggt aaaa cgacagat gg acgct at t ga t gt cacaat g cat t t agat c 7020
   aat t gagaac t gcaat t ct a t t ccaaagaa t aagt gacat t at t gaccgt gt caaat act 7080
   t t gt t at gaa t ct t at t gaa gat t t t aaag t aact gagaa aat caat act t t t agagt t a 7140
   t agt ccgt ga gct aat t gag aaat at gaag t agaccaaca cat ccaggt t t t aat ggat a 7200
   aat cagt aga gt t ggcccac agat at agcc t gagcgagcc t ct t cagaaa ct cagt aat g 7260
   t gct acagcg aat t gagat a aaagat t act at gagaaat t ggt t gggt t t at t gat gat a 7320
   ct gt t gagt g gct t aaagca t t gt ct t t ca aaaat accat t gaagaact a aat agat t ga 7380
   ctgacatgtt ggtgaagaag ttgaaagcat ttgattatca ccagtttgta gacaaaacca 7440
   acagcaaaat ccgtgagatg actcagagaa tcaatgctga aatccaagct ctcaaacttc 7500
   cacaaaaaat ggaagcatta aaactgttgg tagaagactt caaaaccaca gtctccaatt 7560
   ccctggaaag actcaaggac accaaagtaa ctgtggtcat tgattggctg caggatattt 7620
   tgactcaaat gaaagaccat ttccaagata ctctggaaga tgtaagagac cgaatttatc 7680
   aaatggacat tcagagggaa ctggagcact tcttgtctct ggtaaaccaa gtttacagta 7740
   cactggtcac ctatatgtct gactggtgga ctctgactgc taaaaacata acagactttg 7800
   cagagcaata ttccatccaa aactgggctg agagtataaa agtactggtg gaacaaggat 7860
   tcatagttcc tgaaatgcaa acatttctgt ggaccatgcc tgcttttgag gtcagtctcc 7920
   gtgctctcca agaaggtaac tttcagaccc ctgtctttat agtccccttg acagatttga 7980
   ggattccatc aattcggata aactttaaaa tgttaaagaa tataaaaatc ccattgagat 8040
   tttccactcc agaattcact cttctcaaca ccttccatgt ccattccttt acaattgact 8100
   tgctggaaat aaaagcaaag atcattagaa ctatcgacca aattttgagc agtgagctac 8160
   agtggcctct tccagaaatg tatttgagag acctggatgt agtgaacatt cctcttgcaa 8220
   gactgactct gccagacttc catgtaccag aaatcacaat tccagaattc acaatcccaa 8280
   atgtcaatct caaagattta cacgttcctg atcttcacat accagaattc caacttcctc 8340
   acctctcaca tacaattgaa atacctgctt ttggcaaact gcatagcatc cttaagatcc 8400
   aatctcctct ctttatatta gatgctaatg ccaacataca gaatgtaaca acttcaggga 8460
   acaaagcaga gattgtggct tctgtcactg ctaaaggaga gtcccaattt gaagctctca 8520
   attttgattt tcaagcacaa gctcaattcc tggagttaaa tcctcatcct ccagtcctga 8580
   aggaatccat gaacttctcc agtaagcatg tgagaatgga gcatgagggt gagatagtat 8640
   ttgatggaaa ggccattgag gggaaatcag acacagtcgc aagtttacac acagagaaaa 8700
   atgaagtaga gtttaataat ggtatgactg tcaaagtaaa caatcagctc acccttgaca 8760
   gtcacacaaa gtacttccac aagttgagtg ttcctaggct ggacttctcc agtaaggctt 8820
   ctcttaataa tgaaatcaag acactattag aagctggaca tgtggcattg acatcttcag 8880
   ggacagggtc atggaactgg gcctgtccca acttctcgga tgaaggcata cattcgtccc 8940
   aaattagctt tactgtggat ggtcccattg cttttgttgg actatccaat aacataaatg 9000
   gcaaacactt acgggtcatc caaaaactga cttatgaatc tggcttcctc aactattcta 9060
   agtttgaagt tgagtcaaaa gttgaatctc agcacgtggg ctccagcatt ctaacagcca 9120
   atggtcgggc actgctcaag gacgcaaagg cagaaatgac tggtgagcac aatgccaact 9180
   taaatggaaa agttattgga actttgaaaa attctctctt cttttcagca caaccatttg 9240
   agattactgc atccacaaat aatgaaggaa atttgaaagt gggttttcca ctaaagctga 9300
   ctgggaaaat agacttcctg aataactatg cattgtttct gagtccccgt gcccaacaag 9360
   caagctggca agcgagtacc agattcaatc agtacaaata caatcaaaac ttttctgcta 9420
   taaacaatga acacaacata gaagccagta taggaatgaa tggagatgcc aacctggatt 9480
   tcttaaacat acctttaaca attcctgaaa ttaacttgcc ttacacggag ttcaaaactc 9540
   ccttactgaa ggatttctcc atatgggaag aaacaggctt gaaagaattt ttgaagacaa 9600
   caaagcaatc atttgatttg agtgtaaagg ctcaatataa aaagaacagt gacaagcatt 9660
   ccattgttgt ccctctgggt atgttttatg aatttattct caacaatgtc aattcgtggg 9720
   acagaaaatt tgagaaagtc agaaacaatg ctttacattt tcttaccacc tcctataatg 9780
   aagcaaaaat taaggttgat aagtacaaaa ctgaaaattc ccttaatcag ccctctggga 9840
   cctttcaaaa tcatggctac actatcccag ttgtcaacat tgaagtatct ccatttgctg 9900
   tagagacact ggcttccagc catgtgatcc ccacagcaat aagcacccca agtgtcacaa 9960
   tccctggtcc taacatcatg gtgccttcat acaagttagt gctgccaccc ctggagttgc 10020
   cagttttcca tggtcctggg aatctattca agtttttcct cccagatttc aagggattca 10080
   acactattga caatatttat attccagcca tgggcaactt tacctatgac ttttctttta 10140
   aatcaagtgt catcacactg aataccaatg ctggacttta taaccaatca gatatcgttg 10200
   cccatttcct ttcttcctct tcatttgtca ctgacgccct gcagtacaaa ttagagggaa 10260
   catcacgtct gatgcgaaaa aggggattga aactagccac agctgtctct ctaactaaca 10320
   aatttgtaaa gggcagtcat gacagcacca ttagtttaac caagaaaaac atggaagcat 10380
   cagtgagaac aactgccaac ctccatgctc ccatattctc aatgaacttc aagcaggaac 10440
   ttaatggaaa taccaagtca aaacccactg tttcatcatc cattgaacta aactatgact 10500
   tcaattcctc aaagctgcac tctactgcaa caggaggcat tgatcacaag ttcagcttag 10560
   aaagtctcac ttcctacttt tccattgagt cattcaccaa aggaaatatc aagagttcct 10620
   tcctttctca ggaatattca ggaagtgttg ccaatgaagc caatgtatat ctgaattcca 10680
   agggtactcg gtcttcagtg aggctacaag gagcttccaa agttgatggt atctggaacg 10740
   ttgaagtagg agaaaatttt gctggagaag ccaccctcca acgcatctac accacatggg 10800
   agcacaatat gaaaaaccat ttgcaggtat atagctactt cttcacaaaa ggaaagcaaa 10860
   catgcagagc tactttggag ctctccccat ggaccatgtc aaccttgcta caggttcatg 10920
   tgagtcaact cagttccctc cttgacctcc atcactttga ccaggaagtg atcctaaaag 10980
   ctaacactaa gaaccagaag atcagctgga aaggtggggt ccaggttgaa tcacgggttc 11040
   ttcagcacaa tgcacagttc tccaatgacc aagaagaaat acggcttgac cttgcaggat 11100
   ccttagacgg acagctgtgg gaccttgaag ctatcttttt accagtatat ggcaagagct 11160
   tgcaggaact cctacaaatg gatggaaagc gacagtatct tcaagcttca acttctcttc 11220
   tatataccaa aaaccctaat ggctatctcc tctcactccc cgtgcaagaa ctggctgata 11280
   gatttattat accagggata aaactaaatg acttcagtgg agtaaaaatc tataagaagt 11340
   taagtacttc accatttgcc ctcaacctaa caatgctccc caaagtaaaa ttccctggga 11400
   ttgatctgtt aacacagtac tctacaccag agggctcctc tgtccctatt tttgaggcaa 11460
   ctatacctga aattcattta actgtatccc agtttacact tccaaagagc cttccagttg 11520
   gcaacacagt ctttgatctg aataagttgg ccaacatgat tgccgatgtt gacctgccta 11580
   gtgtcaccct gcctgagcag actattgtaa tcccaccctt ggagttctct gtacctgctg 11640
   ggatttttat tcctttcttt ggagaactga ctgcacgtgc tgggatggct tctcccctgt 11700
   ataatgtcac ttggagcgct ggttggaaaa ccaaagcaga tcatgttgaa acgttcctag 11760
   attccatgtg cacttcaacc ttgcagtttc tggagtatgc tttaaaagtt gtagaaacac 11820
   acaaaattga agaagatctg ttaacctata atatcaaagg aacacttcaa cactgtgact 11880
   tcaatgtgga gtataatgaa gatggtctat ttaaaggact ttgggactgg cagggagagg 11940
   ctcacctgga catcaccagc ccagcactga ctgactttca tctgtactac aaagaagaca 12000
   agacaagtct gtctgcctca gcagcctcct cgaccatcgg cactgtgggt ctggattcga 12060
   gcacagatga ccagagtgtg gagctgaatg tctacttcca cccacagtcc cctccagaga 12120
   agaaactcag catattcaaa actgagtgga ggtacaagga gtctgatggt gaaaggtaca 12180
   tcaaaattaa ttgggaagaa gaggcagctt ccagattgct aggctcccta aaaagcaatg 12240
   tgcccaaggc ttctaaggct atttatgatt atgccaataa gtaccacctg gaatacgttt 12300
   cttcagaact aagaaaaagt ctacaggtca atgctgaaca tgccagaagg atggttgatg 12360
   aaatgaacat gagtttccag agagtagccc gtgataccta ccagaatctc tatgaggaga 12420
   tgttggctca gaagagcctg agcatccctg agaatctcaa gaagagggtg ttagacagta 12480
   tagtacatgt tactcagaag taccacatgg cagtcatgtg gctgatggac tcattcattc 12540
   attttctgaa attcaataga gtccagttcc cagggtacgc tggaacatat actgtggacg 12600
   aactctacac tatagtcatg aaggaaacca agaagtcact gtctcagctg tttaatgggt 12660
   taggaaacct actttcctac gttcaaaacc aagtagagaa atcaagatta atcaatgaca 12720
   taacatttaa atgtcctttt ttctcaaaac cttgtaaact aaaagatctc atattgattt 12780
   tcagggagga gttaaacatt ttatcaaaca taggccaaca ggatatcaag tttacaacaa 12840
   tactaagtag tcttcagggc tttttggaga gagttttaga catcatagaa gaacaaatta 12900
   aatgcctaaa ggacaatgaa tctacttgtg ttgctgacca tatcaacatg gttttcaaaa 12960
   tacaggtccc atatgctttt aaatccctaa gagaagacat atactttgtc ctcggtgagt 13020
   tcaatgactt tcttcaatcc atacttcagg aggggtccta caagctacag caggtccatc 13080
   agtatatgaa ggcccttcgt gaagagtatt ttgatccgag catggttggg tggacagtga 13140
   aatattatga aatagaagaa aatatggttg agctgatcaa gaccctttta gtttccttta 13200
   gggatgtcta ctctgaatat agtgtgacag ctgctgattt tgcttccaaa atgtcaactc 13260
   aagttgaaca atttgtgtcc agggatatca gagagtatct tagcatgctt actgatataa 13320
   atggaaagtg gatggaaaag attgcagagc tttctattgt ggcaaaggaa acaatgaaaa 13380
   gctgggtcac tgccgtggcc aaaataatgt ctgattaccc ccagcagttc cactccaatc 13440
   tgcaggattt ttcagaccaa ctctctagct actatgaaaa atttgttggt gagtccacaa 13500
   gattgattga cctgtccatt caaaactacc acgtgtttct cagatacatc accgagttac 13560
   tgagaaagct gcaggtggcc acagccaata atgtgagccc ctatataaag cttgctcaag 13620
   gagagctgat gatcaccttc tgattcatct actaacaaat tcaaattaaa ccttcacata 13680
   gtaggagact ttgtagacta ctataaagac catcctgagc cagacctgca gtcaacagca 13740
   agagcaagaa gcacatagga actatacctg caaccaagct ggcataagaa ccaagacctt 13800
   caaagcagcc tgaactcaag atgacatatt ttacaagtta gagtaaagtc aagagctgag 13860
   ttgttttgtc caactcagga tggagggagg gagggaaggg gaaataaata aatacttcct 13920
   tattgtgc 13928
<210> 3
   <211> 2273
   <212> DNA
   <213> H. sapiens
<400> 3
   gggggcagat cctggggaga atggaggagc acacagaggc aggctcggca ccagagatgg 60
   gggcccagaa ggccctgatt gacaatcctg ctgacatcct agtcattgct gcatatttcc 120
   tgctggtcat tggcgttggc ttgtggtcca tgtgcagaac caacagaggc actgtgggcg 180
   gctacttcct ggcaggacgc agcatggtgt ggtggccggt tggggcctct ctcttcgcca 240
   gcaacat cgg cagt ggccac t t t gt gggcc t ggcagggac t ggcgct gca agt ggct t gg 300
   ct gt t gct gg at t cgagt gg aat gcgct ct t cgt ggt gct gct act gggc t ggct gt t t g 360
   cacccgt gt a cct gacagcg ggggt cat ca cgat gccaca gt acct gcgc aagcgct t cg 420
   gcggccgccg catccgcctc tacctgtctg tgctctccct tttcctgtac atcttcacca 480
   agat ct cagt ggacat gt t c t ccggagct g t at t cat cca gcaggct ct g ggct ggaaca 540
   tctatgcctc cgtcatcgcg cttctgggca tcaccatgat ttacacggtg acaggagggc 600
   t ggccgcgct gat gt acacg gacacggt ac agacct t cgt cat t ct gggg ggcgcct gca 660
   t cct cat ggg t t acgcct t c cacgaggt gg gcgggt at t c gggt ct ct t c gacaaat acc 720
   t gggagcagc gact t cgct g acggt gt ccg aggat ccagc cgt gggaaac at ct ccagct 780
   tctgctatcg accccggccc gactcctacc acctgctccg gcaccccgtg accggggatc 840
   t gccgt ggcc cgcgct gct c ct cggact ca caat cgt ct c gggct ggt ac t ggt gcagcg 900
   accaggtcat cgtgcagcgc tgcctggccg ggaagagcct gacccacatc aaggcgggct 960
   gcat cct gt g t gggt acct g aagct gacgc ccat gt t t ct cat ggt cat g ccaggcat ga 1020
   t cagccgcat t ct gt accca gacgaggt gg cgt gcgt ggt gcct gaggt g t gcaggcgcg 1080
   t gt gcggcac ggaggt gggc t gct ccaaca t cgcct accc gcggct cgt c gt gaagct ca 1140
   t gcccaacgg t ct gcgcgga ct cat gct gg cggt cat gct ggccgcgct c at gt cct cgc 1200
   tggcctccat cttcaacagc agcagcacgc tcttcaccat ggacatctac acgcgcctgc 1260
   ggccacgcgc cggcgaccgc gagctgctgc tggtgggacg gctctgggtg gtgttcatcg 1320
   tggtagtgtc ggtggcctgg cttcccgtgg tgcaggcggc acagggcggg cagctcttcg 1380
   attacatcca ggcagtctct agctacctgg caccgcccgt gtccgccgtc ttcgtgctgg 1440
   cgct ct t cgt gccgcgcgt t aat gagcagg gcgcct t ct g gggact cat c gggggcct gc 1500
   t gat gggcct ggcacgcct g at t cccgagt t ct cct t cgg ct cgggcagc t gt gt gcagc 1560
   cct cggcgt g cccagct t t c ct ct gcggcg t gcact acct ct act t cgcc at t gt gct gt 1620
   tcttctgctc tggcctcctc accctcacgg tctccctgtg caccgcgccc atccccagaa 1680
   agcacctcca ccgcctggtc ttcagtctcc ggcatagcaa ggaggaacgg gaggacctgg 1740
   atgctgatga gcagcaaggc tcctcactcc ctgtacagaa tgggtgccca gagagtgcca 1800
   tggagatgaa tgagccccag gccccggcac caagcctctt ccgccagtgc ctgctctggt 1860
   tttgtggaat gagcagaggt ggggtgggca gtcctccgcc ccttacccag gaggaggcag 1920
   cggcagcagc caggcggctg gaggacatca gcgaggaccc gagctgggcc cgtgtggtca 1980
   acct caat gc cct gct cat g at ggcagt gg ccgt gt t cct ct ggggct t c t at gcct aag 2040
   accaact gcg t t ggacacca t aagccacag cct cacagga agt gggggt g aggagcct gc 2100
   ggtgctcccc agaaaagggg aaggggcagt ggggtgagaa ggtcctggct ccccttctcc 2160
   cggcct t cct ct gcct gggg cccact gcat ct gat t ggca gt cact t ccc at gagggcct 2220
   ggcccacccg ctgcagttgc cctaaggaaa aataaagctg cctttcccct gta        2273
<210> 4
   <211> 3636
   <212> DNA
   <213> H. sapiens
<400> 4
   cagcgacgtc gaggcgctca tggttgcagg cgggcgccgc cgttcagttc agggtctgag 60
   cctggaggag tgagccaggc agtgagactg gctcgggcgg gccgggacgc gtcgttgcag 120
   cagcggctcc cagctcccag ccaggattcc gcgcgcccct tcacgcgccc tgctcctgaa 180
   cttcagctcc tgcacagtcc tccccaccgc aaggctcaag gcgccgccgg cgtggaccgc 240
   gcacggcctc taggtctcct cgccaggaca gcaacctctc ccctggccct catgggcacc 300
   gtcagctcca ggcggtcctg gtggccgctg ccactgctgc tgctgctgct gctgctcctg 360
   ggtcccgcgg gcgcccgtgc gcaggaggac gaggacggcg actacgagga gctggtgcta 420
   gccttgcgtt ccgaggagga cggcctggcc gaagcacccg agcacggaac cacagccacc 480
   ttccaccgct gcgccaagga tccgtggagg ttgcctggca cctacgtggt ggtgctgaag 540
   gaggagaccc acctctcgca gtcagagcgc actgcccgcc gcctgcaggc ccaggctgcc 600
   cgccggggat acctcaccaa gatcctgcat gtcttccatg gccttcttcc tggcttcctg 660
   gtgaagatga gtggcgacct gctggagctg gccttgaagt tgccccatgt cgactacatc 720
   gaggaggact cctctgtctt tgcccagagc atcccgtgga acctggagcg gattacccct 780
   ccacggtacc gggcggatga ataccagccc cccgacggag gcagcctggt ggaggtgtat 840
   ctcctagaca ccagcataca gagtgaccac cgggaaatcg agggcagggt catggtcacc 900
   gacttcgaga atgtgcccga ggaggacggg acccgcttcc acagacaggc cagcaagtgt 960
   gacagtcatg gcacccacct ggcaggggtg gtcagcggcc gggatgccgg cgtggccaag 1020
   ggtgccagca tgcgcagcct gcgcgtgctc aactgccaag ggaagggcac ggttagcggc 1080
   accctcatag gcctggagtt tattcggaaa agccagctgg tccagcctgt ggggccactg 1140
   gtggtgctgc tgcccctggc gggtgggtac agccgcgtcc tcaacgccgc ctgccagcgc 1200
   ctggcgaggg ctggggtcgt gctggtcacc gctgccggca acttccggga cgatgcctgc 1260
   ctctactccc cagcctcagc tcccgaggtc atcacagttg gggccaccaa tgcccaagac 1320
   cagccggtga ccctggggac tttggggacc aactttggcc gctgtgtgga cctctttgcc 1380
   ccaggggagg acatcattgg tgcctccagc gactgcagca cctgctttgt gtcacagagt 1440
   gggacatcac aggctgctgc ccacgtggct ggcattgcag ccatgatgct gtctgccgag 1500
   ccggagctca ccctggccga gttgaggcag agactgatcc acttctctgc caaagatgtc 1560
   atcaatgagg cctggttccc tgaggaccag cgggtactga cccccaacct ggtggccgcc 1620
   ctgcccccca gcacccatgg ggcaggttgg cagctgtttt gcaggactgt atggtcagca 1680
   cactcggggc ctacacggat ggccacagcc gtcgcccgct gcgccccaga tgaggagctg 1740
   ctgagctgct ccagtttctc caggagtggg aagcggcggg gcgagcgcat ggaggcccaa 1800
   gggggcaagc tggtctgccg ggcccacaac gcttttgggg gtgagggtgt ctacgccatt 1860
   gccaggtgct gcctgctacc ccaggccaac tgcagcgtcc acacagctcc accagctgag 1920
   gccagcatgg ggacccgtgt ccactgccac caacagggcc acgtcctcac aggctgcagc 1980
   tcccactggg aggtggagga ccttggcacc cacaagccgc ctgtgctgag gccacgaggt 2040
   cagcccaacc agtgcgtggg ccacagggag gccagcatcc acgcttcctg ctgccatgcc 2100
   ccaggtctgg aatgcaaagt caaggagcat ggaatcccgg cccctcagga gcaggtgacc 2160
   gtggcctgcg aggagggctg gaccctgact ggctgcagtg ccctccctgg gacctcccac 2220
   gtcctggggg cctacgccgt agacaacacg tgtgtagtca ggagccggga cgtcagcact 2280
   acaggcagca ccagcgaagg ggccgtgaca gccgttgcca tctgctgccg gagccggcac 2340
   ctggcgcagg cctcccagga gctccagtga cagccccatc ccaggatggg tgtctgggga 2400
   gggtcaaggg ctggggctga gctttaaaat ggttccgact tgtccctctc tcagccctcc 2460
   atggcctggc acgaggggat ggggatgctt ccgcctttcc ggggctgctg gcctggccct 2520
   tgagtggggc agcctccttg cctggaactc actcactctg ggtgcctcct ccccaggtgg 2580
   aggtgccagg aagctccctc cctcactgtg gggcatttca ccattcaaac aggtcgagct 2640
   gtgctcgggt gctgccagct gctcccaatg tgccgatgtc cgtgggcaga atgactttta 2700
   ttgagctctt gttccgtgcc aggcattcaa tcctcaggtc tccaccaagg aggcaggatt 2760
   cttcccatgg ataggggagg gggcggtagg ggctgcaggg acaaacatcg ttggggggtg 2820
   agtgtgaaag gtgctgatgg ccctcatctc cagctaactg tggagaagcc cctgggggct 2880
   ccctgattaa tggaggctta gctttctgga tggcatctag ccagaggctg gagacaggtg 2940
   cgcccctggt ggtcacaggc tgtgccttgg tttcctgagc cacctttact ctgctctatg 3000
   ccaggctgtg ctagcaacac ccaaaggtgg cctgcgggga gccatcacct aggactgact 3060
   cggcagtgtg cagtggtgca tgcactgtct cagccaaccc gctccactac ccggcagggt 3120
   acacattcgc acccctactt cacagaggaa gaaacctgga accagagggg gcgtgcctgc 3180
   caagctcaca cagcaggaac tgagccagaa acgcagattg ggctggctct gaagccaagc 3240
   ctcttcttac ttcacccggc tgggctcctc atttttacgg gtaacagtga ggctgggaag 3300
   gggaacacag accaggaagc tcggtgagtg atggcagaac gatgcctgca ggcatggaac 3360
   tttttccgtt atcacccagg cctgattcac tggcctggcg gagatgcttc taaggcatgg 3420
   tcgggggaga gggccaacaa ctgtccctcc ttgagcacca gccccaccca agcaagcaga 3480
   catttatctt ttgggtctgt cctctctgtt gcctttttac agccaacttt tctagacctg 3540
   ttttgctttt gtaacttgaa gatatttatt ctgggttttg tagcattttt attaatatgg 3600
   tgacttttta aaataaaaac aaacaaacgt tgtcct 3636
<210> 5
   <211> 874
   <212> DNA
   <213> H. sapiens
<400> 5
   ctgcagcgtc tggggtttcc gttgcagtcc tcggaaccag gacctcggcg tggcctagcg 60
   agttatggcg acgaaggccg tgtgcgtgct gaagggcgac ggcccagtgc agggcatcat 120
   caatttcgag cagaaggaaa gtaatggacc agtgaaggtg tggggaagca ttaaaggact 180
   gactgaaggc ctgcatggat tccatgttca tgagtttgga gataatacag caggctgtac 240
   cagtgcaggt cctcacttta atcctctatc cagaaaacac ggtgggccaa aggatgaaga 300
   gaggcatgtt ggagacttgg gcaatgtgac tgctgacaaa gatggtgtgg ccgatgtgtc 360
   tattgaagat tctgtgatct cactctcagg agaccattgc atcattggcc gcacactggt 420
   ggtccatgaa aaagcagatg acttgggcaa aggtggaaat gaagaaagta caaagacagg 480
   aaacgctgga agtcgtttgg cttgtggtgt aattgggatc gcccaataaa cattcccttg 540
   gatgtagtct gaggcccctt aactcatctg ttatcctgct agctgtagaa atgtatcctg 600
   ataaacatta aacactgtaa tcttaaaagt gtaattgtgt gactttttca gagttgcttt 660
   aaagtacctg tagtgagaaa ctgatttatg atcacttgga agatttgtat agttttataa 720
   aactcagtta aaatgtctgt ttcaatgacc tgtattttgc cagacttaaa tcacagatgg 780
   gtattaaact tgtcagaatt tctttgtcat tcaagcctgt gaataaaaac cctgtatggc 840
   acttattatg aggctattaa aagaatccaa attc 874
<210> 6
   <211> 1631
   <212> DNA
   <213> H. sapiens
<400> 6
   ggacttctag cccctgaact ttcagccgaa tacatctttt ccaaaggagt gaattcaggc 60
   ccttgtatca ctggcagcag gacgtgacca tggagaagct gttgtgtttc ttggtcttga 120
   ccagcctctc tcatgctttt ggccagacag acatgtcgag gaaggctttt gtgtttccca 180
   aagagtcgga tacttcctat gtatccctca aagcaccgtt aacgaagcct ctcaaagcct 240
   tcactgtgtg cctccacttc tacacggaac tgtcctcgac ccggggtaca gtattttctc 300
   gtatgccacc aagagacaag acaatgagat tcttcatatt ttggtctaag gatataggat 360
   acagttttac agtgggtggg tctgaaatat tattcgaggt tcctgaagtc acagtagctc 420
   cagtacacat ttgtacaagc tgggagtccg cctcagggat cgtggagttc tgggtagatg 480
   ggaagcccag ggtgaggaag agtctgaaga agggatacac tgtgggggca gaagcaagca 540
   tcatcttggg gcaggagcag gattccttcg gtgggaactt tgaaggaagc cagtccctgg 600
   tgggagacat tggaaatgtg aacatgtggg actttgtgct gtcaccagat gagattaaca 660
   ccatctatct tggcgggccc ttcagtccta atgtcctgaa ctggcgggca ctgaagtatg 720
   aagtgcaagg cgaagtgttc accaaacccc agctgtggcc ctgaggccca gctgtgggtc 780
   ctgaaggtac ctcccggttt tttacaccgc atgggcccca cgtctctgtc tctggtacct 840
   cccgcttttt tacactgcat ggttcccacg tctctgtctc tgggcctttg ttcccctata 900
   tgcattgcag gcctgctcca ccctcctcag cgcctgagaa tggaggtaaa gtgtctggtc 960
   tgggagctcg ttaactatgc tgggaaacgg tccaaaagaa tcagaatttg aggtgttttg 1020
   ttttcatttt tatttcaagt tggacagatc ttggagataa tttcttacct cacatagatg 1080
   agaaaactaa cacccagaaa ggagaaatga tgttataaaa aactcataag gcaagagctg 1140
   agaaggaagc gctgatcttc tatttaattc cccacccatg acccccagaa agcaggagca 1200
   ttgcccacat tcacagggct cttcagtatc agaatcagga cactggccag gtgtctggtt 1260
   tgggtccaga gtgctcatca tcatgtcata gaactgctgg gcccaggtct cctgaaatgg 1320
   gaagcccagc aataccacgc agtccctcca ctttctcaaa gcacactgga aaggccatta 1380
   gaattgcccc agcagagcag atctgctttt tttccagagc aaaatgaagc actaggtata 1440
   aatatgttgt tactgccaag aacttaaatg actggttttt gtttgcttgc agtgctttct 1500
   taattttatg gctcttctgg gaaactcctc cccttttcca cacgaacctt gtggggctgt 1560
   gaattctttc ttcatccccg cattcccaat atacccaggc cacaagagtg gacgtgaaca 1620
   caggtgccgt g 1631
<210> 7
   <211> 1898
   <212> DNA
   <213> Musmusculus
<400> 7
   cggacgcgtg ggcgaggacc gcgaggagcg cagccctagc cccggcgact gagcacacct 60
   gaggagaggt gcacacactc tgaggaccta ggtgtgcaac ctctgccaga tgtggggcgt 120
   ggctacccag aggcatgccc ctcacccagc tccactgtcc ccacctgctg ctgctgctgt 180
   tggtgctgtc atgtctgcca gaggcaccct ctgcccaggt aatggacttt ttgtttgaga 240
   agtggaagct ctatagtgac caatgccacc acaacctaag cctgctgccc ccacctactg 300
   agctggtctg taacagaacc ttcgacaagt actcctgctg gcctgacacc cctcccaaca 360
   ccactgccaa catttcctgc ccctggtacc taccttggta ccacaaagtg cagcaccgcc 420
   tagtgttcaa gaggtgtggg cccgatgggc agtgggttcg agggccacgg gggcagccgt 480
   ggcgcaacgc ctcccaatgt cagttggatg atgaagagat cgaggtccag aagggggtgg 540
   ccaagatgta tagcagccag caggtgatgt acaccgtggg ctacagtctg tccctggggg 600
   ccttgctcct tgcgctggtc atcctgctgg gcctcaggaa gctgcactgc acccgaaact 660
   acatccatgg gaacctgttt gcgtcctttg tgctcaaggc tggctctgtg ttggtcatcg 720
   attggctgct gaagacacgg tacagccaga agattggcga tgacctcagt gtgagcgtct 780
   ggctcagtga cggggcgatg gccggctgca gagtggccac agtgatcatg cagtacggca 840
   tcatagccaa ctattgctgg ttgctggtag agggcgtgta cctgtacagc ctgctgagcc 900
   ttgccacctt ctctgagagg agcttctttt ccctctacct gggcattggc tggggtgcgc 960
   ccctgctgtt tgtcatcccc tgggtggtgg tcaagtgtct gtttgagaat gttcagtgct 1020
   ggaccagcaa tgacaacatg ggattctggt ggatcctgcg tattcctgtc ttcctggcct 1080
   tactgatcaa ttttttcatc tttgtccaca tcattcacct tcttgtggcc aagctgcgtg 1140
   cccatcagat gcactatgct gactataagt tccggctggc caggtccacg ctgaccctca 1200
   tccctctgct gggggtccac gaggtggtct ttgcctttgt gactgacgag catgcccaag 1260
   gcaccctgcg ctccaccaag ctcttttttg acctgttcct cagctccttc cagggtctgc 1320
   tggtggctgt tctctactgt ttcctcaaca aggaggtgca ggcagagctg atgcggcgtt 1380
   ggaggcaatg gcaagaaggc aaagctcttc aggaggaaag gttggccagc agccatggca 1440
   gccacatggc cccagcaggg ccttgtcatg gtgatccctg tgagaaactt cagcttatga 1500
   gtgcaggcag cagcagtggg actggctgtg tgccctctat ggagacctcg ctggccagta 1560
   gtctcccaag gttggctgac agccccacct gaatctccac tggagcctag ccaggctgcg 1620
   ttcagaaagg gcctcagagg acaacccaga gccagatgcc cggccaaggc tgaagagaca 1680
   aagcagcaag acagcagctt gtactgtgca cactccccta acctgtccta gcctggcaca 1740
   ggccacagtg acagagtagg ggttggatat gatggagaag ccatgttatc tatgaactct 1800
   gagtgttccc atgtgtgttg acatggtccc tgtacccaga tatgtccttc agtaaaaagc 1860
   tcgagtggga aaaaaaaaaa aaaaaaaaaa aaaaaaaa                        1898

<210> 8
   <211> 106000
   <212> DNA
   <213> H. sapiens
<400> 8

<210> 9
   <211> 2034
   <212> DNA
   <213> H. sapiens
<400> 9
   ggatctggca gcgccgcgaa gacgagcggt caccggcgcc cgacccgagc gcgcccagag 60
   gacggcgggg agccaagccg acccccgagc agcgccgcgc gggccctgag gctcaaaggg 120
   gcagcttcag gggaggacac cccactggcc aggacgcccc aggctctgct gctctgccac 180
   t cagct gccc t cggaggagc gt acacacac accaggact g cat t gcccca gt gt gcagcc 240
   cctgccagat gtgggaggca gctagctgcc cagaggcatg cccccctgcc agccacagcg 300
   acccct gct g ct gt t gct gc t gct gct ggc ct gccagcca caggt cccct ccgct caggt 360
   gat ggact t c ct gt t t gaga agt ggaagct ct acggt gac cagt gt cacc acaacct gag 420
   cctgctgccc cctcccacgg agctggtgtg caacagaacc ttcgacaagt attcctgctg 480
   gccggacacc cccgccaata ccacggccaa catctcctgc ccctggtacc tgccttggca 540
   ccacaaagtg caacaccgct tcgtgttcaa gagatgcggg cccgacggtc agtgggtgcg 600
   tggaccccgg gggcagcctt ggcgtgatgc ctcccagtgc cagatggatg gcgaggagat 660
   t gaggt ccag aaggaggt gg ccaagat gt a cagcagct t c caggt gat gt acacagt ggg 720
   ct acagcct g t ccct ggggg ccct gct cct cgcct t ggcc at cct ggggg gcct cagcaa 780
   gct gcact gc acccgcaat g ccat ccacgc gaat ct gt t t gcgt cct t cg t gct gaaagc 840
   cagctccgtg ctggtcattg atgggctgct caggacccgc tacagccaga aaattggcga 900
   cgacct cagt gt cagcacct ggct cagt ga t ggagcggt g gct ggct gcc gt gt ggccgc 960
   ggt gt t cat g caat at ggca t cgt ggccaa ct act gct gg ct gct ggt gg agggcct gt a 1020
   cctgcacaac ctgctgggcc tggccaccct ccccgagagg agcttcttca gcctctacct 1080
   gggcat cggc t ggggt gccc ccat gct gt t cgt cgt cccc t gggcagt gg t caagt gt ct 1140
   gttcgagaac gtccagtgct ggaccagcaa tgacaacatg ggcttctggt ggatcctgcg 1200
   gt t ccccgt c t t cct ggcca t cct gat caa ct t ct t cat c t t cgt ccgca t cgt t cagct 1260
   gctcgtggcc aagctgcggg cacggcagat gcaccacaca gactacaagt tccggctggc 1320
   caagt ccacg ct gaccct ca t ccct ct gct gggcgt ccac gaagt ggt ct t t gcct t cgt 1380
   gacggacgag cacgcccagg gcaccctgcg ctccgccaag ctcttcttcg acctcttcct 1440
   cagct cct t c cagggcct gc t ggt ggct gt cct ct act gc t t cct caaca aggaggt gca 1500
   gtcggagctg cggcggcgtt ggcaccgctg gcgcctgggc aaagtgctat gggaggagcg 1560
   gaacaccagc aaccacaggg cctcatcttc gcccggccac ggccctccca gcaaggagct 1620
   gcagtttggg aggggtggtg gcagccagga ttcatctgcg gagaccccct tggctggtgg 1680
   cctccctaga ttggctgaga gccccttctg aaccctgctg ggaccccagc tagggctgga 1740
   ctctggcacc cagaggcgtc gctggacaac ccagaactgg acgcccagct gaggctgggg 1800
   gcgggggagc caacagcagc ccccacctac cccccacccc cagtgtggct gtctgcgaga 1860
   t t gggcct cc t ct ccct gca cct gcct t gt ccct ggt gca gaggt gagca gaggagt cca 1920
   gggcgggagt gggggctgtg ccgtgaactg cgtgccagtg tccccacgta tgtcggcacg 1980
   t cccat gt gc at ggaaat gt cct ccaacaa t aaagagct c aagt ggt cac cgt g 2034
<210> 10
   <211> 2439
   <212> DNA
   <213> H. sapiens
<400> 10
   ctccgggaac gccagcgccg cggctgccgc ctctgctggg gtctaggctg tttctctcgc 60
   gccaccactg gccgccggcc gcagctccag gtgtcctagc cgcccagcct cgacgccgtc 120
   ccgggacccc t gt gct ct gc gcgaagccct ggccccgggg gccggggcat gggccagggg 180
   cgcggggtga agcggcttcc cgcggggccg tgactgggcg ggcttcagcc atgaagaccc 240
   tcatagccgc ctactccggg gtcctgcgcg gcgagcgtca ggccgaggct gaccggagcc 300
   agcgctctca cggaggacct gcgctgtcgc gcgaggggtc tgggagatgg ggcactggat 360
   ccagcat cct ct ccgccct c caggacct ct t ct ct gt cac ct ggct caat aggt ccaagg 420
   t ggaaaagca gct acaggt c at ct cagt gc t ccagt gggt cct gt cct t c ct t gt act gg 480
   gagt ggcct g cagt gccat c ct cat gt aca t at t ct gcac t gat t gct gg ct cat cgct g 540
   tgctctactt cacttggctg gtgtttgact ggaacacacc caagaaaggt ggcaggaggt 600
   cacagt gggt ccgaaact gg gct gt gt ggc gct act t t cg agact act t t cccat ccagc 660
   t ggt gaagac acacaacct g ct gaccacca ggaact at at ct t t ggat ac cacccccat g 720
   gtatcatggg cctgggtgcc ttctgcaact tcagcacaga ggccacagaa gtgagcaaga 780
   agt t cccagg cat acggcct t acct ggct a cact ggcagg caact t ccga at gcct gt gt 840
   tgagggagta cctgatgtct ggaggtatct gccctgtcag ccgggacacc atagactatt 900
   t gct t t caaa gaat gggagt ggcaat gct a t cat cat cgt ggt cgggggt gcggct gagt 960
   ctctgagctc catgcctggc aagaatgcag tcaccctgcg gaaccgcaag ggctttgtga 1020
   aact ggccct gcgt cat gga gct gacct gg t t cccat ct a ct cct t t gga gagaat gaag 1080
   tgtacaagca ggtgatcttc gaggagggct cctggggccg atgggtccag aagaagttcc 1140
   agaaat acat t ggt t t cgcc ccat gcat ct t ccat ggt cg aggcct ct t c t cct ccgaca 1200
   cctgggggct ggtgccctac tccaagccca tcaccactgt tgtgggagag cccatcacca 1260
   tccccaagct ggagcaccca acccagcaag acatcgacct gtaccacacc atgtacatgg 1320
   aggccctggt gaagctcttc gacaagcaca agaccaagtt cggcctcccg gagactgagg 1380
   t cct ggaggt gaact gagcc agcct t cggg gccaact ccc t ggaggaacc agct gcaaat 1440
   cact t t t t t g ct ct gt aaat t t ggaagt gt cat gggt gt c t gt gggt t at t t aaaagaaa 1500
   t t at aacaat t t t gct aaac cat t acaat g t t aggt ct t t t t t aagaagg aaaaagt cag 1560
   t at t t caagt t ct t t cact t ccagct t gcc ct gt t ct agg t ggt ggct aa at ct gggcct 1620
   aat ct gggt g gct cagct aa cct ct ct t ct t ccct t cct g aagt gacaaa ggaaact cag 1680
   tcttcttggg gaagaaggat tgccattagt gacttggacc agttagatga ttcacttttt 1740
   gcccct aggg at gagaggcg aaagccact t ct cat acaag cccct t t at t gccact accc 1800
   cacgctcgtc tagtcctgaa actgcaggac cagtttctct gccaagggga ggagttggag 1860
   agcacagt t g ccccgt t gt g t gagggcagt agt aggcat c t ggaat gct c cagt t t gat c 1920
   t ccct t ct gc cacccct acc t cacccct ag t cact cat at cggagcct gg act ggcct cc 1980
   aggat gagga t gggggt ggc aat gacaccc t gcaggggaa aggact gccc cccat gcacc 2040
   at t gcaggga ggat gccgcc accat gagct aggt ggagt a act ggt t t t t ct t gggt ggc 2100
   t gat gacat g gat gcagcac agact cagcc t t ggcct gga gcacat gct t act ggt ggcc 2160
   t cagt t t acc t t ccccagat cct agat t ct ggat gt gagg aagagat ccc t ct t cagaag 2220
   gggcctggcc ttctgagcag cagattagtt ccaaagcagg tggcccccga acccaagcct 2280
   cacttttctg tgccttcctg agggggttgg gccggggagg aaacccaacc ctctcctgtg 2340
   t gt t ct gt t a t ct ct t gat g agat cat t gc accat gt cag act t t t gt at at gcct t gaa 2400
   aataaatgaa agtgagaatc caaaaaaaaa aaaaaaaaa 2439
<210> 11
   <211> 3318
   <212> DNA
   <213> H. sapiens
<400> 11
   gtgatgcgta gttccggctg ccggttgaca tgaagaagca gcagcggcta gggcggcggt 60
   agctgcaggg gtcggggatt gcagcgggcc tcggggctaa gagcgcgacg cggcctagag 120
   cggcagacgg cgcagtgggc cgagaaggag gcgcagcagc cgccctggcc cgtcatggag 180
   atggaaaagg agttcgagca gatcgacaag tccgggagct gggcggccat ttaccaggat 240
   atccgacatg aagccagtga cttcccatgt agagtggcca agcttcctaa gaacaaaaac 300
   cgaaataggt acagagacgt cagtcccttt gaccatagtc ggattaaact acatcaagaa 360
   gataatgact atatcaacgc tagtttgata aaaatggaag aagcccaaag gagttacatt 420
   cttacccagg gccctttgcc taacacatgc ggtcactttt gggagatggt gtgggagcag 480
   aaaagcaggg gtgtcgtcat gctcaacaga gtgatggaga aaggttcgtt aaaatgcgca 540
   caatactggc cacaaaaaga agaaaaagag atgatctttg aagacacaaa tttgaaatta 600
   acattgatct ctgaagatat caagtcatat tatacagtgc gacagctaga attggaaaac 660
   cttacaaccc aagaaactcg agagatctta catttccact ataccacatg gcctgacttt 720
   ggagtccctg aatcaccagc ctcattcttg aactttcttt tcaaagtccg agagtcaggg 780
   tcactcagcc cggagcacgg gcccgttgtg gtgcactgca gtgcaggcat cggcaggtct 840
   ggaaccttct gtctggctga tacctgcctc ttgctgatgg acaagaggaa agacccttct 900
   tccgttgata tcaagaaagt gctgttagaa atgaggaagt ttcggatggg gctgatccag 960
   acagccgacc agctgcgctt ctcctacctg gctgtgatcg aaggtgccaa attcatcatg 1020
   ggggactctt ccgtgcagga tcagtggaag gagctttccc acgaggacct ggagccccca 1080
   cccgagcata tccccccacc tccccggcca cccaaacgaa tcctggagcc acacaatggg 1140
   aaatgcaggg agttcttccc aaatcaccag tgggtgaagg aagagaccca ggaggataaa 1200
   gactgcccca tcaaggaaga aaaaggaagc cccttaaatg ccgcacccta cggcatcgaa 1260
   agcatgagtc aagacactga agttagaagt cgggtcgtgg ggggaagtct tcgaggtgcc 1320
   caggctgcct ccccagccaa aggggagccg tcactgcccg agaaggacga ggaccatgca 1380
   ctgagttact ggaagccctt cctggtcaac atgtgcgtgg ctacggtcct cacggccggc 1440
   gcttacctct gctacaggtt cctgttcaac agcaacacat agcctgaccc tcctccactc 1500
   cacctccacc cactgtccgc ctctgcccgc agagcccacg cccgactagc aggcatgccg 1560
   cggtaggtaa gggccgccgg accgcgtaga gagccgggcc ccggacggac gttggttctg 1620
   cactaaaacc catcttcccc ggatgtgtgt ctcacccctc atccttttac tttttgcccc 1680
   ttccactttg agtaccaaat ccacaagcca ttttttgagg agagtgaaag agagtaccat 1740
   gctggcggcg cagagggaag gggcctacac ccgtcttggg gctcgcccca cccagggctc 1800
   cctcctggag catcccaggc gggcggcacg ccaacagccc cccccttgaa tctgcaggga 1860
   gcaactctcc actccatatt tatttaaaca attttttccc caaaggcatc catagtgcac 1920
   tagcattttc ttgaaccaat aatgtattaa aattttttga tgtcagcctt gcatcaaggg 1980
   ctttatcaaa aagtacaata ataaatcctc aggtagtact gggaatggaa ggctttgcca 2040
   tgggcctgct gcgtcagacc agtactggga aggaggacgg ttgtaagcag ttgttattta 2100
   gtgatattgt gggtaacgtg agaagataga acaatgctat aatatataat gaacacgtgg 2160
   gtatttaata agaaacatga tgtgagatta ctttgtcccg cttattctcc tccctgttat 2220
   ctgctagatc tagttctcaa tcactgctcc cccgtgtgta ttagaatgca tgtaaggtct 2280
   tcttgtgtcc tgatgaaaaa tatgtgcttg aaatgagaaa ctttgatctc tgcttactaa 2340
   tgtgccccat gtccaagtcc aacctgcctg tgcatgacct gatcattaca tggctgtggt 2400
   tcctaagcct gttgctgaag tcattgtcgc tcagcaatag ggtgcagttt tccaggaata 2460
   ggcatttgcc taattcctgg catgacactc tagtgacttc ctggtgaggc ccagcctgtc 2520
   ctggtacagc agggtcttgc tgtaactcag acattccaag ggtatgggaa gccatattca 2580
   cacctcacgc tctggacatg atttagggaa gcagggacac cccccgcccc ccacctttgg 2640
   gatcagcctc cgccattcca agtcaacact cttcttgagc agaccgtgat ttggaagaga 2700
   ggcacctgct ggaaaccaca cttcttgaaa cagcctgggt gacggtcctt taggcagcct 2760
   gccgccgtct ctgtcccggt tcaccttgcc gagagaggcg cgtctgcccc accctcaaac 2820
   cctgtggggc ctgatggtgc tcacgactct tcctgcaaag ggaactgaag acctccacat 2880
   taagtggctt tttaacatga aaaacacggc agctgtagct cccgagctac tctcttgcca 2940
   gcattttcac attttgcctt tctcgtggta gaagccagta cagagaaatt ctgtggtggg 3000
   aacattcgag gtgtcaccct gcagagctat ggtgaggtgt ggataaggct taggtgccag 3060
   gctgtaagca ttctgagctg ggcttgttgt ttttaagtcc tgtatatgta tgtagtagtt 3120
   tgggtgtgta tatatagtag catttcaaaa tggacgtact ggtttaacct cctatccttg 3180
   gagagcagct ggctctccac cttgttacac attatgttag agaggtagcg agctgctctg 3240
   ctatatgcct taagccaata tttactcatc aggtcattat tttttacaat ggccatggaa 3300
   taaaccattt ttacaaaa 3318

<210> 12
   <211> 78001
   <212> DNA
   <213> H. sapiens
<400> 12
   cagattccgg gtctcatccc tagaccaaag atatctgaat catttggcga tgggtccagg 60
   aacctgcatt attcaacaca cttcccaggt gaccgtaaat gtcaaaagac aaaattacaa 120
   caaacttaaa catcttaatt ggcttcattc atgattctag aatcgggcaa gacttcattc 180
   ctcaaaatag cacaagtgtc ccaatgagct gagcagagga ggttggtttt atagacagaa 240
   aagggctgaa aaaagcagaa acaaagaaca aaaagcagat tggtcatttc aaagttactt 300
   tccttgtaag gcaggaacag ggaaacagaa caagagagaa ataactgatt ggtcgcatcg 360
   ggttacttca ggttactttt tgttgtaagg attaaagcaa agggaacttc attatgttga 420
   ttaaaacggt ctgcttggga aatcagggtg tgtatctctc ttctgatttt gtgaaaggtt 480
   atcagtctga tgatgtagaa ctttagcatg agtgactcca ttttgatttt tagtctagtc 540
   tgttgagacc ctaatgccag aacttttttt gtttgtttgt ttcgctcttg ttgcccaggc 600
   tggagtgcag tggagctatc ttggctcact gcaacctctg cctccagggt tcaagcgatt 660
   ctcctgcctc agcctcccaa gtagctggga ttacaggcat gcgccaccac gcccggctaa 720
   tttttttttt tttttttttt tttagcagaa accgagtttc accatgttgg tcaggctggt 780
   ctcggactct tgacctcagg tgatccacct gcgtcggtct cccaaagtgc tgggattaca 840
   ggtgtgagcc accacaccca gccctttttt ttcaagacag ggtctctctc tgccacccaa 900
   ggtggagtgc agtggcgcca aaacagctca ctgcagcttc cacctcctgg gctcaggtga 960
   tcttcctgcc tcagcctccc cagcagctgg gccccaccac accggctaat tttttaactt 1020
   ttagtagtga cgaggtctga ttctgttacc caggctggtc tggaactcct ggcctcaaga 1080
   catccgcctg cctctgcctc ccaaagtgct gggattacag atgtaagcca ccgcgcctgg 1140
   gctcctatga tttttattta acataatgca ccatggaatt tgtgctctgc ttagttcagt 1200
   ctgagcagga gttccttgat acttcgggaa acactgaaaa tcattccatc cccatccatt 1260
   cattcctgca gcacccaagt ggaaattctg cgtttcagac agggacacta cccttagaga 1320
   gcagtgggct tccccagcag cgtagtgaaa catgatactc ctgagtttca tgaaaaaagg 1380
   gcagacatct ggccagagct gggaggcagg aaatagagca cggtgccctc ctcccatact 1440
   ccagct t gga t t act gaggc t ggggcccag gccct gcagg aaaggaggt g cat gact act 1500
   t t aaggccac t cact ct gt g act caacggg ccgggt cggg gct ggaact c aat gccct cc 1560
   cgggcctgga gagcccacgc gccgtgggcg gggctcccgg ggtcgcctag gcaacaggcg 1620
   cgcgccgcgc ccgagcccag agccccaaag cggaggaggg aacgcgcgct attagatatc 1680
   tcgcggtgct ggggccactt cccctagcac cgcccccggc tcctccccgc ggaagtgctt 1740
   gtcgaaattc tcgatcgctg attggtcctt ctgcttcagg ggcggagccc ctggcaggcg 1800
   tgatgcgtag ttccggctgc cggttgacat gaagaagcag cagcggctag ggcggcggta 1860
   gctgcagggg tcggggattg cagcgggcct cggggctaag agcgcgacgc ggcctagagc 1920
   ggcagacggc gcagtgggcc gagaaggagg cgcagcagcc gccctggccc gtcatggaga 1980
   t ggaaaagga gt t cgagcag at cgacaagt ccgggagct g ggcggccat t t accaggt gc 2040
   gggagcgccc cggagcgtgg cgggcccttc gcttaggccg cttgaacatc ccctcagacc 2100
   t ccaggcccc agact ccct c t gggt ct t gc cct ct gcct c gct cct act g ct t gaggat t 2160
   cgatgggaca gcgacgcact gcgtcccccc accctttgtc cccggggcgg gcgtgtttct 2220
   cgccgcagcg tcggagcccc cttcgatccc ccacctccct tctgttctcc agctcgggtg 2280
   atctctcaag ccgggggacc gccggtctgt gctctcaacg cgaatccctc gcaccccgac 2340
   cccgccccct gcctgtccac tctttgtccc ctggggtgat ttagcacccc cactatttcc 2400
   t t t t ct ggag t ggaccacct cagact ct ct t cct t t gt ct ccct ggggga aaaggt t act 2460
   ccccccgtcc ctccttcaca tttcctttcc cctagtctca gtgtgcgtcg agtcccagag 2520
   at gacagt cc cct t t cccct t t ct gt t cat t cat t t at t g gat aggagt t ggcaagct t a 2580
   ttctgtgcta ggcaccgctt aggcattgga ggtggtgttt gctaatcagg acaggcaaga 2640
   tcctagcctt agtggggcct agagtcgaat agggcaatca aacacaaaag caaataattt 2700
   cagatagtga caggtgctgt gaagagaacg acttcctaac ggggtacagg gtgactgcat 2760
   agaaggccgg ctgtcttaga gaaggggatc agggaaggcc tgtcaaagga ggagacattt 2820
   gctttgtgag ctgaaccaag aggagcagaa agccgtgaga atatggggct aaagaacctt 2880
   ct agccagga ggcct gcggt acccact cca t t ggggccat gat at t at t c t t t caggcag 2940
   ggact cagga aggt t aacgt t t t aaccct c t ct aaaat ag cat ct t t cct caat gagcag 3000
   ct t agt ct t t ggt cgt ggca gagat gacct t gt ct t agga gt cat ct cct t gt gt gt t aa 3060
   aaagttagga aaggagggtt tctcatatat ctataaaaca agtagttaaa aacacaaaga 3120
   gct ct t cct t t cacaagcag ct gaat aaga t acat act cc caat t aaat g t cat t gcggg 3180
   ggt t gt t aag at t aact aaa accacact t g cacagt at ct t aaat aagcg at at acagaa 3240
   tagagagatt ttgttacttg tgtaaaggga gacagcagat gattctgttt tcagcttata 3300
   ggct caaaag gcaaat t gt g agat ccat ca gct gt agt at t aaaat ct at t t t gagct cc 3360
   gcttagaaag gaaaaaaggt ttaagcagtt ctttggtatg cttgactaac aaaagccttt 3420
   t t t t t t ggca gcct t gat t t t cat gt ggat t t acat caag ct t at t t gac aggat t ct t t 3480
   t t at t t ggac t gt agt gt gt at at t agt t t ct gct agact aat at t t ct a accact gt aa 3540
   t ct at at act aat aagt at g at t gat cagt at at aaaat t t gt at gccat at ct ggt ct c 3600
   t gaat t agct gaat gaat t c cat aagggac t t t gagact g t gt agacaaa t t t t ct gcat 3660
   cagt t t aat g cagt agagt c t aaaat gt ct t t aaat gaaa at t gt t ggt c t gaagt gt t g 3720
   gagttgatta tgatacaccc catcacagtg gaagcattgt ggagagaagt cttttccact 3780
   gaaat t gact gagt t gacaa caagaaat ac gt at t gt aac t t agt t ct t a gt t gaat t t t 3840
   at t t ct t aca at t t t aagcc agagt gggt t gacct gt cac ccaagcat gg t t aaaat t gt 3900
   at t cagcat g caact agcat ggagt gt gt c agt ct t caat t cat t t cct t cat t gt t ct t 3960
   aagtttttct gccacaatta aaccccacaa gttagtcaag gtgttgagat tttcactgct 4020
   t ct t aat gga t t gccacat t ccct gaggt a gt t t ct t t t g gt ct t agaga at t gt caggg 4080
   ccagct t t t c t cacct ccac t gt at ggat a t t t t t ct t t t ct aagat ct t gaaat cagaa 4140
   gct t t t ct cc t aagt gt aaa agt agct ct t t gt cat acaa ct gt agcgt t t t ct gaaaca 4200
   gagt t cagat gacct t gagt ct aaagt ggc t aact t t cca aggt gt gt at cgct t t acca 4260
   aaaccat t at t t t t caagga t t caaagaat gt gt t t acaa t t gat agaaa at ggaagt t t 4320
   aaaaaaat t a at act t t at a gcat gt t gaa at gagggcag cct t at acaa agt cat act t 4380
   t gagct t gcc t agcct at t g t gat cagaga at aat gt aat t t t t gct t ac aact t ggt aa 4440
   gcaggt cagt t at t ct aact t at t t t ct ga t t agaacaaa aagat gt aaa aact t gaaaa 4500
   ctattgggaa aagaacaaag agtgaagagg acttttgagt gctgaggaat gtggcagctt 4560
   ggaaaacaaa ctttttaggc agagattctt tgctaggtca gtttgataaa gtgagcataa 4620
   ccgt at t t t t aat ct t t aat gct aat gaat agcat agat g ct aat aagca t ct aggt ct a 4680
   t aaaaagt ca gct t t gat ag t gt at at aga t ggct t t aaa cat t gt t t t c t agcat t t aa 4740
   acact t t caa at cat ccggt t gct t gat t g ggcct agct g t ct aagagga gagaat gagc 4800
   ccagatgagg aaaagagatt gattttactg agctagaatg agaggagaga gggttgagtg 4860
   aat gaaaaga at agct cat g t gct cccct c cat ct gt agt t t aagagggg t t gggt ccgg 4920
   t gt t t t gct t gt t t t ct cgt ct gt aaat t c t t t gat t ct c t gacaccact cact at at t t 4980
   cat t gt gaat gat t t gat t g t t t cagat aa aggggact gc aat aat acct t gt gacat ga 5040
   aggcaagatt tattcatgtt agaggcaggc tttgtaaaat gggccactct tccaattgac 5100
   at t t gt t t t t at agct gt t t t cat t at gaa at acaat ct a at gcct gact aggt t aaaac 5160
   cat gt t gt aa caat agt t ca ct aaaat t cc t t act gat at acagct t at g t t gt t at at t 5220
   ccaaaaagat gaatattaaa atttgccaat aatgtttatt taaatactat tttcttcaga 5280
   ggaaaaaaaa ctattttatg caaaggagaa agatctatac actatgactc acttcactta 5340
   aaaaaaaaaa gactaacgga aatgacatgg agagactggg aagttctagt catcttgagt 5400
   gacccat t ag at ct aaat gt t ct t gt t t ag ccct ggt t t g agt gaact aa at t t aggt gt 5460
   ct gat cagt a ct t t ggaaat ggt gt aaat g cct t t gt aat t gt ct ggact gat at t agat 5520
   t aact gggag cacaagt aga aat agt gaag gaaagaact t t t t gct at t g t t at t t gaca 5580
   tcactggcat atttatagga atactttggt gtttttggaa gtaagtaaac caaccagtgg 5640
   ttctaaaaag tcagctgggg gataatggta atgccgctgt ttcttagctg caagttatct 5700
   gccgttactt ctcctccatt ttgcatttta tcttgaatag ctcctcaaaa cctattaaaa 5760
   tacctggtat tgaataatgt aattgaatgt gtactgaatt tcacagtgga aatgaataag 5820
   aaatttcctg tggaggtttt ttgacttagc tactgaaata acggcctttt gttgtgtgat 5880
   tctttccctt ttctctttgt taaagaaaac tgtcttgtga tcttgtagat tacagaatcc 5940
   ttttggcaat ttctgttcct agcactgctt tttctttctt tctttctttt aaatagaaat 6000
   ggggttttgc tgtgttgccc aggttggtct tgaactcctg gcttcaagcg atcctcccac 6060
   cttggcctcc tgaagttggg attgcaggcg tgagcaggta ctttttctga ggcctgcctg 6120
   agcctatata tattttgcac aatttggcat tcctccctac agtgtttatg ctgatttgtt 6180
   tctggtaaca actaatactg gcaaatcggc tgggcatgtt actttatgct gcccatattc 6240
   aggaaaattg gaattctagc tgggtcattg ttcccagatg atgtagtttg gcaccagcca 6300
   ttccatgttc acattttgag tatccaggag ggctggggac tttggagtag ttggtgattc 6360
   cctctgccac atttcactgg ttggtcacta tggcatcctt tccaccacac tagtagtcta 6420
   ggttctcaga tgttgcttat gagcctgcaa tggtttctag tttcacactg cagaaatgag 6480
   tgaagccggt tacccgttaa tatggtccca tcatcactag agtaattcat tgttctaaaa 6540
   ccagatctga gtctctcact cctctgcaac tacttctgat tctttcataa cacttgtaaa 6600
   gtccaaactc ctctttagca tggcagccag cttccagtcc ttccctccta tgtggcttcc 6660
   attctagcca gacaagaaag ggcagcgttc tccaaactca tcctcgccct tcattcctct 6720
   ataccattgc tgagcacttt gttgaggatg cctctcccgt tcaatctagc ttgcatcttc 6780
   cagctcgaat gtgtgcttcc ttgcaccaga gttttgttcc gtcacctgtg tgttttcata 6840
   caagctggca catatctctt ctaaagccct gctgtcattg tagctgcgtc tttacaaaca 6900
   tttttttttt aaatttttat aaagtcaagg tctcactata ttgcccaggc tggtctcaaa 6960
   ctcctgggct caagtgatcc tcctgccttg gcctcccaga gtgctgggat tataggtatg 7020
   agacactgtg cccagctgta gctgctactt tatatcccag gtctatctcc aatggagccc 7080
   aagcttcctg aggccacctg ttgtatcttt ctcattcatc ttgaagtcct ctgctcctgg 7140
   cacagagtag gtacctaaca agagttggga ttgaattgat ggtcagtact ttgctagcct 7200
   gatggtataa agatgtacaa aacatgttcc tggctcccac tctagggggg caatgatgga 7260
   aacaaataga ttagcccaca ttagtaccaa tagtagaggt cactctggga gaaggccccc 7320
   accacatttt gagtcatggc ctaatgaggt aatttagtat tgcctgctgc agtggctttg 7380
   gaagaaaggc tggcattctt agccagtaga agctgatacc actgatttgt ttcacagaag 7440
   ctttaaatat aacaataaat ttgtgcttgg cctacggtga actttacagg caacttggag 7500
   gtaatatgtt tgtctctcta agaattgttg aattccctct ccctcatccc tcctgactgg 7560
   ttctcacaag cctagcgggc ctttgcatgt ggttggttca taaaatactt tttgattttg 7620
   ggatataaaa tatagttctc cataaaataa cgactgttac caagtctttg attttttttt 7680
   tcaaactata aatggtaatg acattctttg gcctttgatc agaccaccct taggggcaag 7740
   agagtagttt catgttttgc tttttctagt gtcccctgtg tctgggtata gttgcagtct 7800
   cagctgtcat actaacagtg ctgagtgagt cccttacttt ctttgggttt tggtttctcc 7860
   cttgtaaaaa tgatcctgga ctaactgatc attaagttca ggtcaagtaa taaaaatcct 7920
   taatgtactc acaaatacaa tttaatgttc ctgaataatc cttgtaaaaa ctgcagcagt 7980
   tactcagttt tgtaaggtgt ggttgggtac tattaggctc aaaagtttat aggagctttg 8040
   tgagtatagt taacaactca aaagaatggg gtgttttttc ccgaggggca tgaaatgttt 8100
   ttgataaata gagttcattt gacttggtaa tgtggaaaat gagtagccct gacacgtacg 8160
   ctatgctttt gcagtttttc tctcaagtag caattgggtg gcttttcctg taaaagatag 8220
   aggaactgat tcttgagaat ttacgaaagc ttcaacccta actaggtatg caaagaatag 8280
   ttgcccttta tgttgtaatt ttaggaagaa acctacatct ggtctaagtt tcatttgaat 8340
   aatatgatag tttacacatc tgccatattt gagaagaaag tacctaagtc tccagcattt 8400
   tagaaataat gctttacttt gtgtagaaat ggtctttaga gtttaatagc tgctgccctc 8460
   tcctttttca aagcagcttg acataatcat gagtatcttg ctgacagctt gtaaattttg 8520
   attgtatgaa aactgaaaat aagaccattt cacatggaag attccctcct gccctgaaac 8580
   agccaaagaa aactgtagcc atcaaatcta ttgatctctg ggctttggta caagtcacac 8640
   tactacaaat aaaataatac caagtactta taaatgattt tcagtccttt taaagtttat 8700
   ttttttaata ttttttttga gatggggtct tgctgtgtcg tccaggctgg agtgcagtgg 8760
   cacaatcttg gctcactgca acctccacct cctgggctca agtgatcctc ccacctcagg 8820
   ctcccaagta gctgagacta caggcatgtg ccatcacgcc cagctaattt ttgtattttt 8880
   ttggagtaga gatgggattt tgctgtgttg cccaggctgg tcttgaactc ctgggcttaa 8940
   gccatctgtc tgcctcaggc tcccaaagtg ttgggattac aggtgtgagc cactgtgccc 9000
   ggcccagccc tttttttaag agaaaaacgt atgacatcgt tcgatttact gagtgcttat 9060
   ggttttacta aggcagtaag gttttatgga taccctatgg taattagata gaattagtgc 9120
   tctgaagtca gctctgtaat atggactcag agtaaacatg gcaaagggac acttaaggtc 9180
   tgcattttct ctgggaaata aacgtattct ttactactct gaatctagtg ctgggaaatt 9240
   ctaaatcctt cttgaggatt aaccacttga agtaaagttt tgggtcccaa gtaggcttgt 9300
   gtccctgtct ccttctcttt acttttcaga tgtttcttcc tagagactga ggtatatttt 9360
   acttttacag atgaagaagg aagcctcggc tgtgtttgtg gcttttgtgg gtgagcaaca 9420
   tcacttgcaa agataagatg agcatagcaa aactaggctt tcaaaataat ttttaaaaat 9480
   ttcttagtga ttagaaaagg aaaactcttc ccttgtctct gttaagaaac gtttttcgac 9540
   ttttttcctt tcttaatgga tcttttattg gcacttctct tccttttgca gaatcttact 9600
   taaaagtcac tacgttacat tacagcaaac agcttagcta atttttatcc agatgggccc 9660
   cggttacagg attgtacact attgcgaatt tcttacagga aagtgaacat caagtaatta 9720
   ttccaaatag agttctctta agaacgtgag ttacttaaaa atgtctaagg atgaagtcac 9780
   ttctgaatat aacttcactc aagagaacaa ataagcaaac tgcatttagc ataacatggt 9840
   aaattagctt taactctcct tgatgtttga acatttgtcg ctgttaacta ctgtttcact 9900
   tttcaaatag tcagggctta gtttgcttct gtaaggataa agggaaaata cgccttcact 9960
   gagtcataaa tatttttgtg gctaactttt gcacagagaa aagaggcctc taagaaggta 10020
   cccagtgaat ttttttttcg gggcagggag agaatatgtc attttttggt ttgttgttgt 10080
   tgttgtcatt gttttgcttt gttgttttta ctctgaactg aactgtatct tgacagcact 10140
   tttgaattaa gagcattact cttattgttc tctactacct ggacgccacc tccctgttgc 10200
   catagtgtta aggatcatgc tccgaggtgg ggtgaggcag aatggggcca agatcagaaa 10260
   gttacattaa gctacatcag gtttatacaa gcataaaacc aaatttttgg agcagtcccc 10320
   agaatacaac ctggtttagc cacacctaaa ggttgctctt gaatattcct tgagaatcca 10380
   catccctaga atgctgggtt tcaatgggcc ctttatgtac ctatcatggt gtcatttctg 10440
   agcatttcta aatattcctt catgtcttac tgacagtttt tcttgaataa atcttaggaa 10500
   tattagtgcc attatcagta ttttgtttgg tctgttcaca ccacaaataa ctacccaggt 10560
   ctgctacttg cccctatttc tctacctgct aatgaaaatg cttttgaaag tttgagtaac 10620
   agtattggag tgtgcacagt ggtattggta ggttctgtac tcatccttaa ccacttgttt 10680
   tcatcctttg tgagcttgaa gtttctccaa aaaatttatc acaaaactta tcagacatag 10740
   ttaatacact cagagagaga atcactgaaa aagtagatgt agtttaacaa acccagtgcc 10800
   ttttttttac ccatgaatac atatttgtca actaaacctc attttgcaac ttgttccact 10860
   actcgaatgg taacaaactt ttggtttccc aatagatttg gaagatgttg cttttgaaag 10920
   taggaaatag atggctttag aagatggaag aatattttgt ttgaagtggg agcgtggtat 10980
   gtccttagct gtctgtgaaa tgcagctgaa gatgggtgtg ggccttcatc tgcatttccc 11040
   atcttcagtt tgaggaggta gttacccttc taaccactta agaactgcat ggtacatgct 11100
   gttttattta cagggcaaaa ctgtgctccc gtagtttccc tggtgcttgc cttcacgtta 11160
   acacagtgtc atcgtttggc agtgtttatg tgccagggtc catgttagaa ggaggaaagg 11220
   tatagcgaag ttaaagggtg cagttggcct cccaccttta gttttgtaag tgcctttaaa 11280
   gtttgatttt tgtaggttga tcataaggaa gtgataagta tgttaggtta tttgtggttt 11340
   gagctaattt tagtctcttt ttacagcttg ctttgtatcc tttgccatta aaacatgctt 11400
   tctagaaaga caacttttga atgtaggaca cagtctatat tctatacttg gctacatttc 11460
   aaaaaatatt ttctcagtac tttggaagtt ggacagttgg aagcatagtg acagtattta 11520
   aaaatctttg attccggccg ggcatggtgg ctcacgcctg taatcccagc actttgggag 11580
   gccgaggtgg gtggatcact tgaggtccgg agttcaggac cagcctgacc aacatggtga 11640
   aaccctgtct ctactaaaaa tacaaaatta gccgagcgtg gtggtacatg cctgtaatcc 11700
   cagctactca ggaggctgag gcaggagaat cgcttgaatc tgggaggcgg aggttgcatt 11760
   gagccgagat cataccattg cactacagcc tgggggacaa gagtgaaact ctgtctcaaa 11820
   aaaaaaaaaa aaattaagtg atttctttgc tttgtgacac ttctactttt ccagcaagta 11880
   aattatattc tttcatacag gtatgaaatt cttgttccaa gctagtggtt aaaaaggcac 11940
   agttgatatt agaggatttg taaaagatta tgaccacgcc tgcaatgtac tgaagcaagg 12000
   ctttgctggg ctgtgtatag gaaaccttcc ccagcctgtg cccttgcttg atagaacatt 12060
   ttgctcctaa gggtaggtgc ctgtatctgt ctccagtact ggttagtttc acacagaaca 12120
   gttgtgtttc agagctttag tctcaagctg ccctgctccc ctgaagcagc caccctgagc 12180
   atgtgcactc acaggagggg acatgtgagg tcatggaaga agacgactca ggaagaagaa 12240
   gacttgggtt tgggttctga ctctgccttt gactgttgtg ggattttgag gagttgcata 12300
   caggatctgt aaaatgtagt cattagacta gactagacag ccatatagca ttacctagat 12360
   gtaactttct acaaagacat ggtcacagga gaagaccaga gggtggggtg atctttctgg 12420
   aaaaattggg gcttcatgcc ttactcatgc tagatatggt agcattatat ggctgtgcct 12480
   gatcccccta atctaaaagt gggacagaac tttaaaattt catattaact caaattaaaa 12540
   cttgaaaaaa acccattatt tccttaaaaa taataaaatg ccctgtgggg gcataagtca 12600
   cattatattt taaaattcct gaatgccaca tggatgaatg tagttccttt tgaaattctt 12660
   cttttgtcta aagaggaatg ttggattttg taattggact aaaaaatctt ccatttgaga 12720
   gagaaacagt ctgctgcatg ttctaccctt gttcaggata aaacccacta atagctaaca 12780
   tttattgaat tctgtgttgt gcctcaggca ctgtgcaaag tcctttacat gcaatgctgt 12840
   ttattatata ctgtcaattg gtctataaca gcaggaaatg tttcaggagg acaatgaggt 12900
   cccagaccct cagtcttctc ctgtgtcctg gattcagctt cacaatagca ctatggcagt 12960
   gtggccactg cttcagcttc cacatacatg gctgtgaaga gagacagggg attgtgctaa 13020
   gcctccccga tttattagga cataggagga gagagtttgt agtttttgac ctttgcctag 13080
   ttttctaacc tctttcctag atgtcacaaa ttggccaccc acagtcatat tttgcttgct 13140
   tcacgcaatg ctttttaaaa aagagaagag tttaatttgt gccattgttt ataaatgaat 13200
   caggagaaat gacatgcaac tctggattct ggcctctctt gaaaaatctg aaaatcacac 13260
   cgtctgagct tacactggca gtggtctgct ggactgaggg acacaactcc ttttggatgt 13320
   acatgtgtgc gttgcagagt ttaccacagt cccacagtgg gtcacactgt ccttgtcggt 13380
   gtacactacc tagcacttga gtttgcaacc cctaccccaa gctgagtttt ctcgtcaagc 13440
   ttgatgttaa tgttatgtga tgcttggcct tgtaggtatt tggtatatta tcgttagata 13500
   aaattgaagc aaagggctaa agggttggtg gcctgaggga gtgcccttga cagtaaagtc 13560
   taggataaaa tcattggcca ggtactcctt cccttcccgc ccttcctctt ttctctttat 13620
   cctcagcctc cttctgctat tttgaggaag ttagaagcca ccaccatttt ttcccacctc 13680
   aggcaactga gtgtggctgt atttctgtcc catgttcagt tatttccagg aactattttt 13740
   gatgaccaac ttgaagttac attgggtggg cctaatgggg gctgataaaa gaatgaggtg 13800
   accaaatatg cttgcactga gacggctacg aagtaaggtt tttaatgact tgctttgtga 13860
   cttggtcagg agtgatacca tttgtcatgt gtccaacttc atgactaaat ggttgctcta 13920
   ccttatcctc atagctataa taaaataaaa taaatacata cattgcaggg aggaatgtat 13980
   cttgttaaag gtctctccct tttagcaaca aaagtacata ttatgttgta gaacatgctt 14040
   tttctttgat ccttcttgaa cacctattac tctatagagg tatgttgtgt atggcaaatt 14100
   agaacaagca atagataagg atgattcttt accattataa cccagtcaag gtctttgtcc 14160
   taagttttgt acctttctcc agagggaaag gtatttgtat ttatttattt atttttgagg 14220
   cagagttttg ctcttgttgc ccaggctggg gtgcaatggc acgatctcag ctcactgtaa 14280
   catccgcctc ccgagttcaa gtgattctcc tgcctcagcc tcccgagtag ctgggattac 14340
   aggtgcctgc cacgatgccc ggctaatttt tttttttttt tttgtatttt tagtagagat 14400
   ggggtttcat catgttggcc aggctggtct tgaactcctg acctcaggtg atccatccac 14460
   ctcggcctcc caaagtgttg ggattacagg catcagccac tgcctccggc caggtatttg 14520
   tatttttagt ctctatgcct taccgtctca gatcaggagg atttggtgat ttatcgaatg 14580
   tgggggaagg ggaagaagag gaaacgggag gaatgttcca gattagggaa atagctagat 14640
   ggaagatgca gcccctcatc aaggtgggga cacaggaaaa ggaacgtgtg caaagaagat 14700
   ggtgatctgg ttgtgaccat gttgttagag gacgtccagg gaagcatctg gtaggtggtg 14760
   gggtgtttaa atatagaaca ttcggagaat gctccgaagc ttcagagaac ccttcccaaa 14820
   aggacaaaac cagctcagtg ttttagcact ccgggatcat atggcatgac agcatggctg 14880
   ctttatactt ttttgtgtat gtgaaattaa aaccaaccac tcaggaccaa tttctctgaa 14940
   gctttttgtc aatctttcat ttgcttttct cgtctagatt gtaagctcct tgcagccagt 15000
   gtctgttgat tcagtcattc aaaaaataat acatgaacag ctactaggta ccaggctctg 15060
   tgctgggcag ttgggatatg tggtgaggaa gacaaacttg gtccctgccc ttaggaagtt 15120
   cagtagtcca gcagacaaag tggctgaata aagataatct cagttcacag tgataagagc 15180
   tcttacaggc ctaggctcca ggtgctgtgg ggatgctcag gaaaaggtat ctaattggga 15240
   ttgggagcag gcaaaacaaa taaaggatag tgtataaagg taatatctag ttgaagttct 15300
   gaagggcaag gaggagtgag cctgtatatt ctctgagtct ctccctaatc tgggattgac 15360
   ttcttgtccg tctctgttca tattaagtgt cacctaggct tgaaagggtg agatcatatt 15420
   tcacttcctt cctctttggt cttaaccttt ctctgctacc ccctcacaca atgcatatgc 15480
   attattctct tattgtatat atttttcctc tcttcctttt catgtttcct ctgccattac 15540
   ttttaacctc gactgccata tggcctctaa acgcttccag aagggtagcc tagtggaggt 15600
   tattccatca tggccttgag ctcatgcgac cagatagtga aggcatctgt gtaggtgtct 15660
   tctccaggag ggtgatattt gtttcattgt aaattttgta gccctagaac accaacaaca 15720
   gtgcacagta attagtaggc aggcagtaca ggattcattg aagtgaagtg ataactttta 15780
   tccaagtatg tatgcagata atctttgatt tgtacaaaaa aaattatatt ttaatatgta 15840
   aagatttttt aaaagaatct tcaagtttta gccttcccac taggaatata ttgaaaacat 15900
   gtgcctagtt cactgacttg cagctgccac tatgagaata aaggtctcat ttagttgttg 15960
   tgaattttaa gggatatttt caatgatgtt ggctggttta tcccattatg tggtcttttt 16020
   tttttttttt tttttttttt gaggtggagt ctcgctctgt cacccaggct ggagtgcagt 16080
   ggcgcaatct cgactcactg caacctccgc ctcccgggtt caagcgattc tgctgtctca 16140
   gcctcctaag tagctgggat tacaggcgcc tgccactacg cccagctaat ttttggtatt 16200
   tttggtagag aagggtttca ccatgttggt caggctggtc tcgaactcct gacctcatga 16260
   tccactcact tcagcctccc aaagtgctgg gattacaggc gtgagccacc atgcccagcc 16320
   tatgtgctct tattagcaat tctcagtaca cagatagctt tgagtgattc tttcaagtca 16380
   agtaccttat taaaaaactc aagtgtactg ataattatct tacttttaaa tggctaagtg 16440
   ataagactga atttttaggt actgtaacac ttcagattac agattctgat atttttatgg 16500
   ttatttatat ttatttattt ttgagatgga gttttgctct tgctgcctag gctggagtgc 16560
   aatggcacga tctcggctca ctgcaacctc cgcctcccag gttcaagcga ttctcctgcc 16620
   tcagcctcct gagtagctgg gattacagtc acccgccact acagccggct aatttttgtt 16680
   atttttaata gagacaatgt ttcaccatgt tggccagggt ggtctcgcac ttctgacctc 16740
   tggcgatccg cccgcctcgg cctcccaaag tgctgggatt acaggcgtga gccaccgcac 16800
   ctggcctggt tacttaaatt taaatacaaa aattatgttg attaattctg aatgatttcc 16860
   tgattgctcc ccgtttacca ttcacacatt tattaaattc ttcgcttgcc atatagaagc 16920
   agtctctctg ccatatatgc catatagata acagaactag ctgtctgcaa accactgaaa 16980
   ttgtgaaaac atctcccctt ttttcctgtt tctaattcta gctatgagga ttatatacag 17040
   aagtagtcct ggatttgatt tttttttttt tttgatgatt gttttttgat agttgttgac 17100
   tacaaatcat ttaaacgtct gaaaggggaa aggttttcct taaaaatgga tgacaaagga 17160
   gaataaaaag gtattttgac tatttttttg aatgatgagt tttttttttc tctttcttgt 17220
   tttcttttgg agtcatttat gtgtcactga gtggatacca tggaacatgt ggcagaagta 17280
   gatatatggg gtaaaagaac catagttcat aagctccttg acagaatcac tgaagtgtag 17340
   ccgttatatg gccactgtcg cagggggagg cagcagtttt gaagaagggg atgagtaata 17400
   atgagtgata aaaaggcatc ctggatagaa gaccaaactc tgcagaagac cccagtttga 17460
   ttatgctttt gttttctgat ttgcggagga gagtgaaaat gcctgagggg tgcgggggag 17520
   cacatagggt gtatgtgtgt gtgtgtgcgc gtgcagattc tctctttcac tgtatgtatt 17580
   tgtatgcatg tatgtatctt aggacttaag ctttctagtc aataaattgc catagtgggg 17640
   aattgcttaa ttgcttgcct tctgttgttg tatttaattt aatttatttt ttaatgattt 17700
   ttttggtggg gtacagggtc ttaactatgt tgtccaggct ggtcttgaac tcctaaactc 17760
   aagtgatcct cccgcctcgg gctcccaaaa tgctgggatt acaggtgtga gccaccatgc 17820
   ccagcttagt tgtattttaa atgggcctgt ttgcagcatt ccctactccc cttagtttac 17880
   ctggctcaca acctgtcttt ccatatcaag gcttctgtca cccctggccc atgtcagtgc 17940
   atttgggcag cccacccagc atcatcacct catgtcccag ggaacttcct gttcctctct 18000
   tccagctatt tccttccctg gcagttgaga tagtctctac ctttgaccta ctgttaagct 18060
   cagaccttct gctctctagt tacagcctct gtgctgccag attccctcgc tcagttgctt 18120
   tctctagttt gggttttctc ctttattcag atttccagct gtttctctcc tccccccacc 18180
   gcagcctcct cacttccctc cttatgcatc tgagactgtg gtcagtcact ttagatgctg 18240
   cctctccact gtacttgtgt ccatcttctt acctaccacc tctagccctg gagcaggctc 18300
   ttcccctgtc tttgtcttcc tgggcccagg ctcctaagcg ctgctggaaa aaaaatcccc 18360
   cagtattgag cccctagaaa tccagtcttt aatcccaaat ctgtctcccc cagcatctgg 18420
   ccatcagatc taaagcttac ctgccatcct ttccacctca tttctctcac aggggaaaag 18480
   gagcctttgc tcctagagtc tgcgctcctg accccttccc atctcacctg ttcaaggcat 18540
   cttgcaataa ggggttggtg actctcgagg aatggatccc aggccctccc tattatcatc 18600
   ttatgtatgc cagttcaacg ttctcagctt cctccagccg agacggcccc tccagccact 18660
   gctttatact ctccttctct ggttgaaatt tttgaagtaa ataggtcact ctgcccatcg 18720
   ttcatcttcc agtcactctg tgtgtttatc ttccagggaa gtgaggctct atgctaccaa 18780
   gccactgaaa taattttttt ttttttccag actgagtctt gctctgtcac ccaggctgga 18840
   gtgcagtgcc gcagtcttgg ctcactgcaa cctctgcctc ccggcttcag gcgattctcc 18900
   tgccccagcc tcctgagtag ctgggattac aggtgcctgt catcacgcct ggctaatttt 18960
   ttgtattttt ggtagagatg gggcttcacc atgttggcca ggcttgttgg catgttgacc 19020
   atgttggcca ggctagcctc aagtgatcca cccgtcagcc tcccaaagtg ctgagattac 19080
   aggtgtgagc caccgcacct ggcctgaaat aattcttgac aagatctgct tccttgttac 19140
   taatacagtg gatattttgc atcctaattt taatgcagtt cagtgtggta gacctgtatt 19200
   tgcatattga atattccctt ccctgtttta ataactctat tttttccttt tcttttatat 19260
   ctcctgcttc tctagctagt cctagacctt actcatcggt gtcttctctg tttgttcctc 19320
   aacttgagga gttcctacag ggtttaccca atctgctgct ttcatttagc ccttttgttc 19380
   tttttgagcc atctcattca ctcacccagg atgtagcatc ggcccttgaa ttcagtgtgc 19440
   acacatacac tgtgcactat gggacagcct tcagaggcac tttgttcctg aaattgtggt 19500
   ggtctttgcc tctcatggag ccttgcatat gctgtttcct ctgcctggaa tatcctacct 19560
   tttacttaac tgattctcgt tcttctttcc agtcacattt tgtacatttc ttctgggaag 19620
   ctttctctga tttccccttt ccacaggtcc aagttaactg ccttgtctag gtcctcccat 19680
   ggccctctga aggcctcctt tcatagcacc atgtctgagt atactgtaat aacacgcatt 19740
   gctctgtaat agcctgttta cttacctatt gccaagtaat ctatcaagtc ttataaaggg 19800
   cggggctgct tttgttctag tcatttgtat ctcttagtac ccaatatagt gtttggcata 19860
   tagaaaatac ccaacaaggc cagtcgcagt ggctcatacc tgtaatccga gcactttggt 19920
   aggctgaggt gggcggatca cttgaggtca ggagtttgag accagcctgg ccaacatggt 19980
   gaaaccctgt ctctactaaa aatacaaaaa ttagccaggc gtggtggcgg gtgcctgtag 20040
   tcccagctac ttgggaggct gaggcaggag aatcacttga actggggagg tggaggttgc 20100
   agtgagctga gatcactcca ctgcactcca gcctgggtga cagagtgaga ctccatctta 20160
   aaaaaaaaaa agactccatc ttaaaaaaaa aaaaaaagaa aaaagaaaga aaatacccaa 20220
   taagtagttc ctgaatgaat agatgagaat gctgtttaga aggttcatga attggaaacc 20280
   gtgattgcta gggaggcttt gagttgatgg tattgtgttg aaccatgtgt tacccaggat 20340
   caatttagat tttacacttt gttttctctg ttccttttta tagtaatttt ctgtatgtgg 20400
   tgttttcccc ccatgagatt gtataccatt tctcagcgag aactgtgtgt aatgcttggt 20460
   ggctccctca tggtgccttg catggaattg gacttcgttt cagtggatct gatcccagtt 20520
   atgttaatgc tcgatggagc taagtcttat ctcgaagcag tccatgtctt catcagctgg 20580
   ccctgcctcc atgccctgca cagaccatgc cactctggag aggtagtttc cctgtggctt 20640
   attagtctta tgttccagtg tgctggccaa gtatgagaga catcagtggt atgagagagt 20700
   ctctctcatt caaacttcgt aggttttgta gctgggactg accagtgctg acaggaaata 20760
   gaggcattta ttaaaagcca gagatttttc aagttgcagg aagcaaagct cttgttagct 20820
   atgattttgt ggtgggtttg gtagtccaat ataaaagtaa aaactggatg acaatgggag 20880
   gagcatgctt gggtctccaa agttagatca tttttcctaa gtaatttgtc tttaaacttt 20940
   tactggtttg gaatttcctg agattttgat cttgccagaa agtttatagc aaaagttctg 21000
   agcagatgac acttttgcgt ctgaaaccaa atcattgttt ttgtttttaa cttttttctt 21060
   aatatattat ccttagttca gccctgaaga ttattctgtt atttgtggat ctcaactttc 21120
   cccccatctc ctggatcttt gtgaaatgaa tggtattaat tgaatagaga aggaagatat 21180
   aaacataaac ttagtcaaaa acttgttctt gactaggcaa gttgggcttt atagctttga 21240
   gctgatgaca tgtctattct tgtgaaaaag ggatttttag tgttggtttg gcttcttgtt 21300
   atatttgatt tattattatt atcattatca ttatttttga gacagagtct tgctctgtcg 21360
   cccaggctgg agtgcagtgg ctcaatctcg gctcagtgca acctccgcct cccaggttca 21420
   agcgattctc gtgcctcagc ctctggagta gctgggatta caggcgggtg ccactacacc 21480
   tggctaatat ttgtattttt agtagagaca ggtttcacca tgttggctag gctggtcttg 21540
   aactcctgac ctcaggtgat ccacctgcct tggcctccca aagtgctggg attacaggcc 21600
   ttagccactg tgcctggctg attttttttt tttttttttt tttaggtttg ttttaactgg 21660
   aactttacgt gaatgtaatt gaatttagaa taaaagcact taatttcaca gtgtgcagtg 21720
   aactttctgt tacttatttt aacagtaaaa ccccttgcag taaatgactt ggagcaaaga 21780
   ttgctttttt aaaaaatgtt ttaatttgtt tttcttttct tgagatggag tcttgctctg 21840
   tcaccaggct ggagtatggt ggcgcgatct tggctcactg cagcctcccc gcctcctagg 21900
   ttcaagcgaa tctcctgcct cagcctcctg agtagctggg actacaggca catgccacca 21960
   tgcccagcta atttttgtat ttttagtaga gacagggttt caccatgttg gtcaggatgg 22020
   tcttgatctc ttgaccccgt gatccaccct cctcggcctc ccaaagtgct gggattacaa 22080
   ctgctgggat tacaagtgct gggattacaa gcgtgagcca ccacgcctgg ccaatttttt 22140
   tttttttttt ctttttgaga cagagtttca ctctgtcacc caggctggag tgcagtgtca 22200
   cagtcaaaac tcactggcag ccttaacctc ctgggctcga atgatcctcc tgcctcagcc 22260
   tcccaagtaa ctgagactac aggcatgtac cactgtgccc agctaattgt ttttttattt 22320
   tttatttttt gtagggacag ggtctcgcta ttttgcccag gctagtctac aactcttggg 22380
   ctcaagcagt cctcctgcct tgacctccca aaatgttggg attacaggga caagccactg 22440
   cacctggcca aggattgttt tttaagtgaa ctgagaccca gccttattag tggtcccaga 22500
   gcagacctgg gacctgaagg gaaccctttt cttctggtcc agcgtctttc ctctgatggg 22560
   ctactttcct ggagcctttg attgcctgtc atcagagtaa ctgagtttga acagagtagg 22620
   tagttcctct ccagaccacc acactcacca gctttcattc tgcttctctc gtttagactg 22680
   tggttctgaa tcctcagttc tatttactga gtgtttttaa acataaaaat gccttttaat 22740
   gagattgaag gccagaggtg ggacagttga ggacaaagta gaaataaaac cttcaaggcg 22800
   gggttgttgg tgggagtctt tttttgtttg tttgtttttt gagactgagt ctcgctctgt 22860
   cacccaggct ggagtgcagt ggcacaatct cagctcactg caacctccgc ctcccgagtt 22920
   caagctattc tcctgcctca gcctccttag tagctgggat ttcaggctcc cgccaccatg 22980
   cccagctaat ttttgtattt ttggtagaaa cggggtttca ccatgttggc caggctggtc 23040
   tcaaactcct gacctcaggt gatctgtctg cctcagcctc ccaaagtgct gggattacag 23100
   gcgtgagcca ctgtgcctgg cagggagtct tatagaagct gtcgtggaca atgtgggaag 23160
   tagtgagcct ttgtattcca gtatgctggg ctccactgtg cttgctctgg cccccggtcg 23220
   ctctctgtgt gttattgagt ccccatccac ggccatactc ttcgtcctgc ttctctcctt 23280
   accatcctct ccccgctagt ggtaccacgg ctaccactag caattactga catgtgggat 23340
   cttagggcta cttccctata aggctgcagg gcatgtggtg ttggctacgc gcatggtaac 23400
   catggtagcc ctgtggttct ccacatgtgc gccttgtgac ctgggattgg ctgcagacta 23460
   gtaataaact gcgtcttctg gtatggaatc tgtctgtagt tgtactttct acctctgtat 23520
   ttaaggggag atctgtaacc taccaatgcc agttgaagag gatggatgat agagatgtta 23580
   acaaacagct gaaaaactaa ctacaatggc ctgcaaaata gaacagcagg tttttgtggc 23640
   aaaactttgt gtccatgagt ttgtttttta aatatcctca tataatctgt tttaaatcga 23700
   gaggctttgg gtaaaagcca tggctagtct tacatgtcat ggagtaccta gcttgtgagg 23760
   ttcacagttt attatttaca gagtgtcccc ttaaatcttc tttgggtcgg ttcagcgaat 23820
   gttgctcaga tggacttttt tggctgacat agagtcaaaa tggtaatcaa gcatgaaagt 23880
   acagacagtc cttaacgcac aaatgtgtca tgcttgaaaa gttggaaagt tggttctctg 23940
   gagctctgat tgtattgtcc tgtagaatcc gtgttgtgaa tggtggttaa atcccaaatg 24000
   agtccgtaga acctatataa tctgcaatat acctgcagta ttccaattaa tatgtaattc 24060
   ccccatagaa ctatgttaat gatttgtatg tatggtattt aatattatac ataataatga 24120
   ttgtatgaat aaaaaacatt ctgggctcca tgtggatgat ggggtgtgtg tgtgtgtctg 24180
   tctatgtgtg ggtgggtgtg tgttcataga tcccttttcc tgcaatcctg gcactggaat 24240
   tggttttatc atttccaatt aagtttcatt cccatgaatt ttggagtaca gactgggtcc 24300
   aggtatgcag ggcatagatt agagccctga gaaataggat taggctggaa ttgctgggtt 24360
   ggagatcagt agcttccagg aacacttttt gggcctggct gtcttcatta tccccttttg 24420
   ttttctcctg gggtctgcag gtattgccct gttttgttcc tctaatatca cttttttttt 24480
   ttttctgctt ttgaccaggg tttttgcctc tggtctacaa ctgaatatcc tatcagactc 24540
   tcctgatttt gaaataaata tatagttttt ttgaggtgtt ctagcgaatt tctaaatcta 24600
   aatgttgtgg cagagttatt acatactaat tttgctatga gaggttgtag aatcccagat 24660
   gactaatctt gtaaaccata cacgcatttc catctaattc tccattgtat atcatgttgc 24720
   agaaaataac agcctctaga gtttacattg cctcctttga ctatatttct tatttaagat 24780
   tagttttcag ataagacctt ttcatggcag tacataactg tacagagggc ttccaacttg 24840
   tcttgggagc tctcatctct gggagacatc acattaccca ctgccccctg ccccccgccc 24900
   ccagcctgga tgcactcagc ctgtacccca tttctgtcct cagccaaaca ctgctgaaat 24960
   gcaagagctt tcaattgcta gccagtgaag atgcagacta agggatttcc atgtagaagc 25020
   ccgctctttt cagctggctc gtcgagagct ggaggcccct tgcttgttca catgaggctt 25080
   tttgtccctg acttggtggc tgctgtttca cttctcagca gaaagggaca cccttgcccc 25140
   cccccagaaa ggaagatttg atgtaccact tccgaaaggt tcagtcgggc atcactgtaa 25200
   ccaagaagat aggtcaggtg aggctggagg tggaacaggg ctgctcgcta gaactccaga 25260
   ttgttccaca agtgccttct ggcagagaat gatggaagct tccgtgattt ttttttctcc 25320
   ttaatagtta tgagcacaga agaggagcag attgtctggc tatagaagct gtcttatttt 25380
   ttatttttgt ttttgagatg gagtctttct ctcttgccca ggctaaagtg caatggcgcg 25440
   atctcggctc actgcaacct ccgcctcccg agttcaagcg attctcctgc ctcagcctcc 25500
   tgagtagctg ggaattacag gcatgcgcca ccatgccaga ctgatttttg tattagagac 25560
   agggtttcac catgttggtc agtctggttt cgaactcctg acctcaagat ctgcccacct 25620
   cagcctccca aagtgttggg attacaggtg ttagccactg cacccggccg aagctgtcat 25680
   attaaatagc actttctgct tttagcaaat ttaatccaaa tgagacttta gattttcttg 25740
   ctctgactta ccagcagttc cttgaaacac atttaattat ttttgccaga aaatcactca 25800
   agcacttacg ccattttttt accgtgaaaa tatgctgcat tattttaaaa tatattagaa 25860
   gtcagtaacc ataagatttt atatgttttc taatgtattc tgtaagcttt ctgctgcttt 25920
   tgtttggaag gtgtattttg taacgtagag gactgcttta tctgcttgta agcttgattt 25980
   ttgtttttac tgtaattttt ttttcttttg ctgtattgag aaatacattg agtaattata 26040
   aagtcagtgg catgtttata agttaatatt tgtatctatt ccttagttac tctaactcaa 26100
   aacctaaagt aatcttcaac tctaatttac tctgacatcc agttgactgc caagtcctcc 26160
   aacttaatcc ttatcctttt ttttttaaag agatgcagtc ttgctttgtc acccaggctg 26220
   gagtgcagtg gtgcaatcat agcttactgt aacctcaaat tcctgggctc aaatgatcct 26280
   cccacgtcag cctctggagt agctggggct acaggctctt gctaccatgc ccagctaact 26340
   ttttattttt attttttata gagacagagt ctcactgttg ctcaggctgg ccttgaactc 26400
   ctgccttcag gcggaactcc tgccttcagg cggtcctcct gcattggcct cccaaagtgc 26460
   tggaattaca ggcccaattt tattcttggg atgtatgtct gaaactcttt ccttcacttc 26520
   cttcccaagc cttagttcag gcccttctca tctgtggtct tcaaagtcgc cttcagctgg 26580
   ttcaggtcct tcctttctgc tgtatctttc atgggaggac atgttatgta tcactgtcct 26640
   acttgaaaac ttccattccc cattgatgag ggtgttacct ccagattcct aacacaggtg 26700
   ctgaaggcat gcctggataa aggcactccc ttgatctcct ggccaggtcc ccgtacacct 26760
   gcagcgcatg ctccacattc tgtctttact gatgctgtgt cttctgcctg cggagccacc 26820
   caccattcta ttcacagccc ctgcctcagc ggagcacgtg cctccctctt cctacactga 26880
   gctgtccttt ctattgaatc ccctcttttt tgtagtatgg gaaatatttt attatgaata 26940
   ctcttttctc tgttgcctcc gtgaccacgt taactttgcc ctaattcgcc ttaggactcc 27000
   atctgcttag gggaaagtta ggatttggtt acagaaagca agctgctaga aagaacagtg 27060
   tttagcttct gacaggcaaa ataggatttt gcaacatgct tttccttttt aatgcttaga 27120
   cattttatat gaattaatat ttttatttgg ttgcttatac attactttct ttttagctag 27180
   aatgtgaacc ctataggaac atggggattg cctttcacat ctttgtatcc tcagtaccta 27240
   atgttcagtc accctgtggt cttgtgtcgt atatacattt agccttcctt aattaaacca 27300
   tatgtactgg tccccgtccc ccacccccaa atagagagaa agaaattcct tgaatactac 27360
   attgccagta tcaaaccaca ccttgatatc ctctggggaa agggaggtat cagttgaaaa 27420
   gagaaaagag gttaaaatct aggcattaaa atgtgtaagg cttagatgct ggcaatttaa 27480
   ggtatgtttt cctgaggtta attttgattg tgtgcaaatt ttacctcata tctaactgta 27540
   ggatttagtc accacataag atgggatacc tccataaatc cttcagaaat gtttgtgaaa 27600
   ttaaataaag ccttattgaa gactcagctc ttgagagtca tctacctacc taacagttat 27660
   tcttgaacag aagagtctta cttttcccta taaggcagtg tgatagccat ctgtatattc 27720
   atataattta tgttggcgct tacttcattt aaaaatgtat tccgtgaatg cagttgccag 27780
   gcggtgtgct gatcagaaac gtgtaccaat ggcctctttt ataattataa gaggaagacc 27840
   aacctgaaac agtcacacaa atgattaatt ttaattgtgg aggagtgctg ggaaagaaaa 27900
   ataaaagatg caatgcaagt gtttacaaag gagctttgag cttgtttgaa gtggtccttg 27960
   ggcacttaag caaggcttaa agaatgatgt gattagaagt ggcttagcaa ttctaaagaa 28020
   cacagggaag gcgtgtggcc agaacattgg tccctagagc acatcgcctc ctgacatacc 28080
   atttccttaa gttaatgttt taccactata cataggccct cccctttgtt tacccagatt 28140
   tttttaattt taaggatgtt tttaataact tagaatcctg taatttgttg aacagtcctg 28200
   tattcccttt acttatattc cttgagattt tataaaatat tttttacatg tcccaagtct 28260
   tgattatatc tttttacctc ttgttaagaa atacttactt ttctattttt atgctatatt 28320
   tcatgtttac tgtagaaaac aaaaaaagta aaatttttct ttattcctat cactgcagct 28380
   tataagcact ctaaacattt tgatctatat tttgccaatc atatatttta gttaaaattg 28440
   ttgttgacat aattgtagat tcctgtgcag ttgaaagaaa taatacagag ctgagcgcgg 28500
   tggctcacgc ctgtaatccc agcactttgg gaggccgagg caggcagatc atgaggtcag 28560
   gagtttgaga ccagactggc caacatggcg aaaccctgtc tctactaaaa atacaaaaat 28620
   tagctgggtg tggtggcggg cacctgtaat cccagctagt tgggaggctg aggcaggaga 28680
   atcgtttgaa ctccggaggc agaggttgca gtgagccgag atggtaccat tccactccag 28740
   cctgggcaac aagagcaaga ctgcatctca aaaataataa taataataat aaataaactt 28800
   taaaaataaa acagagagat cccatgtgcg ctttgcctag ttcccccatc cactgcccat 28860
   aacattttgc agaactgcag tacagtatca caaccacaat actgacattg atacagtctg 28920
   ctcatcttat tcatatttcc ccagtgttac tcgtatccac gtgtgtatgc attgtgtttt 28980
   caatactctt ttattataaa gctgttttta atgtgattca attctaggtt gttttgttct 29040
   gccctcaaaa agcattccct ctcctaatca tatctccgtc atacccttgt atgttttctt 29100
   taaacctgtt ttaagaaagc agctacctgt aagagaaatg agattgaaaa cagaattgcc 29160
   aatctgcttg tactttataa gcctgttgat tgtttagata cggtttagcc agtttatagt 29220
   taccctgggt gctgaaaggt atgctggatg atacctaacc aacagagaac cattgaatgc 29280
   cgttcaaaat ggactgaagc atcagcaatg tctgaaaaag gcctgacagt aatgtacatg 29340
   tcaaatggcc cgtaatttaa gcagagtaga gtaagtagaa gaataaacat ggggaaagtt 29400
   ccagcaacag aggaggcttt gagcttttgc tcttcatctt gagtggatgt tgttctcagg 29460
   tggtaatagg ccatcgagct ttctccactg gctgcctctc tggggaacaa ataaccgaaa 29520
   agatactcag caccctggtt ggtacatagg tggtcagttg atttatactt cctggttttc 29580
   agtgttgctt gaattttcta aatggaaaca cagtaccttt ataatcagaa aacaatcccg 29640
   agttttgatt tgagggtgtt gtaaaaagtt aaaaaaaaaa aaacagaaat gtgaaaagga 29700
   agttgtgtta gagtatttgg agttgagaaa gcatgaaaag gacagaagag aagctggttg 29760
   tcaggttgca tggggtagct acaagcacac tgaccagaaa gtcagctgga aaaaaaatgt 29820
   agaaacagga gataaaacgg ccaaggggct atacaagcaa acagcaagga cctgagaaga 29880
   aaaactagtt aggtgtgact gtcagagtga tgtgtacagt gtgatccttt ctgtgtaaaa 29940
   acaagcagta agaattcgct gtttacgttt gcgtgtgttt ggagaagagt ggggaagagt 30000
   aggcactgcc agactgtgaa cactggttag gttattgtta tatctttgta ttatatacac 30060
   tggacatgtt atttgtataa tatgagaaga aattttataa atcattaaat cttttggcat 30120
   ttaggaacat ttgtgttttc taatagttgc ttctatacta ttatctttat tatatgccct 30180
   tcatcttctc agtgtttggc tgttgttgtg attccctttt gtgagcagtg ttgaagttag 30240
   ctaatattca tttcttctcc cttctttcac cctcctccag agtctgattt gaagtattcc 30300
   tagctgctac ctataaaagc aataagcaag attgttttac ttttcacaaa ctcgtcctgt 30360
   tctgtgcctc tgcctcggac atagctgtag tatagagtgt tgtctccctt acatccttct 30420
   atcttagacc tactagtaaa tattaatgct cactctaagt tcttctcaat tctttttttt 30480
   tttttttttt ttttttttga gaaagagttt cgctcttgtt gcccaggctg gagtgcaacg 30540
   gcacgatttc ggctcaccgc aacctccacc ttctgggttt aagcgactct cctgcctcag 30600
   cctcctgagt agctgggatt acagtcacgt gccaccaccc ctggcaaatt ttgtattttt 30660
   agtagagaca aggtttcttc catgttggcc aggctggtct caaactcccg acctcaggtg 30720
   atccacctgc ctcagccttc caaagtgctg ggattccagg cgtgagccac cgcgcccagc 30780
   ctcttctctc aattcttcct gaagctcttt ctgcactaga ttcctcagga agggcttgtg 30840
   ggaacaatct tctgtgaatc aacagtacat attcataata gtttgtcagc agcctattat 30900
   tttaaggcca tttggtctgt atataaaaat gtttggatca cattttcttt ctttaaggta 30960
   aatatgttat tctgttgtct tctggtataa agcattgctg taaatgtttg acagtctaat 31020
   tatcttttgc ttataagtga cttagggttt tttgtctatg tgcccaaagg attttttccc 31080
   tctttctctc tttttttttt tttttttttt tttttaaaca gacaggatct caccctgttg 31140
   cccaggcttt agtgcagtga ggcagtcaga gcttactgaa gttttgaact cctgggcttg 31200
   aggaacaaag gattttttta accttttaat tcaaagtctc atcatttatg caaccatgtc 31260
   ttggtgttgg ctgttttggg ttgttctccc tcaaaaatcc atgtgctctt tcaatatgta 31320
   gttttaaatc tttttttttt aatttcagga aaatcttgaa ttagagtttt ccgtttttcg 31380
   tctggtacat tgcttgggtt tccttcttca ggaactcagc ctgttatgtg tatgtttgat 31440
   cttctttgcc tgtcgtctgt ttctttcact tcctctcact tttttaaact tcatttatta 31500
   aaaaaaaatt tttttttcga gacagagttt cgctcttgtt gcccaggctg gagtgcaatg 31560
   gcgtgatctc ggctcactgc aacctccgcc tcccaggttc aagtgattct cctgcctcag 31620
   tctcccaagt agctgggatt acaggcatgc gccaccacgc ccagctaatt ttttgtattt 31680
   ttagtagaga cagggtttct ccatgttggt caggctggtc ttgaactcct gacctcgtga 31740
   tctgcccgcc tcagcctccc aaagtgctgg gattacaggc gtgagccact gtgcccagcc 31800
   ttattaaaaa ttttaaaaac atacatttaa acttaacaga aaaattatga gagagaaggg 31860
   ggtggtgcca ggctttttta aacaaccagc tcttacatga actcatagag tgataactca 31920
   ttaccatgag gacggcatca agccgttcat gaaggatctg gccccgtgac ccagacacct 31980
   cctactaggt ccatttttaa cattggggat cacatttcaa cgtgagattt ggagggggca 32040
   aaactacaaa ccatgtcact cagggattgg aggagcaagt accacctata ctttggactc 32100
   aggtagaaag gcaaaatatc caggaaataa gctgctaccg tccagggttc agcagaggtg 32160
   cccatcagcc tgccaagtac tcaagagtcc agcctctagg gagctaatca tcatggtgag 32220
   ctcttcgagg cacagggagc tgggaagaca gtgcttgcca cccctgcctg aatagtgttt 32280
   gcacagagag ttctgttgtg tcttgattgg gtcctcctgc cactgggaat gctgtggatt 32340
   atactaggtc tctatctggc ttgtttcagg gctccatgtg aaaaccttct tgatatccta 32400
   gccatccacc tgctcagtcc ctagtttgca aggaggctgt ggggagccta gattctgtgt 32460
   cagatagaat gtactacatt ccgtctcagg aatgtaccac atcagaaaac agtgcgacct 32520
   gcaggagaag tagaggtgaa gaggcacatt cttccgagaa atgtttctct caacacccag 32580
   cattccctgg atatcagcag gaaattactc actgctagaa aatgccccat gagccttctg 32640
   ttaaggaggt caagggagag aacagagaaa gttctcaaag ttgacttggt cactggtact 32700
   ttcttatgcg gttcttattt tgtttgccat cgtcatcatc atgctatgtc tattttctca 32760
   atccaaatcc actgctttca ccttggttct ttctgaccgg tttggcacac tcattcagta 32820
   aatccttatg gagagcccaa tgtctgcata attgtgctgt gctgatgacc aagctagacc 32880
   tacgagtgtc ggctcctttg agatgtacgg gacagctctt ctgtcatctc ttctgggaag 32940
   cctctccagg cttggtgaac agtggcaaga tgtttaacag ttgtacatgt gtcccatgtt 33000
   cctttctaag agcctgggca aaccagaccc ggtcgcaggt catcgtagta tggcgtgagc 33060
   ttcctctctc ctttctgacc ttttgtgtga tggcaagaac ctgcagagtg acacaagcag 33120
   caggcttctg aggttgctct agcctcagaa tggccgtccc ttctccaccc tggccctcat 33180
   tgctgaggtt tcctttgaag caacagtgcc ggaacagact aggggaagca gcttggacat 33240
   agctgtatga tttattacca cccattgagg ccaaccaaag tcggcaagga gaggtagcag 33300
   gtcagtggtg cctggaagct tcctctttcc tttgcaccag atgtgactgc tctgcaatta 33360
   ctcctaaatt tgctactctc gtttttacta gccaaccttg atgtttttcc cttcttcctg 33420
   tagaatagac ttcccctctg atcagtactt tctactcaac actatttgtg gccacagtgg 33480
   gaactcattg aggacaggga ccatgacatt actacctgac ccatcaacac ttggcataac 33540
   ttgaaatgca aggacaaaaa ttggctgcaa gtacaatgtg gtcttcactc tgaaggtgat 33600
   ccttaaaact tggctttggc atcatattgc cttaatatac ctaggggatt gggtaaaacc 33660
   agttacttta aaagagtttt acaattctgg ccttctagct atcttgtctt cttaaacaag 33720
   agcacaagat gaatgtatct tagtgaaatt ttatatggtt tgctttgagt aatcttgcga 33780
   agattgattt ttagcacagt aggaaagaca cattctaata gtgatttttt tccccgagtt 33840
   tatgtactgc tgttgcatga aaatctgact agatttaatg ttcctaaagt tctttgttca 33900
   tcctgatttt tgcaggtcct agggaaagct ttgttttcct cttaacctaa cttagatgtt 33960
   gtcatttcat gagctttgga ggaagagtgt atagccaatt gtgtaatgtc tttaaaggat 34020
   attatctctg caatagttgt ttataaggcc taagttattc atgtaataat agtggccccg 34080
   gatctgtttc tagcaatagg tatatggatt ttggttccta tatagttgta gttgtggctt 34140
   tgagatattg agcaagccct tttaagaaag gatttggcat ccctcagcct tcaaaagctt 34200
   ctcaaaattg atcatatgtt attagcaaag gtttactgcc tgcttccatt gtatagacaa 34260
   tttatttttt atgtattccg ttctaagaag gcagatgacc aaaagatctt gcatctgttg 34320
   cccaaggctt gtgactagag aggaaagaga taagaatact tttttaaaat cccattttac 34380
   taaatatgtt gaggaagtgg taagatatat taatttgttg agatttttct gttatgccta 34440
   ttatatgaaa taggtactct gaacatggct tcttaattaa atatatttga taaaatacaa 34500
   cttgcttccc ctggagttta gaagtcagat aactgccatg gagagctatg ctttctttgt 34560
   tttaaagatc tgcttatgaa catgataaac aggaacaatt taatgttttc aatattttct 34620
   tgtattttac tgcaagttta tacacaacat aaatatgggg gaagggggaa atgtttatac 34680
   cagagccatc ctgcccattc tttccttaca gaaggacaaa ggagcagtat ttattttaac 34740
   tacaaaaata ctattgtagg ttttaaaaat tccgtatatt ttgatatctt gtgttcctct 34800
   tgacctttaa tttgctaaat agttgcaaag aatgaaggta acctgcatca tcttcttaaa 34860
   aaccaactct atctaattat aatagtttgt ctatctctga aaaatagtga tgtgttcatt 34920
   ctgaaatcag aactaccgga tgcagctgca ttttgttact atttgaattt cgggagaggg 34980
   aggaggatgc agcctttcga gctgctgaaa tacacaaaca caaagaagac accaagcata 35040
   gtagaactgt gttaagctga ccaagccaga agaagcacct attctcagca tagtatgaga 35100
   cgtaaaggca atataatggg catagttgaa gatggtagaa ggaaaataga ctctgatggt 35160
   ttaatgttaa atgctttttt taaaaaagtg gtattccaat atcgaagaag aagactttct 35220
   acttttagaa gcaataaagg aaattgcaga ggaaagggtc aataggttgg aatacataaa 35280
   aattaaaaac ttttaaactt tttttttttg agacagagtc tcactctgtc acccaggctg 35340
   gagtgcaatg gtgcaatctc ggctcgctac aacctccgct tcctgagttc aagcaattct 35400
   cctgcctcag cctcccgagt agctgggatt acaggcatgg gccaccactc ctggctaata 35460
   tttgtatttt tagtagagac agggtttcac catgttgtcc aggctgatct caaactcctg 35520
   acctcgtgat ccgcctgcct cggcctccca aagtgctggg attacaggca tgagccaccg 35580
   cgcctggact aaattgtttc agtattaatt ttttttaaaa caagatctta ctgttgccca 35640
   ggctgaagta cagtggccca atcatggcta actgcagcct tgacttctgg gcctcaaggg 35700
   atcctcccac ctcagcgtcc cgagtagctg ggaccacaga catgtaccac cacacccagc 35760
   tacttgtttt atttttattt ttgtagagat gaggtttcac catgttgccc aggctggtct 35820
   cgaactcctg ggcccaagca atcctcctcc cttggcctcc caaagtgctg gtattacagg 35880
   tgtaagccat tgcgccctgc ctgatttttt aaatgtgcaa acagataagt tggaaaagtg 35940
   atttccaata aagataaaga gttgatggtt ttaaaatacg taaagagctt atatgaatga 36000
   gaaaaacact aacattccaa aagattagaa ggcaaaggac agaaagaaac aaatcactat 36060
   gtctgggaag ggacatgaag gagcaggttc ccactgggcc agcggggctc aaacccactg 36120
   gggacgtccg agagactgca agggccatgc cttcacattg ccgtacctga gaagcaagga 36180
   gctggggtat ttatctcttt cacactttgg gaggctgagg tgggcggatc acctgaggtc 36240
   aggagttcga gactagcctg gccaacacag tgaaaccccg tctctactaa aactagaaat 36300
   aattagctgg gtgtggtggc acacacctgt aatcccagct acttggaagg ctgaggcatg 36360
   agaattgctt gagcccagga ggtagaggct gcagtgagca taaattgcac cactgcactc 36420
   cagcctgggt gaaactctgt ctcaaaaagt aataataatc atgataaata aaataacatt 36480
   agattgttag cagaagtagc cacaggtttc tcccacctct ctgcaagttg ctgagtgtga 36540
   ttcccatcaa gaggtacaat gtctttttat ttttatttta tttattttat ttatattgcc 36600
   tatgttgtct aggctggttc caaactcctg agctcaagtg atccttctac gtcagccccc 36660
   caaagtgttg ggattacagg catcagccac tgcacctggc ccagatactt tttcttgagt 36720
   aggaatttcg agtcaccctg aacattgcat gccttcgtag tggggaagac aataggaaac 36780
   cacaggctgt aggctaaaat gggttgtgtt tcttgtaacg tcatgacaag gcataaccca 36840
   tcttggcata gtaaatagta agcactcact gaactgatga ttttaaatct ttgctgttta 36900
   ttcagcaata tcctaaatta gcgctatgtt agtggagttg catctccctc atggattagt 36960
   ctgaaaaaga tgagaaatct gtatgtagac caagttatcc ttaaactgct cataatgtat 37020
   gatgcacgtg gttttacgtg tacagcctgg taccattgtt cttaggcaca tttcagtgcc 37080
   agaactctta atacccagga agaagcaaaa agaaagatgg aggtgcagct agaggttgtg 37140
   gcctttgaac gattcattct gccttaataa gagtggtctg gctgagctcg gtggctcaca 37200
   cctgtaatcc cagcactttg ggaggccaag gcaggcagat cgcttgagcc caggagttca 37260
   agaccagccc aggcagcata gcgagacccc ccctcccccc gtctctacaa aaaaatagaa 37320
   acaatgagcc aggcatggtg gaacgtagtg cgtggtgcct gtagtctcag ctacccagtt 37380
   ggctgaggtg ggaggatcac ctgagcccta gaagtcgagg cttcagtgag cccttattgt 37440
   gccactgcac tccactctag gtgacagagc gagacaggtc ctgtctcgaa aagaaagaag 37500
   aagaattaaa aaaagtgatt agatcccttg tgtttgggac acttgttggc agcagggatg 37560
   gtagcgttta tgagggttgc atgtaacatc gcctagctca gacatctgtt tgactgtctt 37620
   cccccctgaa gcgcaggctc gttgagggca ggtcttttgt ctttcttgtt aatcttcata 37680
   tgcttagtgc ttgccacata gttgatgctc agtcgatatt tggatgaatt gaagggatta 37740
   atgcattgaa tctgaacctt gctttcttaa tgcatatggg gagttctttg gaaagccaca 37800
   cagaggagct tggttgcctg cttcctctct tccccagatt gtctttttat tgttgtggct 37860
   tcactgaagc actctcactt caaataattt tgggcattgg tcgtatttta ttctttgttc 37920
   cttcttcatc cttacccctc agatggtatg tagaaaagta cactacatct agaaagtact 37980
   ttataaactc atttggttga taataataca tatgcctttt ccttggtcct ggtagcagaa 38040
   tcttgtgcca ctcttggaat acaaacgaaa ttcttaacca aagccagttt cattttgatg 38100
   ttctattttc ctcccattca cactccaaat tgtgcaccaa agtatcatcc tagttttgtg 38160
   aggatggttc tccatacttc agggtaggag tatcatgtgg attcctatga tacctttctc 38220
   cctgggacca tggagggcag cagctggtga ttgatagtct gattcccggt gaggaaagct 38280
   gtgagccttc cacttgcaga tgtctgccaa ctacatgtgt ccttagtcaa ctgtaccact 38340
   gtcctccggc aaacagcaga agcccagggc ctgaagttct taagctgtca ttatggaaag 38400
   cagaaggtaa acaaaacaga agtgaaagta gatttaattt tttagactgt tctcttacag 38460
   gaatggtttt gtggttctca gcattttaaa aaaaatagtg gttccaatat gttttattga 38520
   catcaattac tgtaagtctg attcattttc tgcctattga tttctaccca aggtgaaatt 38580
   catgacattt aacagaaagc ataagtgatt ttttaaaagc agacactatt agggacggta 38640
   aaaataagat ttaaagtcgg gacacttgaa aaagcaattt ttataccttt ggtaacgatt 38700
   ctattctgat tctttgtata aataatataa acaaaggctc tagaagctta ctataatgaa 38760
   gttggtgtgc tgtttctaaa ttctggttta aggcccaaat tcattttatc tgcattaact 38820
   tttttttttt tgagagtctc gctctgtcac ccaggctaga gtgcaatggt atgatctcgg 38880
   ctcactgcaa cctctgcctc ccgggttcaa gcgattctcc tgcctcagcc tcccgagtag 38940
   ctgggattat aggtgtgcgc caccacgccc ggctaatttt tgtattttta gtagagacgg 39000
   ggtttcacta tgctggtcag gctggtctca aactcctgac cttgtgatcc gcctgcctcg 39060
   gcctcccaaa gtgctgggat tacaggcgtg agccactgca cccggccgtg ttaaaatttt 39120
   tcagtggtag accactatgt caatatgttg ctttcactga caacagtatt ttcttaaaga 39180
   taggataccc catttctaga tgaatctcat tctagctgga aaataatttt tcagttctga 39240
   aactacatca ggcctcaggg aatcaaaact agctattagc cacacacata taaagtggct 39300
   ttgctttata aacgatttag ggtcaccatc aatgacaatg gtcccttttt attgtatttt 39360
   taagagtttc ttatcttaaa tggctgcata actgtagagt tttaaaaaaa ttaagtaaat 39420
   gaccatgtta atgctctatt aagcttccaa acaatattgt aatttacttt gaagattttt 39480
   ttttattctc aacatcctgc agcttgaccg tttgcctccg tgtctcagtg ctgcttattt 39540
   tgaggtgtgg actggagtcc atctgtcccc cttgcctctg aactgctccg ttttgtgttt 39600
   cgtaattctt catgctgcat cctgggcgca tttctctgta gtagctttca atttgctcat 39660
   gctttgactg ggcttagtct agcgtttatc ctatctctta aggtttttta aaaaattttc 39720
   atgattattc atttatttcc aggatttctc atttcttcag tcacatctcc ttgttctggt 39780
   tttacttctt cctgttttta ttcataacat cttttttata cacgattcct tcatgtattt 39840
   ctaatcttaa gtatatttaa ttgcttattt gattcttttt tttttttatt gagacagggt 39900
   cttactctgc caccaggccg gagtgcagtg acatagtcat agctcactgc agcctcaact 39960
   acttggactc aagcgacctt cccacctcag cctcccaggt agctaggaat acaggtgtga 40020
   gagccgccac acccagctga tttgtcttac tatgttgccc aggctggtct tgaattcctg 40080
   ggctcatgtg atctgccctt cttggcctcc tgaagtgctg agattatagg tgtgaaccac 40140
   tgcacctggc caagtatgtt tatttattta ttctaatttg agagggagtc tcgctctgtc 40200
   gtgcccaggc tgtagtgcag tggcacaatc ccagctcact gcaacctctg cctcctgggt 40260
   tcatgcgatt ctcttgcctc agcctcctga gtacctgggg ttacagttgc gtgccaccac 40320
   acctagctaa tttttgtgtt tttagtacag gcggggtttt accctgttgg ccaggctggt 40380
   cttgaacttg tgacctgaag tgatccgccc gccttggcct cccaaagtgc tgggattaca 40440
   ggcatgagcc accacgcttg gcccaagtat gtttattttt aaagtcccca acaagctata 40500
   caataaattg catatggaat ggatttttgt tctagttgat ttgttggtta tcatttgtag 40560
   aactaactag ttgtcttctg tgtttgatac cttgcttcta ggtcattttg agttgggagc 40620
   cttttgtttt gtttttattc tcatgctgtt tttgagccta gctgtgcctt tatggttttc 40680
   tctaaattta attgaccatt gttttatatt tggagcagtg ggtgtacatc agagtgtgaa 40740
   agcagcccca ccctctccac cagaaggtct ccatgccagt ttcacgaagc atttttcatg 40800
   ccctcattcc tgcccttatc ccttgatttg tggggagttt gtaaagcagt tgattgtttt 40860
   ttttccacgt agttttccaa gtgcacataa ttgttctgtt agtgacttgt agctccatta 40920
   tctattaacc ttgccccaga ccactgtaca agcggaccca acgcttcctc cagctgtggc 40980
   agggacagtt acttggtatc ctgctgcctt ttcaatgctg accagttttg ccccttcctc 41040
   ccctcaaccc ctgtctttca ttcaactatc accaaaccaa aagattctgg tttgcttttt 41100
   agtatgtgtt cttattcagt acatagtcat tttaaaattt aaaccaaaac agacttggta 41160
   ctgattagct taattttaag ctttttcttt attattaaac agtgtagttt atcttagcat 41220
   ttcatattaa gtatatgatt tatttcatat tgcttatatg aatgtacaca taaatataat 41280
   aaaaatattt tcctaaggtt tttgtagtaa attatatcgt ttcattaact ttcatatata 41340
   gcattgcttt tgacctggaa gacattgaac ctctgatgat ttgtatattc ctcggagtat 41400
   actttgttac atagaaattt tctcatttat aatgagattt gtgattaaca aaatttgttc 41460
   aacatgcatt actttgaaga tctggtttct aaaattttat gctagttacc ccaccccccc 41520
   ttctatatat atctccctat tcagcgacta ctgcaagagt tccaggaaat gtacactgtg 41580
   tgttcactta ctgcatttta aatcattgcc tttactatat ttctgcattt cccttcaatc 41640
   tagctctgtc tgtacatttc tgaaagccag tagcttccct gaagaaccag gtaacaaccc 41700
   gaacaatcaa attagataac catttgtaga atggaggttc cgggagatct tagaagatgt 41760
   gatgggtgct aagggacttt gtagttccct gaagttccag tgagtaaaag gtacccttgg 41820
   aattttttat tccttcagac ttttaaaaca gagatcactt tcaaaaatta ctctttctgc 41880
   tttgaatcca tgttttagta actattttga cactgtttgg tcagaaggct gtgtgggtca 41940
   actgcaaata aataaaataa atgtgatttc agtaatttcc attttgtaac aagtaattga 42000
   gaaaatagga ttggatcaga tatttgctta tacacattcc ctttcaggag cacttctgtt 42060
   ctataaagaa tgttggtata ttgttaagga cacttcaagc tttgggaacc tttgaagtat 42120
   ccattgattc agttaacaaa attatgttga gtgcctaccc tgggcctggg cctgtgttag 42180
   gaggggacac taagatgaga gtccaaagca cttcttctca gactcctggc tgctaatggg 42240
   ttgctgcctc tacttcttca cttagcagat agctttaaaa tgagtaatgc attttaccat 42300
   ggagcccgta agagacattc acccagttgt ggaccgagga gaagggtgtt aaacccagat 42360
   tgtgatgttt cacttgatga agtgcttaat ataaacatgg aaatatttcc gcaaggataa 42420
   actggctttt atgcctgtgt gttttcagga gaaatagaaa tctctaatca aatattgcca 42480
   gcttttcacc caagtttgac tttttgccta attgagtttg ggaggtgtct gaataatgga 42540
   taatgagctt tcctgaataa atataaaaat taattaactc caggctctaa ttcattctgt 42600
   taccagagtt ttgtaagcat gttacccctt tgtgttcatt gggagatcat ctgttacctt 42660
   cttaaatgag tggggaagga tgggaaatga ggaagagcta taaaaactat tcaggtgaag 42720
   aaggtttctg cccctccttg ccccttttaa aatctccagc tcagcagatg ctttgtttaa 42780
   acttgatcaa gtgcttgtga atcttcctag cctagctaaa tcataacttt ggaaggactt 42840
   gcttttttct ctcatgacaa tggtttacca cagaaatgat tcagatcact ttgtgtgcct 42900
   gat gcct at g t aaaat gat a cagt gaaat g gaaaccat t t acct gt aagc t t t gggcaca 42960
   cccaagcct g ct t caggagc acat gat cag gcgt gcact c t gggagagcc gt acacat t t 43020
   gacat ct at g at gt gt ggcg t t t t at t ct a t cacat t t ct gaaat ct aca ct aagagaaa 43080
   ggaggctctt aaaaaaccac tgaggtgtgg actgggggaa ggagagatcc gtaaagaacc 43140
   t gt t t gt t ac ct gt t gat ac t at t t cccat t ggt aaaat t t ct aat t t ag t gt gat ccag 43200
   ccct gaaat g ct gaggcaca cact gaat ga ct cct gacat ct t t agt gt t t t t gt t cagg 43260
   ggact ct t ct gggaat ct gt t t cat ggcaa gt t t at t at t ccct t t t ggt t t ggct cat c 43320
   agt t t accca gcagt cat ct t aat cggt t t t aaaggct t t t at t t t at t t t gt t t t ct ct 43380
   gt ggaaat t t t acacat t ca gt agat t aga agt agt t at t t aat ct t t gg t t agcat aat 43440
   aaaagat ct t ct agggacat t t t t t gct t g cagt ggaagg ct agt t aaat gt gt t cat t a 43500
   gt cat gaat c t gct t t t t ct at agct gt t g gaaacgt agc t cccct gt ga t acagt t gt a 43560
   gaat acagaa at ct cgt t t t gct gt t acgg t acggt agt c t act t act t t ct t ccaaacc 43620
   at t aat gt t a t agt t acct t t aat t gcgt a ggt cct at ca cccct caat t t t aagact ct 43680
   aagcct ggca t t t t at ct t a caaaat gaaa t at aaagact t gt act caga gt at gt gt gt 43740
   gt t t t ccat a t accat t ct a aagt agagaa agat gaggga t t cgccagaa act gat t t ct 43800
   aat aaat t at ccagaaact g acccct t ct c acct ct t ct g t t act gt cac t gt ggt t t ca 43860
   gccacagcat cctttgctgc attgttacct tagtttcctg actgtatcct tccttacacc 43920
   attgatccct gcaatcccat ctgcgcgtag cagccagaag ggatccactt actgctgtga 43980
   t cagaaat cc t cagccaggt gcagt ggct c at gcct gt aa t ct cagcact at gggaggct 44040
   gagactggag aattatttga gcccaggagt ttgagaccag cctcaaactg ggtaatataa 44100
   tgagacctca tctctacaaa caggaaaaaa aaaatttttt tttttttttt aactagccag 44160
   gt at agt gct aat at acct g t t ct gggat c cagcat gct c t ccct gacct gcagct t cat 44220
   ct ccaccact t t gcccct ca ct cccaccac aat ggct t t c t t ct ct t cct cagacat gcc 44280
   gt gcgt cct c ct acct ggaa t at t cccct c caaacat t cc cat ggct cac t ccct cacct 44340
   t cat cagat c t ct gt t ccag t gt cact t t t act ggaaggt ct t t t gt gac cat cct act t 44400
   at t at aaaaa aat aat ct gc ccaacct t ct cct t t t at t t cct ct act t g at t t t t caat 44460
   t t agt act t a t cagct gaca t at at t t t gt ct ct ct gt ct ct ct ct gt ct ct cat agaag 44520
   gt aaat t ct a t aaaggaagg aat t t t t at g t t t ggt t ct t t gct gt agct ccaat at t ca 44580
   aaacagt gcc t gacacacag t aggccct t t at at t t gt t g aat aaat gt t gacact ct ga 44640
   t at ct aat t t t t gt ct ggt g act aat acga aaact at aga gt gat aat aa aagcat t acc 44700
   ttagtagact ggaaagggat gagcgctagg atgaactttc tgcctggcga tcttgctgaa 44760
   tttaggaggc agattggggt tcaaaggagg ctgaaatggc taggatttgc agagcagggt 44820
   actaaggatg agcaggctat gacagaaaga actccagaaa tctgcaaagg gatcaccttg 44880
   agt ct ggct g gat acagt gt acact t t gt a gggt gt ct ct t cat gagct t ggat aaagaa 44940
   caactgttgg ggagtggata attcccagca ctcattcaag cttgcatcgg ccagaacgga 45000
   gagagacaga cctctgtaat acgtaggata tttggtagaa acattcaacc gaaaaccatc 45060
   agat at gcaa aaagt aat aa t aat aagt aa acaat gt gat gcat agct ag aagaaaaat c 45120
   agacattaga agcaagccca gaaatgacag atgataaatt agcagataag gacattaaaa 45180
   cagctattat aaataactta gcagatttaa agaaaaacaa cataatgagg ataatggaag 45240
   aaaaacaacc gaataccatt tctaaagaag aaaaatacaa tatctgaaat gagaatttag 45300
   ctggatagga ttaatagttt aggcactgca gaagaaaaaa acagcatcta tatgagaata 45360
   tacccaaggg aagtacagag aggaaaaaaa tgtggattgg ggggtgcctc agtgacatat 45420
   ggaacaat at t aaacaagt c t gccccc aaa at act t gaag gaat aaggt t caagt t t t t t 45480
   ccaggt t t aa t gaaaact at aagcct acag at t caagcat t t caacaaac ct t cagcaaa 45540
   at aaacaaaa ccacagt agg cct ggcacac t gt ct cat gc ct gcaat ccc agcact t t gg 45600
   gagcct gagt caggaggat t gct t gagat c t gct t gggca acat agccag accct gt ct c 45660
   t acaaaaaat aaaat gaaat aaat t agct g gat gt ggagg t ccacacct g t aact ct agc 45720
   t agcct ggag gct aagaagg gaggat t gcc t gagcccagt agt t caaggc t ggagt gagc 45780
   taggactgca tcactgcact ccagcctagg caacagcaag accacatctc tctctctctc 45840
   t ct ct ct ct c t ct caaaagg cagt gaaat a acgact t at t t ggggaaaaa at aaaggcag 45900
   agaatttgtt gccagcagac tagcataaaa aaaaggaagt ccttgaaaca gaagagaaat 45960
   gat aaaagat ggaaat t t gg at at at act a aagaat gagg at t gct aaaa gt gacat aca 46020
   tagataaata tgaaatatat ttttatttta aaatttattt aaagcaaaaa taaaaataca 46080
   t cat at t t at aacat agaaa t aaaaaat gt at gat aat ag cat aaaggat aagt ggacaa 46140
   at gct gt t gt cgt at t t t t g gt aaaat gca ct at t at t t g aaagt agacc at cgt gaat t 46200
   cgat gcat at t gt aaaccaa at agaacact aaaaaat gaa aat aaagaga t at ggct aat 46260
   gtgccaatgg tggagataag atagatgcaa aaaaagaaaa acattcaaaa gaaggcagag 46320
   acagaggaaa aaaggaccaa agatcaaatg agtcaaatag aaagcagcta aactagcaat 46380
   at ggcagat t t aaat ct agc cat gt caat a gt t at at t aa at gt aaat gt t ct aaat acc 46440
   t gaat t aaag gat gaagat t gt cagat t ag at t gaaaaag cat gacccaa ct acat gct g 46500
   tctgtaagaa attagaaaaa gaacaaatta aatccaaagt aagaagaaag gaaatagagt 46560
   agaagttagt gaagtataaa acaaagagca aagaaaatca attaaatgaa aagctggttc 46620
   tttgtaaaga tcagtaaaat tgataaattt ctagctaaac tggccaagaa aaaagaaaag 46680
   acatacaaat taacagtatc aggaagaaaa acagagaatt caaaggagtg taatgcaaac 46740
   t t t at gct ag t aaat gcaat aagt t agat g gt at ggaaaa aaat gt gaac aat acaaagc 46800
   agact gt ggt t gcct t t ggt ggcagt agcg gggt gggagt ggaaggt t ga at t gact gga 46860
   accagaagca caagtgaact ttttggggtg atggaaatgt tttgtatctt ggttgcattg 46920
   at agt t aaat ggt t gt agac at t gct t aaa act cact gaa cact t aagt g ggt at gt t t t 46980
   attatttgta aaatatacct caaaagcagt tttaaaaatg tattcaagta catacttaag 47040
   atctttgcat tttactctga gtatacctta attttaaaat ctgtttttta aaaagtatta 47100
   tgtagatacc ttttattttc ccaatgtctt tattaaatga catctccacg ttttgcttct 47160
   tacctctatt tttttttttt tatttctctg tctctcaggc atgcacacac acacaccaaa 47220
   aaaagtacat atgcataatc cttttggctg aataaaatca gttgcaactg ttatttcggc 47280
   ccttatttgc tccgggtaaa tattcgttag ctgagtggtt tatctgtatc agatatttct 47340
   tacatcttca tccagtcaca ccagctggac tgaccagatt gtttttcact tcaagggcag 47400
   aatttgtact cactgctgaa tgcttccaaa tgatacgtag aataacaaat ttaagactta 47460
   gatttttact ttttcaggtc tttttttttt tttctgtgct gtatagcatt tccctgaaag 47520
   cttaatctca tctgtaagtg atgcagtgga tgtgttacta ttggattaat ttatttactc 47580
   ttaggtaggt ttgtaatctg tcatcatgct gttgtttttt tgtgtgggtt tgtttttggt 47640
   tttgagacag ggtctcactc tgctgcccag gctggagagg ctagagtgca gtgatgtgtt 47700
   tatgggtcac tgcagattca atctcctggg ctcaagtgat cttcctgcct caaccccttg 47760
   tgtagatgga agcacaggtg cacgccacca cacccggcta tttttttaaa tgtattgtag 47820
   agacgaggca tcattttttt gcccaaggct gatcttgaac tcctgggctc aaacaatcct 47880
   cccacctcgg ctcccaaagt gctgggatta cagatgtgaa ccaccactcg agctccatca 47940
   ttctgttatt agttgttctc tagtatgagt caaaaactct tacctgccct tttacagttt 48000
   tataaataag taagcagaat agcagaatgt ggacattttt taaatccaaa ttgaatatgc 48060
   acatgactca aggagtcaaa tagtaccgta atcggtttat gataaaatcc agtggtttgg 48120
   ctgggtgtcg tggctcacac ttgtaatccc agcaccttgg gaggctgagg caggtggatc 48180
   acctgaagtc aggagtttga gaccagtctg acctacatgg tgaaactact aaaatacaaa 48240
   attagctggg catggtggtg catgcctgta atcccagcta cttgggaggc tgaggcagga 48300
   gaattgcttc aacccgggag gcagaggttg tggtgagccg atatcgcatt atttcagaac 48360
   aattttccac aagatcagtg agtgctgtcc aatagacata taatacaacc cacatacatg 48420
   actttacatt ttcttgtagc catagtagaa aaggtcaaaa gaagcagatg aaattaatag 48480
   cctgggcaac aagagcaaaa ccccatcttt taaaaaataa aataaaatat ggtggtttgc 48540
   tgtccccacc tcagaccatt tctctggtct ttctcattga ccaccactcc caatctttgt 48600
   tctgctgatt gattacagct tgtatatatc tccatatttc taagcaaaat gtttatcttt 48660
   tttaaattta taaattcttt ttattatttt tcagagacag ggtcttaact ctgtcgccca 48720
   ggctggagta cagtggcacc atcgtagctc actgtagcct cgaactcctg ggctcaagca 48780
   gtcttcctgc ctccgcctct caggtagctg agactacgct acaggcacat accaccatgc 48840
   ccagctcaaa atgtttatct tttgatacat tattcgagac cattattaag gtggatgatt 48900
   tagttttctt aaacagccat cccctttctt ttcctcccct ctgcttcacc gcccccattt 48960
   tcccaatgtt ttaccttttg gttaaatcag tactcattgt ttacattatt tgcctctgca 49020
   catagtcaca gatagtattg tactgtactg tactgtgttt cttttttaaa cattatttct 49080
   gttgttaata attgactttt taattttttt cctattttgt tttttaaaga gatggggtct 49140
   tactatattg cccaggctag agttcagtgg ctcttcgcgg gcatgatccc actgctgatc 49200
   agtacaggaa tttccacctg ctccatttcc aacctggacc agttcacccc ttcttaggca 49260
   acctggtggt cccccattcc cgggaggtca gcatattgat gccaaactta gtgcggacac 49320
   ccgatcggca taacgcatgc agcccaggac tcctgggctc aagcagtcct cccgggctca 49380
   agcagtcctc ccacctaagc ctcccgcgta gctgagacta cagacacttg ccaccacacc 49440
   aggttaattt ttgtgttttt tgtagaggtg gggttttgcc atgttgtcca gactcatctc 49500
   aaacttctca gctcaagtga gcctcctgcc tcagcttccc aagtagctgg gattatagac 49560
   gcatgccacc acaccccatg ataattgcct ttttttttaa tttgcataat tttctttgta 49620
   gcttttgcta atgttcccat atcttcttat agccttacag aatgattttc cacaagatca 49680
   gtgagtgctg tccagtagac atataataca acccacatac atgattttac ctttttttgt 49740
   agccatagta aaaaaggtca aaagaagcag atgaaattaa tagtatcttt tacttaaccc 49800
   agttcattca aaatgttatt tcaataaatg gtcaatattt aaaatacttg agatattttg 49860
   cttttattta tttcttttgt tactaagtct tcaaaatcca atgtgtattt tacacttaca 49920
   gaacatctct ttttagactg gccacatgta gctcagggtt actgtattgg acagagtggt 49980
   ttcagtttca agtttttcct tggagacatc ctacttgaaa tttccattct ccatgtatct 50040
   gggtggttgg tctatagact tgccactcac agctgtcatc ttgagacttt ctttgctttt 50100
   cttctctatt ggatattcag tttcctggat ttcaggtctt ctcattttcc tctagtagtt 50160
   ttgttaggtc atggttggta tggcatggtt gggatagcgt gttcacacag ctatctcgtg 50220
   agtcatactc ctccaatcca gcctgctcgc ttcccgtgtc tgtcatgtag ttgtcaccct 50280
   gctatctctc cctccagttt ttgcagaaat ttcctttgtc ttcactcttg gtcttcctct 50340
   cccatccccc atgtatccta tatctttctc tttcttggtt tatttcatca ctcaggtgga 50400
   aaagatgctc cagtggatta ctgggaaaag ggggagcatg gatgataaag gtattgagac 50460
   cttacacgtc agggaatttt tttttttttt tttttttgag acggagtttt gctcttgtcc 50520
   aggttggagt gcagtggcgc caactcggct cactgcaacc tccacctcct gggttcaagt 50580
   gattctcctg cctcagcctc ctgagtagct gggattacag gtgcccgcca ccacgcccag 50640
   ctaatttttt gtatttttaa tagagacgag gtttcactgt gttggccagg ctggtcttga 50700
   actcctactt caggcaatcc acccacctcg gaatgttttt attgtccctt ctcatttcat 50760
   gactgctggg ctaggtatag aattccagaa tcattgttct tagaatctcg aaggcattgc 50820
   ttcattgctg gccagctttc agtgttcttg caaagtctga agctgtgcta atcacctcat 50880
   cctttgaaag tgaactgttt tttcttccca gaaacttaca gaacattctc tttgtccgca 50940
   gaattctggg attgcaatta ctgtgcctta gaatgggtct gtttttatca ttatgaagag 51000
   tactggatgg gtcgggaggt tttcttgaat tacttcttga tgtttctttt ccttgtattt 51060
   ttttgtttgc taattttcta tttttttttc ttggtttact ttcttgggca gggggatttc 51120
   ttctacttat atttgattct tcagttgagc ttgtcatttt tgctatcttg tttttaagtt 51180
   tcgagagaca tctttgtttt atataacatt ctgttcttaa tacatagatg caagatcttt 51240
   tctttctgag tatattaata tgtatttgaa atctttctat tctctgcagt ttgtttcccc 51300
   caagggtttt tttttttttc tggtttttgt tttttgtttt tatgttagag actttcctgt 51360
   tatatctggt catcagtggt acctgcatgt ggtggagagt aggggcttat tggagtatga 51420
   gaaccttgag caggtgtaag gagcctgtca acactgcgct ggcctcaggg cctctaggga 51480
   ggctgccagt tgtgcattct gaggatacct tttggttgtg ccttttgtct ggtcagatta 51540
   tctagagatg ctctgcctcc tacctggagg agaagggtct agctgccagc ggtgtgagtg 51600
   tctcttgggg aaaaggactc gagttcctgg tgtttggctt gtgtatggcc gcttacccca 51660
   tttttggtgg agcgctcaca tcttccactg tgccaacagt cttgctgcag ttcatagacc 51720
   ttctggttta catttttcca gaaagtatgt ctttagattt ctgcagaagt ctgaggagca 51780
   tggaaggagc ttggggaatg agatggcaat ccaggtcttc ccagatggct ctacctttat 51840
   cccctgcagg gaatcccact cctccttcct gactgggagc acagccagag ccttgggagg 51900
   aatctggagt ggaaatctcg ggcggtctgg ctttcttact gttcacttgt aattttgctt 51960
   tctcacaact gccaaccact aatcagcctg atttccagct tccagaattc tattgctgtt 52020
   gtctgctctc ctattcccac cgtaggggat ggggctgtct tttttttttt ttttaatttt 52080
   ggtaaaacat acaaaacata aagtgttcca ttttagccat ttttaggtac acagttcagt 52140
   ggcagtaagt acattcacgt tgtgtgtatt tgttttttta gtaataaaca atataaaatt 52200
   ttttaagtaa taaaacacaa ataaaagatt gtttaatgtg attatcgtgg aattttaggt 52260
   gtgatcagga gccatggtgt agtcttctgt tgaaacaggg tgataggatt tgtttaccac 52320
   ctcctaggaa agcagttgga tagtttgttg gcataaaagt acattttatc tatttttaat 52380
   aatcgtagct ttatagaaat tgcagttgga actcccaggc ctggcattca aggctctctg 52440
   agatctgggc tacccaccca tgtcctccag ccgtctgtcg cacctcctac tgcccactca 52500
   ctgttcctgg catgagatgt gatctccagc ccccatgcct ttgctgtgca gggtgttcca 52560
   gagtgaattg tccctcctgt ctgtctctct gccctcttcc tcgtctttcc atcttcctgc 52620
   cccacatcac tgcctcctac ccaaggcctg tgctcattcc tcctcggttt tcccccatgg 52680
   cctggtacat acctctgaat tatcaccttg catttcccat attgcccggc tctctttgat 52740
   gtctgtttct ttgctgggtc ttcctcagtg tctgacggtc agttaaatgt ctttattctt 52800
   ttttgtagga tatccgacat gaagccagtg acttcccatg tagagtggcc aagcttccta 52860
   agaacaaaaa ccgaaatagg tacagagacg tcagtccctg taagtatcca cgtggccggt 52920
   accagtcttg ctcttccttt gctgcaggcc tttttagtca agactccttt cgcctcaggg 52980
   tttagtataa taataaatca atgtagcaga ggtttatgac gcgattgttt cctatagtaa 53040
   aggcattaga gacttatagt aatagctcat ttttccacca ttatagaagg gctcaggttt 53100
   cagtttctgg aaaattcagt gaagttcaaa gcacttttct taagctttga ctgtttttgt 53160
   gatgaatcat tttcctacca gctgaagcag agtatagcag gcataataaa accttttctg 53220
   gatgactcag cagcagcgtc attagggcat gagcactgtg ttccgctgta atgaagcccc 53280
   gcacaggcat tcggggtggg cactgtcgtc ccctgcgctg aatatgcaag gcagctctgt 53340
   ctggagtccc caccgcctcc acccccgcca acctcatcat ttttctccct ctttcctgct 53400
   gttagttctt cctaggattg tcagtgtgcc tgctggcctg tggcagccct gtccgccttc 53460
   tgagtgattg gctgtcagtc tgccggtagc tgaaaagtaa ataacttaac atgttagaat 53520
   ttgcataaag taaggaaaac tggagctgag tacaggactt gaactgcgcc atctcctcta 53580
   ggccacagag gcctttttga cccccttcca ggtctttaga cattgtcagg cagtgagggg 53640
   tcgtagctgc cagtgtctcc atggtagcgt gctctgccag ggatgcagaa gattctccag 53700
   tcattcctcc agtgggcact tcctgcaggt cctgtgccca tggctgggag tggtggctgt 53760
   cattgttctc tgccagaagg gttagcagtg catcctgacc tgacttatgt ggcgcccaga 53820
   ttcctggaag gggtctaaaa atggacctag acttggtgta gaacgtgtgc ctcttggcct 53880
   gccaccatgg ttccctgcct ggttttgtgt gtcagctctg ccgcttaaga actgagtggc 53940
   ttcgggcaag ttgttctctc tcataggagt gtgtgaagat gaagcaacat aagctgctta 54000
   gcccagcgcc cagtacctca cgcagacata agtgctcagt aaatgttgtc tgtggtgggg 54060
   atggttgtca ccaacatctg aagtgcactt ctaggtcatc aggtgacatg attggcgcca 54120
   acacatggta ctcttgattt agcacatctc agctgaggca cctcattgat atttgtttaa 54180
   aaacaaaaac aaaaaacctt ggtgattctg ctgtgaagtc ctggccagaa acctccagac 54240
   cgctgatcaa cacgcaacag aaccatcacc gttcacctct ttgacatggt gccaggatac 54300
   cctggatctc tagcttttgc tatagttgct ctaattaggg aataatcttg tctttaatat 54360
   tcctttgcta cattttttaa catttcttat ctaaatggtt ttatgaatca gttttacaga 54420
   gaaaaaaaac cagtatttaa aatattcttc caggggctgg tccaagtaca gtagtgttta 54480
   caactatgtg atcacaacca gttacagatt tctttgttcc ttctccatcc ccactgcttt 54540
   acttgactag ccaaaaaaaa aaaaaaaaaa agttattcca gggaaacaat tctccaactt 54600
   tttcactccc aatctcactc ctcttatctt cctcccgtac tcctatcctc ctcccgtact 54660
   cctatcctcc tcccctactc ctatcctcca gtagaaacag tcatttgctg tgaaggttat 54720
   gggggagaat gagtcaaggt agaaggtcac ctgctgccca gctcacagtg ctgctggtga 54780
   tgacagcagt ccacagttac aggcacttgc tgaacgaggg gctctgtata cacctcagct 54840
   cattgactct tcccacaacc ctcttgtcac ctaccattta gcaaatgaaa aaaccaaggc 54900
   tctgaggtga gttgtttgcc cagagtcacc cagtgctgtt tgaacccact cacataacca 54960
   accaatacca ttatgtaatt tttgaggtct tttatctctg tgatccactt aaaaattatc 55020
   caagtatctt tatttgtact aagcctccat aatgagaaac agtgttccag atggtggcta 55080
   gttttcaaag acatctctct ttggaattct tctttagaac aaaaagcccc agaccactta 55140
   tccccattca tatccccttt ggacctaggg agaaggtact atttataggt gatcacctga 55200
   gtttattgtc ccttgtgctg tgccagaaat aaaggtcccc acctgctctt attagctcta 55260
   ctaacaggat aaggaaagtg gccctcagag agctactgct tttgtgacaa acaaatgata 55320
   caagaaaaaa aaagtggctt tttaatttta gtgacctggg gcaggacttc caaatgaaag 55380
   tttatttcta aaaactaaaa ggtaaattta atatactttc agtgtttggg cttaaattct 55440
   ctttcaagtg tctttgtgat atgctctgaa ttttaaaaat ttagaatcat tgaagttcat 55500
   tatacttgaa ctttaaaaaa aaaaaacaaa aacctcgtat aaaggtcaag gtatgacttc 55560
   atgctgctgt gtacttaggt catttaatct tcaaaccact ggatagaggt taggttgaag 55620
   ttcgatctta aatcctacct actgtagctc attgtaccag caacagctgt agggactagg 55680
   tggaattcat ggtgggtttt gttccctttt aaagattgaa gccaccatat tttctgccct 55740
   ctaaaagttt atgtcagcca ggcatgggtg gctcacactt gtaatcccag cactttgggg 55800
   aggctgaggt gggtggatca cttgaggcca ggagttcgag accagcctgg ccaacatggt 55860
   gaaaccccat ctctactaaa aatagaaaaa ttaggtgagc atggtggcct gcgcctgtaa 55920
   tcccagctac tcgggaggct gaggcaggag aaacatttga atccgggaga tggaggctgc 55980
   agtgagctga gaacatgcca ctgcactcca gcctgggtga cagagtgaga ctcttgactc 56040
   aaaaaaaaaa gttatgcatc agagaacaga tcctttgatg ccctcctctg ccctgaaagg 56100
   tttttggggg agagtaataa gtatcacaac aagatatgac ctgagaacag atttcccaga 56160
   taggacatga tccatgtttt aatatggctt actgctgttg cttcatagtg tgaagcttca 56220
   gacacttctg aaaacccttt cagaaaatcc cagtcgcccc atactgatga ctaatctcaa 56280
   ctaaaacagg gcttcagcca gtgtgaatgc cactaatgcc accaactcac ctttgctttt 56340
   ctgtagggtg tgcacctgta tgtacacatt cagcttttcc gggattaacc tctgagttct 56400
   ggtttgtctt tcagttgacc atagtcggat taaactacat caagaagata atgactatat 56460
   caacgctagt ttgataaaaa tggaagaagc ccaaaggagt tacattctta cccaggtaag 56520
   cagattgtct gaattttcta tttaatgtca atttaagagt ttgagagtgc tgttatccac 56580
   acctcaaata aaatctgcca catcctttag aaggtcagga tttcagcata ccaaaaagca 56640
   gcaaggaagg gggaaaaatc atccttcaaa ggttcagttt ggttataagg aacgctaatc 56700
   ttttctggga agcataagat gacattgctg gaaatgagag cttatagaaa acaacattaa 56760
   aatgccagag ttgcctgtgt ggtctgttgg cagagacagc agagccatgg ctggaggagg 56820
   gtctgtacct gtgttgcttc cagaagtatt tgtcgtagag cacttgtgat ggcaaatcta 56880
   agaacgttag cagtagacca ggaatctctg tccagagcca ttcagagtag ctcagcatgg 56940
   ttctcattct ttggccagaa gaaaggcatc attggatcat gtgaacaagc atgaaaaatg 57000
   acttaaaatt tctgttggct tttggcatct ttatggaaac aaaatcctga aagtggttta 57060
   ataattgagc ctcttgtaaa acactcagtg gcatgtgacc aaaagggtat ctgggaaaga 57120
   ggataaaaag agtttctttt taattaatct tctcaagtct taacttgtta cctgtaagtt 57180
   ggtctaaaaa gactgggttt cttattttgt ttttcatcat aatttttgtt tctcattcca 57240
   tgtcagcttt cagtcttata tggctttagg ccacagggcg attttgaaca tttgtaattt 57300
   tgcttaataa ttaggaaatt aaaattctgg ggaagacaga atgctctatg aagaaaggct 57360
   gctttgagca aggagctagg tcagggcgcg ttcaactgag gcctttcttc actgcctttt 57420
   tgtcttgtcc cagttcctcc ccatttatga ctaaaatcag cccagatgct tctcgtcatc 57480
   tgggatgcag agcatcagcc cagctgtgtt cagtcctatg gggccattga gtaagttctt 57540
   ggtgcatgga tacagggcag gcctttacca ggccctgagc ccctggtcct cccagcacct 57600
   ctggggtatt taggggaggc tgatggggga gggggttgat aaggcgggag atgtctgggg 57660
   atgaggttga ggcaaaagtg acttcttgag gactttgctt tttggagaag tcaaatttcc 57720
   tacttcttga tttcagccct tcaactctgg tatggagtca ggaagccctt taaatacctg 57780
   ttgtcgggtg tatcatgtca agtgttgcat tagcaaatga ccatgtatcc ttgtgctact 57840
   gtcctgccta ccccgcatcc tagcgcttcc ttgggacatg agaagctctg tctggtttgt 57900
   gaggtggcac tggggatgtt gagaaactgt ttacacagtt tccctttgcc ctggggattt 57960
   actaaaggag tcgaggcagc ctgaccccaa agcatcaccc ctggacacta tgaccgaaac 58020
   atttccccag tgcccaaacc aagaacaccc ttcccatttt tttttcagtg gtgttcatta 58080
   tgtaataata caagtctctc ttctcatttt ttaaaagtca gaagtacaga agagcagaga 58140
   ataatgtcca aggggccctc cttcacctcc cccgtgcagt gtcagctaag tgtggtgcgt 58200
   gtccttgcag atcttagggg attgtgatcc ttcagaccat tctaaactgg ggtggtgctg 58260
   ggagttaggg aaggcatgaa gggagtagtg gagagctgca gtgactgggg tcttcatgcc 58320
   agggtggaga atgcaaggcc caggtggcca gccatgtgcc acgggatttc tggctgccaa 58380
   gagctgttta tctgttcact ggggagggaa gagttaaatg tggtctgctt ttctccgagt 58440
   cccttcagca cagggagtgc tgacttgtct tgttcaggta gtaagttcaa gatgagctca 58500
   ggaaagaaag tgagaggaca ctgagggcta gtggttgagc caagtgtgat gggacttaaa 58560
   gggagaagat ttaaagaata aggagcttat gggccgggga cggtggctta cgcctgtaat 58620
   cccagcactt tgggaggctg aagcaggtgg atcacttggg tcaggagttc gaggccagcc 58680
   tggccaacat ggtgaaaccc cgtctctact aaaaatacag aaattagctg ggtgtggtag 58740
   tgtgcacctg taatcccagc tacttgggag gctgagacag gagaatcgct tgagcccagg 58800
   aggcagaggt tgcagtgagc caggattgcg cccctgtact ccagcctggg tgatggagcg 58860
   agactctgcc tcaaaaaaaa ttaaaaaaaa ataaagaggt taggtgaaaa tagatgagaa 58920
   tggaaaccat gagaagaagt gatgctggcc aaggacatga caggttctga tgtggaggtg 58980
   ataggcaatg tctcttccag ccactgctaa taattgagac aaactcaagg cattcatacc 59040
   ctgtgtccag taaacatctg tgcccattgc caggtgagct ggattgaaat gggccagctg 59100
   ctcagcagac accctcatgc cccagtgact ctgttcccct tgggccacct cattgaccat 59160
   ttatgtttct acatctccta agtttgttgg gccaaggatg gaggctgtct gccgtcaggg 59220
   tcctcattgc tgatggtagg aatagttgct gatgtttcat tggatgttgc tgtattctag 59280
   ggactgtgct aagtacttta tagaaatgaa catacttcat tttcacagtt ttatgaatag 59340
   ggactattat tagtcaagta agcgatgggg aaactggggc agggagcgat gaagtgactt 59400
   gcgcaaggtc acaagatgat gtgattggaa ccaagagaag tgttgtggtt ggccacgccc 59460
   ccacactgcc tctcatctgc accaaggagt tttgtcccat agcccaaggg ccttggggac 59520
   gaatctcagt ggaggccctt agcgggcctg cctgagccag aaagcagaat cggcattttt 59580
   ctgtccttgg ttggcccagc cctgaactga gatgcggaaa tcgcctttcg ctgcctggta 59640
   gaaaatggag ctgcagttac tgaccaccag gcagagagag gtgggtccct gtcccagcct 59700
   cagccaccac tctgcctaag ctgtggggac tgagggcgct gtcgttagct gactgcagaa 59760
   ggtgagcaca cgctgtagca tgttatgttt cagatgtcac atgttgtgtt attgtgtctt 59820
   tgcagggccc tttgcctaac acatgcggtc acttttggga gatggtgtgg gagcagaaaa 59880
   gcaggggtgt cgtcatgctc aacagagtga tggagaaagg ttcggtaagt ctcggcttca 59940
   tttgctgtgt atgtgatcat gcataccact ccatatagtt accattttcg tccagatttt 60000
   taaattattt ttcttgcctt tgtatttcct ttacgtagta tttttattta aaaaaattaa 60060
   aacagcagca tataaatgca tgttggttgt caaccagtta atgaagtgaa taaaagggag 60120
   gaggcggaag aactgcacgg acctcttcgc ccccgccttc tcctgtgtgg tgcgtgtggc 60180
   gctccgccca cctgtgctgc ctgtgcggct ctcatcacag tgtggagttg tgtgtggagt 60240
   tatggagacc tgcttttatc ttgaaaagca agttcttagt gcatcttcat ggtgtctgat 60300
   tttttggctg gtgagagtgt ggctacctct gcggagctgt gggagcggct gactagatga 60360
   gatttgcctc cattcagtac ctagactctt gccctgccac acctcttcgg agtgagcatt 60420
   gacttcagga tgtgtgtcat tctaagttcc tgcaactttt caaacacccc tcgggctagc 60480
   gtgtggctgc acggtgtcca tttgtgcagg ccaccactcc tcttgcatct gggtctagcc 60540
   acctctcctt cttgacttac catagttcat tttgtaccat gctttcagaa tgagctttct 60600
   caaatccaag tctcaccacg gttcttccca gctgaaaacc cttgtgcggt tccctttgcc 60660
   tcacaggata atacatggtg tggcttacgg aaccctgcag gtctggccct aggcccctgg 60720
   acacagacct ctcaccactc ttggaacttt agccaggaca aagttttctg tttttagttt 60780
   cttaccatgt tctctgggcc gaggagtccc agtgcccacg ttcatcccac ttgcaggcac 60840
   ccctggacgg ctgcccccag ctccccaact gcctgcattc tcccctgccc tcctcactct 60900
   gttggaatag ctgagaatag ccgatttctg ggcagccggc ctcctgtgta gactgtcctg 60960
   tgtagactgt cctgtgtaga ttgtctgtgt agactgtcct gtgtagattg tctgtgtaga 61020
   ctgtcctgtg tagactgtcc atgtaaactg tcctgtgtag attgtctgtg tagactgtcc 61080
   tgtgtagact gtcctgtgta gattgtctgt gtagactgtc tctgtagacc gtcgtgtata 61140
   gactgtcctg tgtagactgt ctctgtagac cgtcgtgtat agactgtcct gtgtagactg 61200
   tctctgtaga ccgtcctgtg tagattgtct gtgtagacca tcctgtgtag accatcccat 61260
   ttagaccatc tgcctgtgca ggcgcaggcc agtgttcagc agggccacag gctcctcggc 61320
   ctccctgccc tcgctgctcc ccaacactgc caaccctgct gcggggtcca ggaggagatg 61380
   ggctgaggat cgtggagacc agcaggagcg tgtggcccag gagcagggaa ctgggtgtcc 61440
   ttgggccttg ccaggtccag gctcagctag gacacggctc tcacagctgt cctggttgcc 61500
   tccggccaca gaagaaggtg agggctccag agaggccacc tttccaaaaa aagcacagtc 61560
   atggccctag aatgtaaaaa atccaagtgt taagaaggaa cacatcaaag gaaacttcag 61620
   cagtgaaaac ttgaagcatt aaccacgaag cctctgcctc caccacacac aaagaaacgg 61680
   ctttagttac tcgcagaaag tcttcctctt aggacagcgc gtgtttaaaa tcataggggt 61740
   ttggtttgtt ttgttttggg gttggggttt tttgggggtt ttttaccctt gcctactttt 61800
   taaaaaatga aagtgtttat ttgcccaaca ataacagaca gggagcttgc ctaagtgttc 61860
   tgttgatgat ataatgtatc ttgtcttaga aaaaaacttt ttcagtgaaa ggtggttttt 61920
   aaattttttc ttccctcctt agtagcttga ttagtaaaat gtgaagttac aaatgtgaag 61980
   caaaccccca cccttcacca ctagtcagca attttgagta aagaaacaaa gcatcaggtg 62040
   ctcacagcac acactgtctt agagggaagg ggaagcctgg tggcctgtgg aagccttcag 62100
   catagctcca tctgcaggct tctgacccct agcactactg acacttgggc tggatcattg 62160
   tctgctaggg atccgggcag ggagtggctg tgctgggcgc tgtaggaagt ttagcagcat 62220
   ctctggcctc tatccaccag atgccagtag caccccctcc ccagatgtgg cagtcagatg 62280
   tgtttctgtc tagactccag actttgtcca acgtcccctg gtaggccaaa ttgcccccgg 62340
   ttgagaacca ccgctctaga tggtattgag ggttgggaat tttaaatcaa gacatttatt 62400
   cagaaattac cagatatagt agcatttgct tcttatttat ttctttgttg ctaagtgttt 62460
   ggcaaaacct ctttgctgtg agcacaaggt ttgctttagc aattgttgtc acattacagc 62520
   aaggagtggt gtccagcgct gtagttatgt atttgagcag tgtccagtgc tgtagttatg 62580
   tgttccagcc tcaccaggcc ctgtgcttca ttgtctccca ctcaagactg accacaaatg 62640
   gcccacagat ccactgtgac aacctttccc tttgggttac tgtggtggca tcgagaacat 62700
   ggctggttgg ctttgctgta gtttactgtg ataactgtgc cagcagtccc tgctttcctt 62760
   tgttaagtat cccattccac tggaggatta cttgggcgtg cagattggca tgaaaagcaa 62820
   tgtatggttt gagattgtta aagtttcttt gggatcaaca ttttcaattc tgtatcagca 62880
   ttatccctcc cagagggctg gctgggagaa atcatgagaa gttacagtat cttatttgct 62940
   cagctaatct aattataaat gatccacaca gcttgtggta aaaccagctt ttggggagtt 63000
   ttcatttaat gcatacttgt cttctgattt ccttccttca ccaaatagtg taggatgctc 63060
   cctcttattt ttggcaaaca tgcctgttat cttttgggac cctgggcttc ctggaaacca 63120
   gttatgcaga agatgattgt gtgtgttaga ctggggtcat ccagatggct agagttctca 63180
   ctggttctgt ttaaggattg actttagaca cctcagtgta ggctgcacca tggcgtaagg 63240
   gttgggattg ttgtttagaa gggggaagta agcaaggtga gtttaattgg ccattgcaga 63300
   atctcacccg tatctccctc ctgaaatcct cactaaagct gccgtttgct ttcaggtgct 63360
   ttcatgcaca agacactgca ttttgtatca cagggtccat ataattcatt tttctctcgt 63420
   acttagttct ctgtgttaag aattacttac ttagttctct gtgttaataa tttttggcga 63480
   aaccaaatta cccgtcacag ggttactgta gatgtctttc ataggttttc caaacaccac 63540
   ttgcccactt gtttgggaag gccccaagga ctgtttaaca tctgccttca tggtggaaac 63600
   agcaactatg agagatgcta gcatgttggc actgccatgt tcctctggta ccagcccaag 63660
   ataggactca atttgaggcc tggtgaagta ctgtgttcta ataaaaatcc atctactttt 63720
   catggccgta tatatcaatg taatagggta actggaaatg tgatcttgtg ccttttaaaa 63780
   attttgtgtg tttaaaacaa aaatttctat tggaaatgac agagcatagc ttgttgctgt 63840
   agacacctga gagtccttaa aaataaatat tgggttattg acacttagtt gcatgacaga 63900
   attcctcact tgtacagttc caaagtctta gtctttaccc agattacaga gggttattaa 63960
   gcattaggtt tggttttgaa agtgagtgct tgctgtctgg aggtgagctt taagactcgt 64020
   ctgccctgct tatgagatga ggaagggtgg cctcttcctc ctgcatttct gttcttcgct 64080
   tccttctctg tctgctcact ctgtggaatg cccaccccag cacgggtggg gtggaacctg 64140
   tcagatcagt ctcttgtttc tggggtcttg aggcattata agatctagtt gttagaagtg 64200
   tgggattaat tcatcttttc acattcttct aagttcctgc ttttagctgc cacacccact 64260
   ttggctaagt gggggtcttg ccatgtaatt agcgcctcca tgccaagtgg cagaattgct 64320
   tcaatggtga cagattgtcc ccattcaaga gttcactttt ggcaactcat cattgatcca 64380
   ggaaggtgac atggatgaaa ctggctaaga cttcagacag gcttgtgtcc agactcttga 64440
   gaaagctctg ttggcttctg gtctggcact gtgaagtttg ctgtgatgct ggcaccacaa 64500
   cctggtgttt cctaatttgt ttctcccaca ttttgctttg gttttgtctt ttgggcagct 64560
   tccagctcca gtagagcagg accaataggc atttgtggtt ctatattcac cctcctcacg 64620
   tgcttcctgg ctcctcattg cccccagatg atgccacagg tccctgggcc tgctgccagt 64680
   cgtctgtgat ctgggcctct gctggcccct tctccagctg ctcttttcag cctcttattt 64740
   gcagtcactg cctaggaaat cctagtcatc cttcaaaacc tgcctcttgc acagagcttt 64800
   ctctgatctc tcttttctgt aaccttggct gacctgaaac atttccctct tctgaattcc 64860
   tgctgcatgt ccgtagcatt tccccctcag ccctccccca tagtccacct tgtcactgct 64920
   gggcacagca gtgtcttctg acagacagct ggccctgaag tggttccctt cacccacacc 64980
   atcctttgcc ccagaggagg tattgagtgg gtcagtgcac gtgaactgcc agtgtcattt 65040
   gccaaagagc tgttgacaca cgctgacatt tcttttgctg aaaatcataa gggctttgag 65100
   cttccctctg tccaggcaca tggtcaggct gacccggtag ctctgcccct gctgacctgc 65160
   catttttgtc cacaacagtt atccatgagc agaaacattt gtgtaactga ggcagaaact 65220
   tagttcaagt aaaatgtcac taaattcgag tcagtttttg tcttagaccc taaatgaaac 65280
   caaattttca taaattttct tgttttaaag aaaaatttaa tgagctacat ttaaactgag 65340
   aacatcagat agtgtctgag attatcaaaa tagaacatca aaagtatttt tctgaatgaa 65400
   ctgaaccaaa ccagaatgaa agggcaagcc ctggggagcc tgtctccaag ccttctctga 65460
   aagggagtct gtatttggtg ataactgctc agcctctcca aagggcctca cctgctgtct 65520
   ctcccagttt tatttttaat tgcctgtgag ttttctgtgc agggtaaggc acctacattc 65580
   tatgccagca gcctgatcag gtcctgggta atgtttgaaa tggctacaca gaggagtttc 65640
   aaagcctttt gttcaatctg gcttcacctc gtagacggtg agaaagcgtc agagccctgc 65700
   aggatcccgt tgccacgttt gaccggggag ccgatgggtt tggaagtctg agccctgtct 65760
   gcacaacctg ccccggtcag cagcttcgtg cccccacccc catctcccca tgaggcaggc 65820
   atctgtgctg accatggctt ccatgttcag aaacccccag gcctttgagt tatcatgaag 65880
   cttgtgggat gtgctccaag cctcctgcca tagaaaaact gccatattgc tcacaataat 65940
   tcactattat ttgtttcccc agttaaaatg cgcacaatac tggccacaaa aagaagaaaa 66000
   agagatgatc tttgaagaca caaatttgaa attaacattg atctctgaag atatcaagtc 66060
   atattataca gtgcgacagc tagaattgga aaaccttaca gtgagtatag cacacacttc 66120
   agcacttcag gcggctactg gttcacatgc ctcttccttt atcccttggg tgatattacc 66180
   taatgtcagt gttcctggct tttgtatacc ccgagcaaga tgtggtttgg gcactgtggt 66240
   gagcggagct tacttgtgta cctaccaagt gcccagggag ggtggaggcc acagtgctct 66300
   ctctgacctt taacaacagt taacaccagt tcttagggaa aggagagttt cttacccaaa 66360
   agactggttc ctgcttgtgc agctgcagag ggactggagc ggcagcctgc aagtcccagt 66420
   gaagcatgct gccttctttg tggtcctcag tcttcgagtc tgaagagagg gaagaagggg 66480
   tataggggct cactccagtt tcatagctag tgaaagtttt ctgggccagg tcttgggttt 66540
   ttttgttgtg ggaagagttt ataacaccag ctacttgctt ggtaaaagtt ggtcttggaa 66600
   catggcaagg cattgtggca agcagcactg ccgctgaacg cgctgctcct ggggctttgg 66660
   aataattccc ctggatccgt aacttggggg tgttcatgtc attctgggga acagtggagg 66720
   gagtgcgcgg cagcacctgg gggcaccagt gaagagtggc cagccaccaa cctctagaac 66780
   ctaactgggg tcgaatcctg gccccacctt actagctcat cacagtgtct ccgtttcctc 66840
   ttctgtcaaa ctcaggtttt gcgagggttc tgggaggtcc tatacgggaa gggttagcag 66900
   ttaccatggg tgtgtagcac gggctttatc tgaagggaag gtggagccgt agggagacca 66960
   tgtggagtgg ggctccaggg ctgtgtgggt ggggagggat ctgcttctgg gttaccccat 67020
   gcctcccctt ctcaagtact actttttaat catcatggct cctgccattc atttcatagt 67080
   tgatgtaagc caggtgcggt ggctcacgtc tttaatccca gcacttgggg aggctgaggc 67140
   caggaggatc actcgaggcc aggagttcaa gaccagcttg ggcaacatag tgagaccccc 67200
   gtctctacaa aaaaacaaaa acagttagtc agacatcgtg gtgctcccct atagtccagc 67260
   tactcaggag gctgaggcag gaggattgct tgtgcccggg agttcaaggc tgcagtgagc 67320
   tatgcttgca ccactgcact ctagcctggg tgacagagca agaccctgtc tcaaaaataa 67380
   ataaataaaa aaaatagtag aagtaagatc tagaatgtag cacaggttac caggacgtag 67440
   gcaaggggtt cgggctgcct ggctcttgag gatggtagca gtgcagctga tgtgagtgct 67500
   ttctgccctc tggtggtgac cgcgccggag tcaccagccc tgccatagcc ctgatggggc 67560
   agagggttct gagtacggtg gatggaggtg ctttctggaa gattctcagg agtaacatgg 67620
   gcagtgtgtt ggaatgtgct agaggattta tgcagtagcc ttttaaaaga atgcttttta 67680
   gcat t t gcaa gcct gacat t aagagt gact t ct gggaaac t at t t gct t g t t gagggaaa 67740
   ctgaatttca acagagcaga agagctgtgc gctttttgct tggcagagtg aatacagcca 67800
   gct cagaggt t t t gat gt t a ggat ct gt t t gct ccaacag act t t gt t t t t aaaaggct t 67860
   t t ct cagcca t agct gt ct g t t ct agcaca aggct ggaat gagt t cct t g t gaaagaggt 67920
   gagcaggtgt gagggagggt gtcagtgggc ggtaacccac accttcaagg attaaaggaa 67980
   aacttgcatt tggcatgctt gcttcttatt caattttaaa atacatttta acggccgggc 68040
   acggt ggct a acacct gt aa t cccagcact t t gaggggct gaggt gggt g gt t cacgagg 68100
   ccaggggttc aagaccagcc tggccaagat ggtgaaaccc catctctact aaaaatacaa 68160
   aaaaaaaaaa aattagccgg gcgtggtggc gggcacctgt aatcccagct actcgggagg 68220
   ctgaggcaga gaattgcttg aacccaggag gcggaggttg cattgagccg agatcatgcc 68280
   actgcattcc agcctgggcg gcagagcaag actctgtctc aaaataataa taataatttt 68340
   ttaaaaatac attttaagtc cttttcttcc ccacctgcct ccacccacca aatagaagag 68400
   gtatttcttc ttctttaatg tcattaaggt tatatggata ccattttcta gagaggaaag 68460
   aat gat ggaa t t gcct agt g t gagt ct agc aat t at cct a acat acacaa at t t ct cct t 68520
   gt t ct gt gcc aagat act gt at t t aat at t t aat gaacat t aaat at t at t t act agt gt 68580
   at t t aat ggc t gaggcaggg t t aaat at gt at t at t t t ca t cccagcaga gt t gggggag 68640
   gt cct agt aa ct at gccat g agct ct gt ga gggt gaggt g gt gt ct t t gc ccccgcct cc 68700
   ctggcacagt gactggcaca tgattggcat agtgtggaca ttcgtcaagt gaaggaaggc 68760
   at cat gagca gat ct ct ggc ct gaat cct t ct gccat cag ct gct cgcca ggt ggccct g 68820
   gcactgggcc acagggaaac tctccaggct ggtatggttc ctgtctgtgg ctgtcttccc 68880
   gggcccat gt t aggagact t t cact t ccag agccct t t cc ct ct cagggc ct t gct t acc 68940
   aagt gact gg t t cccat t t a ct aggagct c t t aggt cat t gaagat gt t g cgt act cccc 69000
   ccagtgaggg ctgccttttg atcacagccg ccagaagcct caaggaagga gcagagctgg 69060
   aaacagacgc caggccattg cttctgttcc tctggggcag acccagccac ggaagagaca 69120
   ttctgggaca agggctgggg tccacctttc aaacgtgtct gcagcaggct ctcagcatgg 69180
   act ct ct gcc t ccaaacat c cacct cct ca t cggaaaat g gat gggagt g cct gcct gga 69240
   gcagctggtg ggagagcgca gcgccagcac gtaggacaca ctcggttcat gggctgatgc 69300
   cgt t cgcat t gact gcct ct t cagct gggt gt t gagccac acct t ggagt caccagt ct t 69360
   t ggagaccaa gt ct gct act t t t t t ct ct a aagt gacaat cct ct gaaac ct ccagat ca 69420
   t ct t gaagcc cccgt ct gaa agt t gcccag agccagt gcc t cacct gct g t t cct t gt t c 69480
   act t t t t cac gggaggcct t gcagggct t t at gacaagat t t t at gggt g gct gcccagc 69540
   atcattgtga ctcgtgagac agagagaaac cagttgtaac catgtagaca gtggaagtga 69600
   t agggagaaa agaggt gagg ggact ct t ca at ccgaaggg aaat gaagt c t aagcaggcg 69660
   caccctgcag gttcagtgtc aagcccaggg cctggcccca gggtgtggta tttgttgact 69720
   gggt gt gt gg accct gggag aaagt ct gag aat gaat gt t cct ct t agag gt agagagt g 69780
   gaaggt gact ct gt gt gt ac t t ggaat t ag t gat t t ct gt acagat gat t ct t t t agaat 69840
   cat cat gagt at t t t t ct ct t t cagaccca agaaact cga gagat ct t ac at t t ccact a 69900
   t accacat gg cct gact t t g gagt ccct ga at caccagcc t cat t ct t ga act t t ct t t t 69960
   caaagtccga gagtcagggt cactcagccc ggagcacggg cccgttgtgg tgcactgcag 70020
   t gcaggcat c ggcaggt ct g gaacct t ct g t ct ggct gat acct gcct ct t gct ggt aag 70080
   gaggccctcg cgggtgccct ggggagctcc tctacctgct ctgctgtgat gttttttcct 70140
   aagt agaaac t gaagcgct c ct ct t ccaaa at acagagac t cact gt gt t agt ct gt t t t 70200
   t gcgt t act a at aaaggcgt acct gagact cggt aat t t g t aaagaaaag aggt t t aact 70260
   ggctcccggt tctgcaggct gtacaagcat ggcaccagca tctgctcggc tcctggggag 70320
   gcctcaggga gcttccagtc atggtggaag gtgaagggga gcaggagcaa gagatggggg 70380
   aggtcccaga ctcttaacca gctctcttgt gaatgcattg cctcagggag ggcaccaagc 70440
   ctttcatgag ggacctgtcc ccctgaccca gacacctccc acccagcccc acctccaaca 70500
   ctagggatca catttcagca tgagattggg aggggacaga catctaacgg tgttattaac 70560
   gt t gccct t g agaat t ggac ct ggct gact t at at ct cct ct ct ggct t t cagat ggaca 70620
   agaggaaaga cccttcttcc gttgatatca agaaagtgct gttagaaatg aggaagtttc 70680
   ggatggggct gatccagaca gccgaccagc tgcgcttctc ctacctggct gtgatcgaag 70740
   gt gccaaat t cat cat gggg gact ct t ccg t gcaggt cag cat t gcct t t gt t t gaat cc 70800
   aggtgtgacc attttaactt ttttgtcttt gaaggaggct gtcagttgta aaagttcaaa 70860
   caccgt ct gg t gt cagggga aat agct acc ct t cat gt t t aaaat agct a gaaagt t gt c 70920
   aaaat gt t ca ccat gt t gca ct t t gt gcct t t gaagt gct cacat agaga gcat t gat ag 70980
   gaagacgaga ctttattttc aaaagatttc atcttccaag tacatggctg cagccctgag 71040
   aggccgagag cccctcgcca agccgtcacc tctgctcatg caaagggatt tcctgacaaa 71100
   ccagccgaag tgaacactaa taggacttcc tcttgctgct ctttcaagga tcagtggaag 71160
   gagctttccc acgaggacct ggagccccca cccgagcata tccccccacc tccccggcca 71220
   cccaaacgaa tcctggagcc acacaatggg aaatgcaggg agttcttccc aaatcaccag 71280
   tgggtgaagg aagagaccca ggaggataaa gactgcccca tcaaggaaga aaaaggaagc 71340
   ccct t aaat g ccgcaccct a cggcat cgaa aggt aat at g at t gggt ccc agct t gt t gg 71400
   ggt gagggga aat gact t t c t gt t ct agaa acacacgct g gt act gaaac cct gt ggat g 71460
   cagcctcctg ttggcaagca gcgcttccgc atccttgggg aacagggcgc gtggaccaca 71520
   gccact ccac t cct ggct gc t ggaggt ccg gt at t gggca cagggt ggcc gcaggacat g 71580
   agccact t ct gt gggct t ct agt gccacct t gt ggt gct t gt t ggaat ga ggggct cgga 71640
   gccaccgagt agggtttttc tgccccccct gacgacagcg ccctccccca ggtttccgga 71700
   cagt cct gaa at gt gat gt c caggct t gag t gccct cagt ccccacagt g gt cct t t ggg 71760
   gaat gt aacc t t t t t t at gt ggt ct t gat t aaat cccat t t t act t cct t gcaggt t aac 71820
   aaccat t at t gagt acct at t gat at gt gt ggt gt act ga gt t aact aga acat gt cccc 71880
   tggtctgtgt tctagaccat cttgctggga aaaaggcaga cccaaagcat attttggtgg 71940
   gggcccat gg acagt gat gt gat agaggt g t ccgct gagg t ggt caggga aggct gct t g 72000
   cagt aggt gg ccgt gcacgg aaagt t t gca gaat gagcag gt gt t agt t c cagct ggaga 72060
   t gact gccgg ct gt gccct t ggt acct gct t t ct ggaggg aagt t t t aag acgt gt gcat 72120
   acttgaccca gcagttgtat acatggagaa atttactttg cagcaactct caaaacaagc 72180
   gt gt aaagat gt gt at aggt agt t gt gt t t gt t gt ggcat t gt t t gt agt agt gaaaaat 72240
   tagagacagg ccaatgatat aaccagggac ctgatcaatt atgttctctc ccggtgttgg 72300
   gat at t ct gt agct ct t aaa gaat gagat c t gggt gt act gat gt ggcca gacat t gcaa 72360
   t t gcagt aca t gagaaggca aat cat acag t agt gt gt ac accagt gagt cct ccagcca 72420
   gat aaat cct cacagt gacc agt cgcccag gcacct t gt g aaccct accc t gggt gt ggg 72480
   t gct at ct ga agt acct ggg ggagggggt g acaagt ggac t t caggct ga t gt gggccct 72540
   ggcctggccc tccctccaag cagagggggc tggctcgctg gaaggttaac atcatccaac 72600
   t ct gt ct aca cgt ggct t gt t t t t t cct ag aat t cct gcc acaat agcag cat cct t gcc 72660
   at t cat t t t c t ccaaagt ga gt aacccat c t ct gccct ct gat t cct cag cat gagt caa 72720
   gacact gaag t t agaagt cg ggt cgt gggg ggaagt ct t c gaggt gccca ggct gcct cc 72780
   ccagccaaag gggagccgtc actgcccgag aaggacgagg accatgcact gagttactgg 72840
   aagcccttcc tggtcaacat gtgcgtggct acggtcctca cggccggcgc ttacctctgc 72900
   tacagggtat gtttccactg acagacgcgc tggcgagatg ctcgtgtgca gagagcactg 72960
   gccgct agcc cgat ggt agg at t cagt t ct gt ggt gcat c t gagccagt c t cagaagaaa 73020
   cagatcaaag gtttttaaag tctggaactg tggaagggct aacaagagaa ttaaggatcg 73080
   at gcact ggg gt t t t aagga gccct ct ggt cccaagaat a t aagagt ct a at ct cagggc 73140
   ct t aacct at t caggagt aa gt agagaaaa t gccaaat ac gt ct gt t t ct ct ct ct ct t t 73200
   t t t t t t t t at t cct t t gt t t t t ggaaaaaa at agagt t ac aacacat t gt t gt t t t t aac 73260
   ctttataaaa agcagctttt tgttatttct ggaacaaaaa aaaacaaagt aggcacttat 73320
   gaaact t t ct cat accct t a ggt gat gt aa t cagccat at aat t t at at t t gat t t ccca 73380
   gggaaggaat cccaaacttt tacgaatgta aactcccttg gagaagaggg ttaggacgct 73440
   gttgcgctca agcccccctc agctgtgtgc acactgagcc aggacagggt ctttgagctt 73500
   tcccactata agaagaacag caacaaaagg ccgtctagaa aaacagaacc tgcctctgct 73560
   t ct gct cagg gt gt ccccgc t gggt t t cca t t gt cct t t c t ccat t gct c cct cct gt ga 73620
   cagccat ct t gct cat gt ac cagccct cat caccccat cc ccat aaat gg gt gt cct cga 73680
   ggcctctgcc tgggggtcag aggtcaccac agggtggcca ttggcatgtc aacccgctgt 73740
   t aat t cagag aagt gggct c cacct cat t g ggagaagt gc cat t t cagca gaaat t caca 73800
   cgttagacgt gtgttgctgt taagtaaggg gaagagagag gactagcctc agagctctgg 73860
   ccat ggaaat gacct cct aa gact t t t t cg t ggt t t t aaa t at t t t acct ct t t ccaggt 73920
   ggcat ct gag t acat cagat ggt t t t gcaa aat gcaaaca at t t t t t cct t ggggat gat 73980
   ttttggggag agggggctac tgtaaaaaat aaaaccaaaa ccccctttgc tccctcggag 74040
   gttgaagttg ccggggggtg tggccggggt catgcatgag gcgacagctc tgcaggtgcg 74100
   ggt ct gggct cat ct gaact gt t t ggt t t c at t ccagt t c ct gt t caaca gcaacacat a 74160
   gcctgaccct cctccactcc acctccaccc actgtccgcc tctgcccgca gagcccacgc 74220
   ccgactagca ggcatgccgc ggtaggtaag ggccgccgga ccgcgtagag agccgggccc 74280
   cggacggacg ttggttctgc actaaaaccc atcttccccg gatgtgtgtc tcacccctca 74340
   tccttttact ttttgcccct tccactttga gtaccaaatc cacaagccat tttttgagga 74400
   gagtgaaaga gagtaccatg ctggcggcgc agagggaagg ggcctacacc cgtcttgggg 74460
   ctcgccccac ccagggctcc ctcctggagc atcccaggcg ggcggcacgc caacagcccc 74520
   cccct t gaat ct gcagggag caact ct cca ct ccat at t t at t t aaacaa t t t t t t cccc 74580
   aaaggcat cc at agt gcact agcat t t t ct t gaaccaat a at gt at t aaa at t t t t t gat 74640
   gt cagcct t g cat caagggc t t t at caaaa agt acaat aa t aaat cct ca ggt agt act g 74700
   ggaatggaag gctttgccat gggcctgctg cgtcagacca gtactgggaa ggaggacggt 74760
   t gt aagcagt t gt t at t t ag t gat at t gt g ggt aacgt ga gaagat agaa caat gct at a 74820
   at at at aat g aacacgt ggg t at t t aat aa gaaacat gat gt gagat t ac t t t gt cccgc 74880
   t t at t ct cct ccct gt t at c t gct agat ct agt t ct caat cact gct ccc ccgt gt gt at 74940
   t agaat gcat gt aaggt ct t ct t gt gt cct gat gaaaaat at gt gct t ga aat gagaaac 75000
   t t t gat ct ct gct t act aat gt gccccat g t ccaagt cca acct gcct gt gcat gacct g 75060
   at cat t acat ggct gt ggt t cct aagcct g t t gct gaagt cat t gt cgct cagcaat agg 75120
   gt gcagt t t t ccaggaat ag gcat t t gcct aat t cct ggc at gacact ct agt gact t cc 75180
   t ggt gaggcc cagcct gt cc t ggt acagca gggt ct t gct gt aact caga cat t ccaagg 75240
   gtatgggaag ccatattcac acctcacgct ctggacatga tttagggaag cagggacacc 75300
   ccccgccccc cacctttggg atcagcctcc gccattccaa gtcaacactc ttcttgagca 75360
   gaccgtgatt tggaagagag gcacctgctg gaaaccacac ttcttgaaac agcctgggtg 75420
   acggtccttt aggcagcctg ccgccgtctc tgtcccggtt caccttgccg agagaggcgc 75480
   gt ct gcccca ccct caaacc ct gt ggggcc t gat ggt gct cacgact ct t cct gcaaagg 75540
   gaactgaaga cctccacatt aagtggcttt ttaacatgaa aaacacggca gctgtagctc 75600
   ccgagct act ct ct t gccag cat t t t caca t t t t gcct t t ct cgt ggt ag aagccagt ac 75660
   agagaaat t c t gt ggt ggga acat t cgagg t gt caccct g cagagct at g gt gaggt gt g 75720
   gat aaggct t aggt gccagg ct gt aagcat t ct gagct gg gct t gt t gt t t t t aagt cct 75780
   gt at at gt at gt agt agt t t gggt gt gt at at at agt agc at t t caaaat ggacgt act g 75840
   gt t t aacct c ct at cct t gg agagcagct g gct ct ccacc t t gt t acaca t t at gt t aga 75900
   gaggt agcga gct gct ct gc t at at gcct t aagccaat at t t act cat ca ggt cat t at t 75960
   ttttacaatg gccatggaat aaaccatttt tacaaaaata aaaacaaaaa aagcaaggtg 76020
   ttttggtata ataccttttc aggtgtgtgt ggatacgtgg ctgcatgacc gggtgggtgg 76080
   gggggagtgt ctcagggtct tctgtgacct cacagaactg tcagactgta cagttttcca 76140
   acttgccata ttcatgatgg gtttgcattt tagctgcaac aataaaattt ttttctaaag 76200
   aacatgaatt tggggtgctt cccatttttt tctttgctta atagagctaa accaggatga 76260
   gtaactcctg tttctttcta tccctgctga tgtgaaacag atgttgtcaa tcagctgggg 76320
   ttagagtttt ccacttctaa gaattaacct cagcatccct gcattgccag caccctcagg 76380
   ctggagcgct ttccttgact gtgagcttgt tgaacacctt aggcctcagc ccatttcctt 76440
   cccaaattga cgctttgcct gtgtagggcc ctcagataac ttaacaaact taccagtgtt 76500
   gtttgaagaa cagtgttttg agttgtaatc tcaaaaccat atcccttacc caattacctg 76560
   taagacacaa tggttaccac atctcagtac gtaaagtcca cttgatatag aattgactta 76620
   gaaataagac agattagtat agtttttcat ttgtgtacaa aattaaacaa tgtaaattcc 76680
   ccccaaagtg atttttttga ctttttgaag taattttgga cttgcaaaat gttgccaaaa 76740
   tagtacgaag agttccccag taccctcgaa gtttcctcga ctgtttcaaa gctggctgca 76800
   ggcccaggct catgagactg ggaagaggac aggctgtggt catgtggacc cacaggggcc 76860
   tggggctgca gaagtcagtg tggcttccac catttcaggt ataaaaaagg gcatctaagc 76920
   tttcaagaag agggaggatg ctctagggca gcggtcccca accttttctg gcaccaggaa 76980
   ccggttccat ggaagacaat tttttcacag gcctgggggt ggtgagggat ggttttggga 77040
   tagaaacttc cacctcagat catcaggcat cagattctca taaggagctt gcaacctgat 77100
   ctcttgcaca cattcagttc acaatagggt tcacgctcct aagagaacct gatgctgcag 77160
   ctgatctaac aggagatgga gctcaggtgg tcatgctcag tcgctcgcca ctcacctcct 77220
   gccatgcagt ccagttccta acaggcctca gaccagtacc ggtctgtggc ctgggggttg 77280
   aggacccctg ctctaggctg gtactgctga tgcttaaaaa gagagggttt gccagaaatc 77340
   agatgggaca aaagggcaaa ggccgtgcca cagagtgccc atatagggga gagcacgcct 77400
   ggagccttcg agagcatgca gagaagcctg gagactgcat ttaccggagc tgctgcctga 77460
   ggccaccctc caagtgtccc cacagcgcac cacaagacca caggagtgac ctcctcactg 77520
   gcaggtattt ggggaaacaa ctgctgtcta ctcttttggg taaaaagtga aacaccaata 77580
   gtttaattga aatttcagaa aattgaacat atgaacaagg caaataaata ctaagtaagt 77640
   taaaaacaca aaatatgtcc aggaagtatc gatgagaatg ttcaagttaa agttctccaa 77700
   tgccattgct acagcaacct caaaccctag gttctctctg cactattaac acagacatct 77760
   caggacatgg tttgcttttt tttaagactt aaataggaaa ctaatttttc tttctttaaa 77820
   gcaattgcgt tcttcagtga actctttctt taggccagtt gatggcttct tagcagttta 77880
   ttgacgagat cctagggtag cttccgaagc tgggttgatt gattgcattt gggtgcggat 77940
   ggccaaagtg agtggcccta ctgcctgtgc tgctcagggc tcctgggctg atgtggtggc 78000
   t 78001

<210> 13
   <211> 2160
   <212> DNA
   <213> Musmusculus
<400> 13
   ggcgccctgc tctcccggcg gggcggcgga gggggcgggc tggccggcgc acggtgatgt 60
   ggcgggactc tttgtgcact gcggcaggat acgcgcttgg gcgtcgggac gcggctgcgc 120
   tcagctctct cctctcggaa gctgcagcca tgatggaagt ttgagagttg agccgctgtg 180
   aggccaggcc cggcgcaggc gagggagatg agagacggcg gcggccacgg cccagagccc 240
   ctctcagcgc ctgtgagcag ccgcgggggc agcgccctcg gggagccggc cgggcggcgg 300
   cggcggcagc ggcggcgggc ctcgcctcct cgtcgtctgt tctaaccggg cagcttctga 360
   gcagcttcgg agagagacgg tggaagaagc cgtgggctcg agcgggagcc ggcgcaggct 420
   cggcggctgc acctcccgct cctggagcgg gggggagaag cggcggcggc ggccgcggct 480
   ccggggaggg ggtcggagtc gcctgtcacc attgccaggg ctgggaacgc cggagagttg 540
   ctctctcccc ttctcctgcc tccaacacgg cggcggcggc ggcggcacgt ccagggaccc 600
   gggccggtgt taagcctccc gtccgccgcc gccgcacccc ccctggcccg ggctccggag 660
   gccgccggag gaggcagccg ctgcgaggat tatccgtctt ctccccattc cgctgcctcg 720
   gctgccaggc ctctggctgc tgaggagaag caggcccagt ctctgcaacc atccagcagc 780
   cgccgcagca gccattaccc ggctgcggtc cagggccaag cggcagcaga gcgaggggca 840
   tcagcgaccg ccaagtccag agccatttcc atcctgcaga agaagcctcg ccaccagcag 900
   cttctgccat ctctctcctc ctttttcttc agccacaggc tcccagacat gacagccatc 960
   atcaaagaga tcgttagcag aaacaaaagg agatatcaag aggatggatt cgacttagac 1020
   ttgacctata tttatccaaa tattattgct atgggatttc ctgcagaaag acttgaaggt 1080
   gtatacagga acaatattga tgatgtagta aggtttttgg attcaaagca taaaaaccat 1140
   tacaagatat acaatctatg tgctgagaga cattatgaca ccgccaaatt taactgcaga 1200
   gttgcacagt atccttttga agaccataac ccaccacagc tagaacttat caaacccttc 1260
   tgtgaagatc ttgaccaatg gctaagtgaa gatgacaatc atgttgcagc aattcactgt 1320
   aaagctggaa agggacggac tggtgtaatg atttgtgcat atttattgca tcggggcaaa 1380
   tttttaaagg cacaagaggc cctagatttt tatggggaag taaggaccag agacaaaaag 1440
   ggagtcacaa ttcccagtca gaggcgctat gtatattatt atagctacct gctaaaaaat 1500
   cacctggatt acagacccgt ggcactgctg tttcacaaga tgatgtttga aactattcca 1560
   atgttcagtg gcggaacttg caatcctcag tttgtggtct gccagctaaa ggtgaagata 1620
   tattcctcca attcaggacc cacgcggcgg gaggacaagt tcatgtactt tgagttccct 1680
   cagccattgc ctgtgtgtgg tgatatcaaa gtagagttct tccacaaaca gaacaagatg 1740
   ctcaaaaagg acaaaatgtt tcacttttgg gtaaatacgt tcttcatacc aggaccagag 1800
   gaaacctcag aaaaagtgga aaatggaagt ctttgtgatc aggaaatcga tagcatttgc 1860
   agtatagagc gtgcagataa tgacaaggag tatcttgtac tcaccctaac aaaaaacgat 1920
   cttgacaaag caaacaaaga caaggccaac cgatacttct ctccaaattt taaggtgaaa 1980
   ctatacttta caaaaacagt agaggagcca tcaaatccag aggctagcag ttcaacttct 2040
   gtgactccag atgttagtga caatgaacct gatcattata gatattctga caccactgac 2100
   tctgatccag agaatgaacc ttttgatgaa gatcagcatt cacaaattac aaaagtctga 2160
<210> 14
   <211> 5572
   <212> DNA
   <213> H. sapiens

<400> 14
   cctcccctcg cccggcgcgg tcccgtccgc ctctcgctcg cctcccgcct cccctcggtc 60
   ttccgaggcg cccgggctcc cggcgcggcg gcggaggggg cgggcaggcc ggcgggcggt 120
   gatgtggcgg gactctttat gcgctgcggc aggatacgcg ctcggcgctg ggacgcgact 180
   gcgctcagtt ctctcctctc ggaagctgca gccatgatgg aagtttgaga gttgagccgc 240
   tgtgaggcga ggccgggctc aggcgaggga gatgagagac ggcggcggcc gcggcccgga 300
   gcccctctca gcgcctgtga gcagccgcgg gggcagcgcc ctcggggagc cggccggcct 360
   gcggcggcgg cagcggcggc gtttctcgcc tcctcttcgt cttttctaac cgtgcagcct 420
   cttcctcggc ttctcctgaa agggaaggtg gaagccgtgg gctcgggcgg gagccggctg 480
   aggcgcggcg gcggcggcgg cacctcccgc tcctggagcg ggggggagaa gcggcggcgg 540
   cggcggccgc ggcggctgca gctccaggga gggggtctga gtcgcctgtc accatttcca 600
   gggctgggaa cgccggagag ttggtctctc cccttctact gcctccaaca cggcggcggc 660
   ggcggcggca catccaggga cccgggccgg ttttaaacct cccgtccgcc gccgccgcac 720
   cccccgtggc ccgggctccg gaggccgccg gcggaggcag ccgttcggag gattattcgt 780
   cttctcccca ttccgctgcc gccgctgcca ggcctctggc tgctgaggag aagcaggccc 840
   agtcgctgca accatccagc agccgccgca gcagccatta cccggctgcg gtccagagcc 900
   aagcggcggc agagcgaggg gcatcagcta ccgccaagtc cagagccatt tccatcctgc 960
   agaagaagcc ccgccaccag cagcttctgc catctctctc ctcctttttc ttcagccaca 1020
   ggctcccaga catgacagcc atcatcaaag agatcgttag cagaaacaaa aggagatatc 1080
   aagaggatgg attcgactta gacttgacct atatttatcc aaacattatt gctatgggat 1140
   ttcctgcaga aagacttgaa ggcgtataca ggaacaatat tgatgatgta gtaaggtttt 1200
   tggattcaaa gcataaaaac cattacaaga tatacaatct ttgtgctgaa agacattatg 1260
   acaccgccaa atttaattgc agagttgcac aatatccttt tgaagaccat aacccaccac 1320
   agctagaact tatcaaaccc ttttgtgaag atcttgacca atggctaagt gaagatgaca 1380
   atcatgttgc agcaattcac tgtaaagctg gaaagggacg aactggtgta atgatatgtg 1440
   catatttatt acatcggggc aaatttttaa aggcacaaga ggccctagat ttctatgggg 1500
   aagtaaggac cagagacaaa aagggagtaa ctattcccag tcagaggcgc tatgtgtatt 1560
   attatagcta cctgttaaag aatcatctgg attatagacc agtggcactg ttgtttcaca 1620
   agatgatgtt tgaaactatt ccaatgttca gtggcggaac ttgcaatcct cagtttgtgg 1680
   tctgccagct aaaggtgaag atatattcct ccaattcagg acccacacga cgggaagaca 1740
   agttcatgta ctttgagttc cctcagccgt tacctgtgtg tggtgatatc aaagtagagt 1800
   tcttccacaa acagaacaag atgctaaaaa aggacaaaat gtttcacttt tgggtaaata 1860
   cattcttcat accaggacca gaggaaacct cagaaaaagt agaaaatgga agtctatgtg 1920
   atcaagaaat cgatagcatt tgcagtatag agcgtgcaga taatgacaag gaatatctag 1980
   tacttacttt aacaaaaaat gatcttgaca aagcaaataa agacaaagcc aaccgatact 2040
   tttctccaaa ttttaaggtg aagctgtact tcacaaaaac agtagaggag ccgtcaaatc 2100
   cagaggctag cagttcaact tctgtaacac cagatgttag tgacaatgaa cctgatcatt 2160
   atagatattc tgacaccact gactctgatc cagagaatga accttttgat gaagatcagc 2220
   atacacaaat tacaaaagtc tgaatttttt tttatcaaga gggataaaac accatgaaaa 2280
   taaacttgaa taaactgaaa atggaccttt ttttttttaa tggcaatagg acattgtgtc 2340
   agattaccag ttataggaac aattctcttt tcctgaccaa tcttgtttta ccctatacat 2400
   ccacagggtt ttgacacttg ttgtccagtt gaaaaaaggt tgtgtagctg tgtcatgtat 2460
   ataccttttt gtgtcaaaag gacatttaaa attcaattag gattaataaa gatggcactt 2520
   tcccgtttta ttccagtttt ataaaaagtg gagacagact gatgtgtata cgtaggaatt 2580
   ttttcctttt gtgttctgtc accaactgaa gtggctaaag agctttgtga tatactggtt 2640
   cacatcctac ccctttgcac ttgtggcaac agataagttt gcagttggct aagagaggtt 2700
   tccgaagggt tttgctacat tctaatgcat gtattcgggt taggggaatg gagggaatgc 2760
   tcagaaagga aataatttta tgctggactc tggaccatat accatctcca gctatttaca 2820
   cacacctttc tttagcatgc tacagttatt aatctggaca ttcgaggaat tggccgctgt 2880
   cactgcttgt tgtttgcgca ttttttttta aagcatattg gtgctagaaa aggcagctaa 2940
   aggaagtgaa tctgtattgg ggtacaggaa tgaaccttct gcaacatctt aagatccaca 3000
   aatgaaggga tataaaaata atgtcatagg taagaaacac agcaacaatg acttaaccat 3060
   ataaatgtgg aggctatcaa caaagaatgg gcttgaaaca ttataaaaat tgacaatgat 3120
   ttattaaata tgttttctca attgtaacga cttctccatc tcctgtgtaa tcaaggccag 3180
   tgctaaaatt cagatgctgt tagtacctac atcagtcaac aacttacact tattttacta 3240
   gttttcaatc ataatacctg ctgtggatgc ttcatgtgct gcctgcaagc ttcttttttc 3300
   tcattaaata taaaatattt tgtaatgctg cacagaaatt ttcaatttga gattctacag 3360
   taagcgtttt ttttctttga agatttatga tgcacttatt caatagctgt cagccgttcc 3420
   acccttttga ccttacacat tctattacaa tgaattttgc agttttgcac attttttaaa 3480
   tgtcattaac tgttagggaa ttttacttga atactgaata catataatgt ttatattaaa 3540
   aaggacattt gtgttaaaaa ggaaattaga gttgcagtaa actttcaatg ctgcacacaa 3600
   aaaaaagaca tttgattttt cagtagaaat tgtcctacat gtgctttatt gatttgctat 3660
   tgaaagaata gggttttttt tttttttttt tttttttttt ttaaatgtgc agtgttgaat 3720
   catttcttca tagtgctccc ccgagttggg actagggctt caatttcact tcttaaaaaa 3780
   aatcatcata tatttgatat gcccagactg catacgattt taagcggagt acaactacta 3840
   ttgtaaagct aatgtgaaga tattattaaa aaggtttttt tttccagaaa tttggtgtct 3900
   tcaaattata ccttcacctt gacatttgaa tatccagcca ttttgtttct taatggtata 3960
   aaattccatt ttcaataact tattggtgct gaaattgttc actagctgtg gtctgaccta 4020
   gttaatttac aaatacagat tgaataggac ctactagagc agcatttata gagtttgatg 4080
   gcaaatagat taggcagaac ttcatctaaa atattcttag taaataatgt tgacacgttt 4140
   tccatacctt gtcagtttca ttcaacaatt tttaaatttt taacaaagct cttaggattt 4200
   acacatttat atttaaacat tgatatatag agtattgatt gattgctcat aagttaaatt 4260
   ggtaaagtta gagacaacta ttctaacacc tcaccattga aatttatatg ccaccttgtc 4320
   tttcataaaa gctgaaaatt gttacctaaa atgaaaatca acttcatgtt ttgaagatag 4380
   ttataaatat tgttctttgt tacaatttcg ggcaccgcat attaaaacgt aactttattg 4440
   ttccaatatg taacatggag ggccaggtca taaataatga cattataatg ggcttttgca 4500
   ctgttattat ttttcctttg gaatgtgaag gtctgaatga gggttttgat tttgaatgtt 4560
   tcaatgtttt tgagaagcct tgcttacatt ttatggtgta gtcattggaa atggaaaaat 4620
   ggcattatat atattatata tataaatata tattatacat actctcctta ctttatttca 4680
   gttaccatcc ccatagaatt tgacaagaat tgctatgact gaaaggtttt cgagtcctaa 4740
   ttaaaacttt atttatggca gtattcataa ttagcctgaa atgcattctg taggtaatct 4800
   ctgagtttct ggaatatttt cttagacttt ttggatgtgc agcagcttac atgtctgaag 4860
   ttacttgaag gcatcacttt taagaaagct tacagttggg ccctgtacca tcccaagtcc 4920
   tttgtagctc ctcttgaaca tgtttgccat acttttaaaa gggtagttga ataaatagca 4980
   tcaccattct ttgctgtggc acaggttata aacttaagtg gagtttaccg gcagcatcaa 5040
   atgtttcagc tttaaaaaat aaaagtaggg tacaagttta atgtttagtt ctagaaattt 5100
   tgtgcaatat gttcataacg atggctgtgg ttgccacaaa gtgcctcgtt tacctttaaa 5160
   tactgttaat gtgtcatgca tgcagatgga aggggtggaa ctgtgcacta aagtgggggc 5220
   tttaactgta gtatttggca gagttgcctt ctacctgcca gttcaaaagt tcaacctgtt 5280
   ttcatataga atatatatac taaaaaattt cagtctgtta aacagcctta ctctgattca 5340
   gcctcttcag atactcttgt gctgtgcagc agtggctctg tgtgtaaatg ctatgcactg 5400
   aggatacaca aaaataccaa tatgatgtgt acaggataat gcctcatccc aatcagatgt 5460
   ccatttgtta ttgtgtttgt taacaaccct ttatctctta gtgttataaa ctccacttaa 5520
   aactgattaa agtctcattc ttgtcaaaaa aaaaaaaaaa aaaaaaaaaa aa         5572
<210> 15
   <211> 103886
   <212> DNA
   <213> H. sapiens
<400> 15
<210> 16
   <211> 16
   <212> DNA
   <213> Artificial Sequence

<220>
   <223> Synthetic Oligonucleotide
<400> 16
   ccggaggt gc t t gaat 16
<210> 17
   <211> 14
   <212> DNA
   <213> Artificial Sequence
<220>
   <223> Synthetic Oligonucleotide
<400> 17
   cggaggt gct t gaa 14
<210> 18
   <211> 16
   <212> DNA
   <213> Artificial Sequence
<220>
   <223> Synthetic Oligonucleotide
<400> 18
   gaagccat ac acct ct 16
<210> 19
   <211> 14
   <212> DNA
   <213> Artificial Sequence
<220>
   <223> Synthetic Oligonucleotide
<400> 19
   aagccat aca cct c 14

<210> 20
   <211> 16
   <212> DNA
   <213> Artificial Sequence
<220>
   <223> Synthetic Oligonucleotide
<400> 20
   gt t gaagcca t acacc 16
<210> 21
   <211> 14
   <212> DNA
   <213> Artificial Sequence
<220>
   <223> Synthetic Olgonucleotide
<400> 21
   t t gaagccat acac 14
<210> 22
   <211> 16
   <212> DNA
   <213> Artificial Sequence
<220>
   <223> Synthetic Oligonucleotide
<400> 22
   cct cagggt t gaagcc 16
<210> 23
   <211> 14
   <212> DNA
   <213>Artificial Sequence
<220>
   <223> Synthetic Oligonucleotide
<400> 23
   ct cagggt t g aagc 14

<210> 24
   <211> 16
   <212> DNA
   <213>Artificial Sequence
<220>
   <223> Synthetic Oligonucleotide
<400> 24
   t t t gccct ca gggt t g 16
<210> 25
   <211> 14
   <212> DNA
   <213>Artificial Sequence
<220>
   <223> Synthetic Oligonucleotide
<400> 25
   t t gccct cag ggt t 14
<210> 26
   <211> 16
   <212> DNA
   <213> Artificial Sequence
<220>
   <223> Synthetic Oligonucleotide
<400> 26
   agttcttggt tttctt 16
<210> 27
   <211> 14
   <212> DNA
   <213> Arttificial Sequence
<220>
   <223> Synthetic Oligonucleotide
<400> 27
   gttcttggtt ttct 14
<210> 28
   <211> 16
   <212> DNA
   <213> Artificial Sequence
<220>
   <223> Synthetic Oligonucleotide
<400> 28
   cctcttgatg ttcagg 16
<210> 29
   <211> 14
   <212> DNA
   <213> Artificial Sequence

<220>
   <223> Synthetic Oligonucleotide
<400> 29
   ct ct t gat gt t cag 14
<210> 30
   <211> 16
   <212> DNA
   <213> Artificial Sequence
<220>
   <223> Synthetic Oligonucleotide
<400> 30
   at gcccct ct tgatgt 16
<210> 31
   <211> 14
   <212> DNA
   <213>Artificial Sequence
<220>
   <223> Synthetic Oligonucleotide
<400> 31
   t gcccct ct t gat g 14
<210> 32
   <211> 14
   <212> DNA
   <213>Artifcial Sequence
<220>
   <223> Synthetic Oligonucleotide
<400> 32
   t gccacat t g ccct 14
<210> 33
   <211> 14
   <212> DNA
   <213> Artificial Sequence
<220>
   <223> Synthetic Oligonucleotide
<400> 33
   t t gccacat t gccc 14
<210> 34
   <211> 14
   <212> DNA
   <213> Artificial Sequence
<220>
   <223> Synthetic Oligonucleotide
<400> 34
   gt t gccacat t gcc 14
<210> 35
   <211> 14
   <212> DNA
   <213>Artifcial Sequence
<220>
   <223>Synthetic Oligonucleotide
<400> 35
   t gt t gccaca t t gc 14

<210> 36
   <211> 14
   <212> DNA
   <213> Artificial Sequence
<220>
   <223> Synthetic Oligonucleotide
<400> 36
   ct gt t gccac at t g 14
<210> 37
   <211> 14
   <212> DNA
   <213> Artificial Sequence
<220 >
   <223> Synthetic Oligonucleotide
<400> 37
   t ct gt t gcca cat t 14
<210> 38
   <211> 14
   <212> DNA
   <213> Artificial Sequence
<220>
   <223> Synthetic Oligonucleotide
<400> 38
   t t ct gt t gcc acat 14
<210> 39
   <211> 14
   <212> DNA
   <213> Artificial Sequence
<220>
   <223>Synthetic Oligonucleotide
<400> 39
   tttctgttgc caca 14
<210> 40
   <211> 14
   <212> DNA
   <213> Artificial Sequence
<220>
   <223> Synthetic Oligonucleotide
<400> 40
   atttctgttg ccac 14
<210> 41
   <211> 16
   <212> DNA
   <213> Artificial Sequence
<220>
   <223> Synthetic Oligonucleotide
<400> 41
   gtaggagaaa ggcagg 16
<210> 42
   <211> 14
   <212> DNA
   <213>Artificial Sequence
<220>
   <223> Synthetic Oligonucleotide
<400> 42
   taggagaaag gcag 14
<210> 43
   <211> 16
   <212> DNA
   <213>Artificial Sequence
<220>
   <223> Synthetic Oligonucleotide

<400> 43
   ggct t gt aaa gt gat g 16
<210> 44
   <211> 14
   <212> DNA
   <213>Artificial Sequence
<220>
   <223> Synthetic Oligonucleotide
<400> 44
   gct t gt aaag t gat 14
<210> 45
   <211> 16
   <212> DNA
   <213>Artificial Sequence
<220>
   <223> Synthetic Oligonucleotide
<400> 45
   ccact ggagg at gt ga 16
<210> 46
   <211> 14
   <212> DNA
   <213>Artificial Sequence
<220>
   <223> Synthetic Oligonucleotide
<400> 46
   cact ggagga t gt g 14
<210> 47
   <211> 16
   <212> DNA
   <213> Artificial Sequence
<220>
   <223> Synthetic Oligonucleotide
<400> 47
   tttcagcatg ctttct 16
<210> 48
   <211> 14
   <212> DNA
   <213> Artificial Sequence
<220>
   <223> Synthetic Oligonucleotide
<400> 48
   ttcagcatgc tttc 14
<210> 49
   <211> 16
   <212> DNA
   <213> Artificial Sequence
<220>
   <223> Synthetic Oligonucleotide
<400> 49
   cat at t t gt c acaaac 16

<210> 50
   <211> 14
   <212> DNA
   <213> Artificial Sequence
<220>
   <223> Synthetic Oligonucleotide
<400> 50
   atatttgtca caaa 14
<210> 51
   <211> 16
   <212> DNA
   <213> Artificial Sequence
<220>
   <223> Synthetic Oligonucleotide
<400> 51
   at gcccat at t t gt ca 16
<210> 52
   <211> 14
   <212> DNA
   <213> Artificial Sequence
<220>
   <223> Synthetic Oligonucleotide
<400> 52
   t gcccat at t t gt c 14
<210> 53
<211> 16
<212> DNA
   <213> Artificial Sequence
<220>
   <223> Synthetic Oligonucleotide
<400> 53
   t t t t ggt ggt agagac 16
<210> 54
   <211> 14
   <212> DNA
   <213> Artificial Sequence
<220>
   <223> Synthetic Oligonucleotide
<400> 54
   t t t ggt ggt a gaga 14
<210> 55
   <211> 14
   <212> DNA
   <213> Artificial Sequence
<220>
   <223> Synthetic Oligonucleotide
<400> 55
   tctgcttcgc acct 14
<210> 56
   <211> 14
   <212> DNA
   <213> Artificial Sequence

<220>
   <223> Synthetic Oligonucleotide
<400> 56
   gt ct gct t cg cacc 14
<210> 57
   <211> 14
   <212> DNA
   <213> Artificial Sequence
<220>
   <223> Synthetic Oligonucleotide
<400> 57
   agt ct gct t c gcac 14
<210> 58
   <211> 14
   <212> DNA
   <213> Artificial Sequence
<220>
   <223> Synthetic Oligonucleotide
<400> 58
   cagt ct gct t cgca 14
<210> 59
   <211> 14
   <212> DNA
   <213> Artificial Sequence
<220>
   <223> Synthetic Oligonucleotide
<400> 59
   t cagt ct gct t cgc 14
<210> 60
   <211> 14
   <212> DNA
   <213> Artificial Sequence
<220>
   <223> Synthetic Oligonucleotide
<400> 60
   ctcagtctgc t t cg 14
<210> 61
   <211> 14
   <212> DNA
   <213> Artificial Sequence
<220>
   <223> Synthetic Oligonucleotide
<400> 61
   cct cagt ct g ct t c 14

<210> 62
   <211> 14
   <212> DNA
   <213> Artificial Sequence
<220>
   <223> Synthetic Oligonucleotide
<400> 62
   gcctcagtct gct t 14
<210> 63
   <211> 14
   <212> DNA
   <213> Artificial Sequence <220>
   <223> Synthetic Oligonucleotide
<400> 63
   agcct cagt c t gct 14
<210> 64
   <211> 16
   <212> DNA
   <213> Artificial Sequence
<220>
   <223> Synthetic Oligonucleotide
<400> 64
   aact ct gagg at t gt t 16
<210> 65
   <211> 14
   <212> DNA
   <213> Artificial Sequence
<220>
   <223> Synthetic Oligonucleotide
<400> 65
   actctgagga ttgt 14
<210> 66
   <211> 16
   <212> DNA
   <213> Artificial Sequence
<220>
   <223> Synthetic Oligonucleotide
<400> 66
   t cat t aact c t gagga 16
<210> 67
   <211> 14
   <212> DNA
   <213> Artificial Sequence
<220>
   <223> Synthetic Oligonucleotide
<400> 67
   cat t aact ct gagg 14
<210> 68
   <211> 16
   <212> DNA
   <213> Artificial Sequence
<220>
   <223> Synthetic Oligonucleotide
<400> 68
   at t cat cat t aact ct 16
<210> 69
   <211> 14
   <212> DNA
   <213> Artificial Sequence

<220>
   <223> Synthetic Oligonucleotide
<400> 69
   ttcatcatta act c 14
<210> 70
   <211> 16
   <212> DNA
   <213> Artificial Sequence
<220>
   <223> Synthetic Oligonucleotide
<400> 70
   ttgttctgaa t gt cca 16
<210> 71
   <211> 4
   <212> DNA
   <213> Artificial Sequence
<220>
   <223> Synthetic Oligonucleotide
<400> 71
   act g 4
<210> 72
   <211> 4
   <212> DNA
   <213> Artificial Sequence
<220>
   <223> Synthetic Oligonucleotide
<400> 72
   act g 4
<210> 73
   <211> 14
   <212> DNA
   <213> Artificial Sequence
<220>
   <223> Synthetic Oligonucleotide
<400> 73
   t gt t ct gaat gt cc 14
<210> 74
   <211> 16
   <212> DNA
   <213> Artificial Sequence
<220>
   <223> Synthetic Oligonucleotide
<400> 74
   cagat gagt c cat t t g 16
<210> 75
   <211> 14
   <212> DNA
   <213> Artificial Sequence
<220>
   <223> Synthetic Oligonucleotide
<400> 75
   agat gagt cc at t t 14
<210> 76
   <211> 16
   <212> DNA
   <213> Artificial Sequence
<220>
   <223> Synthetic Oligonucleotide
<400> 76
   at ccacaggg aaat t g 16
<210> 77
   <211> 14
   <212> DNA
   <213> Artificial Sequence

<220>
   <223> Synthetic Oligonucleotide
<400> 77
   tccacaggga aatt 14
<210> 78
   <211> 16
   <212> DNA
   <213> Artificial Sequence
<220>
   <223> Synthetic Oligonucleotide
<400> 78
   cagt t gt aca agt t gc 16
<210> 79
   <211> 14
   <212> DNA
   <213> Artificial Sequence
<220>
   <223> Synthetic Oligonucleotide
<400> 79
   agttgtacaa gttg 14
<210> 80
   <211> 16
   <212> DNA
   <213> Artificial Sequence
<220>
   <223> Synthetic Oligonucleotide
<400> 80
   cacagagt ca gcct t c 16
<210> 81
   <211> 14
   <212> DNA
   <213> Artificial Sequence
<220>
   <223> Synthetic Oligonucleotide
<400> 81
   acagagtcag cct t 14
<210> 82
   <211> 16
   <212> DNA
   <213> Artificial Sequence
<220>
   <223> Synthetic Oligonucleotide
<400> 82
   ggtcaaccac agagtc c 16
<210> 83
   <211> 14
   <212> DNA
   <213> Artificial Sequence
<220>
   <223> Synthetic Oligonucleotide
<400> 83
   gt caaccaca gagt 14
<210> 84
   <211> 14
   <212> DNA
   <213> Artificial Sequence
<220>
   <223> Synthetic Oligonucleotide
<400> 84
   cagccacatg cagc 14
<210> 85
   <211> 14
   <212> DNA
   <213> Artificial Sequence
<220>
   <223> Synthetic Oligonucleotide
<400> 85
   ccagccacat gcag 14
<210> 86
   <211> 14
   <212> DNA
   <213> Artificial Sequence
<220>
   <223> Synthetic Oligonucleotide
<400> 86
   accagccaca tgca 14
<210> 87
   <211> 14
   <212> DNA
   <213> Artificial Sequence
<220>
   <223> Synthetic Oligonucleotide
<400> 87
   t accagccac at gc 14
<210> 88
   <211> 14
   <212> DNA
   <213> Artificial Sequence
<220>
   <223> Synthetic Oligonucleotide
<400> 88
   t t accagcca cat g 14
<210> 89
   <211> 14
   <212> DNA
   <213> Artificial Sequence
<220>
   <223> Synthetic Oligonucleotide
<400> 89
   gt t accagcc acat 14
<210> 90
   <211> 14
   <212> DNA
   <213> Artificial Sequence
<220>
   <223> Synthetic Oligonucleotide

<400> 90
   ggttaccagc caca 14
<210> 91
   <211> 14
   <212> DNA
   <213> Artificial Sequence
<220>
   <223> Synthetic Oligonucleotide
<400> 91
   aggttaccag ccac 14
<210> 92
   <211> 14
   <212> DNA
   <213> Artificial Sequence
<220>
   <223> Synthetic Oligonucleotide
<400> 92
   t aggt t acca gcca 14
<210> 93
   <211> 16
   <212> DNA
   <213> Artificial Sequence
<220>
   <223>Synthetic Oligonucleotide
<400> 93
   aggt t ct gct t t caac 16
<210> 94
   <211> 14
   <212> DNA
   <213> Artificial Sequence
<220>
   <223> Synthetic Oligonucleotide
<400> 94
   ggt t ct gct t t caa 14
<210> 95
   <211> 16
   <212> DNA
   <213> Artificial Sequence
<220>
   <223> Synthetic Oligonucleotide
<400> 95
   t act gat caa at t gt a 16
<210> 96
   <211> 14
   <212> DNA
   <213> Artificial Sequence
<220>
   <223> Synthetic Oligonucleotide
<400> 96
   act gat caaa t t gt 14
<210> 97
   <211> 16
   <212> DNA
   <213> Artificial Sequence
<220>
   <223> Synthetic Oligonucleotide
<400> 97
   tttttcttgt atctgg 16
<210> 98
   <211> 14
   <212> DNA
   <213> Artificial Sequence
<220>
   <223> Synthetic Oligonucleotide
<400> 98
   tttt ct t gt a t ct g 14
<210> 99
   <211> 16
   <212> DNA
   <213> Artificial Sequence
<220>
   <223> Synthetic Oligonucleotide
<400> 99
   at ccat t aaa acct gg 16
<210> 100
   <211> 14
   <212> DNA
   <213> Artificial Sequence
<220>
   <223> Synthetic Oligonucleotide
<400> 100
   t ccat t aaaa cct g 14
<210> 101
   <211> 16
   <212> DNA
   <213> Artificial Sequence
<220>
   <223> Synthetic Oligonucleotide
<400> 101
   at at t gct ct gcaaag 16
<210> 102
   <211> 14
   <212> DNA
   <213> Artificial Sequence

<220>
   <223> Synthetic Oligonucleotide
<400> 102
   t at t gct ct g caaa 14
<210> 103
   <211> 4
   <212> DNA
   <213> Artificial Sequence
<220>
   <223> Synthetic Oligonucleotide
<400> 103
   act g 4
<210> 104
   <211> 14
   <212> DNA
   <213> Artificial Sequence
<220>
   <223> Synthetic Oligonucleotide
<400> 104
   atattgctct gcaa 14
<210> 105
   <211> 14
   <212> DNA
   <213> Artificial Sequence
<220>
   <223> Synthetic Oligonucleotide
<400> 105
   aat at t gct c t gca 14
<210> 106
   <211> 14
   <212> DNA
   <213> Artificial Sequence
<220>
   <223> Synthetic Oligonucleotide
<400> 106
   gaat at t gct ct gc 14
<210> 107
   <211> 14
   <212> DNA
   <213> Artifiicial Sequence
<220>
   <223> Synthetic Oligonucleotide
<400> 107
   agaat at t gc t ct g 14
<210> 108
   <211> 14
   <212> DNA
   <213> Artificial Sequence
<220>
   <223> Synthetic Oligonucleotide
<400> 108
   t agaat at t g ct ct 14
<210> 109
   <211> 14
   <212> DNA
   <213> Artificial Sequence
<220>
   <223> Synthetic Oligonucleotide
<400> 109
   at agaat at t gct c 14
<210> 110
   <211> 14
   <212> DNA
   <213> Artificial Sequence
<220>
   <223> Synthetic Oligonucleotide
<400> 110
   gat agaat at t gct 14
<210> 111
   <211> 14
   <212> DNA
   <213> Artificial Sequence
<220>
   <223> Synthetic Oligonucleotide
<400> 111
   ggat agaat a t t gc 14
<210> 112
   <211> 16
   <212> DNA
   <213> Artificial Sequence <220>
   <223> Synthetic Oligonucleotide
<400> 112
   at ggaat cct caaat c 16
<210> 113
   <211> 14
   <212> DNA
   <213> Artificial Sequence
<220>
   <223> Synthetic Oligonucleotide
<400> 113
   t ggaat cct c aaat 14
<210> 114
   <211> 16
   <212> DNA
   <213> Artificial Sequence
<220>
   <223> Synthetic Oligonucleotide
<400> 114
   gaat t ct ggt at gt ga 16
<210> 115
   <211> 14
   <212> DNA
   <213> Artificial Sequence
<220>
   <223> Synthetic Oligonucleotide
<400> 115
   aat t ct ggt a t gt g 14
<210> 116
   <211> 16
   <212> DNA
   <213> Artificial Sequence
<220>
   <223> Synthetic Oligonucleotide
<400> 116
   agct ggaat t ct ggt a 16
<210> 117
   <211> 14
   <212> DNA
   <213> Artificial Sequence
<220>
   <223> Synthetic Oligonucleotide
<400> 117
   gct ggaat t c t ggt          14
   gct ggaat t c t ggt 14 <210> 118
   <211> 16
   <212> DNA
   <213> Artificial Sequence
<220>
   <223> Synthetic Oligonucleotide
<400> 118
   t gaaaat caa aat t ga 16
<210> 119
   <211> 14
   <212> DNA
   <213> Artificial Sequence
<220>
   <223> Synthetic Oligonucleotide
<400> 119
   gaaaat caaa at t g 14
<210> 120
   <211> 16
   <212> DNA
   <213> Artificial Sequence
<220>
   <223> Synthetic Oligonucleotide
<400> 120
   aaacagt gca t agt t a 16

<210> 121
   <211> 14
   <212> DNA
   <213> Artificial Sequence
<220>
   <223> Synthetic Oligonucleotide
<400> 121
   aacagt gcat agt t 14
<210> 122
   <211> 16
   <212> DNA
   <213> Artificial Sequence
<220>
   <223> Synthetic Oligonucleotide
<400> 122
   t t caggaat t gt t aaa 16
<210> 123
   <211> 14
   <212> DNA
   <213> Artificial Sequence
<220>
   <223> Synthetic Oligonucleotide
<400> 123
   tcaggaattg t t aa 14
<210> 124
   <211> 16
   <212> DNA
   <213> Artificial Sequence
<220>
   <223> Synthetic Oligonucleotide
<400> 124
   ttttgtttca ttatag 16
<210> 125
   <211> 14
   <212> DNA
   <213> Artificial Sequence
<220>
   <223> Synthetic Oligonucleotide
<400> 125
   tttgtttcat tata 14
<210> 126
   <211> 16
   <212> DNA
   <213> Artificial Sequence
<220>
   <223> Synthetic Oligonucleotide
<400> 126
   gatgacactt gattta 16
<210> 127
   <211> 14
   <212> DNA
   <213> Artificial Sequence
<220>
   <223> Synthetic Oligonucleotide
<400> 127
   atgacacttg attt 14
<210> 128
   <211> 16
   <212> DNA
   <213> Artificial Sequence
<220>
   <223> Synthetic Oligonucleotide
<400> 128
   gt gt gat gac act t ga 16
<210> 129
   <211> 14
   <212> DNA
   <213> Artificial Sequence
<220>
   <223> Synthetic Oligonucleotide
<400> 129
   t gt gat gaca cttg 14
<210> 130
   <211> 16
   <212> DNA
   <213> Artificial Sequence
<220>
   <223> Synthetic Oligonucleotide
<400> 130
   t at tcagt gt gat gac 16
<210> 131
   <211> 14
   <212> DNA
   <213> Artificial Sequence
<220>
   <223> Synthetic Oligonucleotide
<400> 131
   at t cagt gt g at ga 14
<210> 132
   <211> 16
   <212> DNA
   <213> Artificial Sequence
<220>
   <223> Synthetic Oligonucleotide
<400> 132
   attggtattc agtgtg 16
<210> 133
   <211> 14
   <212> DNA
   <213> Artificial Sequence
<220>
   <223> Synthetic Oligonucleotide
<400> 133
   ttggtattca gt gt 14
<210> 134
   <211> 14
   <212> DNA
   <213> Artificial Sequence
<220>
   <223> Synthetic Oligonucleotide
<400> 134
   cctctagctgtaag 14
<210> 135
   <211> 14
   <212> DNA
   <213> Artificial Sequence
<220>
   <223> Synthetic Oligonucleotide
<400> 135
   ccct ct agct gt aa 14
<210> 136
   <211> 14
   <212> DNA
   <213> Artificial Sequence
<220>
   <223> Synthetic Oligonucleotide

<400> 136 14
   gccct ct agc t gt a 14
<210> 137
   <211> 14
   <212> DNA
   <213> Artificial Sequence
<220>
   <223> Synthetic Oligonucleotide
<400> 137 14
   ggccct ct ag ct gt 14
<210> 138
   <211> 14
   <212> DNA
   <213> Artificial Sequence
<220>
   <223> Synthetic Oligonucleotide
<400> 138 14
   aggccctcta gctg 14
<210> 139
   <211> 14
   <212> DNA
   <213> Artificial Sequence
<220>
   <223> Synthetic Oligonucleotide
<400> 139 14
   gaggccctct agct 14
<210> 140
   <211> 14
   <212> DNA
   <213> Artificial Sequence
<220>
   <223> Synthetic Oligonucleotide
<400> 140 14
   agaggccct c t agc 14
<210> 141
   <211> 14
   <212> DNA
   <213> Artificial Sequence
<220>
   <223> Synthetic Oligonucleotide
<400> 141 14
   aagaggccct ct ag 14
<210> 142
   <211> 14
   <212> DNA
   <213> Artificial Sequence
<220>
   <223> Synthetic Oligonucleotide
<400> 142
   aaagaggccc tcta 14
<210> 143
   <211> 16
   <212> DNA
   <213> Artificial Sequence
<220>
   <223> Synthetic Oligonucleotide
<400> 143
   gaat ggacag gt caat 16
<210> 144
   <211> 14
   <212> DNA
   <213> Artificial Sequence
<220>
   <223> Synthetic Oligonucleotide
<400> 144
   aat ggacagg t caa 14
<210> 145
   <211> 16
   <212> DNA
   <213> Artificial Sequence
<220>
   <223> Synthetic Oligonucleotide
<400> 145
   gttttgaatg gacagg 16
<210> 146
   <211> 14
   <212> DNA
   <213> Artificial Sequence
<220>
   <223> Synthetic Oligonucleotide
<400> 146
   t t t tgaat gg acag 14
<210> 147
   <211> 16
   <212> DNA
   <213> Artificial Sequence
<220>
   <223> Synthetic Oligonucleotide
<400> 147
   t ggt agt t t t gaat gg 16
<210> 148
   <211> 14
   <212> DNA
   <213> Artificial Sequence
<220>
   <223> Synthetic Oligonucleotide
<400> 148
   ggtagttttg aatg 14
<210> 149
   <211> 14
   <212> DNA
   <213> Artificial Sequence
<220>
   <223> Synthetic Oligonucleotide
<400> 149
   tcactgtatg gttt t 14
<210> 150
   <211> 14
   <212> DNA
   <213> Artificial Sequence
<220>
   <223> Synthetic Oligonucleotide
<400> 150
   ctcact gt at ggt t 14

<210> 151
   <211> 14
   <212> DNA
   <213> Artificial Sequence
<220>
   <223> Synthetic Oligonucleotide
<400> 151
   gctcact gt a t ggt 14
<210> 152
   <211> 14
   <212> DNA
   <213> Artificial Sequence
<220>
   <223> Synthetic Oligonucleotide
<400> 152
   ggct cact gt at gg 14
<210> 153
<211> 14
   <212> DNA
   <213> Artificial Sequence
<220>
   <223> Synthetic Oligonucleotide
<400> 153
   t ggct cact g t at g 14
<210> 154
   <211> 14
   <212> DNA
   <213> Artificial Sequence
<220>
   <223> Synthetic Oligonucleotide
<400> 154
   ctggctcact gt at 14
<210> 155
   <211> 14
   <212> DNA
   <213> Artificial Sequence
<220>
   <223> Synthetic Oligonucleotide
<400> 155
   gct ggct cac t gt a 14
<210> 156
   <211> 14
   <212> DNA
   <213> Artificial Sequence
<220>
   <223> Synthetic Oligonucleotide
<400> 156
   ggct ggct ca ctgt 14
<210> 157
   <211> 14
   <212> DNA
   <213> Artificial Sequence
<220>
   <223> Synthetic Oligonucleotide
<400> 157
   aggctggctc actg 14
<210> 158
   <211> 14
   <212> DNA
   <213> Artificial Sequence
<220>
   <223> Synthetic Oligonucleotide
<400> 158
   caggtccagt tcat 14
<210> 159
   <211> 14
   <212> DNA
   <213> Artificial Sequence
<220>
   <223> Synthetic Oligonucleotide
<400> 159
   gcaggt ccag ttca 14
<210> 160
   <211> 14
   <212> DNA
   <213> Artificial Sequence
<220>
   <223> Synthetic Oligonucleotide
<400> 160
   t gcaggt cca gtt c 14
<210> 161
   <211> 14
   <212> DNA
   <213> Artificial Sequence
<220>
   <223> Synthetic Oligonucleotide
<400> 161
   gt gcaggt cc agtt 14
<210> 162
   <211> 14
   <212> DNA
   <213> Artificial Sequence
<220>
   <223> Synthetic Oligonucleotide
<400> 162
   ggt gcaggt ccagt 14
<210> 163
   <211> 14
   <212> DNA
   <213> Artificial Sequence
<220>
   <223> Synthetic Oligonucleotide
<400> 163
   tggtgcaggt ccag 14
<210> 164
   <211> 14
   <212> DNA
   <213> Artificial Sequence
<220>
   <223> Synthetic Oligonucleotide
<400> 164
   ttggt gcagg tcca 14

<210> 165
   <211> 14
   <212> DNA
   <213> Artificial Sequence
<220>
   <223> Synthetic Oligonucleotide
<400> 165
   tttggtgcag gtcc 14
<210> 166
   <211> 14
   <212> DNA
   <213> Artificial Sequence
<220>
   <223> Synthetic Oligonucleotide
<400> 166
   ctttggtgca ggt c 14
<210> 167
   <211> 14
   <212> DNA
   <213> Artificial Sequence
<220>
   <223> Synthetic Oligonucleotide
<400> 167
   t aact cagat cct g 14
<210> 168
   <211> 14
   <212> DNA
   <213> Artificial Sequence
<220>
   <223> Synthetic Oligonucleotide
<400> 168
   at aact caga t cct 14
<210> 169
   <211> 14
   <212> DNA
   <213> Artificial Sequence
<220>
   <223> Synthetic Oligonucleotide
<400> 169
   aat aact cag at cc 14
<210> 170
   <211> 14
   <212> DNA
   <213> Artificial Sequence
<220>
   <223> Synthetic Oligonucleotide
<400> 170
   aaat aact ca gatc 14
<210> 171
   <211> 14
   <212> DNA
   <213> Artificial Sequence
<220>
   <223> Synthetic Oligonucleotide

<400> 171
   aaaat aactc agat 14
<210> 172
   <211> 14
   <212> DNA
   <213> Artificial Sequence
<220>
   <223> Synthetic Oligonucleotide
<400> 172
   caaaataact caga 14
<210> 173
   <211> 14
   <212> DNA
   <213> Artificial Sequence
<220>
   <23> Synthetic Oligonucleotide
<400> 173
   gcaaaat aac t cag 14
<210> 174
   <211> 14
   <212> DNA
   <213> Artificial Sequence
<220>
   <223> Synthetic Oligonucleotide
<400> 174
   agcaaaat aa ct ca 14
<210> 175
   <211> 14
   <212> DNA
   <213> Artificial Sequence
<220>
   <223> Synthetic Oligonucleotide
<400> 175
   t agcaaaat a act c 14
<210> 176
   <211> 14
   <212> DNA
   <213> Artificial Sequence
<220>
   <223> Synthetic Oligonucleotide
<400> 176
   cagggcctgg agag 14
<210> 177
   <211> 16 <212> DNA
   <213> Artificial Sequence
<220>
   <223> Synthetic Oligonucleotide
<400> 177
   t ct gaagt cc atgat c 16
<210> 178
   <211> 14
   <212> DNA
   <213> Artificial Sequence
<220>
   <223> Synthetic Oligonucleotide
<400> 178
   ctgaagtcca tgat 14
<210> 179
   <211> 16
   <212> DNA
   <213> Artificial Sequence
<220>
   <223> Synthetic Oligonucleotide

<400> 179
   tgggcat gat t ccat t 16
<210> 180
   <211> 14
   <212> DNA
   <213> Artificial Sequence
<220>
   <223> Synthetic Oligonucleotide
<400> 180
   gggcat gat tccat 14
<210> 181
   <211> 14
   <212> DNA
   <213> Artificial Sequence
<220>
   <223> Synthetic Oligonucleotide
<400> 181
   tggagcccac gtgc 14
<210> 182
   <211> 16
   <212> DNA
   <213> Artificial Sequence
<220>
   <223> Synthetic Oligonucleotide
<400> 182
   t t gaagt tgagggct g 16
<210> 183
   <211> 14
   <212> DNA
   <213> Artificial Sequence
<220>
   <223> Synthetic Oligonucleotide
<400> 183
   tgaagt t gag ggct 14
<210> 184
   <211> 16
   <212> DNA
   <213> Artificial Sequence
<220>
   <223> Synthetic Oligonucleotide
<400> 184
   accagtatta attttg 16
<210> 185
   <211> 14
   <212> DNA
   <213> Artificial Sequence
<220>
   <223> Synthetic Oligonucleotide
<400> 185
   ccagtattaa tttt 14
<210> 186
   <211> 16
   <212> DNA
   <213> Artificial Sequence
<220>
   <223> Synthetic Oligonucleotide
<400> 186
   gt gt t ct t t g aagcgg 16
<210> 187
   <211> 14
   <212> DNA
   <213> Artificial Sequence
<220>
   <223> Synthetic Oligonucleotide
<400> 187
   t gt t ct t t ga agcg 14
<210> 188
   <211> 14
   <212> DNA
   <213> Artificial Sequence
<220>
   <223> Synthetic Oligonucleotide
<400> 188
   agttactttg gt gt 14
<210> 189
   <211> 16
   <212> DNA
   <213> Artificial Sequence
<220>
   <223> Synthetic Oligonucleotide
<400> 189
   t ggt acat gg aagt ct 16
<210> 190
   <211> 14
   <212> DNA
   <213> Artificial Sequence
<220>
   <223> Synthetic Oligonucleotide
<400> 190
   ggt acat gga agt c 14
<210> 191
   <211> 4
   <212> DNA
   <213> Artificial Sequence
<220>
   <223> Synthetic Oligonucleotide
<400> 191
   act g 4
<210> 192
   <211> 4
   <212> DNA
   <213> Artificial Sequence
<220>
   <223> Synthetic Oligonucleotide
<400> 192
   act g 4

<210> 193
   <211> 4
   <212> DNA
   <213> Artificial Sequence
<220>
   <223> Synthetic Oligonucleotide
<400> 193
   act g 4
<210> 194
   <211> 4
   <212> DNA
   <213> Artificial Sequence
<220>
   <223> Synthetic Oligonucleotide
<400> 194
   act g 4
<210> 195
   <211> 4
   <212> DNA
   <213> Artificial Sequence
<220>
   <223> Synthetic Oligonucleotide
<400> 195
   act g 4
<210> 196
   <211> 4
   <212> DNA
   <213> Artificial Sequence
<220>
   <223> Synthetic Oligonucleotide
<400> 196
   act g 4
<210> 197
   <211> 4
   <212> DNA
   <213> Artificial Sequence
<220>
   <223> Synthetic Oligonucleotide
<400> 197
   act g 4
<210> 198
   <211> 4
   <212> DNA
   <213> Artificial Sequence
<220>
   <223> Synthetic Oligonucleotide
<400> 198
   act g 4
<210> 199
   <211> 4
   <212> DNA
   <213> Artificial Sequence
<220>
   <223> Synthetic Oligonucleotide
<400> 199
   act g 4
<210> 200
   <211> 14
   <212> DNA
   <213> Artificial Sequence
<220>
   <223> Synthetic Oligonucleotide
<400> 200
   tccatgccat atgt 14
<210> 201
   <211> 16
   <212> DNA
   <213> Artificial Sequence
<220>
   <223> Synthetic Oligonucleotide
<400> 201
   ccct gaagaa gt ccat 16
<210> 202
   <211> 14
   <212> DNA
   <213> Artificial Sequence
<220>
   <223> Synthetic Oligonucleotide
<400> 202
   cctgaagaag tcca 14
<210> 203
   <211> 16
   <212> DNA
   <213> Artificial Sequence
<220>
   <223> Synthetic Oligonucleotide
<400> 203
   gcccagttcc at gacc 16
<210> 204
   <211> 14
   <212> DNA
   <213> Artificial Sequence
<220>
   <223> Synthetic Oligonucleotide
<400> 204
   cccagt t cca t gac 14
<210> 205
   <211> 16
   <212> DNA
   <213> Artificial Sequence
<220>
   <223> Synthetic Oligonucleotide
<400> 205
   ttgaggaagc cagatt 16
<210> 206
   <211> 14
   <212> DNA
   <213> Artificial Sequence
<220>
   <223> Synthetic Oligonucleotide
<400> 206
   t gaggaagcc agat 14
<210> 207
   <211> 16
   <212> DNA
   <213> Artificial Sequence
<220>
   <223> Synthetic Oligonucleotide
<400> 207
   t ggat gcagt aatctc 16
<210> 208
   <211> 14
   <212> DNA
   <213> Artificial Sequence
<220>
   <223> Synthetic Oligonucleotide
<400> 208
   ggat gcagt a at ct 14
<210> 209
   <211> 16
   <212> DNA
   <213> Artificial Sequence
<220>
   <223> Synthetic Oligonucleotide
<400> 209
   t at aaagtcc agcat t 16
<210> 210
   <211> 14
   <212> DNA
   <213> Artificial Sequence
<220>
   <223> Synthetic Oligonucleotide
<400> 210
   at aaagt cca gcat 14
<210> 211
   <211> 16
   <212> DNA
   <213> Artificial Sequence
<220>
   <223> Synthetic Oligonucleotide
<400> 211
   aagt tcct gc t tgaag 16
<210> 212
   <211> 14
   <212> DNA
   <213> Artificial Sequence
<220>
   <223> Synthetic Oligonucleotide
<400> 212
   agt t cct gct t gaa 14
<210> 213
   <211> 14
   <212> DNA
   <213> Artificial Sequence
<220>
   <223> Synthetic Oligonucleotide
<400> 213
   aat ggt gaag t act 14
<210> 214
   <211> 12
   <212> DNA
   <213> Artificial Sequence
<220>
   <223> Synthetic Oligonucleotide
<400> 214
   tgt cagcagg at 12
<210> 215
   <211> 12
   <212> DNA
   <213> Artificial Sequence
<220>
   <223> Synthetic Oligonucleotide
<400> 215
   cagcaggaaa t a 12
<210> 216
   <211> 12
   <212> DNA
   <213> Artificial Sequence
<220>
   <223> Synthetic Oligonucleotide
<400> 216
   ccagcaggaa at 12
<210> 217
   <211> 12
   <212> DNA
   <213> Artificial Sequence
<220>
   <223> Synthetic Oligonucleotide
<400> 217
   accagcagga aa 2
<210> 218
   <211> 12
   <212> DNA
   <213> Artificial Sequence

<220>
   <223> Synthetic Oligonucleotide
<400> 218
   gaccagcagg aa 12
<210> 219
   <211> 12
   <212> DNA
   <213> Artificial Sequence
<220>
   <223> Synthetic Oligonucleotide
<400> 219
   tgaccagcag ga 12
<210> 220
   <211> 4
   <212> DNA
   <213> Artificial Sequence
<220>
   <223> Synthetic Oligonucleotide
<400> 220
   act g 4
<210> 221
   <211> 12
   <212> DNA
   <213> Artificial Sequence
<220>
   <223> Synthetic Oligonucleotide
<400> 221
   atgaccagca gg 12
<210> 222
   <211> 12
   <212> DNA
   <213> Artificial Sequence
<220>
   <223> Synthetic Oligonucleotide
<400> 222
   aat gaccagc ag 12
<210> 223
   <211> 12
   <212> DNA
   <213> Artificial Sequence
<220>
   <223> Synthetic Oligonucleotide
<400> 223
   caatgaccag ca 12
<210> 224
   <211> 12
   <212> DNA
   <213> Artificial Sequence
<220>
   <223> Synthetic Oligonucleotide
<400> 224
   ccaat gacca gc 12
<210> 225
   <211> 12
   <212> DNA
   <213> Artificial Sequence
<220>
   <223> Synthetic Oligonucleotide
<400> 225
   accacaagcc aa 12
<210> 226
   <211> 12
   <212> DNA
   <213> Artificial Sequence
<220>
   <223> Synthetic Oligonucleotide
<400> 226
   tagccgccca ca 12
<210> 227
   <211> 4
   <212> DNA
   <213> Artificial Sequence
<220>
   <223> Synthetic Oligonucleotide
<400> 227
   actg 4
<210> 228
   <211> 12
   <212> DNA
   <213> Artificial Sequence
<220>
   <223> Synthetic Oligonucleotide
<400> 228
   ccggccacca ca 12
<210> 229
   <211> 12
   <212> DNA
   <213> Artificial Sequence
<220>
   <223> Synthetic Oligonucleotide
<400> 229
   accggccacc ac 12
<210> 230
   <211> 12
   <212> DNA
   <213> Artificial Sequence
<220>
   <223> Synthetic Oligonucleotide
<400> 230
   gatgttgct ggc 12
<210> 231
   <211> 4
   <212> DNA
   <213> Artificial Sequence
<220>
   <223> Synthetic Oligonucleotide
<400> 231
   act g 4
<210> 232
   <211> 12
   <212> DNA
   <213> Artificial Sequence
<220>
   <223> Synthetic Oligonucleotide
<400> 232
   cgat gt t gct gg 12
<210> 233
   <211> 12
   <212> DNA
   <213> Artificial Sequence
<220>
   <223> Synthetic Oligonucleotide
<400> 233
   ccgat gt t gc tg 12
<210> 234
   <211> 12
   <212> DNA
   <213> Artificial Sequence
<220>
   <223> Synthetic Oligonucleotide
<400> 234
   gccgat gttg ct 12
<210> 235
   <211> 12
   <212> DNA
   <213> Artificial Sequence
<220>
   <223> Synthetic Oligonucleotide

<400> 235
   t gccgat gttgc 12
<210> 236
   <211> 12
   <212> DNA
   <213> Artificial Sequence
<220>
   <223> Synthetic Oligonucleotide
<400> 236
   ct gccgat gt tg 12
<210> 237
   <211> 12
   <212> DNA
   <213> Artificial Sequence
<220>
   <223> Synthetic Oligonucleotide
<400> 237
   caggcccaca aa 12
<210> 238
   <211> 12
   <212> DNA
   <213> Artificial Sequence
<220>
   <223> Synthetic Oligonucleotide
<400> 238
   ccaggcccac aa 12
<210> 239
   <211> 12
   <212> DNA
   <213> Artificial Sequence
<220>
   <223> Synthetic Oligonucleotide
<400> 239
   gccaggccca ca 12
<210> 240
   <211> 12
   <212> DNA
   <213> Artificial Sequence
<220>
   <223> Synthetic Oligonucleotide
<400> 240
   ccaagccact tg 12
<210> 241
   <211> 12
   <212> DNA
   <213> Artificial Sequence
<220>
   <223> Synthetic Oligonucleotide
<400> 241
   agagcgcatt cc 12
<210> 242
   <211> 12
   <212> DNA
   <213> Artificial Sequence
<220>
   <223> Synthetic Oligonucleotide
<400> 242
   acaggtagag gc 12
<210> 243
   <211> 12
   <212> DNA
   <213> Artificial Sequence
<220>
   <223> Synthetic Oligonucleotide
<400> 243
   agat ct t ggt ga 12
<210> 244
   <211> 4
   <212> DNA
   <213> Artificial Sequence
<220>
   <223> Synthetic Oligonucleotide
<400> 244
   act g 4
<210> 245
   <211> 13
   <212> DNA
   <213> Artificial Sequence
<220>
   <223> Synthetic Oligonucleotide
<400> 245
   tgttccagcc cag 13
<210> 246
   <211> 12
   <212> DNA
   <213> Artificial Sequence
<220>
   <223> Synthetic Oligonucleotide
<400> 246
   tgttccagcc ca 12
<210> 247
   <211> 4
   <212> DNA
   <213> Artificial Sequence
<220>
   <223> Synthetic Oligonucleotide
<400> 247
   act g 4
<210> 248
   <211> 4
   <212> DNA
   <213> Artificial Sequence
<220>
   <223> Synthetic Oligonucleotide
<400> 248
   act g 4
<210> 249
   <211> 4
   <212> DNA
   <213> Artificial Sequence
<220>
   <223> Synthetic Oligonucleotide
<400> 249
   act g 4
<210> 250
   <211> 4
   <212> DNA
   <213> Artificial Sequence
<220>
   <223> Synthetic Oligonucleotide
<400> 250
   act g 4
<210> 251
   <211> 12
   <212> DNA
   <213> Artificial Sequence
<220>
   <223> Synthetic Oligonucleotide
<400> 251
   atgttccagc cc 12

<210> 252
   <211> 12
   <212> DNA
   <213> Artificial Sequence
<220>
   <223> Synthetic Oligonucleotide
<400> 252
   t ggt gat gcc ca 12
<210> 253
   <211> 12
   <212> DNA
   <213> Artificial Sequence
<220>
   <223> Synthetic Oligonucleotide
<400> 253
   atggtgatgc cc 12
<210> 254
   <211> 12
   <212> DNA
   <213> Artificial Sequence
<220>
   <223> Synthetic Oligonucleotide
<400> 254
   catggtgatg cc 12
<210> 255
   <211> 4
   <212> DNA
   <213> Artificial Sequence
<220>
   <223> Synthetic Ogonucleotide
<400> 255
   act g 4
<210> 256
   <211> 12
   <212> DNA
   <213> Artificial Sequence
<220>
   <223> Synthetic Oligonucleotide
<400> 256
   tcatggt gat gc 12
<210> 257
   <211> 12
   <212> DNA
   <213> Artificial Sequence
<220>
   <223> Synthetic Oligonucleotide
<400> 257
   at cat ggt gat g 12
<210> 258
   <211> 12
   <212> DNA
   <213> Artificial Sequence
<220>
   <223> Synthetic Oligonucleotide
<400> 258
   ccct cct gt c ac 12
<210> 259
   <211> 12
   <212> DNA
   <213> Artificial Sequence
<220>
   <223> Synthetic Oligonucleotide
<400> 259
   gccct cct gt ca 12
<210> 260
   <211> 12
   <212> DNA
   <213> Artificial Sequence
<220>
   <223> Synthetic Oligonucleotide
<400> 260
   agccct cct g tc 12
<210> 261
   <211> 12
   <212> DNA
   <213> Artificial Sequence
<220>
   <223> Synthetic Oligonucleotide
<400> 261
   cagccct cct gt 12
<210> 262
   <211> 12
   <212> DNA
   <213> Artificial Sequence
<220>
   <223> Synthetic Oligonucleotide
<400> 262
   ccagccctcc tg 12
<210> 263
   <211> 12
   <212> DNA
   <213> Artificial Sequence
<220>
   <223> Synthetic Oligonucleotide
<400> 263
   gccagccct c ct 12
<210> 264
   <211> 12
   <212> DNA
   <213> Artificial Sequence
<220>
   <223> Synthetic Oligonucleotide
<400> 264
   gacgaaggt c tg 12
<210> 265
   <211> 12
   <212> DNA
   <213> Artificial Sequence
<220>
   <223> Synthetic Oligonucleotide
<400> 265
   gt at t t gt cg aa 12
<210> 266
   <211> 12
   <212> DNA
   <213> Artificial Sequence
<220>
   <223> Synthetic Oligonucleotide
<400> 266
   ggacaccgt c ag 12
<210> 267
   <211> 12
   <212> DNA
   <213> Artificial Sequence
<220>
   <223> Synthetic Oligonucleotide
<400> 267
   cagct t cagg t a 12

<210> 268
   <211> 12
   <212> DNA
   <213> Artificial Sequence
<220>
   <223> Synthetic Oligonucleotide
<400> 268
   catgaccatg ag 12
<210> 269
   <211> 12
   <212> DNA
   <213> Artificial Sequence
<220>
   <223> Synthetic Oligonucleotide
<400> 269
   gcatgaccat ga 12
<210> 270
   <211> 12
   <212> DNA
   <213> Artificial Sequence
<220>
   <223> Synthetic Oligonucleotide
<400> 270
   ggcat gacca t g 12
<210> 271
   <211> 12
   <212> DNA
   <213> Artificial Sequence
<220>
   <223> Synthetic Oligonucleotide
<400> 271
   t ggcat gacc at 12
<210> 272
   <211> 12
   <212> DNA
   <213> Artificial Sequence
<220>
   <223> Synthetic Oligonucleotide
<400> 272
   ct ggcat gac ca 12
<210> 273
   <211> 4
   <212> DNA
   <213> Artificial Sequence
<220>
   <223> Synthetic Oligonucleotide
<400> 273
   act g 4
<210> 274
   <211> 12
   <212> DNA
   <213> Artificial Sequence
<220>
   <223> Synthetic Oligonucleotide
<400> 274
   cctggcatga cc * 12
<210> 275
   <211> 12
   <212> DNA
   <213> Artificial Sequence
<220>
   <223> Synthetic Oligonucleotide
<400> 275
   gcagcccacc tc 12
<210> 276
   <211> 12
   <212> DNA
   <213> Artificial Sequence
<220>
   <223> Synthetic Oligonucleotide
<400> 276
   agcagcccac ct 12
<210> 277
   <211> 12
   <212> DNA
   <213> Artificial Sequence
<220>
   <223> Synthetic Oligonucleotide
<400> 277
   gagcagccca cc 12
<210> 278
   <211> 12
   <212> DNA
   <213> Artificial Sequence
<220>
   <223> Synthetic Oligonucleotide
<400> 278
   cat gagct t c ac 12
<210> 279
   <211> 12
   <212> DNA
   <213> Artificial Sequence
<220>
   <223> Synthetic Oligonucleotide
<400> 279
   gcat gagct t ca 12
<210> 280
   <211> 13
   <212> DNA
   <213> Artificial Sequence
<220>
   <223> Synthetic Oligonucleotide
<400> 280
   ggcat gagct t ca 13
<210> 281
   <211> 12
   <212> DNA
   <213> Artificial Sequence
<220>
   <223> Synthetic Oligonucleotide
<400> 281
   ggcat gagct t c 12
<210> 282
   <211> 4
   <212> DNA
   <213> Artificial Sequence
<220>
   <223> Synthetic Oligonucleotide
<400> 282
   act g 4
<210> 283
   <211> 12
   <212> DNA
   <213> Artificial Sequence
<220>
   <223> Synthetic Oligonucleotide
<400> 283
   gggcat gagc t t 12
<210> 284
   <211> 12
   <212> DNA
   <213> Artificial Sequence
<220>
   <223> Synthetic Oligonucleotide
<400> 284
   cagcatgagt cc 12
<210> 285
   <211> 12
   <212> DNA
   <213> Artificial Sequence
<220>
   <223> Synthetic Oligonucleotide
<400> 285
   ccagcatgag tc 12
<210> 286
   <211> 4
   <212> DNA
   <213> Artificial Sequence
<220>
   <223> Synthetic Oligonucleotide
<400> 286
   act g 4
<210> 287
   <211> 12
   <212> DNA
   <213> Artificial Sequence
<220>
   <223> Synthetic Oligonucleotide
<400> 287
   gccagcatga gt 12
<210> 288
   <211> 12
   <212> DNA
   <213> Artificial Sequence
<220>
   <223> Synthetic Oligonucleotide
<400> 288
   ccat ggt gaa ga 12
<210> 289
   <211> 12
   <212> DNA
   <213> Artificial Sequence

<220>
   <223> Synthetic Oligonucleotide
<400> 289
   cacagctgcc cg 12
<210> 290
   <211> 12
   <212> DNA
   <213> Artificial Sequence
<220>
   <223> Synthetic Oligonucleotide
<400> 290
   acacagctgc cc 12
<210> 291
   <211> 12
   <212> DNA
   <213> Artificial Sequence
<220>
   <223> Synthetic Oligonucleotide
<400> 291
   cacacagctg cc 12
<210> 292
   <211> 13
   <212> DNA
   <213> Artificial Sequence
<220>
   <223> Synthetic Oligonucleotide
<400> 292
   gcacacagct gcc 13
<210> 293
   <211> 12
   <212> DNA
   <213> Artificial Sequence
<220>
   <223> Synthetic Oligonucleotide
<400> 293
   gcacacagct gc 12
<210> 294
   <211> 4
   <212> DNA
   <213> Artificial Sequence
<220>
   <223> Synthetic Oligonucleotide
<400> 294
   act g 4
<210> 295
   <211> 12
   <212> DNA
   <213> Artificial Sequence
<220>
   <223> Synthetic Oligonucleotide
<400> 295
   gccggagact ga 12
<210> 296
   <211> 12
   <212> DNA
   <213> Artificial Sequence
<220>
   <223> Synthetic Oligonucleotide
<400> 296
   attgaggttg ac 12
<210> 297
   <211> 12
   <212> DNA
   <213> Artificial Sequence
<220>
   <223> Synthetic Oligonucleotide
<400> 297
   cat t gaggt t ga 12
<210> 298
   <211> 12
   <212> DNA
   <213> Artificial Sequence
<220>
   <223> Synthetic Oligonucleotide
<400> 298
   gcat t gaggt t g 12
<210> 299
   <211> 12
   <212> DNA
   <213> Artificial Sequence
<220>
   <223> Synthetic Oligonucleotide
<400> 299
   ggcat t gagg t t 12
<210> 300
   <211> 12 <212> DNA
   <213> Artificial Sequence
<220>
   <223> Synthetic Oligonucleotide
<400> 300
   gggcat t gag gt 12
<210> 301
   <211> 4
   <212> DNA
   <213> Artificial Sequence
<220>
   <223> Synthetic Oligonucleotide
<400> 301
   actg 4
<210> 302
   <211> 4
   <212> DNA
   <213> Artificial Sequence
<220>
   <223> Synthetic Oligonucleotide
<400> 302
   act g 4
<210> 303
   <211> 4
   <212> DNA
   <213> Artificial Sequence
<220>
   <223> Synthetic Oligonucleotide
<400> 303
   act g 4
<210> 304
   <211> 4
   <212> DNA
   <213> Artificial Sequence
<220>
   <223> Synthetic Oligonucleotide
<400> 304
   act g 4
<210> 305
   <211> 4
   <212> DNA
   <213> Artificial Sequence

<220>
   <223> Synthetic Oligonucleotide
<400> 305
   act g 4
<210> 306
   <211> 4
   <212> DNA
   <213> Artificial Sequence
<220>
   <223> Synthetic Oligonucleotide
<400> 306
   act g 4
<210> 307
   <211> 4
   <212> DNA
   <213> Artificial Sequence
<220>
   <223> Synthetic Oligonucleotide
<400> 307
   act g 4
<210> 308
   <211> 4
   <212> DNA
   <213> Artificial Sequence
<220>
   <223> Synthetic Oligonucleotide
<400> 308
   act g 4
<210> 309
   <211> 4
   <212> DNA
   <213> Artificial Sequence
<220>
   <223> Synthetic Oligonucleotide
<400> 309
   act g 4
<210> 310
   <211> 4
   <212> DNA
   <213> Artificial Sequence
<220>
   <223> Synthetic Oligonucleotide
<400> 310
   act g 4
<210> 311
   <211> 4
   <212> DNA
   <213> Artificial Sequence
<220>
   <223> Synthetic Oligonucleotide
<400> 311
   act g 4
<210> 312
   <211> 4
   <212> DNA
   <213> Artificial Sequence
<220>
   <223> Synthetic Oligonucleotide
<400> 312
   act g 4
<210> 313
   <211> 4
   <212> DNA
   <213> Artificial Sequence
<220>
   <223> Synthetic Oligonucleotide
<400> 313
   act g 4
<210> 314
   <211> 4
   <212> DNA
   <213> Artificial Sequence
<220>
   <223> Synthetic Oligonucleotide
<400> 314
   act g 4
<210> 315
   <211> 4
   <212> DNA
   <213> Artificial Sequence
<220>
   <223> Synthetic Oligonucleotide
<400> 315
   act g 4
<210> 316
   <211> 4
   <212> DNA
   <213> Artificial Sequence
<220>
   <223> Synthetic Oligonucleotide
<400> 316
   act g 4
<210> 317
   <211> 4
   <212> DNA
   <213> Artificial Sequence
<220>
   <223> Synthetic Oligonucleotide
<400> 317
   act g 4
<210> 318
   <211> 4
   <212> DNA
   <213> Artificial Sequence
<220>
   <223> Synthetic Oligonucleotide
<400> 318
   act g 4
<210> 319
   <211> 4
   <212> DNA
   <213> Artificial Sequence
<220>
   <223> Synthetic Oligonucleotide
<400> 319
   act g 4
<210> 320
   <211> 4
   <212> DNA
   <213> Artificial Sequence
<220>
   <223> Synthetic Oligonucleotide
<400> 320
   act g 4
<210> 321
   <211> 4
   <212> DNA
   <213> Artificial Sequence
<220>
   <223> Synthetic Oligonucleotide
<400> 321
   act g 4
<210> 322
   <211> 4
   <212> DNA
   <213> Artificial Sequence
<220>
   <223> Synthetic Oligonucleotide
<400> 322
   act g 4
<210> 323
   <211> 4
   <212> DNA
   <213> Artificial Sequence
<220>
   <223> Synthetic Oligonucleotide
<400> 323
   act g 4
<210> 324
   <211> 4
   <212> DNA
   <213> Artificial Sequence
<220>
   <223> Synthetic Oligonucleotide
<400> 324
   act g 4
<210> 325
   <211> 4
   <212> DNA
   <213> Artificial Sequence
<220>
   <223> Synthetic Oligonucleotide
<400> 325
   act g 4
<210> 326
   <211> 4
   <212> DNA
   <213> Artificial Sequence
<220>
   <223> Synthetic Oligonucleotide

<400> 326
   act g 4
<210> 327
   <211> 4 <212> DNA
   <213> Artificial Sequence
<220>
   <223> Synthetic Oligonucleotide
<400> 327
   act g 4
<210> 328
   <211> 4
   <212> DNA
   <213> Artificial Sequence
<220>
   <223> Synthetic Oligonucleotide
<400> 328
   act g 4
<210> 329
   <211> 14
   <212> DNA
   <213> Artificial Sequence
<220>
   <223> Synthetic Oligonucleotide
<400> 329
   at ggggcaac t t ca 14
<210> 330
   <211> 14
   <212> DNA
   <213> Artificial Sequence
<220>
   <223> Synthetic Oligonucleotide
<400> 330
   cat ggggcaa ct t c 14
<210> 331
   <211> 14
   <212> DNA
   <213> Artificial Sequence
<220>
   <223> Synthetic Oligonucleotide
<400> 331
   acat ggggca act t 14
<210> 332
   <211> 14
   <212> DNA
   <213> Artificial Sequence
<220>
   <223> Synthetic Oligonucleotide
<400> 332
   gggatgctct gggc 14
<210> 333
   <211> 14
   <212> DNA
   <213> Artificial Sequence
<220>
   <223> Synthetic Oligonucleotide
<400> 333
   cgct ccaggt t cca 14
<210> 334
   <211> 14
   <212> DNA
   <213> Artificial Sequence
<220>
   <223> Synthetic Oligonucleotide
<400> 334
   gat acacct c cacc 14
<210> 335
   <211> 14
   <212> DNA
   <213> Artificial Sequence
<220>
   <223> Synthetic Oligonucleotide
<400> 335
   agatacacct ccac 14
<210> 336
   <211> 14
   <212> DNA
   <213> Artificial Sequence
<220>
   <223> Synthetic Oligonucleotide
<400> 336
   gagat acacc t cca 14
<210> 337
   <211> 14
   <212> DNA
   <213> Artificial Sequence
<220>
   <223> Synthetic Oligonucleotide
<400> 337
   gcct gt ct gt ggaa 14
<210> 338
   <211> 14
   <212> DNA
   <213> Artificial Sequence
<220>
   <223> Synthetic Oligonucleotide
<400> 338
   ct gt cacact t gct 14
<210> 339
   <211> 14
   <212> DNA
   <213> Artificial Sequence
<220>
   <223> Synthetic Oligonucleotide
<400> 339
   cggccgctga ccac 14
<210> 340
   <211> 14
   <212> DNA
   <213> Artificial Sequence
<220>
   <223> Synthetic Oligonucleotide
<400> 340
   ccggccgctg acca 14
<210> 341
   <211> 14
   <212> DNA
   <213> Artificial Sequence
<220>
   <223> Synthetic Oligonucleotide
<400> 341
   cccggccgct gacc 14
<210> 342
   <211> 14
   <212> DNA
   <213> Artificial Sequence
<220>
   <223> Synthetic Oligonucleotide
<400> 342
   tcccggccgc tgac 14
<210> 343
   <211> 14
   <212> DNA,
   <213> Artificial Sequence
<220>
   <223> Synthetic Oligonucleotide
<400> 343
   atcccggccg ctga 14
<210> 344
   <211> 14
   <212> DNA
   <213> Artificial Sequence
<220>
   <223> Synthetic Oligonucleotide
<400> 344
   catcccggcc gct g 14
<210> 345
   <211> 14
   <212> DNA
   <213> Artificial Sequence
<220>
   <223> Synthetic Oligonucleotide
<400> 345
   gcatcccggc cgct 14
<210> 346
   <211> 14
   <212> DNA
   <213> Artificial Sequence
<220>
   <223> Synthetic Oligonucleotide
<400> 346
   gt gccct t cc ct t g 14
<210> 347
   <211> 14
   <212> DNA
   <213> Artificial Sequence
<220>
   <223> Synthetic Oligonucleotide
<400> 347
   at gagggt gc cgct 14
<210> 348
   <211> 14
   <212> DNA
   <213> Artificial Sequence
<220>
   <223> Synthetic Oligonucleotide
<400> 348
   t at gagggt g ccgc 14
<210> 349
   <211> 14
   <212> DNA
   <213> Artificial Sequence
<220>
   <223> Synthetic Oligonucleotide
<400> 349
   ct at gagggt gccg 14
<210> 350
   <211> 14
   <212> DNA
   <213> Artificial Sequence
<220>
   <223> Synthetic Oligonucleotide
<400> 350
   cct at gaggg t gcc 14
<210> 351
   <211> 14
   <212> DNA
   <213> Artificial Sequence
<220>
   <223> Synthetic Oligonucleotide
<400> 351
   cgaataaact ccag 14
<210> 352
   <211> 14
   <212> DNA
   <213> Artificial Sequence
<220>
   <223> Synthetic Oligonucleotide
<400> 352
   ccgaat aaac t cca 14
<210> 353
   <211> 14
   <212> DNA
   <213> Artificial Sequence
<220>
   <223> Synthetic Oligonucleotide
<400> 353
   t ccgaat aaa ct cc 14
<210> 354
   <211> 14
   <212> DNA
   <213> Artificial Sequence
<220>
   <223> Synthetic Oligonucleotide
<400> 354
   t t ccgaat aa act c 14
<210> 355
   <211> 14
   <212> DNA
   <213> Artificial Sequence
<220>
   <223> Synthetic Oligonucleotide
<400> 355
   gccaggggca gcag 14
<210> 356
   <211> 14
   <212> DNA
   <213> Artificial Sequence
<220>
   <223> Synthetic Oligonucleotide
<400> 356
   gagt agaggc aggc 14
<210> 357
   <211> 14
   <212> DNA
   <213> Artificial Sequence
<220>
   <223> Synthetic Oligonucleotide
<400> 357
   ccccaaagtc ccca 14
<210> 358
   <211> 14
   <212> DNA
   <213> Artificial Sequence
<220>
   <223> Synthetic Oligonucleotide
<400> 358
   tccccaaagt cccc 14
<210> 359
   <211> 14
   <212> DNA
   <213> Artificial Sequence
<220>
   <223> Synthetic Oligonucleotide
<400> 359
   gt ccccaaag t ccc 14
<210> 360
   <211> 14
   <212> DNA
   <213> Artificial Sequence
<220>
   <223> Synthetic Oligonucleotide
<400> 360
   acgtgggcag cagc 14
<210> 361
   <211> 14
   <212> DNA
   <213> Artificial Sequence
<220>
   <223> Synthetic Oligonucleotide
<400> 361
   cacgtgggca gcag 14
<210> 362
   <211> 14
   <212> DNA
   <213> Artificial Sequence
<220>
   <223> Synthetic Oligonucleotide
<400> 362
   ccacgtgggc agca 14
<210> 363
   <211> 14
   <212> DNA
   <213> Artificial Sequence
<220>
   <223> Synthetic Oligonucleotide
<400> 363
   gccacgtggg cagc 14
<210> 364
   <211> 14
   <212> DNA
   <213> Artificial Sequence
<220>
   <223> Synthetic Oligonucleotide
<400> 364
   cagggaacca ggcc 14
<210> 365
   <211> 14
   <212> DNA
   <213> Artificial Sequence
<220>
   <223> Synthetic Oligonucleotide
<400> 365
   tcagggaacc aggc 14
<210> 366
   <211> 14
   <212> DNA
   <213> Artificial Sequence
<220>
   <223> Synthetic Oligonucleotide
<400> 366
   ct cagggaac cagg 14
<210> 367
   <211> 14
   <212> DNA
   <213> Artificial Sequence
<220>
   <223> Synthetic Oligonucleotide
<400> 367
   cctcagggaa ccag 14
<210> 368
   <211> 14
   <212> DNA
   <213> Artificial Sequence
<220>
   <223> Synthetic Oligonucleotide
<400> 368
   tcctcaggga acca 14
<210> 369
   <211> 14
   <212> DNA
   <213> Artificial Sequence
<220>
   <223> Synthetic Oligonucleotide
<400> 369
   gt cct caggg aacc 14
<210> 370
   <211> 14
   <212> DNA
   <213> Artificial Sequence
<220>
   <223> Synthetic Oligonucleotide
<400> 370
   ggt cct cagg gaac 14
<210> 371
   <211> 14
   <212> DNA
   <213> Artificial Sequence
<220>
   <223> Synthetic Oligonucleotide
<400> 371
   t ggt cct cag ggaa 14
<210> 372
   <211> 14
   <212> DNA
   <213> Artificial Sequence
<220>
   <223> Synthetic Oligonucleotide
<400> 372
   ctggtcctca ggga 14
<210> 373
   <211> 14
   <212> DNA
   <213> Artificial Sequence
<220>
   <223> Synthetic Oligonucleotide
<400> 373
   gct ggt cct c aggg 14
<210> 374
   <211> 14
   <212> DNA
   <213> Artificial Sequence
<220>
   <223> Synthetic Oligonucleotide
<400> 374
   gtgctggggg gcag 14
<210> 375
   <211> 14
   <212> DNA
   <213> Artificial Sequence
<220>
   <223> Synthetic Oligonucleotide
<400> 375
   ggt gct gggg ggca 14
<210> 376
   <211> 14
   <212> DNA
   <213> Artificial Sequence
<220>
   <223> Synthetic Oligonucleotide
<400> 376
   gggt gct ggg gggc 14
<210> 377
   <211> 14
   <212> DNA
   <213> Artificial Sequence
<220>
   <223> Synthetic Oligonucleotide
<400> 377
   t gggt gct gg gggg 14
<210> 378
   <211> 14
   <212> DNA
   <213> Artificial Sequence
<220>
   <223> Synthetic Oligonucleotide
<400> 378
   gagcagct ca gcag 14
<210> 379
   <211> 14
   <212> DNA
   <213> Artificial Sequence
<220>
   <223> Synthetic Oligonucleotide
<400> 379
   ggagcagct c agca 14
<210> 380
   <211> 14
   <212> DNA
   <213> Artificial Sequence
<220>
   <223> Synthetic Oligonucleotide
<400> 380
   tggagcagct cagc 14
<210> 381
   <211> 14
   <212> DNA
   <213> Artificial Sequence
<220>
   <223> Synthetic Oligonucleotide
<400> 381
   ct ggagcagc t cag 14
<210> 382
   <211> 14
   <212> DNA
   <213> Artificial Sequence
<220>
   <223> Synthetic Oligonucleotide
<400> 382
   ccct cacccc caaa 14
<210> 383
   <211> 14
   <212> DNA
   <213> Artificial Sequence
<220>
   <223> Synthetic Oligonucleotide
<400> 383
   accctcaccc ccaa 14
<210> 384
   <211> 14
   <212> DNA
   <213> Artificial Sequence
<220>
   <223> Synthetic Oligonucleotide
<400> 384
   caccctcacc ccca 14
<210> 385
   <211> 14
   <212> DNA
   <213> Artificial Sequence
<220>
   <223> Synthetic Oligonucleotide
<400> 385
   acaccctcac cccc 14
<210> 386
   <211> 14
   <212> DNA
   <213> Artificial Sequence
<220>
   <223> Synthetic Oligonucleotide
<400> 386
   gacaccctca cccc 14
<210> 387
   <211> 14
   <212> DNA
   <213> Artificial Sequence
<220>
   <223> Synthetic Oligonucleotide
<400> 387
   agacaccct c accc 14
<210> 388
   <211> 14
   <212> DNA
   <213> Artificial Sequence
<220>
   <223> Synthetic Oligonucleotide
<400> 388
   tagacaccct cacc 14
<210> 389
   <211> 14
   <212> DNA
   <213> Artificial Sequence
<220>
   <223> Synthetic Oligonucleotide
<400> 389
   tggcagcagg aagc 14
<210> 390
   <211> 14
   <212> DNA
   <213> Artificial Sequence
<220>
   <223> Synthetic Oligonucleotide

<400> 390
   at ggcagcag gaag 14
<210> 391
   <211> 14
   <212> DNA
   <213> Artificial Sequence
<220>
   <223> Synthetic Oligonucleotide
<400> 391
   cat ggcagca ggaa 14
<210> 392
   <211> 14
   <212> DNA
   <213> Artificial Sequence
<220>
   <223> Synthetic Oligonucleotide
<400> 392
   gcat ggcagc agga 14
<210> 393
   <211> 14
   <212> DNA
   <213> Artificial Sequence
<220>
   <223> Synthetic Oligonucleotide
<400> 393
   ggcagcagat ggca 14
<210> 394
   <211> 14
   <212> DNA
   <213> Artificial Sequence
<220>
   <223> Synthetic Oligonucleotide
<400> 394
   cggcagcaga tggc 14
<210> 395
   <211> 14
   <212> DNA
   <213> Artificial Sequence
<220>
   <223> Synthetic Oligonucleotide
<400> 395
   ccggcagcag atgg 14
<210> 396
   <211> 14
   <212> DNA
   <213> Artificial Sequence
<220>
   <223> Synthetic Oligonucleotide
<400> 396
   tccggcagca gatg 14

<210> 397
   <211> 14
   <212> DNA
   <213> Artificial Sequence
<220>
   <223> Synthetic Oligonucleotide
<400> 397
   ctccggcagc agat 14
<210> 398
   <211> 14
   <212> DNA
   <213> Artificial Sequence
<220>
   <223> Synthetic Oligonucleotide
<400> 398
   gct ccggcag caga 14
<210> 399
   <211> 14
   <212> DNA
   <213> Artificial Sequence
<220>
   <223> Synthetic Oligonucleotide
<400> 399
   ggctccggca gcag 14
<210> 400
   <211> 14
   <212> DNA
   <213> Artificial Sequence
<220>
   <223> Synthetic Oligonucleotide
<400> 400
   cggctccggc agca 14
<210> 401
   <211> 14
   <212> DNA
   <213> Artificial Sequence
<220>
   <223> Synthetic Oligonucleotide
<400> 401
   ccggctccgg cagc 14
<210> 402
   <211> 14
   <212> DNA
   <213> Artificial Sequence
<220>
   <223> Synthetic Oligonucleotide
<400> 402
   gccggct ccg gcag 14
<210> 403
   <211> 14
   <212> DNA
   <213> Artificial Sequence
<220>
   <223> Synthetic Oligonucleotide
<400> 403
   agttacaaaa gcaa 14
<210> 404
   <211> 4
   <212> DNA
   <213> Artificial Sequence
<220>
   <223> Synthetic Oligonucleotide
<400> 404
   actg 4
<210> 405
   <211> 4
   <212> DNA
   <213> Artificial Sequence
<220>
   <223> Synthetic Oligonucleotide
<400> 405
   actg 4
<210> 406
   <211> 16
   <212> DNA
   <213> Artificial Sequence
<220>
   <223>Synthetic Oligonucleotide
<400> 406
   gtcgcccttc agcacg 16
<210> 407
   <211> 14
   <212> DNA
   <213> Artificial Sequence
<220>
   <223> Synthetic Oligonucleotide
<400> 407
   t cgccct t ca gcac 14
<210> 408
   <211> 12
   <212> DNA
   <213> Artificial Sequence
<220>
   <223> Synthetic Oligonucleotide
<400> 408
   cgcccttcag ca 12
<210> 409
   <211> 16
   <212> DNA
   <213> Artificial Sequence
<220>
   <223> Synthetic Oligonucleotide
<400> 409
   actctggacc caaacc 16
<210> 410
   <211> 14
   <212> DNA
   <213> Artificial Sequence
<220>
   <223> Synthetic Oligonucleotide
<400> 410
   ct ct ggaccc aaac 14
<210> 411
   <211> 16
   <212> DNA
   <213> Artificial Sequence
<220>
   <223> Synthetic Oligonucleotide
<400> 411
   ccatttcagg agacct 16
<210> 412
   <211> 14
   <212> DNA
   <213> Artificial Sequence
<220>
   <223> Synthetic Oligonucleotide
<400> 412
   catttcagga gacc 14
<210> 413
   <211> 14
   <212> DNA
   <213> Artificial Sequence
<220>
   <223> Synthetic Oligonucleotide
<400> 413
   t t tgggaggt ggt c 14
<210> 414
   <211> 14
   <212> DNA
   <213> Artificial Sequence
<220>
   <223> Synthetic Oligonucleotide
<400> 414
   cacaccaggc agag 14
<210> 415
   <211> 14
   <212> DNA
   <213> Artificial Sequence
<220>
   <223> Synthetic Oligonucleotide
<400> 415
   ctttacagct tcca 14
<210> 416
   <211> 14
   <212> DNA
   <213> Artificial Sequence
<220>
   <223> Synthetic Oligonucleotide
<400> 416
   cact acct t ccact 14
<210> 417
   <211> 14
   <212> DNA
   <213> Artificial Sequence
<220>
   <223> Synthetic Oligonucleotide
<400> 417
   aacacacact acct 14
<210> 418
   <211> 14
   <212> DNA
   <213> Artificial Sequence
<220>
   <223> Synthetic Oligonucleotide
<400> 418
   ctcttcaaaa caca 14
<210> 419
   <211> 14
   <212> DNA
   <213> Artificial Sequence
<220>
   <223> Synthetic Oligonucleotide
<400> 419
   gt aat tgtgc t gt c 14
<210> 420
   <211> 14
   <212> DNA
   <213> Artificial Sequence
<220>
   <223> Synthetic Oligonucleotide
<400> 420
   tttttcttcg aatt t 14
<210> 421
   <211> 14
   <212> DNA
   <213> Artificial Sequence
<220>
   <223> Synthetic Oligonucleotide
<400> 421
   cattttcgat agcg 14
<210> 422
   <211> 14
   <212> DNA
   <213> Artificial Sequence
<220>
   <223> Synthetic Oligonucleotide
<400> 422
   accttccagg ttca 14
<210> 423
   <211> 14
   <212> DNA
   <213> Artificial Sequence
<220>
   <223> Synthetic Oligonucleotide
<400> 423
   at aggaagca t aaa 14
<210> 424
   <211> 14
   <212> DNA
   <213> Artificial Sequence
<220>
   <223> Synthetic Oligonucleotide
<400> 424
   tcttttaaag aaga 14
<210> 425
   <211> 14
   <212> DNA
   <213> Artificial Sequence
<220>
   <223> Synthetic Oligonucleotide
<400> 425
   aaggat at t t taaa 14
<210> 426
   <211> 4
   <212> DNA
   <213> Artificial Sequence
<220>
   <223> Synthetic Oligonucleotide
<400> 426
   act g 4
<210> 427
   <211> 14
   <212> DNA
   <213> Artificial Sequence
<220>
   <223> Synthetic Oligonucleotide
<400> 427
   gaacaaaaat t aaa 14
<210> 428
   <211> 14
   <212> DNA
   <213> Artificial Sequence
<220>
   <223> Synthetic Oligonucleotide
<400> 428
   t t ccacagat ctgt 14
<210> 429
   <211> 14
   <212> DNA
   <213> Artificial Sequence
<220>
   <223> Synthetic Oligonucleotide
<400> 429
   t t tataaagt aaag 14
<210> 430
   <211> 4
   <212> DNA
   <213> Artificial Sequence
<220>
   <223> Synthetic Oligonucleotide
<400> 430
   act g 4

<210> 431
   <211> 4
   <212> DNA
   <213> Artificial Sequence
<220>
   <223> Synthetic Oligonucleotide
<400> 431
   actg 4
<210> 432
   <211> 4
   <212> DNA
   <213> Artificial Sequence
<220>
   <223> Synthetic Oligonucleotide
<400> 432
   actg 4
<210> 433
   <211> 14
   <212> DNA
   <213> Artificial Sequence
<220>
   <223> Synthetic Oligonucleotide
<400> 433
   t act gt gaga aat a 14
<210> 434
   <211> 4
   <212> DNA
   <213> Artificial Sequence
<220>
   <223> Synthetic Oligonucleotide
<400> 434
   actg 4
<210> 435
   <211> 14
   <212> DNA
   <213> Artificial Sequence
<220>
   <223> Synthetic Oligonucleotide
<400> 435
   t t ccagcttg aaga 14
<210> 436
   <211> 14
   <212> DNA
   <213> Artificial Sequence
<220>
   <223> Synthetic Oligonucleotide
<400> 436
   gat cagt t ct catg 14
<210> 437
   <211> 4
   <212> DNA
   <213> Artificial Sequence
<220>
   <223> Synthetic Oligonucleotide
<400> 437
   act g 4
<210> 438
   <211> 4
   <212> DNA
   <213> Artificial Sequence
<220>
   <223> Synthetic Oligonucleotide
<400> 438
   act g 4
<210> 439
   <211> 14
   <212> DNA
   <213> Artificial Sequence
<220>
   <223> Synthetic Oligonucleotide
<400> 439
   ttatcaatga t gca 14
<210> 440
   <211> 14
   <212> DNA
   <213> Artificial Sequence
<220>
   <223> Synthetic Oligonucleotide
<400> 440
   gcat gct gga cagt 14
<210> 441
   <211> 4
   <212> DNA
   <213> Artificial Sequence
<220>
   <223> Synthetic Oligonucleotide
<400> 441
   act g 4
<210> 442
   <211> 4
   <212> DNA
   <213> Artificial Sequence
<220>
   <223> Synthetic Oligonucleotide
<400> 442
   act g 4
<210> 443
   <211> 14
   <212> DNA
   <213> Artificial Sequence
<220>
   <223> Synthetic Oligonucleotide
<400> 443
   t t gcacct ga act a 14
<210> 444
   <211> 14
   <212> DNA
   <213> Artificial Sequence
<220>
   <223> Synthetic Oligonucleotide
<400> 444
   cagaat at at t t ct 14
<210> 445
   <211> 4
   <212> DNA
   <213> Artificial Sequence
<220>
   <223> Synthetic Oligonucleotide
<400> 445
   act g 4
<210> 446
   <211> 4
   <212> DNA
   <213> Artificial Sequence
<220>
   <223> Synthetic Oligonucleotide
<400> 446
   act g 4
<210> 447
   <211> 4
   <212> DNA
   <213> Artificial Sequence
<220>
   <223> Synthetic Oligonucleotide
<400> 447
   act g 4
<210> 448
   <211> 4
   <212> DNA
   <213> Artificial Sequence
<220>
   <223> Synthetic Oligonucleotide
<400> 448
   act g 4
<210> 449
   <211> 4
   <212> DNA
   <213> Artificial Sequence
<220>
   <223> Synthetic Oligonucleotide
<400> 449
   act g 4
<210> 450
   <211> 14
   <212> DNA
   <213> Artificial Sequence
<220>
   <223> Synthetic Oligonucleotide

<400> 450
   at aagagat t aaaa 14
<210> 451
   <211> 14
   <212> DNA
   <213> Artificial Sequence
<220>
   <223> Synthetic Oligonucleotide
<400> 451
   t ccccct t ct cat t 14
<210> 452
   <211> 14
   <212> DNA
   <213> Artificial Sequence
<220>
   <223> Synthetic Oligonucleotide
<400> 452
   gggcat t gt t aaaa 14
<210> 453
   <211> 4
   <212> DNA
   <213> Artificial Sequence
<220>
   <223> Synthetic Oligonucleotide
<400> 453
   actg 4
<210> 454
   <211> 4
   <212> DNA
   <213> Artificial Sequence
<220>
   <223> Synthetic Oligonucleotide
<400> 454
   actg 4
<210> 455
   <211> 14
   <212> DNA
   <213> Artificial Sequence
<220>
   <223> Synthetic Oligonucleotide
<400> 455 14
   agaactcaca t ct g 14
<210> 456
   <211> 14
   <212> DNA
   <213> Artificial Sequence
<220>
   <223> Synthetic Oligonucleotide
<400> 456 14
   gagctggacg gagg 14
<210> 457
   <211> 14
   <212> DNA
   <213> Artificial Sequence
<220>
   <223> Synthetic Oligonucleotide
<400> 457 14
   aagct t cat c ggag 14
<210> 458
   <211> 14
   <212> DNA
   <213> Artificial Sequence
<220>
   <223> Synthetic Oligonucleotide
<400> 458 14
   at aat ggcat cccg 14
<210> 459
   <211> 4
   <212> DNA
   <213> Artificial Sequence
<220>
   <223> Synthetic Oligonucleotide
<400> 459 4
   actg 4
<210> 460
   <211> 14
   <212> DNA
   <213> Artificial Sequence
<220>
   <223> Synthetic Oligonucleotide
<400> 460 14
   t gct gt cct a t aag 14
<210> 461
   <211> 14
   <212> DNA
   <213> Artificial Sequence
<220>
   <223> Synthetic Oligonucleotide
<400> 461 14
   cacaaaggt a at t g 14
<210> 462
   <211> 14
   <212> DNA
   <213> Artificial Sequence
<220>
   <223> Synthetic Oligonucleotide
<400> 462
   atcatttctt t ccag 14
<210> 463
   <211> 4
   <212> DNA
   <213> Artificial Sequence
<220>
   <223> Synthetic Oligonucleotide
<400> 463
   act g 4
<210> 464
   <211> 4
   <212> DNA
   <213> Artificial Sequence
<220>
   <223> Synthetic Oligonucleotide
<400> 464
   act g 4
<210> 465
   <211> 4
   <212> DNA
   <213> Artificial Sequence
<220>
   <223> Synthetic Oligonucleotide
<400> 465
   act g 4
<210> 466
   <211> 14
   <212> DNA
   <213> Artificial Sequence
<220>
   <223> Synthetic Oligonucleotide
<400> 466
   ccat t acct t ccag 14
<210> 467
   <211> 14
   <212> DNA
   <213> Artificial Sequence
<220>
   <223> Synthetic Oligonucleotide
<400> 467
   gcataaacag ggtt 14
<210> 468
   <211> 4
   <212> DNA
   <213> Artificial Sequence
<220>
   <223> Synthetic Oligonucleotide
<400> 468
   act g 4
<210> 469
   <211> 4
   <212> DNA
   <213> Artificial Sequence
<220>
   <223> Synthetic Oligonucleotide
<400> 469
   act g 4
<210> 470
   <211> 4
   <212> DNA
   <213> Artificial Sequence
<220>
   <223> Synthetic Oligonucleotide
<400> 470
   act g 4
<210> 471
   <211> 4
   <212> DNA
   <213> Artificial Sequence
<220>
   <223> Synthetic Oligonucleotide
<400> 471
   act g 4
<210> 472
   <211> 14
   <212> DNA
   <213> Artificial Sequence
<220>
   <223> Synthetic Oligonucleotide
<400> 472
   t at gaaagga at gt 14
<210> 473
   <211> 4
   <212> DNA
   <213> Artificial Sequence
<220>
   <223> Synthetic Oligonucleotide
<400> 473
   act g 4
<210> 474
   <211> 14
   <212> DNA
   <213> Artificial Sequence
<220>
   <223> Synthetic Oligonucleotide
<400> 474
   ttccttaagc ttcc 14
<210> 475
   <211> 4
   <212> DNA
   <213> Artificial Sequence
<220>
   <223> Synthetic Oligonucleotide
<400> 475
   act g 4
<210> 476
   <211> 4
   <212> DNA
   <213> Artificial Sequence
<220>
   <223> Synthetic Oligonucleotide
<400> 476
   actg 4
<210> 477
   <211> 4
   <212> DNA
   <213> Artificial Sequence
<220>
   <223> Synthetic Oligonucleotide
<400> 477
   actg 4
<210> 478
   <211> 4
   <212> DNA
   <213> Artificial Sequence
<220>
   <223> Synthetic Oligonucleotide
<400> 478
   actg 4
<210> 479
   <211> 4
   <212> DNA
   <213> Artificial Sequence
<220>
   <223> Synthetic Oligonucleotide
<400> 479
   act g 4
<210> 480
   <211> 4
   <212> DNA
   <213> Artificial Sequence
<220>
   <223> Synthetic Oligonucleotide
<400> 480
   act g 4
<210> 481
   <211> 4
   <212> DNA
   <213> Artificial Sequence
<220>
   <223> Synthetic Oligonucleotide
<400> 481
   act g 4
<210> 482
   <211> 4
   <212> DNA
   <213> Artificial Sequence
<220>
   <223> Synthetic Oligonucleotide
<400> 482
   act g 4
<210> 483
   <211> 4
   <212> DNA
   <213> Artificial Sequence
<220>
   <223> Synthetic Oligonucleotide
<400> 483
   act g 4
<210> 484
   <211> 4
   <212> DNA
   <213> Artificial Sequence
<220>
   <223> Synthetic Oligonucleotide
<400> 484
   act g 4
<210> 485
   <211> 14
   <212> DNA
   <213> Artificial Sequence
<220>
   <223> Synthetic Oligonucleotide
<400> 485
   gaacagttaa acat 14
<210> 486
   <211> 16
   <212> DNA
   <213> Artificial Sequence
<220>
   <223> Synthetic Oligonucleotide
<400> 486
   t agagct t cc act t ct 16
<210> 487
   <211> 14
   <212> DNA
   <213> Artificial Sequence
<220>
   <223> Synthetic Oligonucleotide
<400> 487
   gagct t ccac tt ct 14
<210> 488
   <211> 14
   <212> DNA
   <213> Artificial Sequence
<220>
   <223> Synthetic Oligonucleotide
<400> 488
   t gt t ggccgt ggt a 14
<210> 489
   <211> 14
   <212> DNA
   <213> Artificial Sequence
<220>
   <223> Synthetic Oligonucleotide
<400> 489
   at gt t ggccg t ggt 14

<210> 490
   <211> 14
   <212> DNA
   <213> Artificial Sequence
<220>
   <223> Synthetic Oligonucleotide
<400> 490
   gat gt t ggcc gt gg 14
<210> 491
   <211> 14
   <212> DNA
   <213> Artificial Sequence
<220>
   <223> Synthetic Oligonucleotide
<400> 491
   agat gttggc cgt g 14
<210> 492
   <211> 14
   <212> DNA
   <213> Artificial Sequence
<220>
   <223> Synthetic Oligonucleotide
<400> 492
   gagatgttgg ccgt 14
<210> 493
   <211> 14
   <212> DNA
   <213> Artificial Sequence
<220>
   <223> Synthetic Oligonucleotide
<400> 493
   ggagatgttg gccg 14
<210> 494
   <211> 14
   <212> DNA
   <213> Artificial Sequence
<220>
   <223> Synthetic Oligonucleotide
<400> 494
   aggagatgt tggcc 14
<210> 495
   <211> 14
   <212> DNA
   <213> Artificial Sequence
<220>
   <223> Synthetic Oligonucleotide
<400> 495
   caggagatgt tggc 14
<210> 496
   <211> 14
   <212> DNA
   <213> Artificial Sequence
<220>
   <223> Synthetic Oligonucleotide
<400> 496
   gcaggagat g t t gg 14
<210> 497
   <211> 14
   <212> DNA
   <213> Artificial Sequence
<220>
   <223> Synthetic Oligonucleotide
<400> 497
   ggcaggagat gt t g 14
<210> 498
   <211> 14
   <212> DNA
   <213> Artificial Sequence
<220>
   <223> Synthetic Oligonucleotide
<400> 498
   gt ggt gccaa ggca 14
<210> 499
   <211> 14
   <212> DNA
   <213> Artificial Sequence
<220>
   <223> Synthetic Oligonucleotide
<400> 499
   tgtggtgcca aggc 14
<210> 500
   <211> 14
   <212> DNA
   <213> Artificial Sequence
<220>
   <223> Synthetic Oligonucleotide
<400> 500
   t t gt ggt gcc aagg 14
<210> 501
   <211> 14
   <212> DNA
   <213> Artificial Sequence
<220>
   <223> Synthetic Oligonucleotide
<400> 501
   ttt gt ggt gc caag 14
<210> 502
   <211> 14
   <212> DNA
   <213> Artificial Sequence
<220>
   <223> Synthetic Oligonucleotide
<400> 502
   ct tt gt ggt g ccaa 14
<210> 503
   <211> 14 <212> DNA
   <213> Artificial Sequence
<220>
   <223> Synthetic Oligonucleotide
<400> 503
   act t t gt ggt gcca 14
<210> 504
   <211> 14
   <212> DNA
   <213> Artificial Sequence
<220>
   <223> Synthetic Oligonucleotide
<400> 504
   cact t t gt gg t gcc 14
<210> 505
   <211> 14
   <212> DNA
   <213> Artificial Sequence
<220>
   <223> Synthetic Oligonucleotide
<400> 505
   gcact tt gt g gt gc 14
<210> 506
   <211> 14
   <212> DNA
   <213> Artificial Sequence
<220>
   <223> Synthetic Oligonucleotide
<400> 506
   t gcactttgt ggt g 14
<210> 507
   <211> 14
   <212> DNA
   <213> Artificial Sequence
<220>
   <223> Synthetic Oligonucleotide
<400> 507
   ttgcactttg t ggt 14
<210> 508
   <211> 14
   <212> DNA
   <213> Artificial Sequence
<220>
   <223> Synthetic Oligonucleotide
<400> 508
   cggt gt t gca ct t t 14
<210> 509
   <211> 14
   <212> DNA
   <213> Artificial Sequence
<220>
   <223> Synthetic Oligonucleotide
<400> 509
   gcggt gt t gc act t 14
<210> 510
   <211> 14
   <212> DNA
   <213> Artificial Sequence
<220>
   <223> Synthetic Oligonucleotide
<400> 510
   agcggt gt t g cact 14
<210> 511
   <211> 14
   <212> DNA
   <213> Artificial Sequence
<220>
   <223> Synthetic Oligonucleotide
<400> 511
   aagcggtgtt gcac 14
<210> 512
   <211> 14
<212> DNA
   <213> Artificial Sequence
<220>
   <223> Synthetic Oligonucleotide
<400> 512
   gaagcggt gt t gca 14
<210> 513
   <211> 14 <212> DNA
   <213> Artificial Sequence
<220>
   <223> Synthetic Oligonucleotide
<400> 513
   cgaagcggtg t t gc 14
<210> 514
   <211> 14
   <212> DNA
   <213> Artificial Sequence
<220>
   <223> Synthetic Oligonucleotide
<400> 514
   acgaagcggt gttg 14
<210> 515
   <211> 14
   <212> DNA
   <213> Artificial Sequence
<220>
   <223> Synthetic Oligonucleotide
<400> 515
   cacgaagcgg t gt t 14
<210> 516
   <211> 14
   <212> DNA
   <213> Artificial Sequence
<220>
   <223> Synthetic Oligonucleotide
<400> 516
   acacgaagcg gt gt 14
<210> 517
   <211> 14
   <212> DNA
   <213> Artificial Sequence
<220>
   <223> Synthetic Oligonucleotide
<400> 517
   aacacgaagc ggtg 14
<210> 518
   <211> 14
   <212> DNA
   <213> Artificial Sequence
<220>
   <223> Synthetic Oligonucleotide
<400> 518
   gctgctgtac atct 14
<210> 519
   <211> 4
   <212> DNA
   <213> Artificial Sequence
<220>
   <223> Synthetic Oligonucleotide
<400> 519
   act g 4
<210> 520
   <211> 14
   <212> DNA.
   <213> Artificial Sequence
<220>
   <223> Synthetic Oligonucleotide
<400> 520
   agct gct gt a cat c 14
<210> 521
   <211> 14
   <212> DNA
   <213> Artificial Sequence
<220>
   <223> Synthetic Oligonucleotide
<400> 521
   aagctgctgt acat 14
<210> 522
   <211> 14
   <212> DNA
   <213> Artificial Sequence
<220>
   <223> Synthetic Oligonucleotide
<400> 522
   gaagctgctg taca 14
<210> 523
   <211> 14
   <212> DNA
   <213> Artificial Sequence
<220>
   <223> Synthetic Oligonucleotide
<400> 523
   ggaagct gct gt ac 14
<210> 524
   <211> 14
   <212> DNA
   <213> Artificial Sequence
<220>
   <223> Synthetic Oligonucleotide
<400> 524
   t ggaagct gc t gt a 14
<210> 525
   <211> 14
   <212> DNA
   <213> Artificial Sequence
<220>
   <223> Synthetic Oligonucleotide
<400> 525
   ct ggaagct g ctgt 14
<210> 526
   <211> 14
   <212> DNA
   <213> Artificial Sequence
<220>
   <223> Synthetic Oligonucleotide
<400> 526
   cct ggaagct gct g 14
<210> 527
   <211> 14
   <212> DNA
   <213> Artificial Sequence
<220>
   <223> Synthetic Oligonucleotide
<400> 527
   acctggaagc tgct 14
<210> 528
   <211> 14
   <212> DNA
   <213> Artificial Sequence
<220>
   <223> Synthetic Oligonucleotide
<400> 528
   cacctggaag ctgc 14
<210> 529
   <211> 14
   <212> DNA
   <213> Artificial Sequence
<220>
   <223> Synthetic Oligonucleotide
<400> 529
   tcacctggaa gctg 14
<210> 530
   <211> 14
   <212> DNA
   <213> Artificial Sequence
<220>
   <223> Synthetic Oligonucleotide
<400> 530
   at cacct gga agct 14
<210> 531
   <211> 14
   <212> DNA
   <213> Artificial Sequence
<220>
   <223> Synthetic Oligonucleotide
<400> 531
   cat cacct gg aagc 14
<210> 532
   <211> 14
   <212> DNA
   <213> Artificial Sequence
<220>
   <223> Synthetic Oligonucleotide
<400> 532
   acat cacct g gaag 14
<210> 533
   <211> 14
   <212> DNA
   <213> Artificial Sequence
<220>
   <223> Synthetic Oligonucleotide
<400> 533
   t acat cacct ggaa 14
<210> 534
   <211> 14
   <212> DNA
   <213> Artificial Sequence
<220>
   <223> Synthetic Oligonucleotide
<400> 534
   gt acat cacc tgga 14
<210> 535
   <211> 14
   <212> DNA
   <213> Artificial Sequence
<220>
   <223> Synthetic Oligonucleotide
<400> 535
   tgt acat cac ctgg 14
<210> 536
   <211> 14
   <212> DNA
   <213> Artificial Sequence
<220>
   <223> Synthetic Oligonucleotide
<400> 536
   gt gt acat ca cct g 14
<210> 537
   <211> 14
   <212> DNA
   <213> Artificial Sequence
<220>
   <223> Synthetic Oligonucleotide
<400> 537
   t agcgggt cc t gag 14
<210> 538
   <211> 14
   <212> DNA
   <213> Artificial Sequence
<220>
   <223> Synthetic Oligonucleotide
<400> 538
   gt agcgggt c ct ga 14
<210> 539
   <211> 14
   <212> DNA
   <213> Artificial Sequence
<220>
   <223> Synthetic Oligonucleotide
<400> 539
   t gt agcgggt cct g 14
<210> 540
   <211> 14
   <212> DNA
   <213> Artificial Sequence
<220>
   <223> Synthetic Oligonucleotide
<400> 540
   ct gt agcggg t cct 14
<210> 541
   <211> 14
   <212> DNA
   <213> Artificial Sequence
<220>
   <223> Synthetic Oligonucleotide
<400> 541
   gctgtagcgg gtcc 14
<210> 542
   <211> 14
   <212> DNA
   <213> Artificial Sequence
<220>
   <223> Synthetic Oligonucleotide
<400> 542
   ggctgtagcg ggt c 14
<210> 543
   <211> 14
   <212> DNA
   <213> Artificial Sequence
<220>
   <223> Synthetic Oligonucleotide
<400> 543
   t ggct gt agc gggt 14
<210> 544
   <211> 14
   <212> DNA
   <213> Artificial Sequence
<220>
   <223> Synthetic Oligonucleotide
<400> 544
   ct ggct gt ag cggg 14
<210> 545
   <211> 14
   <212> DNA
   <213> Artificial Sequence
<220>
   <223> Synthetic Oligonucleotide
<400> 545
   t ct ggct gt a gcgg 14
<210> 546
   <211> 14
   <212> DNA
   <213> Artificial Sequence
<220>
   <223> Synthetic Oligonucleotide
<400> 546
   t t ct ggct gt agcg 14
<210> 547
   <211> 14
   <212> DNA
   <213> Artificial Sequence
<220>
   <223> Synthetic Oligonucleotide
<400> 547
   tgaacaccgc ggcc 14
<210> 548
   <211> 14
   <212> DNA
   <213> Artificial Sequence
<220>
   <223> Synthetic Oligonucleotide
<400> 548
   atgaacaccg cggc 14
<210> 549
   <211> 14
   <212> DNA,
   <213> Artificial Sequence
<220>
   <223> Synthetic Oligonucleotide
<400> 549
   catgaacacc gcgg 14
<210> 550
   <211> 14
   <212> DNA
   <213> Artificial Sequence
<220>
   <223> Synthetic Oligonucleotide
<400> 550
   gcatgaacac cgcg 14
<210> 551
   <211> 14
   <212> DNA
   <213> Artificial Sequence
<220>
   <223> Synthetic Oligonucleotide
<400> 551
   tgcatgaaca ccgc 14
<210> 552
   <211> 14
   <212> DNA
   <213> Artificial Sequence
<220>
   <223> Synthetic Oligonucleotide
<400> 552
   t t gcat gaac accg 14
<210> 553
   <211> 14
   <212> DNA
   <213> Artificial Sequence
<220>
   <223> Synthetic Oligonucleotide
<400> 553
   at t gcat gaa cacc 14
<210> 554
   <211> 14
   <212> DNA
   <213> Artificial Sequence
<220>
   <223> Synthetic Oligonucleotide
<400> 554
   t at t gcat ga acac 14
<210> 555
   <211> 14
   <212> DNA
   <213> Artificial Sequence
<220>
   <223> Synthetic Oligonucleotide
<400> 555
   at at t gcat g aaca 14
<210> 556
   <211> 14
   <212> DNA
   <213> Artificial Sequence

<220>
   <223> Synthetic Oligonucleotide
<400> 556
   cat at t gcat gaac 14
<210> 557
   <211> 14
   <212> DNA
   <213> Artificial Sequence
<220>
   <223> Synthetic Oligonucleotide
<400> 557
   aggt t gt gca ggt a 14
<210> 558
   <211> 14
   <212> DNA
   <213> Artificial Sequence
<220>
   <223> Synthetic Oligonucleotide
<400> 558
   caggt t gt gc aggt 14
<210> 559
   <211> 14
   <212> DNA
   <213> Artificial Sequence
<220>
   <223> Synthetic Oligonucleotide
<400> 559
   gcaggt t gt g cagg 14
<210> 560
   <211> 14
   <212> DNA
   <213> Artificial Sequence
<220>
   <223> Synthetic Oligonucleotide
<400> 560
   agcaggt t gt gcag 14
<210> 561
   <211> 14
   <212> DNA
   <213> Artificial Sequence
<220>
   <223> Synthetic Oligonucleotide
<400> 561
   cagcaggttg tgca 14
<210> 562
   <211> 14
   <212> DNA
   <213> Artificial Sequence
<220>
   <223> Synthetic Oligonucleotide
<400> 562
   ccagcaggt t gt gc 14
<210> 563
   <211> 14
   <212> DNA
   <213> Artificial Sequence
<220>
   <223> Synthetic Oligonucleotide
<400> 563
   cccagcaggt tgtg 14
<210> 564
   <211> 14
   <212> DNA
   <213> Artificial Sequence
<220>
   <223> Synthetic Oligonucleotide
<400> 564
   gcccagcagg t t gt 14
<210> 565
   <211> 14
   <212> DNA
   <213> Artificial Sequence
<220>
   <223> Synthetic Oligonucleotide
<400> 565
   ggcccagcag gttg 14
<210> 566
   <211> 14
   <212> DNA,
   <213> Artificial Sequence
<220>
   <223> Synthetic Oligonucleotide
<400> 566
   aggcccagca ggtt 14
<210> 567
   <211> 16
   <212> DNA
   <213> Artificial Sequence
<220>
   <223> Synthetic Oligonucleotide
<400> 567
   t gt cat gct ggt cca 16
<210> 568
   <211> 14
   <212> DNA
   <213> Artificial Sequence
<220>
   <223> Synthetic Oligonucleotide
<400> 568
   t cat t gct gg t cca 14
<210> 569
   <211> 16
   <212> DNA
   <213> Artificial Sequence
<220>
   <223> Synthetic Oligonucleotide
<400> 569
   tggccagccg gaactt 16
<210> 570
   <211> 14
   <212> DNA
   <213> Artificial Sequence
<220>
   <223> Synthetic Oligonucleotide
<400> 570
   gccagccgga act t 14
<210> 571
   <211> 16
   <212> DNA
   <213> Artificial Sequence
<220>
   <223> Synthetic Oligonucleotide
<400> 571
   gggatgaggg t cagcg 16
<210> 572
   <211> 14
   <212> DNA
   <213> Artificial Sequence
<220>
   <223> Synthetic Oligonucleotide
<400> 572
   gatgagggtc c agcg 14
<210> 573
   <211> 14
   <212> DNA
   <213> Artificial Sequence
<220>
   <223> Synthetic Oligonucleotide
<400> 573
   aaggcaaaga ccac 14
<210> 574
   <211> 14
   <212> DNA
   <213> Artifiicial Sequence
<220>
   <223> Synthetic Oligonucleotide
<400> 574
   ggagcgcagg gtgc 14
<210> 575
   <211> 14
   <212> DNA
   <213> Artificial Sequence
<220>
   <223> Synthetic Oligonucleotide
<400> 575
   t gcacct cct t gt t 14
<210> 576
   <211> 14
   <212> DNA
   <213> Artificial Sequence
<220>
   <223> Synthetic Oligonucleotide
<400> 576
   cagcagaccc tgga 14
<210> 577
   <211> 16
   <212> DNA
   <213> Artificial Sequence
<220>
   <223> Synthetic Oligonucleotide
<400> 577
   acat ct ggca gaggt t 16
<210> 578
   <211> 14
   <212> DNA
   <213> Artificial Sequence
<220>
   <223> Synthetic Oligonucleotide
<400> 578
   at ct ggcaga ggt t 14
<210> 579
   <211> 16
   <212> DNA
   <213> Artificial Sequence
<220>
   <223> Synthetic Oligonucleotide
<400> 579
   caggccagca ggagta 16
<210> 580
   <211> 14
   <212> DNA
   <213> Artificial Sequence
<220>
   <223> Synthetic Oligonucleotide
<400> 580
   ggccagcagg agta 14
<210> 581
   <211> 16
   <212> DNA
   <213> Artificial Sequence
<220>
   <223> Synthetic Oligonucleotide
<400> 581
   ct caaacaaa aagt cc 16
<210> 582
   <211> 14
   <212> DNA
   <213> Artificial Sequence
<220>
   <223> Synthetic Oligonucleotide
<400> 582
   caaacaaaaa gtcc 14
<210> 583
   <211> 16
   <212> DNA
   <213> Artificial Sequence
<220>
   <223> Synthetic Oligonucleotide
<400> 583
   gt ggt gacat t ggt ca 16
<210> 584
   <211> 14
   <212> DNA
   <213> Artificial Sequence
<220>
   <223> Synthetic Oligonucleotide
<400> 584
   ggt gacat t g gt ca 14
<210> 585
   <211> 4
   <212> DNA
   <213> Artificial Sequence
<220>
   <223> Synthetic Oligonucleotide
<400> 585
   act g 4
<210> 586
   <211> 4
   <212> DNA
   <213> Artificial Sequence
<220>
   <223> Synthetic Oligonucleotide
<400> 586
   act g 4
<210> 587
   <211> 14
   <212> DNA
   <213> Artificial Sequence
<220>
   <223> Synthetic Oligonucleotide
<400> 587
   t t ggcagt gg t gt t 14
<210> 588
   <211> 14
   <212> DNA
   <213> Artificial Sequence
<220>
   <223> Synthetic Oligonucleotide
<400> 588
   atgttggcag tggt 14
<210> 589
   <211> 16
   <212> DNA
   <213> Artificial Sequence
<220>
   <223> Synthetic Oligonucleotide
<400> 589
   aggaaat gt t ggcagt 16
<210> 590
   <211> 14
   <212> DNA
   <213> Artificial Sequence
<220>
   <223> Synthetic Oligonucleotide
<400> 590
   gaaat gt t gg cagt 14
<210> 591
   <211> 14
   <212> DNA
   <213> Artificial Sequence
<220>
   <223> Synthetic Oligonucleotide
<400> 591
   caggaaat gt t ggc 14
<210> 592
   <211> 14
   <212> DNA
   <213> Artificial Sequence
<220>
   <223> Synthetic Oligonucleotide
<400> 592
   gggcaggaaa t gt t 14
<210> 593
   <211> 14
   <212> DNA
   <213> Artificial Sequence
<220>
   <223> Synthetic Oligonucleotide
<400> 593
   aaggtaggta ccag 14
<210> 594
   <211> 14
   <212> DNA
   <213> Artificial Sequence
<220>
   <223> Synthetic Oligonucleotide
<400> 594
   accaaggtag gt ac 14
<210> 595
   <211> 14
   <212> DNA
   <213> Artificial Sequence
<220>
   <223> Synthetic Oligonucleotide
<400> 595
   ct t t gt ggca ccaa 14
<210> 596
   <211> 14
   <212> DNA
   <213> Artificial Sequence
<220> <223> Synthetic Oligonucleotide
<400> 596
   gcact t t gt g gcac 14
<210> 597
   <211> 14
   <212> DNA
   <213> Artificial Sequence
<220>
   <223> Synthetic Oligonucleotide
<400> 597
   t gcact t t gt ggca 14
<210> 598
   <211> 14
   <212> DNA
   <213> Artificial Sequence
<220>
   <223> Synthetic Oligonucleotide

<400> 598
   gct gcact t t gt gg 14
<210> 599
   <211> 14
   <212> DNA
   <213> Artificial Sequence
<220>
   <223> Synthetic Oligonucleotide
<400> 599
   ggt gct gcac t t t g 14
<210> 600
   <211> 14
   <212> DNA
   <213> Artificial Sequence
<220>
   <223> Synthetic Oligonucleotide
<400> 600
   ggcggt gct g cact 14
<210> 601
   <211> 14
   <212> DNA
   <213> Artificial Sequence
<220>
   <223> Synthetic Oligonucleotide
<400> 601
   t gaacact ag gcgg 14
<210> 602
   <211> 14
   <212> DNA
   <213> Artificial Sequence
<220>
   <223> Synthetic Oligonucleotide
<400> 602
   t ct t gaacac t agg 14
<210> 603
   <211> 16
   <212> DNA
   <213> Artificial Sequence
<220>
   <223> Synthetic Oligonucleotide
<400> 603
   cacct ct t ga acact a 16
<210> 604
   <211> 14
   <212> DNA
   <213> Artificial Sequence
<220>
   <223> Synthetic Oligonucleotide
<400> 604
   cct ct t gaac act a 14
<210> 605
   <211> 14
   <212> DNA
   <213> Artificial Sequence
<220>
   <223> Synthetic Oligonucleotide
<400> 605
   acctcttgaa cact 14
<210> 606
   <211> 14
   <212> DNA
   <213> Artificial Sequence
<220>
   <223> Synthetic Oligonucleotide
<400> 606
   cacacctctt t gaac 14
<210> 607
   <211> 14
   <212> DNA
   <213> Artificial Sequence
<220>
   <223> Synthetic Oligonucleotide
<400> 607
   cct cgaaccc actg 14
<210> 608
   <211> 16
   <212> DNA
   <213> Artificial Sequence
<220>
   <223> Synthetic Oligonucleotide
<400> 608
   ct t ct ggacc t cgat c 16
<210> 609
   <211> 14
   <212> DNA
   <213> Artificial Sequence
<220>
   <223> Synthetic Oligonucleotide
<400> 609
   t ct ggacct c gat c 14
<210> 610
   <211> 16
   <212> DNA
   <213> Artificial Sequence
<220>
   <223> Synthetic Oligonucleotide
<400> 610
   ct gct at aca t ct t gg 16
<210> 611
   <211> 14
   <212> DNA
   <213> Artificial Sequence
<220>
   <223> Synthetic Oligonucleotide
<400> 611
   gct at acat c t t gg 14
<210> 612
   <211> 16 <212> DNA
   <213> Artificial Sequence
<220>
   <223> Synthetic Oligonucleotide
<400> 612
   cacggt gt ac at cacc 16
<210> 613
   <211> 14
   <212> DNA
   <213> Artificial Sequence
<220>
   <223> Synthetic Oligonucleotide
<400> 613
   cggt gt acat cacc 14
<210> 614
   <211> 16
   <212> DNA
   <213> Artificial Sequence
<220>
   <223> Synthetic Oligonucleotide
<400> 614
   ggacagactg tagccc 16
<210> 615
   <211> 14
   <212> DNA
   <213> Artificial Sequence
<220>
   <223> Synthetic Oligonucleotide
<400> 615
   acagact gt a gccc 14
<210> 616
   <211> 16
   <212> DNA
   <213> Artificial Sequence
<220>
   <223> Synthetic Oligonucleotide
<400> 616
   gt cat cgcca at ct t c 16
<210> 617
   <211> 14
   <212> DNA
   <213> Artificial Sequence <220>
   <223> Synthetic Oligonucleotide
<400> 617
   catcgccaat ct t c 14
<210> 618
   <211> 16
   <212> DNA
   <213> Artificial Sequence
<220>
   <223> Synthetic Oligonucleotide
<400> 618
   t cacact gag gt cat c 16
<210> 619
   <211> 14
   <212> DNA
   <213> Artificial Sequence
<220>
   <223> Synthetic Oligonucleotide
<400> 619
   acact gaggt cat c 14
<210> 620
   <211> 14
   <212> DNA
   <213> Artificial Sequence
<220>
   <223> Synthetic Oligonucleotide
<400> 620
   cgccccgt ca ct ga 14
<210> 621
   <211> 16
   <212> DNA
   <213> Artificial Sequence
<220>
   <223> Synthetic Oligonucleotide
<400> 621
   ccagcaacca gcaata 16
<210> 622
   <211> 14
   <212> DNA
   <213> Artificial Sequence
<220>
   <223> Synthetic Oligonucleotide
<400> 622
   agcaaccagc aata 14
<210> 623 <211> 16
   <212> DNA
   <213> Artificial Sequence
<220>
   <223> Synthetic Oligonucleotide
<400> 623
   caggct gt ac aggt ac 16
<210> 624
   <211> 14
   <212> DNA
   <213> Artificial Sequence
<220>
   <223> Synthetic Oligonucleotide
<400> 624
   ggct gt acag gt ac 14
<210> 625
   <211> 16
   <212> DNA
   <213> Artificial Sequence
<220>
   <223> Synthetic Oligonucleotide
<400> 625
   gaagct cct c t cagag 16
<210> 626
   <211> 14
   <212> DNA
   <213> Artificial Sequence
<220>
   <223> Synthetic Oligonucleotide
<400> 626
   agct cct ct c agag 14
<210> 627
   <211> 16
   <212> DNA
   <213> Artificial Sequence
<220>
   <223> Synthetic Oligonucleotide
<400> 627
   cccagccaat gcccag 16
<210> 628
   <211> 14
   <212> DNA
   <213> Artificial Sequence
<220>
   <223> Synthetic Oligonucleotide
<400> 628
   cagccaatgc ccag 14
<210> 629
   <211> 16
   <212> DNA
   <213> Artificial Sequence
<220>
   <223> Synthetic Oligonucleotide
<400> 629
   t caaacagac act t ga 16
<210> 630
   <211> 14
   <212> DNA
   <213> Artificial Sequence
<220>
   <223> Synthetic Oligonucleotide
<400> 630
   aaacagacac t t ga 14
<210> 631
   <211> 16
   <212> DNA
   <213> Artificial Sequence
<220>
   <223> Synthetic Oligonucleotide
<400> 631
   agcact gaac at t ct c 16
<210> 632
   <211> 14
   <212> DNA
   <213> Artificial Sequence
<220>
   <223> Synthetical Oligonucleotide
<400> 632
   cact gaacat t ct c 14
<210> 633
   <211> 16
   <212> DNA
   <213> Artificial Sequence
<220>
   <223> Synthetic Oligonucleotide
<400> 633
   ggatccacca gaatcc 16
<210> 634
   <211> 14
   <212> DNA
   <213> Artificial Sequence
<220>
   <223> Synthetic Oligonucleotide
<400> 634
   at ccaccaga at cc 14
<210> 635
   <211> 14
   <212> DNA
   <213> Artificial Sequence
<220>
   <223> Synthetic Oligonucleotide
<400> 635
   t gat cagt aa ggcc 14
<210> 636
   <211> 16
   <212> DNA
   <213> Artificial Sequence
<220>
   <223> Synthetic Oligonucleotide
<400> 636
   ggacaaagat gaaaaa 16
<210> 637
   <211> 14
   <212> DNA
   <213> Artificial Sequence
<220>
   <223> Synthetic Oligonucleotide
<400> 637
   acaaagatga aaaa 14
<210> 638
   <211> 16
   <212> DNA
   <213> Artificial Sequence
<220>
   <223> Synthetic Oligonucleotide
<400> 638
   ggcacgcagc ttggcc 16
<210> 639
   <211> 14
   <212> DNA
   <213> Artificial Sequence
<220>
   <223> Synthetic Oligonucleotide
<400> 639
   cacgcagct t ggcc 14
<210> 640
   <211> 16
   <212> DNA
   <213> Artificial Sequence
<220>
   <223> Synthetic Oligonucleotide
<400> 640
   tggaccccca gcagag 16
<210> 641
   <211> 14
   <212> DNA
   <213> Artificial Sequence
<220>
   <223> Synthetic Oligonucleotide
<400> 641
   gacccccagc agag 14
<210> 642
   <211> 14
   <212> DNA
   <213> Artificial Sequence
<220>
   <223> Synthetic Oligonucleotide
<400> 642
   caaaggcaaa gacc 14
<210> 643
   <211> 14
   <212> DNA
   <213> Artificial Sequence
<220>
   <223> Synthetic Oligonucleotide
<400> 643
   t cacaaaggc aaag 14
<210> 644
   <211> 14
   <212> DNA
   <213> Artificial Sequence
<220>
   <223> Synthetic Oligonucleotide
<400> 644
   cagt cacaaa ggca 14
<210> 645
   <211> 14
   <212> DNA
   <213> Artificial Sequence
<220>
   <223> Synthetic Oligonucleotide
<400> 645
   cgt cagt cac aaag 14
<210> 646
   <211> 14
   <212> DNA
   <213> Artificial Sequence
<220>
   <223> Synthetic Oligonucleotide
<400> 646
   gct cgt cagt caca 14
<210> 647
   <211> 14
   <212> DNA
   <213> Artificial Sequence
<220>
   <223> Synthetic Oligonucleotide
<400> 647
   cat gct cgt c agt c 14
<210> 648
   <211> 14
   <212> DNA
   <213> Artificial Sequence
<220>
   <223> Synthetic Oligonucleotide
<400> 648
   gggcat gct c gt ca 14
<210> 649
   <211> 4
   <212> DNA
   <213> Artificial Sequence
<220>
   <223> Synthetic Oligonucleotide
<400> 649
   act g 4
<210> 650
   <211> 4
   <212> DNA
   <213> Artificial Sequence
<220>
   <223> Synthetic Oligonucleotide
<400> 650
   act g 4
<210> 651
   <211> 4
   <212> DNA
   <213> Artificial Sequence
<220>
   <223> Synthetical Oligonucleotide
<400> 651
   act g 4
<210> 652
   <211> 4
   <212> DNA
   <213> Artificial Sequence
<220>
   <223> Synthetic Oligonucleotide
<400> 652
   act g 4
<210> 653
   <211> 4
   <212> DNA
   <213> Artificial Sequence
<220>
   <223> Synthetic Oligonucleotide
<400> 653
   act g 4
<210> 654
   <211> 14
   <212> DNA
   <213> Artificial Sequence
<220>
   <223> Synthetic Oligonucleotide
<400> 654
   cttgggcatg ctcg 14
<210> 655
   <211> 4
   <212> DNA
   <213> Artificial Sequence
<220>
   <223> Synthetic Oligonucleotide
<400> 655
   act g 4
<210> 656
   <211> 4
   <212> DNA
   <213> Artificial Sequence
<220>
   <223> Synthetic Oligonucleotide
<400> 656
   act g 4
<210> 657
   <211> 14
   <212> DNA
   <213> Artificial Sequence
<220>
   <223> Synthetic Oligonucleotide
<400> 657
   tgccttgggc atgc 14
<210> 658
   <211> 14
   <212> DNA
   <213> Artificial Sequence
<220>
   <223> Synthetic Oligonucleotide
<400> 658
   gggt gcct t g ggca 14
<210> 659
   <211> 14
   <212> DNA,
   <213> Artificial Sequence
<220>
   <223> Synthetic Oligonucleotide
<400> 659
   gcagggtgcc ttgg 14
<210> 660
   <211> 14
   <212> DNA
   <213> Artificial Sequence
<220>
   <223> Synthetic Oligonucleotide
<400> 660
   agcgcagggt gcct 14
<210> 661
   <211> 16
   <212> DNA
   <213> Artificial Sequence
<220>
   <223> Synthetic Oligonucleotide
<400> 661
   gtggagcgca gggtgc 16
<210> 662
   <211> 14
   <212> DNA
   <213> Artificial Sequence
<220>
   <223> Synthetic Oligonucleotide
<400> 662
   t ggagcgcag ggt g 14
<210> 663
   <211> 14
   <212> DNA
   <213> Artificial Sequence
<220>
   <223> Synthetic Oligonucleotide
<400> 663
   tggtggagcg cagg 14
<210> 664
   <211> 14
   <212> DNA
   <213> Artificial Sequence
<220>
   <223> Synthetic Oligonucleotide

<400> 664
   gcttggtgga gcgc 14
<210> 665
   <211> 14
   <212> DNA
   <213> Artificial Sequence
<220>
   <223> Synthetic Oligonucleotide
<400> 665
   agagct t ggt ggag 14
<210> 666
   <211> 16
   <212> DNA
   <213> Artificial Sequence
<220>
   <223> Synthetic Oligonucleotide
<400> 666
   aaaagagctt ggt gga 16
<210> 667
   <211> 14
   <212> DNA
   <213> Artificial Sequence
<220>
   <223> Synthetic Oligonucleotide
<400> 667
   aagagct t gg t gga 14
<210> 668
   <211> 14
   <212> DNA
   <213> Artificial Sequence
<220>
   <223> Synthetic Oligonucleotide
<400> 668
   aaaagagctt ggt g 14
<210> 669
   <211> 14
   <212> DNA
   <213> Artificial Sequence
<220>
   <223> Synthetic Oligonucleotide
<400> 669
   caaaaaagag cttg 14
<210> 670
   <211> 16
   <212> DNA
   <213> Artificial Sequence
<220>
   <223> Synthetic Oligonucleotide
<400> 670
   aaggagctga ggaaca 16
<210> 671
   <211> 14
   <212> DNA
   <213> Artificial Sequence
<220>
   <223> Synthetic Oligonucleotide
<400> 671
   ggagctgagg aaca 14
<210> 672
   <211> 16
   <212> DNA
   <213> Artificial Sequence
<220>
   <223> Synthetic Oligonucleotide
<400> 672
   gcagaccctg gaagga 16
<210> 673
   <211> 14
   <212> DNA
   <213> Artificial Sequence
<220>
   <223> Synthetic Oligonucleotide
<400> 673
   agaccctgga agga 14
<210> 674
   <211> 16
   <212> DNA
   <213> Synthetic Oligonucleotide
<220>
   <223> Synthetic Oligonucleotide
<400> 674
   accagcagac cctgga 16
<210> 675
   <211> 4
   <212> DNA
   <213> Artificial Sequence
<220>
   <223> Synthetic Oligonucleotide
<400> 675
   act g 4
<210> 676
   <211> 16
   <212> DNA
   <213> Artificial Sequence
<220>
   <223> Synthetic Oligonucleotide
<400> 676
   cagtagagaa cagcca 16
<210> 677
   <211> 14
   <212> DNA
   <213> Artifcial Sequence

<220>
   <223> Synthetic Oligonucleotide
<400> 677
   gtagagaaca gcca 14
<210> 678
   <211> 16
   <212> DNA
   <213> Artificial Sequence
<220>
   <223> Synthetic Oligonucleotide
<400> 678
   t gt t gaggaa acagt a 16
<210> 679
   <211> 14
   <212> DNA
   <213> Artificial Sequence
<220>
   <223> Synthetic Oligonucleotide
<400> 679
   t t gaggaaac agt a 14
<210> 680
   <211> 16
   <212> DNA
   <213> Artificial Sequence
<220>
   <223> Synthetic Oligonucleotide
<400> 680
   cct gcacct c ct t gt t 16
<210> 681
   <211> 14
   <212> DNA
   <213> Artificial Sequence
<220>
   <223> Synthetic Oligonucleotide

<400> 681
   at gagggt ct t cat 14
<210> 682
   <211> 14
   <212> DNA
   <213> Artificial Sequence
<220>
   <223> Synthetic Oligonucleotide
<400> 682
   accccggagt aggc 14
<210> 683
   <211> 12
   <212> DNA
   <213> Artificial Sequence
<220>
   <223> Synthetic Oligonucleotide
<400> 683
   cccggagt ag gc 12
<210> 684
   <211> 16
   <212> DNA
   <213> Artificial Sequence
<220>
   <223> Synthetic Oligonucleotide
<400> 684
   caggaccccg gagtag 16
<210> 685
   <211> 16
   <212> DNA
   <213> Artificial Sequence
<220>
   <223> Synthetic Oligonucleotide
<400> 685
   gcaggacccc ggagta 16
<210> 686
   <211> 14
   <212> DNA
   <213> Artificial Sequence
<220>
   <223> Synthetic Oligonucleotide
<400> 686
   aggaccccgg agta 14
<210> 687
   <211> 14
   <212> DNA
   <213> Artificial Sequence
<220>
   <223> Synthetic Oligonucleotide
<400> 687
   caggaccccg gagt 14
<210> 688
   <211> 12
   <212> DNA
   <213> Artificial Sequence
<220>
   <223> Synthetic Oligonucleotide
<400> 688
   cagacccctc gc 12
<210> 689
   <211> 12
   <212> DNA
   <213> Artificial Sequence
<220>
   <223> Synthetic Oligonucleotide
<400> 689
   agaggatgct gg 12
<210> 690
   <211> 16
   <212> DNA
   <213> Artificial Sequence
<220>
   <223> Synthetic Oligonucleotide
<400> 690
   gagccaggtg acagag 16
<210> 691
   <211> 14
   <212> DNA
   <213> Artificial Sequence
<220>
   <223> Synthetic Oligonucleotide
<400> 691
   agccaggtga caga 14
<210> 692
   <211> 12
   <212> DNA
   <213> Artificial Sequence
<220>
   <223> Synthetic Oligonucleotide
<400> 692
   t gagccaggt ga 12
<210> 693
   <211> 14
   <212> DNA
   <213> Artificial Sequence
<220>
   <223> Synthetic Oligonucleotide
<400> 693
   ttttccacct t gga 14
<210> 694
   <211> 12
   <212> DNA
   <213> Artificial Sequence
<220>
   <223> Synthetic Oligonucleotide
<400> 694
   ct gcaggcca ct 12
<210> 695
   <211> 16
   <212> DNA
   <213> Artificial Sequence
<220>
   <223> Synthetic Oligonucleotide
<400> 695
   t caccagct g gatggg 16
<210> 696
   <211> 16
   <212> DNA
   <213> Artificial Sequence
<220>
   <223> Synthetic Oligonucleotide
<400> 696
   t t caccagct ggat gg 16
<210> 697
   <211> 14
   <212> DNA
   <213> Artificial Sequence
<220>
   <223> Synthetic Oligonucleotide
<400> 697
   caccagct gg at gg 14
<210> 698
   <211> 14
   <212> DNA
   <213> Artificial Sequence
<220>
   <223> Synthetic Oligonucleotide
<400> 698
   t caccagct g gat g 14
<210> 699
   <211> 12
   <212> DNA
   <213> Artificial Sequence
<220>
   <223> Synthetic Oligonucleotide
<400> 699
   tcaccagctg ga 12
<210> 700
   <211> 16
   <212> DNA
   <213> Artificial Sequence
<220>
   <223> Synthetic Oligonucleotide
<400> 700
   tgtcttcacc agct gg 16
<210> 701
   <211> 14
   <212> DNA
   <213> Artificial Sequence
<220>
   <223> Synthetic Oligonucleotide
<400> 701
   gt ct tcacca gct g 14
<210> 702
   <211> 16
   <212> DNA
   <213> Artificial Sequence
<220>
   <223> Synthetic Oligonucleotide
<400> 702
   gtgtgtcttc accagc 16
<210> 703
   <211> 14
   <212> DNA
   <213> Artificial Sequence
<220>
   <223> Synthetic Oligonucleotide
<400> 703
   t gt gt ct t ca ccag 14
<210> 704
   <211> 16
   <212> DNA
   <213> Artificial Sequence
<220>
   <223> Synthetic Oligonucleotide
<400> 704
   ttgtgtgtct tcacca 16
<210> 705
   <211> 14
   <212> DNA
   <213> Artificial Sequence
<220>
   <223> Synthetic Oligonucleotide
<400> 705
   t gt gt gt ct t cacc 14
<210> 706
   <211> 16
   <212> DNA
   <213> Artificial Sequence
<220>
   <223> Synthetic Oligonucleotide
<400> 706
   aggt t gt gt g t ct t ca 16
<210> 707
   <211> 14
   <212> DNA
   <213> Artificial Sequence
<220>
   <223> Synthetic Oligonucleotide
<400> 707
   ggt t gt gt gt ct tc 14
<210> 708
   <211> 16
   <212> DNA
   <213> Artificial Sequence
<220>
   <223> Synthetic Oligonucleotide
<400> 708
   gcaggt t gt g t gt ct t 16
<210> 709
   <211> 14
   <212> DNA
   <213> Artificial Sequence
<220>
   <223> Synthetic Oligonucleotide
<400> 709
   caggt t gt gt gt ct 14
<210> 710
   <211> 16
   <212> DNA
   <213> Artificial Sequence
<220>
   <223> Synthetic Oligonucleotide
<400> 710 t cagcaggt t gt gt gt 16
<210> 711
   <211> 14
   <212> DNA
   <213> Artificial Sequence
<220>
   <223> Synthetic Oligonucleotide
<400> 711
   cagcaggt t g t gt g 14
<210> 712
   <211> 16
   <212> DNA
   <213> Artificial Sequence
<220>
   <223> Synthetic Oligonucleotide
<400> 712
   ggtcagcagg t t gt gt 16
<210> 713
   <211> 16
   <212> DNA
   <213> Artificial Sequence
<220>
   <223> Synthetic Oligonucleotide
<400> 713
   t ggt cagcag gt t gt g 16
<210> 714
   <211> 14
   <212> DNA
   <213> ArtificialSequence
<220>
   <223> Synthetic Oligonucleotide
<400> 714
   gt cagcaggt t gt g 14
<210> 715
   <211> 14
   <212> DNA
   <213> Artificial Sequence
<220>
   <223> Synthetic Oligonucleotide
<400> 715
   ggt cagcagg t tgt 14
<210> 716
   <211> 16
   <212> DNA
   <213> Artificial Sequence
<220>
   <223> Synthetic Oligonucleotide
<400> 716
   ct ggt ggt ca gcaggt 16
<210> 717
   <211> 14
   <212> DNA
   <213> Artificial Sequence
<220>
   <223> Synthetic Oligonucleotide
<400> 717
   t ggt ggt cag cagg 14
<210> 718
   <211> 12
   <212> DNA
   <213> Artificial Sequence
<220>
   <223> Synthetic Oligonucleotide
<400> 718
   ct ggt ggt ca gc 12
<210> 719
   <211> 16
   <212> DNA
   <213> Artificial Sequence
<220>
   <223> Synthetic Oligonucleotide
<400> 719
   agt t cct ggt ggt cag 16
<210> 720
   <211> 14
   <212> DNA
   <213> Artificial Sequence
<220>
   <223> Synthetic Oligonucleotide
<400> 720
   gt t cct ggt g gt ca 14
<210> 721
   <211> 16
   <212> DNA
   <213> Artificial Sequence
<220>
   <223> Synthetic Oligonucleotide
<400> 721
   t at agt t cct ggt ggt 16
<210> 722
   <211> 14
   <212> DNA
   <213> Artificial Sequence
<220>
   <223> Synthetic Oligonucleotide
<400> 722
   at agt t cct g gt gg 14
<210> 723
   <211> 16
   <212> DNA
   <213> Artificial Sequence
<220>
   <223> Synthetic Oligonucleotide
<400> 723
   gat at agt t c ct ggt g 16
<210> 724
   <211> 14
   <212> DNA
   <213> Artificial Sequence
<220>
   <223> Synthetic Oligonucleotide
<400> 724
   at at agt t cc t ggt 14
<210> 725
   <211> 16
   <212> DNA
   <213> Artificial Sequence
<220>
   <223> Synthetic Oligonucleotide
<400> 725 16
   ccaaagat at agt t cc 16
<210> 726
   <211> 16
   <212> DNA
   <213> Artificial Sequence
<220>
   <223> Synthetic Oligonucleotide
<400> 726
   t ccaaagat a t agt t c 16
<210> 727
   <211> 14
   <212> DNA
   <213> Artificial Sequence
<220>
   <223> Synthetic Oligonucleotide
<400> 727
   caaagat at a gt t c 14
<210> 728
   <211> 14
   <212> DNA
   <213> Artificial Sequence
<220>
   <223> Synthetic Oligonucleotide
<400> 728
   ccaaagat at agt t 14
<210> 729
   <211> 12
   <212> DNA
   <213> Artificial Sequence
<220>
   <223> Synthetic Oligonucleotide
<400> 729
   ccaggcccat ga 12
<210> 730
   <211> 16
   <212> DNA
   <213> Artificial Sequence
<220>
   <223> Synthetic Oligonucleotide
<400> 730
   ggcacccagg cccatg 16
<210> 731
   <211> 14
   <212> DNA
   <213> Artificial Sequence
<220>
   <223> Synthetic Oligonucleotide
<400> 731
   gcacccaggc ccat 14
<210> 732
   <211> 16
   <212> DNA
   <213> Artificial Sequence
<220>
   <223> Synthetic Oligonucleotide
<400> 732
   cagaaggcac ccaggc 16
<210> 733
   <211> 14
   <212> DNA
   <213> Artificial Sequence
<220>
   <223> Synthetic Oligonucleotide
<400> 733
   agaaggcacc cagg 14
<210> 734
   <211> 12
   <212> DNA
   <213> Artificial Sequence
<220>
   <223> Synthetic Oligonucleotide
<400> 734
   ccagacatca gg 12
<210> 735
   <211> 12
   <212> DNA
   <213> Artificial Sequence
<220>
   <223> Synthetic Oligonucleotide
<400> 735
   gacagggcag at 12
<210> 736
   <211> 12
   <212> DNA
   <213> Artificial Sequence
<220>
   <223> Synthetic Oligonucleotide
<400> 736
   tgacagggca ga 12
<210> 737
   <211> 12
   <212> DNA
   <213> Artificial Sequence <220>
   <223> Synthetic Oligonucleotide
<400> 737
   ccact cccat t c 12
<210> 738
   <211> 16
   <212> DNA
   <213> Artificial Sequence
<220>
   <223> Synthetic Oligonucleotide
<400> 738
   gccaggcatg gagctc 16
<210> 739
   <211> 14
   <212> DNA
   <213> Artificial Sequence <220>
   <223> Synthetic Oligonucleotide
<400> 739
   ccaggcatgg agct 14
<210> 740
   <211> 12
   <212> DNA
   <213> Artificial Sequence
<220>
   <223> Synthetical gonucleotide
<400> 740
   ccaggcatgg ag 12
<210> 741
   <211> 12
   <212> DNA
   <213> Artificial Sequence
<220>
   <223> Synthetic Oligonucleotide
<400> 741
   cagggt gact gc 12
<210> 742
   <211> 16
   <212> DNA
   <213> Artificial Sequence
<220>
   <223> Synthetic Oligonucleotide
<400> 742
   gt t ccgcagg gtgact 16
<210> 743
   <211> 14
   <212> DNA
   <213> Artificial Sequence
<220>
   <223> Synthetic Oligonucleotide
<400> 743
   t t ccgcaggg t gac 14
<210> 744
   <211> 16
   <212> DNA
   <213> Artificial Sequence
<220>
   <223> Synthetic Oligonucleotide
<400> 744
   gcggt t ccgc agggt g 16
<210> 745
   <211> 14
   <212> DNA
   <213> Artificial Sequence
<220>
   <223> Synthetic Oligonucleotide
<400> 745
   cggt t ccgca gggt 14
<210> 746
   <211> 16
   <212> DNA
   <213> Artificial Sequence
<220>
   <223> Synthetic Oligonucleotide
<400> 746
   tcggccccag gagccc 16
<210> 747
   <211> 14
   <212> DNA
   <213> Artificial Sequence
<220>
   <223> Synthetic Oligonucleotide
<400> 747
   cggccccagg agcc 14
<210> 748
   <211> 16
   <212> DNA
   <213> Artificial Sequence
<220>
   <223> Synthetic Oligonucleotide
<400> 748
   ccat cggccc caggag 16
<210> 749
   <211> 14
   <212> DNA
   <213> Artificial Sequence
<220>
   <223> Synthetic Oligonucleotide
<400> 749
   cat cggcccc agga 14
<210> 750
   <211> 16
   <212> DNA
   <213> Artificial Sequence
<220>
   <223> Synthetic Oligonucleotide
<400> 750
   gacccatcgg ccccag 16
<210> 751
   <211> 14
   <212> DNA
   <213> Artificial Sequence
<220>
   <223> Synthetic Oligonucleotide
<400> 751
   acccatcggc ccca 14
<210> 752
   <211> 16
   <212> DNA
   <213> Artificial Sequence
<220>
   <223> Synthetic Oligonucleotide
<400> 752
   t t ct ggaccc at cggc 16
<210> 753
   <211> 12
   <212> DNA
   <213> Artificial Sequence
<220>
   <223> Synthetical gonucleotide
<400> 753
   ggacccat cg gc 12
<210> 754
   <211> 14
   <212> DNA
   <213> Artificial Sequence
<220>
   <223> Synthetic Oligonucleotide
<400> 754
   t ct ggaccca t cgg 14
<210> 755
   <211> 16
   <212> DNA
   <213> Artificial Sequence
<220>
   <223> Synthetic Oligonucleotide
<400> 755
   caccagcccc caggt g 16
<210> 756
   <211> 14
   <212> DNA
   <213> Artificial Sequence
<220>
   <223> Synthetic Oligonucleotide
<400> 756
   accagccccc aggt 14
<210> 757
   <211> 16
   <212> DNA
   <213> Artificial Sequence
<220>
   <223> Synthetic Oligonucleotide
<400> 757
   tagggcacca gccccc 16
<210> 758
   <211> 14
   <212> DNA
   <213> Artificial Sequence
<220>
   <223> Synthetic Oligonucleotide
<400> 758
   agggcaccag cccc 14
<210> 759
   <211> 16
   <212> DNA
   <213> Artificial Sequence
<220>
   <223> Synthetic Oligonucleotide
<400> 759
   tggagtaggg caccag 16
<210> 760
   <211> 14
   <212> DNA
   <213> Artificial Sequence
<220>
   <223> Synthetic Oligonucleotide
<400> 760
   ggagt agggc acca 14
<210> 761
   <211> 12
   <212> DNA
   <213> Artificial Sequence
<220>
   <223> Synthetic Oligonucleotide
<400> 761
   ct t ggagt ag gg 12
<210> 762
   <211> 16
   <212> DNA
   <213> Artificial Sequence
<220>
   <223> Synthetic Oligonucleotide
<400> 762
   tgatgggcttggagta 16
<210> 763
   <211> 14
   <212> DNA
   <213> Artificial Sequence
<220>
   <223> Synthetic Oligonucleotide
<400> 763
   gat gggct t g gagt 14
<210> 764
   <211> 16
   <212> DNA
   <213> Artificial Sequence
<220>
   <223> Synthetic Oligonucleotide
<400> 764
   tgtggtacag gtcgat 16
<210> 765
   <211> 14
   <212> DNA
   <213> Artificial Sequence
<220>
   <223> Synthetic Oligonucleotide
<400> 765
   gt ggt acagg t cga 14
<210> 766
   <211> 12
   <212> DNA
   <213> Artificial Sequence
<220>
   <223> Synthetic Oligonucleotide
<400> 766
   ggt acaggt c ga 12
<210> 767
   <211> 16
   <212> DNA
   <213> Artificial Sequence
<220>
   <223> Synthetic Oligonucleotide
<400> 767
   ggt gt ggt ac aggt cg 16
<210> 768
   <211> 14
   <212> DNA
   <213> Artificial Sequence
<220>
   <223> Synthetic Oligonucleotide
<400> 768
   gt gt ggt aca ggt c 14
<210> 769
   <211> 16
   <212> DNA
   <213> Artificial Sequence
<220>
   <223> Synthetic Oligonucleotide
<400> 769
   cat ggt gt gg t acagg 16
<210> 770
   <211> 14
   <212> DNA
   <213> Artificial Sequence
<220>
   <223> Synthetic Oligonucleotide
<400> 770
   at ggt gt ggt acag 14
<210> 771
   <211> 16
   <212> DNA
   <213> Artificial Sequence
<220>
   <223> Synthetic Oligonucleotide
<400> 771
   t acat ggt gt ggt aca 16
<210> 772
   <211> 14
   <212> DNA
   <213> Artificial Sequence
<220>
   <223> Synthetic Oligonucleotide
<400> 772
   acat ggt gt g gt ac 14
<210> 773
   <211> 16
   <212> DNA
   <213> Artificial Sequence
<220>
   <223> Synthetic Oligonucleotide
<400> 773
   at gt acat gg t gt ggt 16
<210> 774
   <211> 14
   <212> DNA
   <213> Artificial Sequence
<220>
   <223> Synthetic Oligonucleotide
<400> 774
   t gt acat ggt gt gg 14
<210> 775
   <211> 12
   <212> DNA
   <213> Artificial Sequence
<220>
   <223> Synthetic Oligonucleotide
<400> 775
   gcct ccat gt ac 12
<210> 776
   <211> 12
   <212> DNA
   <213> Synthetic Oligonucleotide
<220>
   <223> Synthetic Oligonucleotide
<400> 776
   agct t cacca gg 12
<210> 777
   <211> 12
   <212> DNA
   <213> Artificial Sequence
<220>
   <223> Synthetic Oligonucleotide
<400> 777
   gt t cacct cc ag 12
<210> 778
   <211> 16
   <212> DNA
   <213> Artificial Sequence
<220>
   <223> Synthetic Oligonucleotide
<400> 778
   cat gccccag ccgccg 16
<210> 779
   <211> 14
   <212> DNA
   <213> Artificial Sequence
<220>
   <223> Synthetic Oligonucleotide
<400> 779
   at gccccagc cgcc 14
<210> 780
   <211> 16
   <212> DNA
   <213> Artificial Sequence
<220>
   <223> Synthetic Oligonucleotide
<400> 780
   gat gagggt c tt cat g 16
<210> 781
   <211> 16
   <212> DNA
   <213> Artificial Sequence
<220>
   <223> Synthetic Oligonucleotide
<400> 781
   gaccccggag t aggca 16
<210> 782
   <211> 16
   <212> DNA
   <213> Artificial Sequence
<220>
   <223> Synthetic Oligonucleotide
<400> 782
   at gct ggagc cagt gc 16
<210> 783
   <211> 14
   <212> DNA
   <213> Artificial Sequence
<220>
   <223> Synthetic Oligonucleotide
<400> 783
   t gct ggagcc agtg 14
<210> 784
   <211> 14
   <212> DNA
   <213> Artificial Sequence
<220>
   <223> Synthetic Oligonucleotide
<400> 784
   gt ct t ggagg gccg 14
<210> 785
   <211> 14
   <212> DNA
   <213> Artificial Sequence
<220>
   <223> Synthetic Oligonucleotide
<400> 785
   cccaggt gt c agag 14
<210> 786
   <211> 16
   <212> DNA
   <213> Artificial Sequence
<220>
   <223> Synthetic Oligonucleotide
<400> 786
   t t t t ccacct t ggat c 16
<210> 787
   <211> 14
   <212> DNA
   <213> Artificial Sequence
<220>
   <223> Synthetic Oligonucleotide
<400> 787
   t t t ccacct t ggat 14
<210> 788
   <211> 16
   <212> DNA
   <213> Artificial Sequence
<220>
   <223> Synthetic Oligonucleotide
<400> 788
   t t t t t ccacc t t ggat 16
<210> 789
   <211> 16
   <212> DNA
   <213> Artificial Sequence
<220>
   <223> Synthetic Oligonucleotide
<400> 789
   aggt gt t t t t ccacct 16
<210> 790
   <211> 14
   <212> DNA
   <213> Artificial Sequence
<220>
   <223> Synthetic Oligonucleotide
<400> 790
   ggt gt t t t t c cacc 14
<210> 791
   <211> 16
   <212> DNA
   <213> Artificial Sequence
<220>
   <223> Synthetic Oligonucleotide
<400> 791
   ccaggaagga t aggac 16
<210> 792
   <211> 14
   <212> DNA
   <213> Artificial Sequence
<220>
   <223> Synthetic Oligonucleotide
<400> 792
   caggaaggat agga 14
<210> 793
   <211> 16
   <212> DNA
   <213> Artificial Sequence
<220>
   <223> Synthetic Oligonucleotide
<400> 793
   t gacactgca ggccac 16
<210> 794
   <211> 14
   <212> DNA
   <213> Artificial Sequence
<220>
   <223> Synthetic Oligonucleotide
<400> 794
   gacactgcag gcca 14
<210> 795
   <211> 16
   <212> DNA
   <213> Artificial Sequence
<220>
   <223> Synthetic Oligonucleotide
<400> 795
   acat gaggat gacact 16
<210> 796
   <211> 14
   <212> DNA
   <213> Artificial Sequence
<220>
   <223> Synthetic Oligonucleotide
<400> 796
   catgaggatg acac 14
<210> 797
   <211> 16
   <212> DNA
   <213> Artificial Sequence
<220>
   <223> Synthetic Oligonucleotide
<400> 797
   gcagaaggtg t acat g 16
<210> 798
   <211> 14
   <212> DNA
   <213> Artificial Sequence
<220>
   <223> Synthetic Oligonucleotide
<400> 798
   cagaaggt gt acat 14
<210> 799
   <211> 16
   <212> DNA
   <213> Artificial Sequence
<220>
   <223> Synthetic Oligonucleotide
<400> 799
   gcagt cagt g cagaag 16
<210> 800
   <211> 14
   <212> DNA
   <213> Artificial Sequence
<220>
<223> Synthetic Oligonucleotide
<400> 800
   cagt cagt gc agaa 14
<210> 801
   <211> 16
   <212> DNA
   <213> Artificial Sequence
<220>
   <223> Synthetic Oligonucleotide
<400> 801
   acacggccca gtttcg 16
<210> 802
   <211> 14
   <212> DNA
   <213> Artificial Sequence
<220>
   <223> Synthetic Oligonucleotide
<400> 802
   cacggcccag tttc 14
<210> 803
   <211> 16
   <212> DNA
   <213> Artificial Sequence
<220>
   <223> Synthetic Oligonucleotide
<400> 803
   ggcccat gat gccat g 16
<210> 804
   <211> 14
   <212> DNA
   <213> Artificial Sequence
<220>
   <223> Synthetic Oligonucleotide
<400> 804
   gcccat gat g ccat 14
<210> 805
   <211> 16
   <212> DNA
   <213> Artificial Sequence
<220>
   <223> Synthetical Oligonucleotide
<400> 805
   t acagaaggc acccag 16
<210> 806
   <211> 14
   <212> DNA
   <213> Artificial Sequence
<220>
   <223>Synthetic Oligonucleotide
<400> 806
   acagaaggca ccca 14
<210> 807
   <211> 16
   <212> DNA
   <213> Artificial Sequence

<220>
   <223> Synthetic Oligonucleotide
<400> 807
   gaagt t gcca gccaat 16
<210> 808
   <211> 14
   <212> DNA
   <213> Artificial Sequence
<220>
   <223> Synthetic Oligonucleotide
<400> 808
   aagt t gccag ccaa 14
<210> 809
   <211> 14
   <212> DNA
   <213>Artificial Sequence
<220>
   <223> Synthetic Oligonucleotide
<400> 809
   cagggcagat cct t 14
<210> 810
   <211> 16
   <212> DNA
   <213> Artificial Sequence
<220>
   <223> Synthetic Oligonucleotide
<400> 810
   caagt agt ct at ggt g 16
<210> 811
   <211> 14
   <212> DNA
   <213> Artificial Sequence
<220>
   <223> Synthetic Oligonucleotide
<400> 811
   aagt agt ct a t ggt 14
<210> 812
   <211> 16
   <212> DNA
   <213> Artificial Sequence
<220>
   <223> Synthetic Oligonucleotide
<400> 812
   t ggaaagcaa gt agt c 16
<210> 813
   <211> 14
   <212> DNA
   <213> Artificial Sequence
<220>
   <223> Synthetic Oligonucleotide
<400> 813
   ggaaagcaag t agt 14
<210> 814
   <211> 16
   <212> DNA
   <213> Artificial Sequence
<220>
   <223> Synthetical Oligonucleotide
<400> 814
   ggccagct t t t acaaag 16
<210> 815
   <211> 14
   <212> DNA
   <213> Artificial Sequence
<220>
   <223> Synthetic Oligonucleotide
<400> 815
   gccagct t t a caaa 14
<210> 816
   <211> 14
   <212> DNA
   <213> Artificial Sequence
<220>
   <223> Synthetic Oligonucleotide
<400> 816
   cgcagggcca gctt 14
<210> 817
   <211> 16
   <212> DNA
   <213> Artificial Sequence
<220>
   <223> Synthetic Oligonucleotide
<400> 817
   aaaggaat ag gt ggga 16
<210> 818
   <211> 14
   <212> DNA
   <213> Artificial Sequence
<220>
   <223>Synthetic Oligonucleotide

<400> 818
   aaggaat agg t ggg 14
<210> 819
   <211> 16
   <212> DNA
   <213> Artificial Sequence
<220>
   <223> Synt hetic Oligonucleotide
<400> 819
   gcgaaaccaa t at act 16
<210> 820
   <211> 14
   <212> DNA
   <213> Artificial Sequence
<220>
   <223> Synthetic Oligonucleotide
<400> 820
   cgaaaccaat at ac 14
<210> 821
   <211> 16
   <212> DNA
   <213> Artificial Sequence
<220>
   <223> Synthetic Oligonucleotide
<400> 821
   ccccaggt gt cagagg 16
<210> 822
   <211> 4
   <212> DNA
   <213> Artificial Sequence
<220>
   <223> Synthetic Oligonucleotide
<400> 822
   act g 4
<210> 823
   <211> 16
   <212> DNA
   <213> Artificial Sequence
<220>
   <223> Synthetic Oligonucleotide

<400> 823
   gat t gt caaa gagct t 16
<210> 824
   <211> 14
   <212> DNA
   <213> Artificial Sequence
<220>
   <223> Synthetic Oligonucleotide
<400> 824
   at t gt caaag agct 14
<210> 825
   <211> 16
   <212> DNA
   <213> Artificial Sequence
<220>
   <223> Synthetic Oligonucleotide
<400> 825
   t t ggt ct t gt gat t gt 16
<210> 826
   <211> 14
   <212> DNA
   <213> Artificial Sequence
<220>
   <223> Synthetic Oligonucleotide
<400> 826
   t ggt ct t gt g at t g 14
<210> 827
   <211> 16
   <212> DNA
   <213> Artificial Sequence
<220>
   <223> Synthetic Oligonucleotide
<400> 827
   aaggccgaat t t ggt c 16
<210> 828
   <211> 14
   <212> DNA
   <213> Artificial Sequence
<220>
   <223> Synthetic Oligonucleotide
<400> 828
   aggccgaa t t ggt 14
<210> 829
   <211> 12
   <212> DNA
   <213> Artificial Sequence
<220>
   <223> Synthetic Oligonucleotide
<400> 829
   aat ccgact g tg 12
<210> 830
   <211> 12
   <212> DNA
   <213> Artificial Sequence
<220>
   <223> Synthetic Oligonucleotide
<400> 830
   aatttaatcc ga 12
<210> 831
   <211> 14
   <212> DNA
   <213> Artificial Sequence
<220>
   <223> Synthetic Oligonucleotide
<400> 831
   acct t cgat c acag 14
<210> 832
   <211> 12
   <212> DNA
   <213> Artificial Sequence
<220>
   <223> Synthetic Oligonucleotide
<400> 832
   act gat cct g ca 12
<210> 833
   <211> 12
   <212> DNA
   <213> Artificial Sequence
<220>
   <223> Synthetic Oligonucleotide
<400> 833
   act gt ggt ca aa 12
<210> 834
   <211> 12
   <212> DNA
   <213> Artificial Sequence
<220>
   <223> Synthetic Oligonucleotide
<400> 834
   agccgcccag tt 12
<210> 835
   <211> 12
   <212> DNA
   <213> Artificial Sequence
<220>
   <223> Synthetic Oligonucleotide
<400> 835
   agcct t gt cg at 12
<210> 836
   <211> 12
   <212> DNA
   <213> Artificial Sequence
<220>
   <223> Synthetic Oligonucleotide
<400> 836
   agt t cccagc ct 12
<210> 837
   <211> 12
   <212> DNA
   <213> Artificial Sequence
<220>
   <223> Synthetic Oligonucleotide
<400> 837
   at ccgact gt gg 12
<210> 838
   <211> 12
   <212> DNA
   <213> Artificial Sequence
<220>
   <223> Synthetic Oligonucleotide
<400> 838
   at cct gcact ga 12
<210> 839
   <211> 12
   <212> DNA
   <213> Artificial Sequence
<220>
   <223> Synthetic Oligonucleotide
<400> 839
   at t t aat ccg ac 12
<210> 840
   <211> 12
   <212> DNA
   <213> Artificial Sequence
<220>
   <223> Synthetic Oligonucleotide
<400> 840
   caat t t aat c cg 12
<210> 841
   <211> 12
   <212> DNA
   <213> Artificial Sequence
<220>
   <223> Synthetic Oligonucleotide
<400> 841
   cact gacgag t c 12
<210> 842
   <211> 12
   <212> DNA
   <213> Artificial Sequence
<220>
   <223> Synthetic Oligonucleotide
<400> 842
   cact gat cct gc 12
<210> 843
   <211> 12
   <212> DNA
   <213> Artificial Sequence
<220>
   <223> Synthetic Oligonucleotide
<400> 843
   cagcct t gt c ga 12
<210> 844
   <211> 12
   <212> DNA
   <213> Artificial Sequence
<220>
   <223> Synthetic Oligonucleotide
<400> 844
   cagt t cccag cc 12
<210> 845
   <211> 12
   <212> DNA
   <213> Artificial Sequence
<220>
   <223> Synthetic Oligonucleotide
<400> 845
   ccactgatcc t g 12
<210> 846
   <211> 12
   <212> DNA
   <213> Artificial Sequence
<220>
   <223> Synthetic Oligonucleotide
<400> 846
   ccagcct t gt cg 12
<210> 847
   <211> 12
   <212> DNA
   <213> Artificial Sequence
<220>
   <223> Synthetic Oligonucleotide
<400> 847
   ccagt t ccca gc 12
<210> 848
   <211> 12
   <212> DNA
   <213> Artificial Sequence
<220>
   <223> Synthetic Oligonucleotide
<400> 848
   cccagcct tg tc 12
<210> 849
   <211> 12
   <212> DNA
   <213> Artificial Sequence
<220>
   <223> Synthetic Oligonucleotide
<400> 849
   cccagttccc ag 12
<210> 850
   <211> 12
   <212> DNA
   <213> Artificial Sequence
<220>
   <223> Synthetic Oligonucleotide
<400> 850
   ccgcccagt t cc 12
<210> 851
   <211> 12
   <212> DNA
   <213> Artificial Sequence
<220>
   <223> Synthetic Oligonucleotide
<400> 851
   cct gcact ga cg 12
<210> 852
   <211> 14
   <212> DNA
   <213> Artificial Sequence
<220>
   <223> Synthetic Oligonucleotide
<400> 852
   ccttggaatg t ct g 14
<210> 853
   <211> 12
   <212> DNA
   <213> Artificial Sequence
<220>
   <223> Synthetic Oligonucleotide
<400> 853
   cct t gt cgat ct 12
<210> 854
   <211> 12
   <212> DNA
   <213> Artificial Sequence
<220>
   <223> Synthetic Oligonucleotide
<400> 854
   cgact gt ggt ca 12
<210> 855
   <211> 12
   <212> DNA
   <213> Artificial Sequence
<220>
   <223> Synthetic Oligonucleotide
<400> 855
   cgcccagttc cc 12
<210> 856
   <211> 12
   <212> DNA
   <213> Artificial Sequence
<220>
   <223> Synthetic Oligonucleotide
<400> 856
   ctgatcctgc ac 12
<210> 857
   <211> 12
   <212> DNA
   <213> Artificial Sequence
<220>
   <223> Synthetic Oligonucleotide
<400> 857
   ctgcactgac ga 12
<210> 858
   <211> 12
   <212> DNA
   <213> Artificial Sequence
<220>
   <223> Synthetic Oligonucleotide
<400> 858
   ct gt ggt caa aa 12
<210> 859
   <211> 12
   <212> DNA
   <213> Artificial Sequence
<220>
   <223> Synthetic Oligonucleotide
<400> 859
   ct t gt cgat c t c 12
<210> 860
   <211> 12
   <212> DNA
   <213> Artificial Sequence
<220>
   <223> Synthetic Oligonucleotide
<400> 860
   gatcctgcac tg 12
<210> 861
   <211> 12
   <212> DNA
   <213> Artificial Sequence
<220>
   <223> Synthetic Oligonucleotide
<400> 861
   gcaatttaat cc 12
<210> 862
   <211> 12
   <212> DNA
   <213> Artificial Sequence
<220>
   <223> Synthetic Oligonucleotide
<400> 862
   gcact gacga gt 12
<210> 863
   <211> 12
   <212> DNA
   <213> Artificial Sequence
<220>
   <223> Synthetic Oligonucleotide
<400> 863
   gcccagttcc ca 12
<210> 864
   <211> 12
   <212> DNA
   <213> Artificial Sequence
<220>
   <223> Synthetic Oligonucleotide
<400> 864
   gccgcccagt tc 12
<210> 865
   <211> 12
   <212> DNA
   <213> Artificial Sequence
<220>
   <223> Synthetic Oligonucleotide
<400> 865
   gcct t gt cga t c 12
<210> 866
   <211> 12
   <212> DNA
   <213> Artificial Sequence
<220>
   <223> Synthetic Oligonucleotide
<400> 866
   ggt caaaagg gc 12
<210> 867
   <211> 14
   <212> DNA
   <213> Artificial Sequence
<220>
   <223> Synthetic Oligonucleotide
<400> 867
   ggt cat gcac aggc 14
<210> 868
   <211> 12
   <212> DNA
   <213> Artificial Sequence
<220>
   <223> Synthetic Oligonucleotide
<400> 868
   gtcgatctcc tc 12
<210> 869
   <211> 12
   <212> DNA
   <213> Artificial Sequence
<220>
   <223> Synthetic Oligonucleotide
<400> 869
   gtggtcaaaa gg 12
<210> 870
   <211> 12
   <212> DNA
   <213> Artificial Sequence
<220>
   <223> Synthetic Oligonucleotide
<400> 870
   gt t cccagcc t t 12
<210> 871
   <211> 12
   <212> DNA
   <213> Artificial Sequence
<220>
   <223> Synthetic Oligonucleotide
<400> 871
   t aat ccgact gt 12
<210> 872
   <211> 14
   <212> DNA
   <213> Artificial Sequence
<220>
   <223> Synthetic Oligonucleotide
<400> 872
   tattccatgg ccat 14
<210> 873
   <211> 14
   <212> DNA
   <213> Artificial Sequence
<220>
   <223> Synthetic Oligonucleotide
<400> 873
   t caggt cat g caca 14
<210> 874
   <211> 12
   <212> DNA
   <213> Artificial Sequence
<220>
   <223> Synthetic Oligonucleotide
<400> 874
   t cccagcct t gt 12
<210> 875
   <211> 12
   <212> DNA
   <213> Artificial Sequence
<220>
   <223> Synthetic Oligonucleotide
<400> 875
   t ccgact gt g gt 12
<210> 876
   <211> 12
   <212> DNA
   <213> Artificial Sequence
<220>
   <223> Synthetic Oligonucleotide
<400> 876
   tcctgcactg ac 12
<210> 877
   <211> 12
   <212> DNA
   <213> Artificial Sequence
<220>
   <223> Synthetic Oligonucleotide
<400> 877
   tcgatctcct cg 12
<210> 878
   <211> 12
   <212> DNA
   <213> Artificial Sequence
<220>
   <223> Synthetic Oligonucleotide
<400> 878
   t gat cct gca ct 12
<210> 879
   <211> 12
   <212> DNA
   <213> Artificial Sequence
<220>
   <223> Synthetic Oligonucleotide
<400> 879
   tgcaatttaa t c 12
<210> 880
   <211> 12
   <212> DNA
   <213> Artificial Sequence
<220>
   <223> Synthetic Oligonucleotide
<400> 880
   tgcactgacg ag 12
<210> 881
   <211> 12
   <212> DNA
   <213> Artificial Sequence
<220>
   <223> Synthetic Oligonucleotide
<400> 881
   tggtcaaaag gg 12
<210> 882
   <211> 12
   <212> DNA
   <213> Artificial Sequence
<220>
   <223> Synthetic Oligonucleotide
<400> 882
   t gt cgat ct c ct 12
<210> 883
   <211> 12
   <212> DNA
   <213> Artificial Sequence
<220>
   <223> Synthetic Oligonucleotide

<400> 883
   tgtggtcaaa ag 12
<210> 884
   <211> 12
   <212> DNA
   <213> Artificial Sequence
<220>
   <223> Synthetic Oligonucleotide
<400> 884
   t t aat ccgac t g 12
<210> 885
   <211> 12
   <212> DNA
   <213> Artificial Sequence
<220>
   <223> Synthetic Oligonucleotide
<400> 885
   t t cccagcct tg 12
<210> 886
   <211> 12
   <212> DNA
   <213> Artificial Sequence
<220>
   <223> Synthetic Oligonucleotide
<400> 886
   ttgtcgatct cc 12
<210> 887
   <211> 12
   <212> DNA
   <213> Artificial Sequence
<220>
   <223> Synthetic Oligonucleotide
<400> 887
   t t taat ccga ct 12
<210> 888
   <211> 12 <212> DNA
   <213> Artificial Sequence
<220>
   <223> Synthetic Oligonucleotide
<400> 888
   gact gt ggt caa 12
<210> 889
   <211> 12
   <212> DNA
   <213> Artificial Sequence
<220>
   <223> Synthetic Oligonucleotide
<400> 889
   ccgactgtgg tc 12
<210> 890
   <211> 14
   <212> DNA
   <213> Artificial Sequence
<220>
   <223> Synthetic Oligonucleotide
<400> 890
   cccagtgggt ttga 14
<210> 891
   <211> 12
   <212> DNA
   <213> Artificial Sequence
<220>
   <223> Synthetic Oligonucleotide
<400> 891
   ttcttgatgt cc 12
<210> 892
   <211> 12
   <212> DNA
   <213> Artificial Sequence
<220>
   <223> Synthetic Oligonucleotide
<400> 892
   gact ccaaag t c 12
<210> 893
   <211> 16
   <212> DNA
   <213> Artificial Sequence
<220>
   <223> Synthetic Oligonucleotide
<400> 893
   t cct gcact g acgagt 16
<210> 894
   <211> 16
   <212> DNA
   <213> Artificial Sequence
<220>
   <223> Synthetic Oligonucleotide
<400> 894
   cact gat cct gcact g 16
<210> 895
   <211> 16
   <212> DNA
   <213> Artificial Sequence
<220>
   <223> Synthetic Oligonucleotide
<400> 895
   ct t ccact ga t cct t a 16
<210> 896
   <211> 16
   <212> DNA
   <213> Artificial Sequence
<220>
   <223> Synthetic Oligonucleotide
<400> 896
   gaaagct cct t ccact 16
<210> 897
   <211> 14
   <212> DNA
   <213> Artificial Sequence
<220>
   <223> Synthetic Oligonucleotide
<400> 897
   ggcagt ct t t at cc 14
<210> 898
   <211> 12
   <212> DNA
   <213> Artificial Sequence
<220>
   <223> Synthetic Oligonucleotide
<400> 898
   ct ggt aaat a gc 12
<210> 899
   <211> 14
   <212> DNA
   <213> Artificial Sequence
<220>
   <223> Synthetic Oligonucleotide
<400> 899
   ct acct gagg at t t 14
<210> 900
   <211> 14
   <212> DNA
   <213> Artificial Sequence
<220>
   <223> Synthetic Oligonucleotide
<400> 900
   cccagt act a cct g 14
<210> 901
   <211> 12
   <212> DNA
   <213> Artificial Sequence
<220>
   <223> Synthetic Oligonucleotide
<400> 901
   t gt t ccct ct ac 12
<210> 902
   <211> 14
   <212> DNA
   <213> Artificial Sequence
<220>
   <223> Synthetic Oligonucleotide
<400> 902
   t at t cct gga aaac 14
<210> 903
   <211> 14
   <212> DNA
   <213> Artificial Sequence
<220>
   <223> Synthetic Oligonucleotide
<400> 903
   caagaagt gt ggt t 14
<210> 904
   <211> 14
   <212> DNA
   <213> Artificial Sequence
<220>
   <223> Synthetic Oligonucleotide
<400> 904
   gt gaaaat gc t ggc 14
<210> 905
   <211> 14
   <212> DNA
   <213> Artificial Sequence
<220>
   <223> Synthetic Oligonucleotide
<400> 905
   aggaggt t aa acca 14
<210> 906
   <211> 12
   <212> DNA
   <213> Artificial Sequence
<220>
   <223> Synthetic Oligonucleotide
<400> 906
   t ggat gat t g gc 12
<210> 907
   <211> 12
   <212> DNA
   <213> Artificial Sequence
<220>
   <223> Synthetic Oligonucleotide
<400> 907
   caaaaggatc c cc 12
<210> 908
   <211> 12
   <212> DNA
   <213> Artificial Sequence
<220>
   <223> Synthetic Oligonucleotide
<400> 908
   at gt caaccg gc 12
<210> 909
   <211> 12
   <212> DNA
   <213> Artificial Sequence
<220>
   <223> Synthetic Oligonucleotide
<400> 909
   tcagccagac ag 12
<210> 910
   <211> 14
   <212> DNA
   <213> Artificial Sequence
<220>
   <223> Synthetic Oligonucleotide
<400> 910
   taagtgtccc tttg 14
<210> 911
   <211> 14
   <212> DNA
   <213> Artificial Sequence
<220>
   <223> Synthetic Oligonucleotide
<400> 911
   ctaaatttag ttca 14
<210> 912
   <211> 14
   <212> DNA
   <213> Artificial Sequence
<220>
   <223> Synthetic Oligonucleotide
<400> 912
   gact acat t t t aca 14
<210> 913
   <211> 14
   <212> DNA
   <213> Artificial Sequence
<220>
   <223> Synthetic Oligonucleotide
<400> 913
   cagt aaggaa t t t t 14
<210> 914
   <211> 14
   <212> DNA
   <213> Artificial Sequence
<220>
   <223> Synthetic Oligonucleotide
<400> 914
   t gt gt ccct c agt c 14
<210> 915
   <211> 12
   <212> DNA
   <213> Artificial Sequence
<220>
   <223> Synthetic Oligonucleotide
<400> 915
   ccgt t ggacc cc 12
<210> 916
   <211> 14
   <212> DNA
   <213> Artificial Sequence
<220>
   <223> Synthetic Oligonucleotide
<400> 916
   gacagct t ct at aa 14
<210> 917
   <211> 14
   <212> DNA
   <213> Artificial Sequence
<220>
   <223> Synthetic Oligonucleotide
<400> 917
   agt gact gac caca 14
<210> 918
   <211> 14
   <212> DNA
   <213> Artificial Sequence
<220>
   <223> Synthetic Oligonucleotide
<400> 918
   gt agcat aga gcct 14
<210> 919
   <211> 14
   <212> DNA
   <213> Artificial Sequence
<220>
   <223> Synthetic Oligonucleotide
<400> 919
   cagat ct t gt caag 14
<210> 920
   <211> 12
   <212> DNA
   <213> Artificial Sequence
<220>
   <223> Synthetic Oligonucleotide
<400> 920
   gtaagaggca gg 12
<210> 921
   <211> 12
   <212> DNA
   <213> Artificial Sequence
<220>
   <223> Synthetic Oligonucleotide
<400> 921
   ccatggcggg ac 12
<210> 922
   <211> 12
   <212> DNA
   <213> Artificial Sequence
<220>
   <223> Synthetic Oligonucleotide
<400> 922
   gct t acgat t gt 12
<210> 923
   <211> 14
   <212> DNA
   <213> Artificial Sequence
<220>
   <223> Synthetic Oligonucleotide
<400> 923
   ccagcacact ggaa 14
<210> 924
   <211> 14
   <212> DNA
   <213> Artificial Sequence
<220>
   <223> Synthetic Oligonucleotide
<400> 924
   gt cagt ccca gct a 14
<210> 925 <211> 14
   <212> DNA
   <213> Artificial Sequence
<220>
   <223> Synthetic Oligonucleotide
<400> 925
   t t agt at gac agct 14
<210> 926
   <211> 14
   <212> DNA
   <213> Artificial Sequence
<220>
   <223> Synthetic Oligonucleotide
<400> 926
   t cagt gt agg aaga 14
<210> 927
   <211> 14
   <212> DNA
   <213> Artificial Sequence
<220>
   <223> Synthetic Oligonucleotide
<400> 927
   t gaat at aca gat g 14
<210> 928
   <211> 12
   <212> DNA
   <213> Artificial Sequence
<220>
   <223> Synthetic Oligonucleotide
<400> 928
   cccgccacca cc 12
<210> 929
   <211> 12
   <212> DNA
   <213> Artificial Sequence
<220>
   <223> Synthetic Oligonucleotide
<400> 929
   ttgttccctc ta 12
<210> 930
   <211> 14
   <212> DNA
   <213> Artificial Sequence
<220>
   <223> Synthetic Oligonucleotide
<400> 930
   t ct acct gag tcca 14
<210> 931
   <211> 14
   <212> DNA
   <213> Artificial Sequence
<220>
   <223> Synthetic Oligonucleotide
<400> 931
   aacat caagc t t ga 14
<210> 932
   <211> 14
   <212> DNA
   <213> Artificial Sequence
<220>
   <223> Synthetic Oligonucleotide

<400> 932
   gat cacctt c agag 14
<210> 933
   <211> 14
   <212> DNA
   <213> Artificial Sequence
<220>
   <223> Synthetic Oligonucleotide
<400> 933
   t gaacacat c act a 14
<210> 934
   <211> 12
   <212> DNA
   <213> Artificial Sequence
<220>
   <223> Synthetic Oligonucleotide
<400> 934
   tttcctcttg tc 12
<210> 935
   <211> 14
   <212> DNA
   <213> Artificial Sequence
<220>
   <223> Synthetic Oligonucleotide
<400> 935
   t aat t gat gt caat 14
<210> 936
   <211> 14
   <212> DNA
   <213> Artificial Sequence
<220>
   <223> Synthetic Oligonucleotide
<400> 936
   agt aat t gat gt ca 14
<210> 937
   <211> 14
   <212> DNA
   <213> Artificial Sequence
<220>
   <223> Synthetic Oligonucleotide
<400> 937
   acagt aat t g at gt 14
<210> 938
   <211> 14
   <212> DNA
   <213> Artificial Sequence
<220>
   <223> Synthetic Oligonucleotide
<400> 938
   t t acagt aat t gat 14
<210> 939
   <211> 14
   <212> DNA
   <213> Artificial Sequence
<220>
   <223> Synthetic Oligonucleotide
<400> 939
   acttacagta attg 14
<210> 940
   <211> 14
   <212> DNA
   <213> Artificial Sequence
<220>
   <223> Synthetic Oligonucleotide
<400> 940
   agact t acag t aat 14
<210> 941
   <211> 14
   <212> DNA
   <213> Artificial Sequence
<220>
   <223> Synthetic Oligonucleotide
<400> 941
   t cagact t ac agt a 14
<210> 942
   <211> 14
   <212> DNA
   <213> Artificial Sequence
<220>
   <223> Synthetic Oligonucleotide
<400> 942
   aat cagact acag 14
<210> 943
   <211> 14
   <212> DNA
   <213> Artificial Sequence
<220>
   <223> Synthetic Oligonucleotide
<400> 943
   t gaat cagac t t ac 14
<210> 944
   <211> 14
   <212> DNA
   <213> Artificial Sequence
<220>
   <223> Synthetic Oligonucleotide
<400> 944
   aat gaat cag act t 14
<210> 945
   <211> 14
   <212> DNA
   <213> Artificial Sequence
<220>
   <223> Synthetic Oligonucleotide
<400> 945
   t gcaggat gt t gag 14
<210> 946
   <211> 14
   <212> DNA
   <213> Artificial Sequence
<220>
   <223> Synthetic Oligonucleotide
<400>.946
   ct ggt cagca t t ga 14
<210> 947
   <211> 14
   <212> DNA
   <213> Artificial Sequence
<220>
   <223> Synthetic Oligonucleotide
<400> 947
   caaagt ccct t agc 14
<210> 948
   <211> 14
   <212> DNA
   <213> Artificial Sequence
<220>
   <223> Synthetic Oligonucleotide
<400> 948
   atttctctta cagg 14
<210> 949
   <211> 12
   <212> DNA
   <213> Artificial Sequence
<220>
   <223> Synthetic Oligonucleotide
<400> 949
   t t aacgagcc t t 12
<210> 950
   <211> 12
   <212> DNA
   <213> Artificial Sequence
<220>
   <223> Synthetic Oligonucleotide
<400> 950
   cgacacggga ac 12
<210> 951
   <211> 12
   <212> DNA
   <213> Artificial Sequence
<220>
   <223> Synthetic Oligonucleotide
<400> 951
   caagt aggat gt 12
<210> 952
   <211> 14
   <212> DNA
   <213> Artificial Sequence
<220>
   <223> Synthetic Oligonucleotide
<400> 952
   gttccctttg cagg 14
<210> 953
   <211> 14
   <212> DNA
   <213> Artificial Sequence
<220>
   <223> Synthetic Oligonucleotide
<400> 953
   at t agccat a t ct c 14
<210> 954
   <211> 14
   <212> DNA
   <213> Artificial Sequence
<220>
   <223> Synthetic Oligonucleotide
<400> 954
   agcat t cagc agt g 14
<210> 955
   <211> 12
   <212> DNA
   <213> Artificial Sequence
<220>
   <223> Synthetic Oligonucleotide
<400> 955
   tt ccct ct ac ac 12
<210> 956
   <211> 12
   <212> DNA
   <213> Artificial Sequence
<220>
   <223> Synthetic Oligonucleotide
<400> 956
   t ccct ct aca cc 12
<210> 957
   <211> 14
   <212> DNA
   <213> Artificial Sequence
<220>
   <223> Synthetic Oligonucleotide
<400> 957
   ct acaggaca at ac 14
<210> 958
   <211> 14
   <212> DNA
   <213> Artificial Sequence
<220>
   <223> Synthetic Oligonucleotide
<400> 958
   aact gggt t a agt a 14
<210> 959
   <211> 14
   <212> DNA
   <213> Artificial Sequence
<220>
   <223> Synthetic Oligonucleotide
<400> 959
   t gaacacgct at cc 14
<210> 960
   <211> 14
   <212> DNA
   <213> Artificial Sequence
<220>
   <223> Synthetic Oligonucleotide
<400> 960
   t t t ccact t g ggtg 14
<210> 961
   <211> 12
   <212> DNA
   <213> Artificial Sequence
<220>
   <223> Synthetic Oligonucleotide
<400> 961
   t ct acaccag gt 12
<210> 962
   <211> 14
   <212> DNA
   <213> Artificial Sequence
<220>
   <223> Synthetic Oligonucleotide
<400> 962
   gaaagct cct t cca 14
<210> 963
   <211> 12
   <212> DNA,
   <213> Artificial Sequence
<220>
   <223> Synthetic Oligonucleotide
<400> 963
   aggt aggaga ag 12
<210> 964
   <211> 12
   <212> DNA
   <213> Artificial Sequence
<220>
   <223> Synthetic Oligonucleotide
<400> 964
   acccagtcag gg 12
<210> 965
   <211> 12
   <212> DNA
   <213> Artificial Sequence
<220>
   <223> Synthetic Oligonucleotide
<400> 965
   at gt cat t aa ac 12
<210> 966
   <211> 12
   <212> DNA
   <213> Artificial Sequence
<220>
   <223> Synthetic Oligonucleotide
<400> 966
   gaggtgggaa aa 12
<210> 967
   <211> 14
   <212> DNA
   <213> Artificial Sequence
<220>
   <223> Synthetic Oligonucleotide
<400> 967
   t t t cct agga ggt g 14
<210> 968
   <211> 12
   <212> DNA
   <213> Artificial Sequence
<220>
   <223> Synthetic Oligonucleotide
<400> 968
   gt t ct t agga ag 12
<210> 969
   <211> 12
   <212> DNA
   <213> Artificial Sequence
<220>
   <223> Synthetic Oligonucleotide
<400> 969
   ct ct acacca gg 12
<210> 970
   <211> 14
   <212> DNA
   <213> Artificial Sequence
<220>
   <223> Synthetic Oligonucleotide
<400> 970
   tgtttttaca caga 14
<210> 971
   <211> 12
   <212> DNA
   <213> Artificial Sequence
<220>
   <223> Synthetic Oligonucleotide
<400> 971
   t ggct cat g t c 12
<210> 972
   <211> 14
   <212> DNA
   <213> Artificial Sequence
<220>
   <223> Synthetic Oligonucleotide
<400> 972
   ttgttcttag gaag 14
<210> 973
   <211> 12
   <212> DNA
   <213> Artificial Sequence
<220>
   <223> Synthetic Oligonucleotide
<400> 973
   tacaccaggt ca 12
<210> 974
   <211> 14
   <212> DNA
   <213> Artificial Sequence
<220>
   <223> Synthetic Oligonucleotide
<400> 974
   t gagct acag t agg 14
<210> 975
   <211> 12
   <212> DNA
   <213> Artificial Sequence
<220>
   <223> Synthetic Oligonucleotide
<400> 975
   ggct gacat t ca 12
<210> 976
   <211> 14
   <212> DNA
   <213> Artificial Sequence
<220>
   <223> Synthetic Oligonucleotide
<400> 976
   t ggt caact g aaag 14
<210> 977
   <211> 12
   <212> DNA
   <213> Artificial Sequence
<220>
   <223> Synthetic Oligonucleotide
<400> 977
   t agt cat t at ct 12
<210> 978
   <211> 12
   <212> DNA
   <213> Artificial Sequence
<220>
   <223> Synthetic Oligonucleotide
<400> 978
   ccatttttat ca 12
<210> 979
   <211> 14
   <212> DNA
   <213> Artificial Sequence
<220>
   <223> Synthetic Oligonucleotide
<400> 979
   gggct t ct t c cat t 14
<210> 980
   <211> 14
   <212> DNA
   <213> Artificial Sequence
<220>
   <223> Synthetic Oligonucleotide
<400> 980
   ggt gt ggat a acag 14
<210> 981
   <211> 14
   <212> DNA
   <213> Artificial Sequence
<220>
   <223> Synthetic Oligonucleotide
<400> 981
   t cat aact at taag 14
<210> 982
   <211> 12
   <212> DNA
   <213> Artificial Sequence
<220>
   <223> Synthetic Oligonucleotide
<400> 982
   cctctacacc ag 12
<210> 983
   <211> 14
   <212> DNA
   <213> Artificial Sequence
<220>
   <223> Synthetic Oligonucleotide
<400> 983
   cagct t aggc agag 14
<210> 984
   <211> 12
   <212> DNA
   <213> Artificial Sequence
<220>
   <223> Synthetic Oligonucleotide
<400> 984
   t cat t cccca ct 12
<210> 985
   <211> 12
   <212> DNA
   <213> Artificial Sequence
<220>
   <223> Synthetic Oligonucleotide
<400> 985
   ctcccacacc at 12
<210> 986
   <211> 12
   <212> DNA
   <213> Artificial Sequence
<220>
   <223> Synthetic Oligonucleotide
<400> 986
   t t ct gct ccc ac 12
<210> 987
   <211> 12
   <212> DNA
   <213> Artificial Sequence
<220>
   <223> Synthetic Oligonucleotide
<400> 987
   cagagaaggt ct 12
<210> 988
   <211> 12
   <212> DNA
   <213> Artificial Sequence
<220>
   <223> Synthetic Oligonucleotide
<400> 988
   gt at gcact g ct 12
<210> 989
   <211> 14
   <212> DNA
   <213> Artificial Sequence
<220>
   <223> Synthetic Oligonucleotide
<400> 989
   caatgaagca cagg 14
<210> 990
   <211> 12
   <212> DNA
   <213> Artificial Sequence
<220>
   <223> Synthetic Oligonucleotide
<400> 990
   tcccaaacaa at 12
<210> 991
   <211> 14
   <212> DNA
   <213> Artificial Sequence
<220>
   <223> Synthetic Oligonucleotide
<400> 991
   at t ct t aaca caga 14
<210> 992
   <211> 12
   <212> DNA
   <213> Artificial Sequence
<220>
   <223> Synthetic Oligonucleotide
<400> 992
   cgggt act at gg 12
<210> 993
   <211> 12
   <212> DNA
   <213> Artificial Sequence
<220>
   <223> Synthetic Oligonucleotide
<400> 993
   ctacaccagg t c 12
<210> 994
   <211> 12
   <212> DNA
   <213> Artificial Sequence
<220>
   <223> Synthetic Oligonucleotide
<400> 994
   t at agct cct ct 12
<210> 995
   <211> 14
   <212> DNA
   <213> Artificial Sequence
<220>
   <223> Synthetic Oligonucleotide
<400> 995
   cat t t agggt ct aa 14
<210> 996
   <211> 12
   <212> DNA
   <213> Artificial Sequence
<220>
   <223> Synthetic Oligonucleotide
<400> 996
   at ct t cagag at 12
<210> 997
   <211> 12
   <212> DNA
   <213> Artificial Sequence
<220>
   <223> Synthetic Oligonucleotide
<400> 997
   ttgatatagt ca 12
<210> 998
   <211> 12
   <212> DNA
   <213> Artificial Sequence
<220>
   <223> Synthetic Oligonucleotide
<400> 998
   t aat at gact t g 12
<210> 999
   <211> 14
   <212> DNA
   <213> Artificial Sequence
<220>
   <223> Synthetic Oligonucleotide
<400> 999
   agccgcctga agtg 14
<210> 1000
   <211> 12
   <212> DNA
   <213> Artificial Sequence
<220>
   <223> Synthetic Oligonucleotide
<400> 1000
   ccccagcagc gg 12
<210> 1001
   <211> 12
   <212> DNA
   <213> Artificial Sequence
<220>
   <223> Synthetic Oligonucleotide
<400> 1001
   t cct t ccact ga 12
<210> 1002
   <211> 12
   <212> DNA
   <213> Artificial Sequence
<220>
   <223> Synthetic Oligonucleotide
<400> 1002
   cggtttttgt tc 12
<210> 1003
   <211> 14
   <212> DNA
   <213> Artificial Sequence
<220>
   <223> Synthetic Oligonucleotide
<400> 1003
   t aaat cct ct agca 14
<210> 1004
   <211> 12
   <212> DNA
   <213> Artificial Sequence
<220>
   <223> Synthetic Oligonucleotide
<400> 1004
   gt t ccct ct a ca 12
<210> 1005
   <211> 12
   <212> DNA
   <213> Artificial Sequence
<220>
   <223> Synthetic Oligonucleotide
<400> 1005
   acaccat ct c cc 12
<210> 1006
   <211> 12
   <212> DNA
   <213> Artificial Sequence
<220>
   <223> Synthetic Oligonucleotide
<400> 1006
   gct cct t cca ct 12
<210> 1007
   <211> 14
   <212> DNA
   <213> Artificial Sequence
<220>
   <223> Synthetic Oligonucleotide
<400> 1007
   cactgatcct gcac 14
<210> 1008
   <211> 12
   <212> DNA
   <213> Artificial Sequence
<220>
   <223> Synthetic Oligonucleotide
<400> 1008
   t cggact t t g aa 12
<210> 1009
   <211> 14
   <212> DNA
   <213> Artificial Sequence
<220> .
   <223> Synthetic Oligonucleotide
<400> 1009
   t cggact t t g aaaa 14
<210> 1010
   <211> 14
   <212> DNA
   <213> Artificial Sequence
<220>
   <223> Synthetic Oligonucleotide
<400> 1010
   at cagccaga caga 14
<210> 1011
   <211> 14
   <212> DNA
   <213> Artificial Sequence
<220>
   <223> Synthetic Oligonucleotide
<400> 1011
   cat cagcaag aggc 14
<210> 1012
   <211> 12
   <212> DNA
   <213> Artificial Sequence
<220>
   <223> Synthetic Oligonucleotide
<400> 1012
   ttgttcttag ga 12
<210> 1013
   <211> 12
   <212> DNA
   <213> Artificial Sequence
<220>
   <223> Synthetic Oligonucleotide
<400> 1013
   gccagacaga ag 12
<210> 1014
   <211> 12
   <212> DNA
   <213> Artificial al Sequence
<220>
   <223> Synthetic Oligonucleotide
<400> 1014
   t t gt cgat ct gc 12
<210> 1015
   <211> 14
   <212> DNA
   <213> Artificial al Sequence
<220>
   <223> Synthetic Oligonucleotide
<400> 1015
   ggagaagcgc agct 14
<210> 1016
   <211> 12
   <212> DNA
   <213> Artificial Sequence
<220>
   <223> Synthetic Oligonucleotide
<400> 1016
   ggagaagcgc ag 12
<210> 1017
   <211> 14
   <212> DNA
   <213> Artificial Sequence
<220>
   <223> Synthetic Oligonucleotide
<400> 1017
   ct gcact gac gagt 14
<210> 1018
   <211> 12
   <212> DNA
   <213> Artificial Sequence
<220>
   <223> Synthetic Oligonucleotide
<400> 1018
   ct gcaacat g at 12
<210> 1019
   <211> 14
   <212> DNA
   <213> Artificial Sequence
<220>
   <223> Synthetic Oligonucleotide
<400> 1019
   ct gat cct t a gaag 14
<210> 1020
   <211> 16
   <212> DNA
   <213> Artificial Sequence
<220>
   <223> Synthetic Oligonucleotide
<400> 1020
   cct t ccact g at cct g 16
<210> 1021
   <211> 12
   <212> DNA
   <213> Artificial Sequence
<220>
   <223> Synthetic Oligonucleotide
<400> 1021
   ct cct t ccac t g 12
<210> 1022
   <211> 14
   <212> DNA
   <213> Artificial Sequence
<220>
   <223> Synthetic Oligonucleotide
<400> 1022
   t gcagccat g t act 14
<210> 1023
   <211> 12
   <212> DNA
   <213> Artificial Sequence
<220>
   <223> Synthetic Oligonucleotide
<400> 1023
   cgt t t gggt g gc 12
<210> 1024
   <211> 14
   <212> DNA
   <213> Artificial Sequence
<220>
   <223> Synthetic Oligonucleotide
<400> 1024
   t ccact gat c ct gc 14
<210> 1025
   <211> 14
   <212> DNA
   <213> Artificial Sequence
<220>
   <223> Synthetic Oligonucleotide
<400> 1025
   t t ccact gat cct g 14
<210> 1026
   <211> 12
   <212> DNA
   <213> Artificial Sequence
<220>
   <223> Synthetic Oligonucleotide
<400> 1026
   t ccact gat c ct 12
<210> 1027
   <211> 14
   <212> DNA
   <213> Artificial Sequence
<220>
   <223> Synthetic Oligonucleotide
<400> 1027
   ct t ccact ga t cct 14
<210> 1028
   <211> 16
   <212> DNA
   <213> Artificial Sequence
<220>
   <223> Synthetic Oligonucleotide
<400> 1028
   t cct t ccact gat cct 16
<210> 1029
   <211> 12
   <212> DNA
   <213> Artificial Sequence
<220>
   <223> Synthetic Oligonucleotide
<400> 1029
   t t ccact gat cc 12
<210> 1030
   <211> 16
   <212> DNA
   <213> Artificial Sequence
<220>
   <223> Synthetic Oligonucleotide
<400> 1030
   ct cct t ccac t gat cc 16
<210> 1031
   <211> 14
   <212> DNA
   <213> Artificial Sequence
<220>
   <223> Synthetic Oligonucleotide
<400> 1031
   t cct t ccact gat c 14
<210> 1032
   <211> 16
   <212> DNA
   <213> Artificial Sequence
<220>
   <223> Synthetic Oligonucleotide
<400> 1032
   gct cct t cca ct gat c 16
<210> 1033
   <211> 14
   <212> DNA
   <213> Artificial Sequence
<220>
   <223> Synthetic Oligonucleotide
<400> 1033 14
   gctccttcca ctga 14
<210> 1034
   <211> 16
   <212> DNA
   <213> Artificial Sequence
<220>
   <223> Synthetic Oligonucleotide
<400> 1034 16
   aagct cct t c cact ga 16
<210> 1035
   <211> 14
   <212> DNA
   <213> Artificial Sequence
<220>
   <223> Synthetic Oligonucleotide
<400> 1035 14
   aagct cct t c cact 14
<210> 1036
   <211> 12
   <212> DNA
   <213> Artificial Sequence
<220>
   <223> Synthetic Oligonucleotide
<400> 1036 12
   agctccttcc ac 12
<210> 1037
   <211> 16
   <212> DNA
   <213> Artificial Sequence
<220>
   <223> Synthetic Oligonucleotide

<400> 1037 16
   gggaaagct c ct t cca 16
<210> 1038
   <211> 14
   <212> DNA
   <213> Artificial al Sequence
<220>
   <223> Synthetic Oligonucleotide
<400> 1038 14
   t t gcaat gt c t ggc 14
<210> 1039
   <211> 14
   <212> DNA
   <213> Artificial Sequence
<220>
   <223> Synthetic Oligonucleotide
<400> 1039
   gat t t at ct g gct g 14
<210> 1040
   <211> 12
   <212> DNA
   <213> Artificial Sequence
<220>
   <223> Synthetic Oligonucleotide
<400> 1040
   aagggccct g gg 12
<210> 1041
   <211> 12
   <212> DNA
   <213> Artificial Sequence
<220>
   <223> Synthetic Oligonucleotide
<400> 1041
   aacttcagtg tc 12
<210> 1042
   <211> 12
   <212> DNA
   <213> Artificial Sequence

<220>
   <223> Synthetic Oligonucleotide
<400> 1042
   agggct t cca gt 12
<210> 1043
   <211> 12
   <212> DNA
   <213> Artificial Sequence
<220>
   <223> Synthetic Oligonucleotide
<400> 1043
   aggaagggct tc c 12
<210> 1044
   <211> 12
   <212> DNA
   <213> Artificial Sequence
<220>
   <223> Synthetic Oligonucleotide
<400> 1044
   cct t ccact g at 12
<210> 1045
   <211> 14
   <212> DNA
   <213> Artificial Sequence
<220>
   <223> Synthetic Oligonucleotide
<400> 1045
   ggaaacatac cctg 14
<210> 1046
   <211> 14
   <212> DNA
   <213> Artificial Sequence
<220>
   <223> Synthetic Oligonucleotide
<400> 1046
   cct t ccact g at cc 14
<210> 1047
   <211> 12
   <212> DNA
   <213> Artificial Sequence
<220>
   <223> Synthetic Oligonucleotide
<400> 1047
   ct t ccact ga t c 12
<210> 1048
   <211> 14
   <212> DNA
   <213> Artificial Sequence
<220>
   <223> Synthetic Oligonucleotide
<400> 1048
   gaat aggt t a aggc 14
<210> 1049
   <211> 14
   <212> DNA
   <213> Artificial Sequence
<220>
   <223> Synthetic Oligonucleotide
<400> 1049
   aacaat gt gt t gt a 14
<210> 1050
   <211> 12
   <212> DNA
   <213> Artificial Sequence
<220>
   <223> Synthetic Oligonucleotide
<400> 1050
   ccct ct acac ca 12
<210> 1051
   <211> 14
   <212> DNA
   <213> Artificial Sequence
<220>
   <223> Synthetic Oligonucleotide
<400> 1051
   gcacacagct gagg 14
<210> 1052
   <211> 14
   <212> DNA
   <213> Artificial Sequence
<220>
   <223> Synthetic Oligonucleotide
<400> 1052
   gt gcacacag ct ga 14
<210> 1053
   <211> 14
   <212> DNA
   <213> Artificial Sequence
<220>
   <223> Synthetic Oligonucleotide
<400> 1053
   cagtgtgcac acag 14
<210> 1054
   <211> 14
   <212> DNA
   <213> Artificial Sequence
<220>
   <223> Synthetic Oligonucleotide
<400> 1054
   ct cagt gt gc acac 14
<210> 1055
   <211> 12
   <212> DNA
   <213> Artificial Sequence
<220>
   <223> Synthetic Oligonucleotide
<400> 1055
   cacccactgg tg 12
<210> 1056
   <211> 14
   <212> DNA
   <213> Artificial Sequence
<220>
   <223> Synthetic Oligonucleotide
<400> 1056
   cat t t ccat g gcca 14
<210> 1057
   <211> 14
   <212> DNA
   <213> Artificial Sequence
<220>
   <223> Synthetic Oligonucleotide
<400> 1057
   accaaacagt t cag 14
<210> 1058
   <211> 12
   <212> DNA
   <213> Artificial Sequence

<220>
   <223> Synthetic Oligonucleotide
<400> 1058
   ttgaccagga ag 12
<210> 1059
   <211> 12
   <212> DNA
   <213> Artificial Sequence
<220>
   <223> Synthetic Oligonucleotide
<400> 1059
   caggccatgt gg 12
<210> 1060
   <211> 14
   <212> DNA
   <213> Artificial Sequence
<220>
   <223> Synthetic Oligonucleotide
<400> 1060
   agt caggcca t gt g 14
<210> 1061
   <211> 12
   <212> DNA
   <213> Artificial Sequence
<220>
   <223> Synthetic Oligonucleotide
<400> 1061
   gcgcgagccc ga 12
<210> 1062
   <211> 12
   <212> DNA
   <213> Artificial Sequence
<220>
   <223> Synthetic Oligonucleotide
<400> 1062
   t gt gaggct c ca 12
<210> 1063
   <211> 12
   <212> DNA
   <213> Artificial Sequence
<220>
   <223> Synthetic Oligonucleotide
<400> 1063
   ccctgaaggt t c 12
<210> 1064
   <211> 14
   <212> DNA
   <213> Artificial Sequence
<220>
   <223> Synthetic Oligonucleotide
<400> 1064
   t t t gat aaag ccct 14
<210> 1065
   <211> 14
   <212> DNA
   <213> Artificial Sequence
<220>
   <223> Synthetic Oligonucleotide
<400> 1065
   caagaagacc t t ac 14
<210> 1066
   <211> 14 <212> DNA
   <213> Artificial Sequence
<220>
   <223> Synthetic Oligonucleotide
<400> 1066
   tgcacaggca ggtt 14
<210> 1067
   <211> 12
   <212> DNA
   <213> Artificial Sequence
<220>
   <223> Synthetic Oligonucleotide
<400> 1067
   acagccaggt ag 12
<210> 1068
   <211> 14
   <212> DNA
   <213> Artificial Sequence
<220>
   <223> Synthetic Oligonucleotide
<400> 1068
   t ggaaaact g cacc 14
<210> 1069
   <211> 12
   <212> DNA
   <213> Artificial Sequence
<220>
   <223> Synthetic Oligonucleotide
<400> 1069
   t gggt ggccg gg 12
<210> 1070
   <211> 12
   <212> DNA
   <213> Synthetic Oligonucleotide
<220>
   <223> Synthetic Oligonucleotide
<400> 1070
   gggctt ct t c ca 12
<210> 1071
   <211> 12
   <212> DNA
   <213> Artificial Sequence
<220>
   <223> Synthetic Oligonucleotide
<400> 1071
   gct gacat ct cg 12
<210> 1072
   <211> 14
   <212> DNA
   <213> Artificial Sequence
<220>
   <223> Synthetic Oligonucleotide
<400> 1072
   cagcctggca ccta 14
<210> 1073
   <211> 12
   <212> DNA
   <213> Artificial Sequence
<220>
   <223> Synthetic Oligonucleotide
<400> 1073
   taaaaacaac aa 12
<210> 1074
   <211> 12
   <212> DNA
   <213> Artificial Sequence
<220>
   <223> Synthetic Oligonucleotide
<400> 1074
   t t t at aaaac t g 12
<210> 1075
   <21 1> 4
   <212> DNA
   <213> Artificial Sequence
<220>
   <223> Synthetic Oligonucleotide
<400> 1075
   act g 4
<210> 1076
   <211> 14
   <212> DNA
   <213> Artificial Sequence
<220>
   <223> Synthetic Oligonucleotide
<400> 1076
   agt aaat at t ggct 14
<210> 1077
   <211> 12
   <212> DNA
   <213> Artificial Sequence
<220>
   <223> Synthetic Oligonucleotide
<400> 1077
   gt t gagcat g ac 12
<210> 1078
   <211> 12
   <212> DNA
   <213> Artificial Sequence
<220>
   <223> Synthetic Oligonucleotide
<400> 1078
   gt gcgct ccc at 12
<210> 1079
   <211> 14
   <212> DNA
   <213> Artificial Sequence
<220>
   <223> Synthetic Oligonucleotide
<400> 1079
   t cct gcact g acga 14
<210> 1080
   <211> 16
   <212> DNA
   <213> Artificial Sequence
<220>
   <223> Synthetic Oligonucleotide
<400> 1080
   gatcctgcac t gacga 16
<210> 1081
   <211> 14
   <212> DNA
   <213> Artificial Sequence
<220>
   <223> Synthetic Oligonucleotide
<400> 1081
   gat cct gcac t gac 14
<210> 1082
   <211> 16
   <212> DNA
   <213> Artificial Sequence
<220>
   <223> Synthetic Oligonucleotide
<400> 1082
   ct gat cct gc act gac 16
<210> 1083
   <211> 16
   <212> DNA
   <213> Artificial Sequence
<220>
   <223> Synthetic Oligonucleotide
<400> 1083
   t ccact gat c ct gcac 16
<210> 1084
   <211> 14
   <212> DNA
   <213> Artificial Sequence
<220>
   <223> Synthetic Oligonucleotide
<400> 1084
   cgcgagat at ct aa 14
<210> 1085
   <211> 14
   <212> DNA
   <213> Artificial Sequence
<220>
   <223> Synthetic Oligonucleotide
<400> 1085
   cgcacctggt aaat 14
<210> 1086
   <211> 14
   <212> DNA
   <213> Artificial Sequence
<220>
   <223> Synthetic Oligonucleotide
<400> 1086
   gt t caagcgg cct a 14
<210> 1087
   <211> 14
   <212> DNA
   <213> Artificial Sequence
<220>
   <223> Synthetic Oligonucleotide
<400> 1087
   ccct t t acac aagt 14
<210> 1088
   <211> 14
   <212> DNA
   <213> Artificial Sequence
<220>
   <223> Synthetic Oligonucleotide
<400> 1088
   ct caaaat ag at t t 14
<210> 1089
   <211> 14
   <212> DNA
   <213> Artificial Sequence
<220>
   <223> Synthetic Oligonucleotide
<400> 1089
   cacat gagct at t c 14
<210> 1090
   <211> 14
   <212> DNA
   <213> Artificial Sequence
<220>
   <223> Synthetic Oligonucleotide
<400> 1090
   gt gaagt gag t cat 14
<210> 1091
   <211> 14
   <212> DNA
   <213> Artificial Sequence
<220>
   <223> Synthetic Oligonucleotide
<400> 1091
   ggt cact caa gat g 14
<210> 1092
   <211> 14
   <212> DNA
   <213> Artificial Sequence
<220>
   <223> Synthetic Oligonucleotide
<400> 1092
   ggact cact c agca 14
<210> 1093
   <211> 14
   <212> DNA
   <213> Artificial Sequence
<220>
   <223> Synthetic Oligonucleotide
<400> 1093
   t cagggct ac t cat 14
<210> 1094
   <211> 14
   <212> DNA
   <213> Artificial Sequence
<220>
   <223> Synthetic Oligonucleotide
<400> 1094
   cagcact aga t t ca 14
<210> 1095
   <211> 14
   <212> DNA
   <213> Artificial Sequence
<220>
   <223> Synthetic Oligonucleotide
<400> 1095
   t agct t aat g t aac 14
<210> 1096
   <211> 14
   <212> DNA
   <213> Artificial Sequence
<220>
   <223> Synthetic Oligonucleotide
<400> 1096
   t t gt gacat c t agg 14
<210> 1097
   <211> 14
   <212> DNA
   <213> Artificial Sequence
<220>
   <223> Synthetic Oligonucleotide
<400> 1097
   gt gcct agca caga 14
<210> 1098
   <211> 12
   <212> DNA
   <213> Artificial Sequence
<220>
   <223> Synthetic Oligonucleotide
<400> 1098
   cagcct acca gt 12
<210> 1099
   <211> 14
   <212> DNA
   <213> Artificial Sequence
<220>
   <223> Synthetic Oligonucleotide
<400> 1099
   acat gt cagt aat t 14
<210> 1100
   <211> 14
   <212> DNA
   <213> Artificial Sequence
<220>
   <223> Synthetic Oligonucleotide
<400> 1100
   ctcatggaca caaa 14
<210> 1101
   <211> 14
   <212> DNA
   <213> Artificial Sequence
<220>
   <223> Synthetic Oligonucleotide
<400> 1101
   ggaagt t t t c aagt 14
<210> 1102
   <211> 14
   <212> DNA
   <213> Artificial Sequence
<220>
   <223> Synthetic Oligonucleotide
<400> 1102 14
   gaat ct ggag gt aa 14
<210> 1103
   <211> 14
   <212> DNA
   <213> Artificial Sequence
<220>
   <223> Synthetic Oligonucleotide

<400> 1103 14
   agcccct t gg ccgt 14
<210> 1104
   <211> 14
   <212> DNA
   <213> Artificial Sequence
<220>
   <223> Synthetic Oligonucleotide
<400> 1104 14
   gaat act t ca aat c 14
<210> 1105
   <211> 14
   <212> DNA
   <213> Artificial Sequence
<220>
   <223> Synthetic Oligonucleotide
<400> 1105
   ctgatcctgc actg 14
<210> 1106
   <211> 14
   <212> DNA
   <213> Artificial Sequence
<220>
   <223> Synthetic Oligonucleotide
<400> 1106
   tttgaggagc t at t 14
<210> 1107
   <211> 12
   <212> DNA
   <213> Artificial Sequence
<220>
   <223> Synthetic Oligonucleotide
<400> 1107 12
   ttgttgttcc ct 12
<210> 1108
   <211> 12
   <212> DNA
   <213> Artificial Sequence
<220>
   <223> Synthetic Oligonucleotide
<400> 1108
   gagt t gt t gt t c 12
<210> 1109
   <211> 12
   <212> DNA
   <213> Artificial Sequence
<220>
   <223> Synthetic Oligonucleotide
<400> 1109
   cgagttgttg t t 12
<210> 1110
   <211> 12
   <212> DNA
   <213> Artificial Sequence
<220>
   <223> Synthetic Oligonucleotide
<400> 1110
   gcgctaggcc gc 12
<210> 1111
   <211> 12
   <212> DNA
   <213> Artificial Sequence
<220>
   <223> Synthetic Oligonucleotide
<400> 1111
   gttgttgttc cc 12
<210> 1112
   <211> 12
   <212> DNA
   <213> Artificial Sequence
<220>
   <223> Synthetic Oligonucleotide
<400> 1112
   agt t gt t gt t cc 12
<210> 1113
   <211> 12
   <212> DNA
   <213> Artificial Sequence
<220>
   <223> Synthetic Oligonucleotide
<400> 1113
   ct cacct t ca t g 12
<210> 1114
   <211> 14
   <212> DNA
   <213> Artificial Sequence
<220>
   <223> Synthetic Oligonucleotide
<400> 1114
   ctt agaaggc agca 14
<210> 1115
   <211> 12
   <212> DNA
   <213> Artificial Sequence
<220>
   <223> Synthetic Oligonucleotide
<400> 1115
   gt t gt t ccct ct 12
<210> 1116
   <211> 12
   <212> DNA
   <213> Artificial Sequence
<220>
   <223> Synthetic Oligonucleotide
<400> 1116
   ccaactccaa ct 12
<210> 1117
   <211> 12
   <212> DNA
   <213> Artificial Sequence
<220>
   <223> Synthetic Oligonucleotide
<400> 1117
   gat ct ct cga gt 12
<210> 1118
   <211> 12
   <212> DNA
   <213> Artificial Sequence
<220>
   <223> Synthetic Oligonucleotide
<400> 1118
   aat gcaggat ct 12
<210> 1119
   <211> 12
   <212> DNA
   <213> Artificial Sequence
<220>
   <223> Synthetic Oligonucleotide
<400> 1119
   ct cggactt tga 12
<210> 1120
   <211> 12
   <212> DNA
   <213> Artificial Sequence
<220>
   <223> Synthetic Oligonucleotide
<400> 1120
   t gact ct cgg ac 12
<210> 1121
   <211> 12
   <212> DNA
   <213> Artificial Sequence
<220>
   <223> Synthetic Oligonucleotide
<400> 1121
   cttgtccatc ag 12
<210> 1122
   <211> 12
   <212> DNA
   <213> Artificial Sequence
<220>
   <223> Synthetic Oligonucleotide
<400> 1122
   gggt ct t t cc t c 12
<210> 1123
   <211> 16
   <212> DNA
   <213> Artificial Sequence
<220>
   <223> Synthetic Oligonucleotide
<400> 1123
   cact gat cct t agaag 16
<210> 1124
   <211> 14
   <212> DNA
   <213> Artificial Sequence
<220>
   <223> Synthetic Oligonucleotide
<400> 1124
   cact gat cct t aga 14
<210> 1125
   <211> 16
   <212> DNA
   <213> Artificial Sequence
<220>
   <223> Synthetic Oligonucleotide
<400> 1125
   t ccact gat c ct t aga 16
<210> 1126
   <211> 16
   <212> DNA
   <213> Artificial Sequence
<220>
   <223> Synthetic Oligonucleotide
<400> 1126
   ct t ccact ga t cct gc 16
<210> 1127
   <211> 14
   <212> DNA
   <213> Artificial Sequence
<220>
   <223> Synthetic Oligonucleotide
<400> 1127
   t ccact gat c ct ta 14
<210> 1128
   <211> 12
   <212> DNA
   <213> Artificial Sequence
<220>
   <223> Synthetic Oligonucleotide
<400> 1128
   cagt ggacca ca 12
<210> 1129
   <211> 12
   <212> DNA
   <213> Artificial Sequence
<220>
   <223> Synthetic Oligonucleotide
<400> 1129
   t gcgagt t gt t g 12
<210> 1130
   <211> 12
   <212> DNA
   <213> Artificial Sequence
<220>
   <223> Synthetic Oligonucleotide
<400> 1130
   aaagt caggc ca 12
<210> 1131
   <211> 14
   <212> DNA
   <213> Artificial Sequence
<220>
   <223> Synthetic Oligonucleotide
<400> 1131
   t aact t cagt gt ct 14
<210> 1132
   <211> 14
   <212> DNA
   <213> Artificial Sequence
<220>
   <223> Synthetic Oligonucleotide
<400> 1132
   gaagggcttc cagt 14
<210> 1133
   <211> 14
   <212> DNA
   <213> Artificial Sequence
<220>
   <223> Synthetic Oligonucleotide
<400> 1133
   t gaccaggaa gggc 14
<210> 1134
   <211> 14
   <212> DNA
   <213> Artificial Sequence
<220>
   <223> Synthetic Oligonucleotide
<400> 1134
   aggccct gag at t a 14
<210> 1135
   <211> 14
   <212> DNA
   <213> Artificial Sequence
<220>
   <223> Synthetic Oligonucleotide
<400> 1135
   ggt t aaggcc ct ga 14
<210> 1136
   <211> 14
   <212> DNA
   <213> Artificial Sequence
<220>
   <223> Synthetic Oligonucleotide
<400> 1136
   ggaat gt ct g agt t 14
<210> 1137
   <211> 14
   <212> DNA
   <213> Artificial Sequence
<220>
   <223> Synthetic Oligonucleotide
<400> 1137
   t aat gacct g at ga 14
<210> 1138
   <211> 12
   <212> DNA
   <213> Artificial Sequence
<220>
   <223> Synthetic Oligonucleotide
<400> 1138
   t gaagt t aat t c 12
<210> 1139
   <211> 12
   <212> DNA
   <213> Artificial Sequence
<220>
   <223> Synthetic Oligonucleotide
<400> 1139
   gaaattgagg aa 12
<210> 1140
   <211> 12
   <212> DNA
   <213>Artificial Sequence
<220>
   <223> Synthetic Oligonucleotide
<400> 1140
   gt at t caagt aa 12
<210> 1141
   <211> 12
   <212> DNA
   <213>Artifical Sequence
<220>
   <223> Synthetic Oligonucleotide
<400> 1141
   acaat t at gg ca 12
<210> 1142
   <211> 12
   <212> DNA
   <213> Artificial Sequence
<220>
   <223> Synthetic Oligonucleotide
<400> 1142
   cagt t t at t c aa 12
<210> 1143
   <211> 12
   <212> DNA
   <213> Artificial Sequence
<220>
   <223> Synthetic Oligonucleotide
<400> 1143
   gaagct gct g gt 12
<210> 1144
   <211> 12
   <212> DNA
   <213> Artificial Sequence
<220>
   <223> Synthetic Oligonucleotide
<400> 1144
   gt gaaacat t t t 12
<210> 1145
   <211> 14
   <212> DNA
   <213> Artificial Sequence
<220>
   <223> Synthetic Oligonucleotide
<400> 1145
   aaaagt gaaa cat t 14
<210> 1146
   <211> 12
   <212> DNA
   <213> Artificial Sequence
<220>
   <223> Synthetic Oligonucleotide
<400> 1146
   ttcttaaagg tg 12
<210> 1147
   <211> 12
   <212> DNA
   <213> Artificial Sequence
<220>
   <223> Synthetic Oligonucleotide
<400> 1147
   gcacaaat t t tc c 12
<210> 1148
   <211> 12
   <212> DNA
   <213> Artificial Sequence
<220>
   <223> Synthetic Oligonucleotide
<400> 1148
   gt t t agt gca ca 12
<210> 1149
   <211> 12
   <212> DNA
   <213> Artificial Sequence
<220>
   <223> Synthetic Oligonucleotide
<400> 1149
   t ct ggat cag ag 12
<210> 1150
   <211> 12
   <212> DNA
   <213> Artificial Sequence
<220>
   <223> Synthetic Oligonucleotide
<400> 1150
   t gct gcacat cc 12
<21 0> 1151
   <211> 12
   <212> DNA
   <213> Artificial Sequence
<220>
   <223> Synthetic Oligonucleotide
<400> 1151
   t ct gact ggg aa 12
<210> 1152
   <211> 12
   <212> DNA
   <213> Artificial Sequence
<220>
   <223> Synthetic Oligonucleotide
<400> 1152
   acaaagagt c cc 12
<210> 1153
   <211> 12
   <212> DNA
   <213> Artificial Sequence
<220>
   <223> Synthetic Oligonucleotide
<400> 1153
   agagagct ga gc 12
<210> 1154
   <211> 12
   <212> DNA
   <213> Artificial Sequence
<220>
   <223> Synthetic Oligonucleotide
<400> 1154
   t ggcct caca gc 12
<210> 1155
   <211> 12
   <212> DNA
   <213> Artificial Sequence
<220>
   <223> Synthetic Oligonucleotide
<400> 1155
   ggaccgcagc cg 12
<210> 1156
   <211> 12
   <212> DNA
   <213> Artificial Sequence
<220>
   <223> Synthetic Oligonucleotide
<400> 1156
   gt t agaacag ac 12
<210> 1157
   <211> 12
   <212> DNA
   <213> Artificial Sequence
<220>
   <223> Synthetic Oligonucleotide
<400> 1157
   cct gaaact g ca 12
<210> 1158
   <211> 12
   <212> DNA
   <213> Artificial Sequence
<220>
   <223> Synthetic Oligonucleotide
<400> 1158
   t gct cacagg cg 12
<210> 1159
   <211> 12 <212> DNA
   <213> Artificial Sequence
<220>
   <223> Synthetic Oligonucleotide
<400> 1159
   agagagcaac t c 12
<210> 1160
   <211> 12
   <212> DNA
   <213> Artificial Sequence
<220>
   <223> Synthetic Oligonucleotide
<400> 1160
   at ggct gct g cg 12
<210> 1161
   <211> 12
   <212> DNA
   <213> ArtificialSequence
<220>
   <223> Synthetic Oligonucleotide
<400> 1161
   cat ggct gca gc 12
<210> 1162
   <211> 14
   <212> DNA
   <213> Artificial Sequence
<220>
   <223> Synthetic Oligonucleotide
<400> 1162
   ct t ggccct g gacc 14
<210> 1163
   <211> 13
   <212> DNA
   <213> Artificial Sequence
<220>
   <223> Synthetic Oligonucleotide
<400> 1163
   gct agcct ct gga 13
<210> 1164
   <211> 12
   <212> DNA
   <213> Artificial Sequence
<220>
   <223> Synthetic Oligonucleotide
<400> 1164
   t ggccct gga cc 12
<210> 1165
   <211> 12
   <212> DNA
   <213> Artificial Sequence
<220>
   <223> Synthetic Oligonucleotide
<400> 1165
   t t t ct gcagg aa 12
<210> 1166
   <211> 14
   <212> DNA
   <213> Artificial Sequence
<220>
   <223> Synthetic Oligonucleotide
<400> 1166
   t acaggt caa gt ct 14
<210> 1167
   <211> 12
   <212> DNA
   <213> Artificial Sequence
<220>
   <223> Synthetic Oligonucleotide
<400> 1167
   acaggt caag tc 12
<210> 1168
   <211> 14
   <212> DNA
   <213> ArtificialSequence
<220>
   <223> Synthetic Oligonucleotide
<400> 1168
   t t ggat aaat at ct 14
<210> 1169
   <211> 12
   <212> DNA
   <213> Artificial Sequence
<220>
   <223> Synthetic Oligonucleotide
<400> 1169
   t ggat aaat a t c 12
<210> 1170
   <211> 12
   <212> DNA
   <213> Artificial Sequence
<220>
   <223> Synthetic Oligonucleotide
<400> 1170
   t t ct gcagga t g 12
<210> 1171
   <211> 12
   <212> DNA
   <213> Artificial Sequence
<220>
   <223> Synthetic Oligonucleotide
<400> 1171
   cacct t caag t c 12
<210> 1172
   <211> 12
   <212> DNA
   <213> Artificial Sequence
<220>
   <223> Synthetic Oligonucleotide
<400> 1172
   cat agat tgt at 12
<210> 1173
   <211> 12
   <212> DNA
   <213> Artificial Sequence
<220>
   <223> Synthetic Oligonucleotide
<400> 1173
   gt cagaat at ct 12
<210> 1174
   <211> 12
   <212> DNA
   <213> Artificial Sequence
<220>
   <223> Synthetic Oligonucleotide
<400> 1174
   t ct gcagt t a aa 12
<210> 1175
   <211> 12
   <212> DNA
   <213> Artificial Sequence
<220>
   <223> Synthetic Oligonucleotide
<400> 1175
   at ct t gt gaa ac 12
<210> 1176
   <211> 12
   <212> DNA
   <213> Artificial Sequence
<220>
   <223> Synthetic Oligonucleotide
<400> 1176
   t cat caaaag gt 12
<210> 1177
   <211> 12
   <212> DNA
   <213> Artificial Sequence
<220>
   <223> Synthetic Oligonucleotide
<400> 1177
   ct t t gt caag at 12
<210> 1178
   <211> 12
   <212> DNA
   <213> Artificial Sequence
<220>
   <223> Synthetic Oligonucleotide
<400> 1178
   t at gcacaaa t c 12
<210> 1179
   <211> 12
   <212> DNA
   <213> Artificial Sequence
<220>
   <223> Synthetic Oligonucleotide
<400> 1179
   t aat ccaggt ga 12
<210> 1180
   <211> 12
   <212> DNA
   <213> Artificial Sequence
<220>
   <223> Synthetic Oligonucleotide
<400> 1180
   cacacaggca at 12
<210> 1181
   <211> 12
   <212> DNA
   <213> Artificial Sequence
<220>
   <223> Synthetic Oligonucleotide
<400> 1181
   t t gagcat ct tg 12
<210> 1182
   <211> 14
   <212> DNA
   <213> Artificial Sequence
<220>
   <223> Synthetic Oligonucleotide
<400> 1182
   cat t t t gt cc t t t t 14
<210> 1183
   <211> 12
   <212> DNA
   <213> ArtificialSequence
<220>
   <223> Synthetic Oligonucleotide

<400> 1183
   gat t t cct ga t c 12
<210> 1184
   <211> 15
   <212> DNA
   <213> Artificial Sequence
<220>
   <223> Synthetic Oligonucleotide
<400> 1184
   ct ggat t t ga t ggct 15
<210> 1185
   <211> 15
   <212> DNA
   <213> Artificial Sequence
<220>
   <223> Synthetic Oligonucleotide
<400> 1185
   t ct ggat t t g at ggc 15
<210> 1186
   <211> 12
   <212> DNA
   <213> Artificial Sequence
<220>
   <223> Synthetic Oligonucleotide
<400> 1186
   t ggact t ggc gg 12
<210> 1187
   <211> 15
   <212> DNA
   <213> Artificial Sequence
<220>
   <223> Synthetic Oligonucleotide
<400> 1187
   ct ct ggat t t gat gg 15
<210> 1188
   <211> 15
   <212> DNA
   <213> Artificial Sequence
<220>
   <223> Synthetic Oligonucleotide
<400> 1188
   cct ct ggat t t gat g 15
<210> 1189
   <211> 15
   <212> DNA
   <213> Artificial Sequence
<220>
   <223> Synthetic Oligonucleotide
<400> 1189
   gcct ct ggat t t gat 15
<210> 1190
   <211> 16
   <212> DNA
   <213> Artificial Sequence
<220>
   <223> Synthetic Oligonucleotide
<400> 1190
   t t t agcct ct ggat t t 16
<210> 1191
   <211> 16
   <212> DNA
   <213> Artificial Sequence
<220>
   <223> Synthetic Oligonucleotide
<400> 1191
   t ct agcct ct ggat t t 16
<210> 1192
   <211> 12
   <212> DNA
   <213> Artificial Sequence
<220>
   <223> Synthetic Oligonucleotide
<400> 1192
   agt agt t gt a ct 12
<210> 1193
   <211> 12
   <212> DNA
   <213> Artificial Sequence
<220>
   <223> Synthetic Oligonucleotide
<400> 1193
   t ct ggagt ca ca 12
<210> 1194
   <211> 12
   <212> DNA
   <213> Artificial Sequence
<220>
   <223> Synthetic Oligonucleotide
<400> 1194
   gt cat aat gt ct 12
<210> 1195
   <211> 14
   <212> DNA
   <213> Artificial Sequence
<220>
   <223> Synthetic Oligonucleotide
<400> 1195
   gt gt cat aat gt ct 14
<210> 1196
   <211> 12
   <212> DNA
   <213> Artificial Sequence
<220>
   <223> Synthetic Oligonucleotide
<400> 1196
   ct t caaaagg at 12
<210> 1197
   <211> 12
   <212> DNA
   <213> Artificial Sequence
<220>
   <223> Synthetic Oligonucleotide
<400> 1197
   gt ct t caaaa gg 12
<210> 1198
   <211> 12
   <212> DNA
   <213>Artificial Sequence
<220>
   <223> Synthetic Oligonucleotide
<400> 1198
   atcctcttga t a 12
<210> 1199
   <211> 12
   <212> DNA
   <213> Artificial Sequence
<220>
   <223> Synthetic Oligonucleotide
<400> 1199
   gt gcct t t aa aa 12
<210> 1200
   <211> 12
   <212> DNA
   <213> Artificial Sequence
<220>
   <223> Synthetic Oligonucleotide
<400> 1200
   t gt cat aat g t c 12
<210> 1201
   <211> 12
   <212> DNA
   <213> Artificial Sequence
<220>
   <223> Synthetic Oligonucleotide
<400> 1201
   caat ct gaca ca 12
<210> 1202
   <211> 12
   <212> DNA
   <213> Artificial Sequence
<220>
   <223> Synthetic Oligonucleotide
<400> 1202
   aat at t gt t c ct 12
<210> 1203
   <211> 12
   <212> DNA
   <213> Artificial Sequence
<220>
   <223> Synthetic Oligonucleotide
<400> 1203
   gt caggagaa ga 12
<210> 1204
   <211> 12
   <212> DNA
   <213>Artificial Sequence
<220>
   <223> Synthetic Oligonucleotide
<400> 1204
   t gt caaaacc ac 12
<210> 1205
   <211> 12
   <212> DNA
   <213>Artificial Sequence
<220>
   <223> Synthetic Oligonucleotide
<400> 1205
   gt cct at t gc ca 12
<210> 1206
   <211> 12
   <212> DNA
   <213>Artificial Sequence
<220>
   <223> Synthetic Oligonucleotide
<400> 1206
   aggt at at ac at 12
<210> 1207
   <211> 12
   <212> DNA
   <213> Artificial Sequence
<220>
   <223> Synthetic Oligonucleotide
<400> 1207
   cagct ggt ga ca 12
<210> 1208
   <211> 12
   <212> DNA
   <213>Artificial Sequence
<220>
   <223> Synthetic Oligonucleotide
<400> 1208
   t t cact t agc ca 12
<210> 1209
   <211> 12
   <212> DNA
   <213> Artificial Sequence
<220>
   <223> Synthetic Oligonucleotide
<400> 1209
   aaccct gcag aa 12
<210> 1210
   <211> 12
   <212> DNA
   <213> Artificial Sequence
<220>
   <223> Synthetic Oligonucleotide
<400> 1210
   t gt caaaacc ct 12
<210> 1211
   <211> 12
   <212> DNA
   <213> Artificial Sequence
<220>
   <223> Synthetic Oligonucleotide
<400> 1211
   gt gt caaaac cc 12
<210> 1212
   <211> 14
   <212> DNA
   <213> Artificial Sequence
<220>
   <223> Synthetic Oligonucleotide
<400> 1212
   at ggt ccaga gccc 14
<210> 1213
   <211> 12
   <212> DNA
   <213>Artificial Sequence
<220>
   <223> Synthetic Oligonucleotide
<400> 1213
   tggtccagag cc 12
<210> 1214
   <211> 12
   <212> DNA
   <213> Artificial Sequence
<220>
   <223> Synthetic Oligonucleotide
<400> 1214
   t gggt t at gg t c 12
<210> 1215
   <211> 12
   <212> DNA
   <213> Artificial Sequence
<220>
   <223> Synthetic Oligonucleotide
<400> 1215
   caaagaat gg tg 12
<210> 1216
   <211> 12
   <212> DNA
   <213> Artificial Sequence
<220>
   <223> Synthetic Oligonucleotide
<400> 1216
   gt t t agcat g ct 12
<210> 1217
   <211> 12
   <212> DNA
   <213> Artificial Sequence
<220>
   <223> Synthetic Oligonucleotide
<400> 1217
   t gcct t t aaa aa 12
<210> 1218
   <211> 12
   <212> DNA
   <213>Artificial Sequence
<220>
   <223> Synthetic Oligonucleotide
<400> 1218
   accaat at gc t c 12
<210> 1219
   <211> 12
   <212> DNA
   <213>Artificial Sequence
<220>
   <223> Synthetic Oligonucleotide
<400> 1219
   caccaat aag t t 12
<210> 1220
   <211> 12
   <212> DNA
   <213> Artificial Sequence
<220>
   <223> Synthetic Oligonucleotide
<400> 1220
   cct t t agct g gc 12
<210> 1221
   <211> 12
   <212> DNA
   <213> Artificial Sequence
<220>
   <223> Synthetic Oligonucleotide
<400> 1221
   gt t gaaagcc t c 12
<210> 1222
   <211> 12
   <212> DNA
   <213> Artificial Sequence
<220>
   <223> Synthetic Oligonucleotide
<400> 1222.
   t t t gat gat g gc 12
<210> 1223
   <211> 12
   <212> DNA
   <213>Artificial Sequence
<220>
   <223> Synthetic Oligonucleotide
<400> 1223
   t cat t gt caa at 12
<210> 1224
   <211> 12
   <212> DNA
   <213> Artificial Sequence
<220>
   <223> Synthetic Oligonucleotide
<400> 1224
   t t acaact ga ga 12
<210> 1225
   <211> 12
   <212> DNA
   <213> Artificial Sequence
<220>
   <223> Synthetic Oligonucleotide
<400> 1225
   agat ggagca gt 12
<210> 1226
   <211> 14
   <212> DNA
   <213> Artificial Sequence
<220>
   <223> Synthetic Oligonucleotide
<400> 1226
   ct t agcact g gcct 14
<210> 1227
   <211> 12
   <212> DNA
   <213> Artificial Sequence
<220>
   <223> Synthetic Oligonucleotide
<400> 1227
   agagt at ct g aa 12
<210> 1228
   <211> 12
   <212> DNA
   <213> Artificial Sequence
<220>
   <223> Synthetic Oligonucleotide
<400> 1228
   act gat gt ag gg 12
<210> 1229
   <211> 12
   <212> DNA
   <213> Artificial Sequence
<220>
   <223> Synthetic Oligonucleotide
<400> 1229
   ccacagt agg t a 12
<210> 1230
   <211> 14
   <212> DNA
   <213> Artificial Sequence
<220>
   <223> Synthetic Oligonucleotide
<400> 1230
   ct ggcagcca gcac 14
<210> 1231
   <211> 12
   <212> DNA
   <213> Artificial Sequence
<220>
   <223> Synthetic Oligonucleotide
<400> 1231
   t ggcagccag ca 12
<210> 1232
   <211> 12
   <212> DNA
   <213> Artificial Sequence
<220>
   <223> Synthetic Oligonucleotide
<400> 1232
   gcat cat caa t c 12
<210> 1233
   <211> 12
   <212> DNA
   <213> Artificial Sequence
<220>
   <223> Synthetic Oligonucleotide
<400> 1233
   aaat cat t gt ca 12
<210> 1234
   <211> 12
   <212> DNA
   <213> Artificial Sequence
<220>
   <223> Synthetic Oligonucleotide
<400> 1234
   gcaaaact ca t t 12
<210> 1235
   <211> 14
   <212> DNA
   <213> Artificial Sequence
<220>
   <223> Synthetic Oligonucleotide
<400> 1235
   t gt gcaact c t gca 14
<210> 1236
   <211> 12
   <212> DNA
   <213> Artificial Sequence
<220>
   <223> Synthetic Oligonucleotide
<400> 1236
   gt gcaact ct gc 12
<210> 1237
<211> 12
   <212> DNA
   <213> Artificial Sequence
<220>
   <223> Synthetic Oligonucleotide
<400> 1237
   aat at t cat g t a 12
<210> 1238
   <211> 12
   <212> DNA
   <213> Artificial Sequence
<220>
   <223> Synthetic Oligonucleotide
<400> 1238
   ggat t gcaag t t 12
<210> 1239
   <211> 12
   <212> DNA
   <213> Artificial Sequence
<220>
   <223> Synthetic Oligonucleotide
<400> 1239
   at aaagcat c t g 12
<210> 1240
   <211> 12
   <212> DNA
   <213> Artificial Sequence
<220>
   <223> Synthetic Oligonucleotide
<400> 1240
   ccact gaaca t t 12
<210> 1241
   <211> 12
   <212> DNA
   <213> Artificial Sequence
<220>
   <223> Synthetic Oligonucleotide
<400> 1241
   gaagccct aa t c 12
<210> 1242
   <211> 12
   <212> DNA
   <213> Artificial Sequence
<220>
   <223> Synthetic Oligonucleotide
<400> 1242
   t aaat t gt at gc 12
<210> 1243
   <211> 12
   <212> DNA
   <213> Artificial Sequence
<220>
   <223> Synthetic Oligonucleotide
<400> 1243
   aagat ct t ca ca 12
<210> 1244
   <211> 12
   <212> DNA
   <213> Artificial Sequence
<220>
   <223> Synthetic Oligonucleotide
<400> 1244
   caagat ct t c ac 12
<210> 1245
   <211> 12
   <212> DNA
   <213> Artificial Sequence
<220>
   <223> Synthetic Oligonucleotide
<400> 1245
   at gt caaggt ca 12
<210> 1246
   <211> 12
   <212> DNA
   <213> Artificial Sequence
<220>
   <223> Synthetic Oligonucleotide
<400> 1246
   at aat ggct g ga 12
<210> 1247
   <211> 14
   <212> DNA
   <213> Artificial Sequence
<220>
   <223> Synthetic Oligonucleotide
<400> 1247
   agcaccaat a t gct 14
<210> 1248
   <211> 12
   <212> DNA
   <213> Artificial Sequence
<220>
   <223> Synthetic Oligonucleotide
<400> 1248
   gcaccaat at gc 12
<210> 1249
   <211> 12
   <212> DNA
   <213> Artificial Sequence
<220>
   <223> Synthetic Oligonucleotide
<400> 1249
   t ggcagacca ca 12
<210> 1250
   <211> 12
   <212> DNA
   <213> Artificial Sequence
<220>
   <223> Synthetic Oligonucleotide
<400> 1250
   t cct at gcaa t c 12
<210> 1251
   <211> 12 <212> DNA
   <213> Artificial Sequence
<220>
   <223> Synthetic Oligonucleotide
<400> 1251
   t at aaat gct t c 12
<210> 1252
   <211> 12
   <212> DNA
   <213> Artificial Sequence
<220>
   <223> Synthetic Oligonucleotide
<400> 1252
   gt caaat t ct at 12
<210> 1253
   <211> 12
   <212> DNA
   <213> Artificial Sequence
<220>
   <223> Synthetic Oligonucleotide
<400> 1253
   t t caggct aa t c 12
<210> 1254
   <211> 12
   <212> DNA
   <213> Artificial Sequence
<220>
   <223> Synthetic Oligonucleotide
<400> 1254
   ct gat gaggt at 12
<210> 1255
   <211> 12
   <212> DNA
   <213> Artificial Sequence
<220>
   <223> Synthetic Oligonucleotide
<400> 1255
   ggt gt cagaa t a 12
<210> 1256
   <211> 12
   <212> DNA
   <213> Artificial Sequence
<220>
   <223> Synthetic Oligonucleotide

<400> 1256
   t t t gaaagac ac 12
<210> 1257
   <211> 4 <212> DNA
   <213> Artificial Sequence
<220>
   <223> Synthetic Oligonucleotide
<400> 1257
   act g 4
<210> 1258
   <211> 12
   <212> DNA
   <213> Artificial Sequence
<220>
   <223> Synthetic Oligonucleotide
<400> 1258
   t gcaacagcc ca 12
<210> 1259
   <211> 14
   <212> DNA
   <213> Artificial Sequence
<220>
   <223> Synthetic Oligonucleotide
<400> 1259
   aaagagt ccc gcca 14
<210> 1260
   <211> 12
   <212> DNA
   <213> Artificial Sequence
<220>
   <223> Synthetic Oligonucleotide
<400> 1260
   aagagt cccg cc 12
<210> 1261
   <211> 14
   <212> DNA
   <213> Artificial Sequence
<220>
   <223> Synthetic Oligonucleotide
<400> 1261
   t ccgagagga gaga 14
<210> 1262
   <211> 12
   <212> DNA
   <213> Artificial Sequence
<220>
   <223> Synthetic Oligonucleotide
<400> 1262
   ccgagaggag ag 12
<210> 1263
   <211> 14
   <212> DNA
   <213> Artificial Sequence
<220>
   <223> Synthetic Oligonucleotide
<400> 1263
   t cat ggct gc agct 14
<210> 1264
   <211> 14
   <212> DNA
   <213> Artificial Sequence
<220>
   <223> Synthetic Oligonucleotide
<400> 1264
   acagcggct c aact 14
<210> 1265
   <211> 12
   <212> DNA
   <213> Artificial Sequence
<220>
   <223> Synthetic Oligonucleotide
<400> 1265
   cagcggctca ac 12
<210> 1266
   <211> 14
   <212> DNA
   <213> Artificial Sequence
<220>
   <223> Synthetic Oligonucleotide
<400> 1266
   ggt gacaggc gact 14
<210> 1267
   <211> 12
   <212> DNA
   <213> Artificial Sequence
<220>
   <223> Synthetic Oligonucleotide
<400> 1267
   gt gacaggcg ac 12
<210> 1268
   <211> 12
   <212> DNA
   <213> Artificial Sequence
<220>
   <223> Synthetic Oligonucleotide
<400> 1268
   at ggt gacag gc 12
<210> 1269
   <211> 12
   <212> DNA
   <213> Artificial Sequence
<220>
   <223> Synthetic Oligonucleotide
<400> 1269
   agaggcct gg ca 12
<210> 1270
   <211> 12
   <212> DNA
   <213> Artificial Sequence
<220>
   <223> Synthetic Oligonucleotide
<400> 1270
   ct ggat ggt t gc 12
<210> 1271
   <211> 14
   <212> DNA
   <213> Artificial Sequence
<220>
   <223> Synthetic Oligonucleotide
<400> 1271
   aat ggct gct gcgg 14
<210> 1272
   <211> 12
   <212> DNA
   <213> Artificial Sequence
<220>
   <223> Synthetic Oligonucleotide
<400> 1272
   ccgggt aat g gc 12
<210> 1273
   <211> 14
   <212> DNA
   <213> Artificial Sequence
<220>
   <223> Synthetic Oligonucleotide
<400> 1273
   t ggaccgcag ccgg 14
<210> 1274
   <211> 14
   <212> DNA
   <213> Artificial Sequence
<220>
   <223> Synthetic Oligonucleotide
<400> 1274
   t gat gcccct cgct 14
<210> 1275
   <211> 12
   <212> DNA
   <213> Artificial Sequence
<220>
   <223> Synthetic Oligonucleotide
<400> 1275
   gat gcccct c gc 12
<210> 1276
   <211> 14
   <212> DNA
   <213> Artificial Sequence
<220>
   <223> Synthetic Oligonucleotide
<400> 1276
   ct ggact t gg cggt 14
<210> 1277
   <211> 14
   <212> DNA
   <213> Artificial Sequence
<220>
   <223> Synthetic Oligonucleotide
<400> 1277
   ggaaat ggct ct gg 14
<210> 1278
   <211> 12
   <212> DNA
   <213> Artificial Sequence
<220>
   <223> Synthetic Oligonucleotide
<400> 1278
   gaaat ggct c t g 12
<210> 1279
   <211> 14
   <212> DNA
   <213> Artificial Sequence
<220>
   <223> Synthetic Oligonucleotide
<400> 1279
   agaagct gct ggt g 14
<210> 1280
   <211> 12
   <212> DNA
   <213> Artificial Sequence
<220>
   <223> Synthetic Oligonucleotide
<400> 1280
   gat ggcagaa gc 12
<210> 1281
   <211> 14
   <212> DNA
   <213> Artificial Sequence
<220>
   <223> Synthetic Oligonucleotide
<400> 1281
   agagagat gg caga 14
<210> 1282
   <211> 12
   <212> DNA
   <213> Artificial Sequence
<220>
   <223> Synthetic Oligonucleotide
<400> 1282
   gagagat ggc ag 12
<210> 1283
   <211> 14
   <212> DNA
   <213> Artificial Sequence
<220>
   <223> Synthetic Oligonucleotide
<400> 1283
   gt ggct gaag aaaa 14
<210> 1284
   <211> 12
   <212> DNA
   <213> Artificial Sequence
<220>
   <223> Synthetic Oligonucleotide
<400> 1284
   t ggct gaaga aa 12
<210> 1285
   <211> 14
   <212> DNA
   <213> Artificial Sequence
<220>
   <223> Synthetic Oligonucleotide
<400> 1285
   at gt ct ggga gcct 14
<210> 1286
   <211> 12
   <212> DNA
   <213> Artificial Sequence
<220>
   <223> Synthetic Oligonucleotide
<400> 1286
   t gt ct gggag cc 12
<210> 1287
   <211> 14
   <212> DNA
   <213> Artificial Sequence
<220>
   <223> Synthetic Oligonucleotide
<400> 1287
   at gat ggct g t cat 14
<210> 1288
   <211> 12
   <212> DNA
   <213> Artificial Sequence
<220>
   <223> Synthetic Oligonucleotide
<400> 1288
   t gat gat ggc t g 12
<210> 1289
   <211> 14
   <212> DNA
   <213> Artificial Sequence
<220>
   <223> Synthetic Oligonucleotide
<400> 1289
   ct t t gat gat ggct 14
<210> 1290
   <211> 14
   <212> DNA
   <213> Artificial Sequence
<220>
   <223> Synthetic Oligonucleotide
<400> 1290
   acgat ct ct t t gat 14
<210> 1291
   <211> 14
   <212> DNA
   <213> Artificial Sequence
<220>
   <223> Synthetic Oligonucleotide
<400> 1291
   t gt t t ct gct aacg 14
<210> 1292
   <211> 12
   <212> DNA
   <213> Artificial Sequence
<220>
   <223> Synthetic Oligonucleotide
<400> 1292
   gt t t ct gct a ac 12
<210> 1293
   <211> 14
   <212> DNA
   <213> Artificial Sequence
<220>
   <223> Synthetic Oligonucleotide
<400> 1293
   t t t gt t t ct g ct aa 14

<210> 1294
   <211> 14
   <212> DNA
   <213> Artificial Sequence
<220>
   <223> Synthetic Oligonucleotide
<400> 1294
   ct t gat at ct cct t 14
<210> 1295
   <211> 14
   <212> DNA
   <213> Artificial Sequence
<220>
   <223> Synthetic Oligonucleotide
<400> 1295
   cat cct ct t g at at 14
<210> 1296
   <211> 14
   <212> DNA
   <213> Artificial Sequence
<220>
   <223> Synthetic Oligonucleotide
<400> 1296
   aat ccat cct ct t g 14
<210> 1297
   <211> 12
   <212> DNA
   <213> Artificial Sequence
<220>
   <223> Synthetic Oligonucleotide
<400> 1297
   gaat ccat cc t c 12
<210> 1298
   <211> 14
   <212> DNA
   <213> Artificial Sequence
<220>
   <223> Synthetic Oligonucleotide
<400> 1298
   gt ct aagt cg aat c 14
<210> 1299
   <211> 12
   <212> DNA
   <213> Artificial Sequence
<220>
   <223> Synthetic Oligonucleotide
<400> 1299
   caagt ct aag t c 12
<210> 1300
   <211> 12
   <212> DNA
   <213> Artificial Sequence
<220>
   <223> Synthetic Oligonucleotide
<400> 1300
   aggt caagt c t a 12
<210> 1301
   <211> 14
   <212> DNA
   <213> Artificial Sequence
<220>
   <223> Synthetic Oligonucleotide
<400> 1301
   cgcagaaat g gat a 14
<210> 1302
   <211> 12
   <212> DNA
   <213> Artificial Sequence
<220>
   <223> Synthetic Oligonucleotide
<400> 1302
   gcagaaat gg at 12
<210> 1303
   <211> 14
   <212> DNA
   <213> Artificial Sequence
<220>
   <223> Synthetic Oligonucleotide
<400> 1303
   t t cgcat ccg t ct a 14
<210> 1304
   <211> 12
   <212> DNA
   <213> Artificial Sequence
<220>
   <223> Synthetic Oligonucleotide
<400> 1304
   t cgcat ccgt ct 12
<210> 1305
   <211> 14
   <212> DNA
   <213> Artificial Sequence
<220>
   <223> Synthetic Oligonucleotide
<400> 1305
   ccct aggt t g aat a 14
<210> 1306
   <211> 12
   <212> DNA
   <213> Artificial Sequence
<220>
   <223> Synthetic Oligonucleotide
<400> 1306
   cct aggt t ga at 12
<210> 1307
   <211> 14
   <212> DNA
   <213> Artificial Sequence
<220>
   <223> Synthetic Oligonucleotide
<400> 1307
   gt t at gcaaa t cag 14
<210> 1308
   <211> 12
   <212> DNA
   <213> Artificial Sequence
<220>
   <223> Synthetic Oligonucleotide
<400> 1308
   t t at gcaaat ca 12
<210> 1309
   <211> 14
   <212> DNA,
   <213> Artificial Sequence
<220>
   <223> Synthetic Oligonucleotide
<400> 1309
   t gact cagt a aat t 14
<210> 1310
   <211> 12
   <212> DNA
   <213> Artificial Sequence
<220>
   <223> Synthetic Oligonucleotide
<400> 1310
   gact cagt aa at 12
<210> 1311
   <211> 14
   <212> DNA
   <213> Artificial Sequence
<220>
   <223> Synthetic Oligonucleotide
<400> 1311
   t t aaaat t ct t ggg 14
<210> 1312
   <211> 12
   <212> DNA
   <213> Artificial Sequence
<220>
   <223> Synthetic Oligonucleotide
<400> 1312
   t aaaat t ct t gg 12
<210> 1313
   <211> 14
   <212> DNA
   <213> Artificial Sequence
<220>
   <223> Synthetic Oligonucleotide
<400> 1313
   cct aact t t t agac 14
<210> 1314
   <211> 12
   <212> DNA
   <213> Artificial Sequence
<220>
   <223> Synthetic Oligonucleotide
<400> 1314
   ct aact t t t a ga 12
<210> 1315
   <211> 14
   <212> DNA
   <213> Artificial Sequence
<220>
   <223> Synthetic Oligonucleotide
<400> 1315
   acct gaaact gcaa 14
<210> 1316
   <211> 14
   <212> DNA
   <213> Artificial Sequence
<220>
   <223> Synthetic Oligonucleotide
<400> 1316
   gt gt caaaac cact 14
<210> 1317
   <211> 14
   <212> DNA
   <213> Artificial Sequence
<220>
   <223> Synthetic Oligonucleotide
<400> 1317
   cct at t ccca ct ga 14
<210> 1318
   <211> 12
   <212> DNA
   <213> Artificial Sequence
<220>
   <223> Synthetic Oligonucleotide
<400> 1318
   ct at t cccac t g 12
<210> 1319
   <211> 14
   <212> DNA
   <213> Artificial Sequence
<220>
   <223> Synthetic Oligonucleotide
<400> 1319
   cccat agcaa t aat 14
<210> 1320
   <211> 12
   <212> DNA
   <213> Artificial Sequence
<220>
   <223> Synthetic Oligonucleotide
<400> 1320
   ccat agcaat aa 12
<210> 1321
   <211> 12
   <212> DNA
   <213> Artificial Sequence
<220>
   <223> Synthetic Oligonuceotide
<400> 1321
   at cccat agc aa 12
<210> 1322
   <211> 14
   <212> DNA
   <213> Artificial Sequence
<220>
   <223> Synthetic Oligonucleotide
<400> 1322
   t ct gcaggaa at cc 14
<210> 1323
   <211> 12
   <212> DNA
   <213> Artificial Sequence
<220>
   <223> Synthetic Oligonucleotide

<400> 1323
   ct gcaggaaa t c 12
<210> 1324
   <211> 14
   <212> DNA
   <213> Artificial Sequence
<220>
   <223> Synthetic Oligonucleotide
<400> 1324
   t tct gcagga aat c 14
<210> 1325
   <211> 14
   <212> DNA
   <213> Artificial Sequence
<220>
   <223> Synthetic Oligonucleotide
<400> 1325
   cct t caagt c t t t c 14
<210> 1326
   <211> 14
   <212> DNA
   <213> Artificial Sequence
<220>
   <223> Synthetic Oligonucleotide
<400> 1326
   t t gt t cct gt at ac 14
<210> 1327
   <211> 14
   <212> DNA
   <213> Artificial Sequence
<220>
   <223> Synthetic Oligonucleotide
<400> 1327
   caat at tgt t cct g 14
<210> 1328
   <211> 12
   <212> DNA
   <213> Artificial Sequence
<220>
   <223> Synthetic Oligonucleotide
<400> 1328
   caat at t gt t cc 12
<210> 1329
   <211> 14
   <212> DNA
   <213> Artificial Sequence
<220>
   <223> Synthetic Oligonucleotide
<400> 1329
   acat cat caa tat t 14
<210> 1330
   <211> 14
   <212> DNA
   <213> Artificial Sequence
<220>
   <223> Synthetic Oligonucleotide
<400> 1330
   ct t t gaat cc aaaa 14
<210> 1331
   <211> 12
   <212> DNA
   <213> Artificial Sequence
<220>
   <223> Synthetic Oligonucleotide
<400> 1331
   t t t gaat cca aa 12
<210> 1332
   <211> 14
   <212> DNA
   <213> Artificial Sequence
<220>
   <223> Synthetic Oligonucleotide
<400> 1332
   t at gct t t ga at cc 14
<210> 1333
   <211> 14
   <212> DNA
   <213> Artificial Sequence
<220>
   <223> Synthetic Oligonucleotide
<400> 1333
   t t gt aat ggt t t t t 14
<210> 1334
   <211> 12
   <212> DNA
   <213> Artificial Sequence
<220>
   <223> Synthetic Oligonucleotide
<400> 1334
   at ct tgt aat gg 12
<210> 1335
   <211> 14
   <212> DNA
   <213> Artificial Sequence
<220>
   <223> Synthetic Oligonucleotide
<400> 1335
   agat t gt at a t ct t 14
<210> 1336
   <211> 14
   <212> DNA
   <213> Artificial Sequence
<220>
   <223> Synthetic Oligonucleotide
<400> 1336
   cggt gt cat a at gt 14
<210> 1337
   <211> 14
   <212> DNA
   <213> Artificial Sequence
<220>
   <223> Synthetic Oligonucleotide
<400> 1337
   aaat t t ggcg gt gt 14
<210> 1338
   <211> 14
   <212> DNA
   <213> Artificial Sequence
<220>
   <223> Synthetic Oligonucleotide
<400> 1338
   t t aaat t t gg cggt 14
<210> 1339
   <211> 12
   <212> DNA
   <213> Artificial Sequence
<220>
   <223> Synthetic Oligonucleotide
<400> 1339
   t aaat t t ggc gg 12
<210> 1340
   <211> 14
<212> DNA
   <213> Artificial Sequence
<220>
   <223> Synthetic Oligonucleotide
<400> 1340
   t ct tcaaaag gat a 14
<210> 1341
   <211> 12
   <212> DNA
   <213> Artificial Sequence
<220>
   <223> Synthetic Oligonucleotide
<400> 1341
   t ggt ct tcaa aa 12
<210> 1342
   <211> 14
   <212> DNA
   <213> Artificial Sequence
<220>
   <223> Synthetic Oligonucleotide
<400> 1342
   gt gggt tat ggt ct 14
<210> 1343
   <211> 14
   <212> DNA
   <213> Artificial Sequence
<220>
   <223> Synthetic Oligonucleotide
<400> 1343
   aagt t ct agc t gt g 14
<210> 1344
   <211> 12
   <212> DNA
   <213> Artificial Sequence
<220>
   <223> Synthetic Oligonucleotide
<400> 1344
   at aagt t ct a gc 12
<210> 1345
   <211> 14
   <212> DNA
   <213> Artificial Sequence
<220>
   <223> Synthetic Oligonucleotide
<400> 1345
   aagggt t t ga t aag 14
<210> 1346
   <211> 14
   <212> DNA
   <213> Artificial Sequence
<220>
   <223> Synthetic Oligonucleotide
<400> 1346
   t caagat ct t caca 14
<210> 1347
   <211> 14
   <212> DNA
   <213> Artificial Sequence
<220>
   <223> Synthetic Oligonucleotide
<400> 1347
   agccat t ggt caag 14
<210> 1348
   <211> 14
   <212> DNA .
   <213> Artificial Sequence
<220>
   <223> Synthetic Oligonucleotide
<400> 1348
   t ct t cact t a gcca 14
<210> 1349
   <211> 14
   <212> DNA
   <213> Artificial Sequence
<220>
   <223> Synthetic Oligonucleotide
<400> 1349
   t gct gcaaca t gat 14
<210> 1350
   <211> 12
   <212> DNA
   <213> Artificial Sequence
<220>
   <223> Synthetic Oligonucleotide
<400> 1350
   gctgcaacat ga 12
<210> 1351
   <211> 14
   <212> DNA
   <213> Artificial Sequence
<220>
   <223> Synthetic Oligonucleotide
<400> 1351
   t t acagt gaa t tgc 14
<210> 1352
   <211> 12
   <212> DNA
   <213> Artificial Sequence
<220>
   <223> Synthetic Oligonucleotide
<400> 1352 12
   ccagct t t ac ag 12
<210> 1353
   <211> 12
   <212> DNA
   <213> Artificial Sequence
<220>
   <223> Synthetic Oligonucleotide
<400> 1353 12
   cat t acacca gt 12
<210> 1354
   <211> 14
   <212> DNA
   <213> Artificial Sequence
<220>
   <223> Synthetic Oligonucleotide
<400> 1354 14
   at cat t acac cagt 14
<210> 1355
   <211> 12
   <212> DNA
   <213> Artificial Sequence
<220>
   <223> Synthetic Oligonucleotide
<400> 1355 12
   t cat t acacc ag 12
<210> 1356
   <211> 14
   <212> DNA
   <213> Artificial Sequence
<220>
   <223> Synthetic Oligonucleotide
<400> 1356 14
   aat aaat at g caca 14
<210> 1357
   <211> 14
   <212> DNA
   <213> Artificial Sequence
<220>
   <223> Synthetic Oligonucleotide
<400> 1357 14
   t gt gcct t t a aaaa 14
<210> 1358
   <211> 14
   <212> DNA
   <213> Artificial Sequence
<220>
   <223> Synthetic Oligonucleotide
<400> 1358 14
   t t gt gcct t t aaaa 14
<210> 1359
   <211> 14
   <212> DNA
   <213> Artificial Sequence
<220>
   <223> Synthetic Oligonucleotide
<400> 1359
   gggcct ct t g t gcc 14
<210> 1360
   <211> 14
   <212> DNA
   <213> Artificial Sequence
<220>
   <223> Synthetic Oligonucleotide
<400> 1360
   cct t act t cc ccat 14
<210> 1361
   <211> 14
   <212> DNA
   <213> Artificial Sequence
<220>
   <223> Synthetic Oligonucleotide
<400> 1361
   ct ct ggt cct t act 14
<210> 1362
   <211> 16
   <212> DNA
   <213> Artificial Sequence
<220>
   <223> Synthetic Oligonucleotide
<400> 1362
   gt ct ct ggt c ct t act 16
<210> 1363
   <211> 14
   <212> DNA
   <213> Artificial Sequence
<220>
   <223> Synthetic Oligonucleotide
<400> 1363
   t ct ct ggt cc t tac 14
<210> 1364
   <211> 14
   <212> DNA
   <213> Artificial Sequence
<220>
   <223> Synthetic Oligonucleotide
<400> 1364
   cct ct gact g ggaa 14
<210> 1365
   <211> 14
   <212> DNA
   <213> Artificial Sequence
<220>
   <223> Synthetic Oligonucleotide
<400> 1365
   cat agcgcct ct ga 14
<210> 1366
   <211> 14
   <212> DNA
   <213> Artificial Sequence
<220>
   <223> Synthetic Oligonucleotide
<400> 1366
   caggt agct a t aat 14
<210> 1367
   <211> 12
   <212> DNA
   <213> Artificial Sequence
<220>
   <223> Synthetic Oligonucleotide
<400> 1367
   caggt agct a t a 12
<210> 1368
   <211> 14
   <212> DNA
   <213> Artificial Sequence
<220>
   <223> Synthetic Oligonucleotide
<400> 1368
   t cat ct t gt g aaac 14
<210> 1369
   <211> 12
   <212> DNA <213> Artificial Sequence
<220>
   <223> Synthetic Oligonucleotide
<400> 1369
   cat ct t gt ga aa 12
<210> 1370
   <211> 14
   <212> DNA
   <213> Artificial Sequence
<220>
   <223> Synthetic Oligonucleotide
<400> 1370
   gt t t caaaca t cat 14
<210> 1371
   <211> 14
   <212> DNA
   <213> Artificial Sequence
<220>
   <223> Synthetic Oligonucleotide
<400> 1371
   t agt t t caaa cat c 14
<210> 1372
   <211> 12
   <212> DNA
   <213> Artificial Sequence
<220>
   <223> Synthetic Oligonucleotide
<400> 1372
   agt t t caaac at 12
<210> 1373
   <211> 12
   <212> DNA
   <213> Artificial Sequence
<220>
   <223> Synthetic Oligonucleotide
<400> 1373
   gaat agt t t c aa 12
<210> 1374
   <211> 14
   <212> DNA
   <213> Artificial Sequence
<220>
   <223> Synthetic Oligonucleotide
<400> 1374
   cat t ggaat a gt t t 14
<210> 1375
   <211> 14
   <212> DNA
   <213> Artificial Sequence
<220>
   <223> Synthetic Oligonucleotide
<400> 1375
   cact gaacat t gga 14
<210> 1376
   <211> 12
   <212> DNA
   <213> Artificial Sequence
<220>
   <223> Synthetic Oligonucleotide
<400> 1376
   act gaacat t gg 12
<210> 1377
   <211> 14
   <212> DNA
   <213> Artificial Sequence
<220>
   <223> Synthetic Oligonucleotide
<400> 1377
   ccgccactga acat 14
<210> 1378
   <211> 14
   <212> DNA
   <213> Artificial Sequence
<220>
   <223> Synthetic Oligonucleotide
<400> 1378
   cagaccacaa act g 14
<210> 1379
   <211> 12
   <212> DNA
   <213> Artificial Sequence
<220>
   <223> Synthetic Oligonucleotide
<400> 1379
   agaccacaaa ct 12
<210> 1380
   <211> 14
   <212> DNA
   <213> Artificial Sequence
<220>
   <223> Synthetic Oligonucleotide
<400> 1380
   ct ggcagacc acaa 14
<210> 1381
   <211> 14
   <212> DNA
   <213> Artificial Sequence
<220>
   <223> Synthetic Oligonucleotide
<400> 1381
   agct ggcaga ccac 14
<210> 1382
   <211> 14
   <212> DNA
   <213> Artificial Sequence
<220>
   <223> Synthetic Oligonucleotide
<400> 1382
   acct t t agct ggca 14
<210> 1383
   <211> 14
   <212> DNA
   <213> Artificial Sequence
<220>
   <223> Synthetic Oligonucleotide
<400> 1383
   t ct t cacct t t agc 14
<210> 1384
   <211> 12
   <212> DNA
   <213> Artificial Sequence

<220>
   <223> Synthetic Oligonucleotide
<400> 1384
   t caaagt aca t g 12
<210> 1385
   <211> 14
   <212> DNA
   <213> Artificial Sequence
<220>
   <223> Synthetic Oligonucleotide
<400> 1385
   gaact caaag t aca 14
<210> 1386
   <211> 12
   <212> DNA
   <213> Artificial Sequence
<220>
   <223> Synthetic Oligonucleotide
<400> 1386
   ggaact caaa gt 12
<210> 1387
   <211> 14
   <212> DNA
   <213> Artificial Sequence
<220>
   <223> Synthetic Oligonucleotide
<400> 1387
   agggaact ca aagt 14
<210> 1388
   <211> 12
   <212> DNA
   <213> Artificial Sequence
<220>
   <223> Synthetic Oligonucleotide
<400> 1388
   gggaact caa ag 12
<210> 1389
   <211> 14
   <212> DNA
   <213> Artificial Sequence
<220>
   <223> Synthetic Oligonucleotide
<400> 1389
   gct gagggaa ct ca 14
<210> 1390
   <211> 14
   <212> DNA
   <213> Artificial Sequence
<220>
   <223> Synthetic Oligonucleotide
<400> 1390
   t caccacaca cagg 14
<210> 1391
   <211> 14
   <212> DNA
   <213> Artificial Sequence
<220>
   <223> Synthetic Oligonucleotide
<400> 1391
   gaact ct act t t ga 14
<210> 1392
   <211> 12
   <212> DNA
   <213> Artificial Sequence
<220>
   <223> Synthetic Oligonucleotide
<400> 1392
   gaagaact ct ac 12
<210> 1393
   <211> 14
   <212> DNA
   <213> Artificial Sequence
<220>
   <223> Synthetic Oligonucleotide
<400> 1393
   gt ggaagaac t ct a 14
<210> 1394
   <211> 12
   <212> DNA
   <213> Artificial Sequence
<220>
   <223> Synthetic Oligonucleotide
<400> 1394
   t ggaagaact ct 12
<210> 1395
   <211> 14
   <212> DNA
   <213> Artificial Sequence
<220>
   <223> Synthetic Oligonucleotide
<400> 1395
   t gt t t gt gga agaa 14
<210> 1396
   <211> 14
   <212> DNA
   <213> Artificial Sequence
<220>
   <223> Synthetic Oligonucleotide
<400> 1396
   cat ct t gt t c t gt t 14
<210> 1397
   <211> 14
   <212> DNA
   <213> Artificial Sequence
<220>
   <223> Synthetic Oligonucleotide
<400> 1397
   agt gaaacat t t t g 14
<210> 1398
   <211> 12
   <212> DNA
   <213> Artificial Sequence
<220>
   <223> Synthetic Oligonucleotide
<400> 1398
   ttacccaaaa gt 12
<210> 1399
   <211> 14
   <212> DNA
   <213> Artificial Sequence
<220>
   <223> Synthetic Oligonucleotide
<400> 1399
   t at t t accca aaag 14
<210> 1400
   <211> 14
   <212> DNA
   <213> Artificial Sequence
<220>
   <223> Synthetic Oligonucleotide
<400> 1400
   gt at t t accc aaaa 14
<210> 1401
   <211> 12
   <212> DNA
   <213> Artificial Sequence
<220>
   <223> Synthetic Oligonucleotide
<400> 1401
   t at t t accca aa 12
<210> 1402
   <211> 14
   <212> DNA
   <213> Artificial Sequence
<220>
   <223> Synthetic Oligonucleotide
<400> 1402
   t cct ggt at g aaga 14
<210> 1403
   <211> 12
   <212> DNA
   <213> Artificial Sequence
<220>
   <223> Synthetic Oligonucleotide
<400> 1403
   cct ggt at ga ag 12
<210> 1404
   <211> 12
   <212> DNA
   <213> Artificial Sequence
<220>
   <223> Synthetic Oligonucleotide
<400> 1404
   ggt cct ggt a t g 12
<210> 1405
   <211> 14
   <212> DNA
   <213> Artificial Sequence
<220>
   <223> Synthetic Oligonucleotide
<400> 1405
   t t cct ct ggt cct g 14
<210> 1406
   <211> 14
   <212> DNA
   <213> Artificial Sequence
<220>
   <223> Synthetic Oligonucleotide
<400> 1406
   aggt t t cct c t ggt 14
<210> 1407
   <211> 12
   <212> DNA
   <213> Artificial Sequence
<220>
   <223> Synthetic Oligonucleotide
<400> 1407
   ggt t t cct ct gg 12
<210> 1408
   <211> 14
   <212> DNA
   <213> Artificial Sequence
<220>
   <223> Synthetic Oligonucleotide
<400> 1408
   t t t t ct gagg t t t c 14
<210> 1409
   <211> 14
   <212> DNA
   <213> Artificial Sequence
<220>
   <223> Synthetic Oligonucleotide
<400> 1409
   agact t ccat t t t c 14
<210> 1410
   <211> 12
   <212> DNA
   <213> Artificial Sequence
<220>
   <223> Synthetic Oligonucleotide
<400> 1410
   agact t ccat t t 12
<210> 1411
   <211> 14
   <212> DNA
   <213> Artificial Sequence
<220>
   <223> Synthetic Oligonucleotide
<400> 1411
   caaat gct at cgat 14
<210> 1412
   <211> 14
   <212> DNA
   <213> Artificial Sequence
<220>
   <223> Synthetic Oligonucleotide
<400> 1412
   gcacgct ct a t act 14
<210> 1413
   <211> 12
   <212> DNA
   <213> Artificial Sequence
<220>
   <223> Synthetic Oligonucleotide
<400> 1413
   cacgct ct at ac 12
<210> 1414
   <211> 14 <212> DNA
   <213> Artificial Sequence
<220>
   <223> Synthetic Oligonucleotide
<400> 1414
   t cct t gt cat t at c 14
<210> 1415
   <211> 14
   <212> DNA
   <213> Artificial Sequence
<220>
   <223> Synthetic Oligonucleotide
<400> 1415
   gct t t gt caa gatc 14
<210> 1416
   <211> 14
   <212> DNA
   <213> Artificial Sequence
<220>
   <223> Synthetic Oligonucleotide
<400> 1416
   t gct t t gt ca agat 14
<210> 1417
   <211> 14
   <212> DNA
   <213> Artificial Sequence
<220>
   <223> Synthetic Oligonucleotide
<400> 1417
   aagt at cggt t ggc 14
<210> 1418
   <211> 12
   <212> DNA
   <213> Artificial Sequence
<220>
   <223> Synthetic Oligonucleotide
<400> 1418
   agt at cggt t gg 12
<210> 1419
   <211> 14
   <212> DNA
   <213> Artificial Sequence
<220>
   <223> Synthetic Oligonucleotide
<400> 1419
   t t aaaat t t g gaga 14
<210> 1420
   <211> 14
   <212> DNA
   <213> Artificial Sequence
<220>
   <223> Synthetic Oligonucleotide
<400> 1420
   cct t aaaat t t gga 14
<210> 1421
   <211> 12
   <212> DNA
   <213> Artificial Sequence
<220>
   <223> Synthetic Oligonucleotide
<400> 1421
   ct t aaaat t t gg 12
<210> 1422
   <211> 14
   <212> DNA
   <213> Artificial Sequence
<220>
   <223> Synthetic Oligonucleotide
<400> 1422
   t ct act gt t t t t gt 14
<210> 1423
   <211> 14
   <212> DNA
   <213> Artificial Sequence
<220>
   <223> Synthetic Oligonucleotide
<400> 1423
   ggct cct ct a ct gt 14
<210> 1424
   <211> 15
   <212> DNA
   <213> Artificial Sequence
<220>
   <223> Synthetic Oligonucleotide
<400> 1424
   agcct ct gga t t t ga 15
<210> 1425
   <211> 14
   <212> DNA
   <213> Artificial Sequence
<220>
   <223> Synthetic Oligonucleotide
<400> 1425
   agcct ct gga t t t g 14
<210> 1426
   <211> 15
   <212> DNA
   <213> Artificial Sequence
<220>
   <223> Synthetic Oligonucleotide
<400> 1426
   t agcct ct gg at t t g 15
<210> 1427
   <211> 14
   <212> DNA
   <213> Artificial Sequence
<220>
   <223> Synthetic Oligonucleotide
<400> 1427
   t agcct ct gg at t t 14
<210> 1428
   <211> 16
   <212> DNA
   <213> Artificial Sequence
<220>
   <223> Synthetic Oligonucleotide
<400> 1428
   gct agcct ct ggat t t 16
<210> 1429
   <211> 15
   <212> DNA
   <213> Artificial Sequence
<220>
   <223> Synthetic Oligonucleotide
<400> 1429
   ct agcct ct g gat t t 15
<210> 1430
   <211> 15
   <212> DNA
   <213> Artificial Sequence
<220>
   <223> Synthetic Oligonucleotide
<400> 1430
   gct agcct ct ggat t 15
<210> 1431
   <211> 14
   <212> DNA
   <213> Artificial Sequence
<220>
   <223> Synthetic Oligonucleotide
<400> 1431
   ct agcct ct g gat t 14
<210> 1432
   <211> 13
   <212> DNA
   <213> Artificial Sequence
<220>
   <223> Synthetic Oligonucleotide
<400> 1432
   t agcct ct gg at t 13
<210> 1433
   <211> 15
   <212> DNA
   <213> Artificial Sequence
<220>
   <223> Synthetic Oligonucleotide
<400> 1433
   t gct agcct c t ggat 15
<210> 1434
   <211> 12
   <212> DNA
   <213> Artificial Sequence
<220>
   <223> Synthetic Oligonucleotide
<400> 1434
   t agcct ct gg at 12
<210> 1435
   <211> 15
   <212> DNA
   <213> Artificial Sequence
<220>
   <223> Synthetic Oligonucleotide
<400> 1435
   ct gct agcct ct gga 15
<210> 1436
   <211> 15
   <212> DNA
   <213> Artificial Sequence
<220>
   <223> Synthetic Oligonucleotide
<400> 1436
   act gct agcc t ct gg 15
<210> 1437
   <211> 15
   <212> DNA
   <213> Artificial Sequence
<220>
   <223> Synthetic Oligonucleotide
<400> 1437
   aact gct agc ct ct g 15
<210> 1438
   <211> 15
   <212> DNA
   <213> Artificial Sequence
<220>
   <223> Synthetic Oligonucleotide
<400> 1438
   gaact gct ag cct ct 15
<210> 1439
   <211> 15
   <212> DNA
   <213> Artificial Sequence
<220>
   <223> Synthetic Oligonucleotide
<400> 1439
   t gaact gct a gcct c 15
<210> 1440
   <211> 15
   <212> DNA
   <213> Artificial Sequence
<220>
   <223> Synthetic Oligonucleotide
<400> 1440
   t t gaact gct agcct 15
<210> 1441
   <211> 14
   <212> DNA
   <213> Artificial Sequence
<220>
   <223> Synthetic Oligonucleotide
<400> 1441
   t gaact gct a gcct 14
<210> 1442
   <211> 14
   <212> DNA
   <213> Artificial Sequence
<220>
   <223> Synthetic Oligonucleotide
<400> 1442
   agt t gaact g ct ag 14
<210> 1443
   <211> 12
   <212> DNA
   <213> Artificial Sequence
<220>
   <223> Synthetic Oligonucleotide
<400> 1443
   gt t gaact gc t a 12
<210> 1444
   <211> 12
   <212> DNA
   <213> Artificial Sequence
<220>
   <223> Synthetic Oligonucleotide
<400> 1444
   gaagt t gaac t g 12
<210> 1445
   <211> 14
   <212> DNA
   <213> Artificial Sequence
<220>
   <223> Synthetic Oligonucleotide
<400> 1445
   acagaagt t g aact 14
<210> 1446
   <211> 14
   <212> DNA
   <213> Artificial Sequence
<220>
   <223> Synthetic Oligonucleotide
<400> 1446
   t cat t gt cac t aac 14

<210> 1447
   <211> 12
   <212> DNA
   <213> Artificial Sequence
<220>
   <223> Synthetic Oligonucleotide
<400> 1447
   cat t gt cact aa 12
<210> 1448
   <211> 12
   <212> DNA
   <213> Artificial Sequence
<220>
   <223> Synthetic Oligonucleotide
<400> 1448
   tcaggttcat tg 12
<210> 1449
   <211> 14
   <212> DNA
   <213> Artificial Sequence
<220>
   <223> Synthetic Oligonucleotide
<400> 1449
   at gat caggt t cat 14
<210> 1450
   <211> 14
   <212> DNA
   <213> Artificial Sequence
<220>
   <223> Synthetic Oligonucleotide
<400> 1450
   t at aat gat c aggt 14
<210> 1451
   <211> 12
   <212> DNA
   <213> Artificial Sequence
<220>
   <223> Synthetic Oligonucleotide
<400> 1451
   at aat gat ca gg 12
<210> 1452
   <211> 14
   <212> DNA
   <213> Artificial Sequence
<220>
   <223> Synthetic Oligonucleotide
<400> 1452
   t ggt gt caga at at 14
<210> 1453
   <211> 14
   <212> DNA
   <213> Artificial Sequence
<220>
   <223> Synthetic Oligonucleotide
<400> 1453
   t cagt ggt gt caga 14
<210> 1454
   <211> 14
   <212> DNA
   <213> Artificial Sequence
<220>
   <223> Synthetic Oligonucleotide
<400> 1454
   ct ct ggat ca gagt 14
<210> 1455
   <211> 14
   <212> DNA
   <213> Artificial Sequence
<220>
   <223> Synthetic Oligonucleotide
<400> 1455
   aaggt t cat t ct ct 14
<210> 1456
   <211> 14
   <212> DNA
   <213> Artificial Sequence
<220>
   <223> Synthetic Oligonucleotide
<400> 1456
   t t cat caaaa ggt t 14
<210> 1457
   <211> 14
   <212> DNA
   <213> Artificial Sequence
<220>
   <223> Synthetic Oligonucleotide
<400> 1457
   ct t cat caaa aggt 14
<210> 1458
   <211> 12
   <212> DNA
   <213> Artificial Sequence
<220>
   <223> Synthetic Oligonucleotide
<400> 1458
   ct t cat caaa ag 12
<210> 1459
   <211> 14
   <212> DNA
   <213> Artificial Sequence
<220>
   <223> Synthetic Oligonucleotide
<400> 1459
   at gct gat ct t cat 14
<210> 1460
   <211> 14
   <212> DNA
   <213> Artificial Sequence
<220>
   <223> Synthetic Oligonucleotide
<400> 1460
   t t t t gt aat t t gt g 14
<210> 1461
   <211> 14
   <212> DNA
   <213> Artificial Sequence
<220>
   <223> Synthetic Oligonucleotide
<400> 1461
   t cagact t t t gt aa 14
<210> 1462
   <211> 14
   <212> DNA
   <213> Artificial Sequence
<220>
   <223> Synthetic Oligonucleotide
<400> 1462
   t gt cct at t g ccat 14
<210> 1463
   <211> 14
   <212> DNA
   <213> Artificial Sequence
<220>
   <223> Synthetic Oligonucleotide
<400> 1463
   t ct gacacaa t gt c 14
<210> 1464
   <211> 12
   <212> DNA
   <213> Artificial Sequence
<220>
   <223> Synthetic Oligonucleotide
<400> 1464
   ct gacacaat gt 12
<210> 1465
   <211> 14
   <212> DNA
   <213> Artificial Sequence
<220>
   <223> Synthetic Oligonucleotide
<400> 1465
   tgttcctata actg 14
<210> 1466
   <211> 12
   <212> DNA
   <213> Artificial Sequence
<220>
   <223> Synthetic Oligonucleotide
<400> 1466
   gt t cct at aa ct 12
<210> 1467
   <211> 14
   <212> DNA
   <213> Artificial Sequence
<220>
   <223> Synthetic Oligonucleotide
<400> 1467
   aagat t ggt c agga 14
<210> 1468
   <211> 12
   <212> DNA
   <213> Artificial Sequence
<220>
   <223> Synthetic Oligonucleotide
<400> 1468
   agat t ggt ca gg 12
<210> 1469
   <211> 14
   <212> DNA
   <213> Artificial Sequence
<220>
   <223> Synthetic Oligonucleotide
<400> 1469
   gt gt caaaac cct g 14
<210> 1470
   <211> 12
   <212> DNA
   <213> Artificial Sequence
<220>
   <223> Synthetic Oligonucleotide
<400> 1470
   agct acacaa cc 12
<210> 1471
   <211> 14
   <212> DNA
   <213> Artificial Sequence
<220>
   <223> Synthetic Oligonucleotide
<400> 1471
   cacagct aca caac 14
<210> 1472
   <211> 12
   <212> DNA
   <213> Artificial Sequence
<220>
   <223> Synthetic Oligonucleotide
<400> 1472
   acagct acac aa 12
<210> 1473
   <211> 14
   <212> DNA
   <213> Artificial Sequence
<220>
   <223> Synthetic Oligonucleotide
<400> 1473
   t at at acat g acac 14
<210> 1474
   <211> 12
   <212> DNA
   <213> Artificial Sequence
<220>
   <223> Synthetic Oligonucleotide
<400> 1474
   at at acat ga ca 12
<210> 1475
   <211> 14
   <212> DNA
   <213> Artificial Sequence
<220>
   <223> Synthetic Oligonucleotide
<400> 1475
   aat t t t aaat gt cc 14
<210> 1476
   <211> 12
   <212> DNA
   <213> Artificial Sequence
<220>
   <223> Synthetic Oligonucleotide
<400> 1476
   at t t t aaat g t c 12
<210> 1477
   <211> 12
   <212> DNA
   <213> Artificial Sequence
<220>
   <223> Synthetic Oligonucleotide
<400> 1477
   t cct aat t ga at 12
<210> 1478
   <211> 12
   <212> DNA
   <213> Artificial Sequence
<220>
   <223> Synthetic Oligonucleotide
<400> 1478
   aaagt gccat ct 12
<210> 1479
   <211> 14
   <212> DNA
   <213> Artificial Sequence
<220>
   <223> Synthetic Oligonucleotide
<400> 1479
   t t t at aaaac t gga 14
<210> 1480
   <211> 12
   <212> DNA
   <213> Artificial Sequence
<220>
   <223> Synthetic Oligonucleotide
<400> 1480
   t t at aaaact gg 12
<210> 1481
   <211> 14
   <212> DNA
   <213> Artificial Sequence
<220>
   <223> Synthetic Oligonucleotide
<400> 1481
   t gcaaact t a t ct g 14
<210> 1482
   <211> 12
   <212> DNA
   <213> Artificial Sequence
<220>
   <223> Synthetic Oligonucleotide
<400> 1482
   gcaaact t at ct 12
<210> 1483
   <211> 12
   <212> DNA
   <213> Artificial Sequence
<220>
   <223> Synthetic Oligonucleotide
<400> 1483
   agccaact gc aa 12
<210> 1484
   <211> 14
   <212> DNA
   <213> Artificial Sequence
<220>
   <223> Synthetic Oligonucleotide
<400> 1484
   ct t agccaac t gca 14
<210> 1485
   <211> 12
   <212> DNA
   <213> Artificial Sequence
<220>
   <223> Synthetic Oligonucleotide
<400> 1485
   t t agccaact gc 12
<210> 1486
   <211> 14
   <212> DNA
   <213> Artificial Sequence
<220>
   <223> Synthetic Oligonucleotide
<400> 1486
   t aaat cat tg t caa 14
<210> 1487
   <211> 14
   <212> DNA
   <213> Artificial Sequence
<220>
   <223> Synthetic Oligonucleotide
<400> 1487
   gcact ggcct t gat 14
<210> 1488
   <211> 12
   <212> DNA
   <213> Artificial Sequence
<220>
   <223> Synthetic Oligonucleotide
<400> 1488
   cact ggcct t ga 12
<210> 1489
   <211> 12
   <212> DNA
   <213> Artificial Sequence
<220>
   <223> Synthetic Oligonucleotide
<400> 1489
   t t agcact gg cc 12
<210> 1490
   <211> 12
   <212> DNA
   <213> Artificial Sequence
<220>
   <223> Synthetic Oligonucleotide
<400> 1490
   t gt gt aaggt ca 12
<210> 1491
   <211> 12 <212> DNA
   <213> Artificial Sequence
<220>
   <223> Synthetic Oligonucleotide
<400> 1491
   gt t aat gaca t t 12
<210> 1492
   <211> 12
   <212> DNA
   <213> Artificial Sequence
<220>
   <223> Synthetic Oligonucleotide
<400> 1492
   gacaat t t ct ac 12
<210> 1493
   <211> 12
   <212> DNA
   <213> Artificial Sequence
<220>
   <223> Synthetic Oligonucleotide
<400> 1493
   aacact gcac at 12
<210> 1494
   <211> 14
   <212> DNA
   <213> Artificial Sequence
<220>
   <223> Synthetic Oligonucleotide
<400> 1494
   t aggt caagt ct aa 14
<210> 1495
   <211> 14
   <212> DNA
   <213> Artificial Sequence
<220>
   <223> Synthetic Oligonucleotide
<400> 1495
   t at aggt caa gt ct 14
<210> 1496
   <211> 14
   <212> DNA
   <213> Artificial Sequence
<220>
   <223> Synthetic Oligonucleotide
<400> 1496
   t t t ggat aaa t at a 14
<210> 1497
   <211> 14
   <212> DNA
   <213> Artificial Sequence
<220>
   <223> Synthetic Oligonucleotide
<400> 1497
   t gggact t ca cacc 14
<210> 1498
   <211> 12
   <212> DNA
   <213> Artificial Sequence
<220>
   <223> Synthetic Oligonucleotide
<400> 1498
   accacagct a gt 12
<210> 1499
   <211> 12
   <212> DNA
   <213> Artificial Sequence
<220>
   <223> Synthetic Oligonucleotide
<400> 1499
   gggact t cac ac 12
<210> 1500
   <211> 14
   <212> DNA
   <213> Artificial Sequence
<220>
   <223> Synthetic Oligonucleotide
<400> 1500
   ccaaaaacct t act 14
<210> 1501
   <211> 12
   <212> DNA
   <213> Artificial Sequence
<220>
   <223> Synthetic Oligonucleotide
<400> 1501
   gt ggcaacca ca 12
<210> 1502
   <211> 12
   <212> DNA
   <213> Artificial Sequence
<220>
   <223> Synthetic Oligonucleotide
<400> 1502
   aaggct t aaa gt 12
<210> 1503
   <211> 12
   <212> DNA
   <213> Artificial Sequence
<220>
   <223> Synthetic Oligonucleotide
<400> 1503
   aggact t ggg at 12
<210> 1504
   <211> 12
   <212> DNA
   <213> Artificial Sequence
<220>
   <223> Synthetic Oligonucleotide
<400> 1504
   gggaat caga gc 12
<210> 1505
   <211> 12
   <212> DNA
   <213> Artificial Sequence
<220>
   <223> Synthetic Oligonucleotide
<400> 1505
   at t t gat gct gc 12
<210> 1506
   <211> 12
   <212> DNA
   <213> Artificial Sequence
<220>
   <223> Synthetic Oligonucleotide
<400> 1506
   t t ccaat gac t a 12
<210> 1507
   <211> 12
   <212> DNA
   <213> Artificial Sequence
<220>
   <223> Synthetic Oligonucleotide
<400> 1507
   at caacagct gc 12
<210> 1508
   <211> 14
   <212> DNA
   <213> Artificial Sequence
<220>
   <223> Synthetic Oligonucleotide
<400> 1508
   gat t gcaagt t ccg 14
<210> 1509
   <211> 12
   <212> DNA
   <213> Artificial Sequence
<220>
   <223> Synthetic Oligonucleotide
<400> 1509
   t gct t ggaag ga 12

<210> 1510
   <211> 14
   <212> DNA
   <213> Artificial Sequence
<220>
   <223> Synthetic Oligonucleotide
<400> 1510
   ctgctgcaca tcca 14
<210> 1511
   <211> 12
   <212> DNA
   <213> Artificial Sequence
<220>
   <223> Synthetic Oligonucleotide
<400> 1511
   cacat t aaca gt 12
<210> 1512
   <211> 12
   <212> DNA
   <213> Artificial Sequence
<220>
   <223> Synthetic Oligonucleotide
<400> 1512
   agt t gaaat t t c 12
<210> 1513
   <211> 12
   <212> DNA
   <213> Artificial Sequence
<220>
   <223> Synthetic Oligonucleotide
<400> 1513
   accct cat t c ag 12
<210> 1514
   <211> 12
   <212> DNA
   <213> Artificial Sequence
<220>
   <223> Synthetic Oligonucleotide
<400> 1514
   agaat gagac t t 12
<210> 1515
   <211> 12
   <212> DNA
   <213> Artificial Sequence
<220>
   <223> Synthetic Oligonucleotide
<400> 1515
   gt aaat cct a ag 12
<210> 1516
   <211> 12
   <212> DNA
   <213> Artificial Sequence
<220>
   <223> Synthetic Oligonucleotide
<400> 1516
   t aaat at agg t c 12
<210> 1517
   <211> 12
   <212> DNA
   <213> Artificial Sequence
<220>
   <223> Synthetic Oligonucleotide
<400> 1517
   aat gcct t at t a 12
<210> 1518
   <211> 14
   <212> DNA
   <213> Artificial Sequence
<220>
   <223> Synthetic Oligonucleotide
<400> 1518
   cacct t aaaa t t t g 14
<210> 1519
   <211> 12
   <212> DNA
   <213> Artificial Sequence
<220>
   <223> Synthetic Oligonucleotide
<400> 1519
   gt gt acagat t t 12
<210> 1520
   <211> 12
   <212> DNA
   <213> Artificial Sequence
<220>
   <223> Synthetic Oligonucleotide
<400> 1520
   ct t t cagt ca t a 12
<210> 1521
   <211> 12
   <212> DNA
   <213> Artificial Sequence
<220>
   <223> Synthetic Oligonucleotide
<400> 1521
   gggcat at ca aa 12
<210> 1522
   <211> 12
   <212> DNA
   <213> Artificial Sequence
<220>
   <223> Synthetic Oligonucleotide
<400> 1522
   t gaggcat t a t c 12
<210> 1523
   <211> 4
   <212> DNA
   <213> Artificial Sequence
<220>
   <223> Synthetic Oligonucleotide
<400> 1523
   act g 4
<210> 1524
   <211> 19
   <212> DNA
   <213> Artificial Sequence
<220>
   <223> Primer
<400> 1524
   cgt gggct cc agcat t ct a 19
<210> 1525
   <211> 21 <212> DNA
   <213> Artificial Sequence
<220>
   <223> Primer
<400> 1525
   agt cat t t ct gcc t t t gcgt c 21
<210> 1526
   <211> 22
   <212> DNA
   <213> Artificial Sequence
<220>
   <223> Pr obe
<400> 1526
   ccaat ggt cg ggcact gct c aa 22
<210> 1527
   <211> 30
   <212> DNA
   <213> Artificial Sequence
<220>
   <223> Primer
<400> 1527
   gaaaat agac t t cct gaat a act at gcat t 30
<210> 1528
   <211> 18
   <212> DNA
   <213> Artificial Sequence
<220>
   <223> Primer
<400> 1528
   act cgct t gc cagct t gc 18
<210> 1529
   <211> 23 <212> DNA
   <213> Artificial Sequence
<220>
   <223> Pr obe
<400> 1529
   t t t ct gagt c cccgt gccca aca 23
<210> 1530
   <211> 20
   <212> DNA
   <213> Artificial Sequence
<220>
   <223> Primer
<400> 1530
   t gagcct t gc cacct t ct ct 20
<210> 1531
   <211> 15
   <212> DNA
   <213> Artificial Sequence
<220>
   <223> Primer
<400> 1531
   gcgcacccca gccaa 15
<210> 1532
   <211> 29
   <212> DNA
   <213> Artificial Sequence
<220>
   <223> Probe
<400> 1532
   agaggagct t ct t t t ccct c t acct gggc 29
<210> 1533
   <211> 20
   <212> DNA
   <213> Artificial Sequence
<220>
   <223> Primer
<400> 1533
   at t t cct gcc cct ggt acct 20
<210> 1534
   <211> 17
   <212> DNA
   <213> Artificial Sequence
<220>
   <223> Primer
<400> 1534
   cgggcccaca cct ct t g 17
<210> 1535
   <211> 26
   <212> DNA
   <213> Artificial Sequence
<220>
   <223> Probe
<400> 1535
   ccacaaagt g cagcaccgcc t agt gt 26
<210> 1536
   <211> 19
   <212> DNA
   <213> Artificial Sequence
<220>
   <223> Primer
<400> 1536
   gccacaggct cccagacat 19
<210> 1537
   <211> 25
   <212> DNA
   <213> Artificial Sequence
<220>
   <223> Primer
<400> 1537
   t ccat cct ct t gat at ct cc t t t tg 25
<210> 1538
   <211> 31
   <212> DNA
   <213> Artificial Sequence
<220>
   <223> Probe
<400> 1538
   acagccat ca t caaagagat cgt t agcaga a 31
<210> 1539
   <211> 24
   <212> DNA
   <213> Artificial Sequence
<220>
   <223> Primer
<400> 1539
   at gacaat ca t gt t gcagca at t c 24
<210> 1540
   <211> 25
   <212> DNA
   <213> Artificial Sequence
<220>
   <223> Primer
<400> 1540
   cgat gcaat a aat at gcaca aat ca 25
<210> 1541
   <211> 28
   <212> DNA
   <213> Artificial Sequence
<220>
   <223> Probe
<400> 1541
   ct gt aaagct ggaaagggac ggact ggt 28
<210> 1542
   <211> 14 <212> DNA
   <213> Artificial Sequence
<220>
   <223> Synthetic Oligonucleotide

<400> 1542
   gt gt gcacac agct 14
<210> 1543
   <211> 12
   <212> DNA
   <213> Artificial Sequence
<220>
   <223> Synthetic Oligonucleotide
<400> 1543
   cagcct gggc ac 12
<210> 1544
   <211> 14
   <212> DNA
   <213> Artificial Sequence
<220>
   <223> Synthetic Oligonucleotide
<400> 1544
   t gct cgaact cct t 14
<210> 1545
   <211> 14
   <212> DNA
   <213> Artificial Sequence
<220>
   <223> Synthetic Oligonucleotide
<400> 1545
   gaagt cact g gct t 14
<210> 1546
   <211> 14
   <212> DNA
   <213> Artificial Sequence
<220>
   <223> Synthetic Oligonucleotide
<400> 1546
   gggaagt cac t ggc 14
<210> 1547
   <211> 14
   <212> DNA
   <213> Artificial Sequence
<220>
   <223> Synthetic Oligonucleotide
<400> 1547
   gt t aggcaaa gggc 14
<210> 1548
   <211> 14
   <212> DNA
   <213> Artificial Sequence
<220>
   <223> Synthetic Oligonucleotide
<400> 1548
   gggct gagt g accc 14
<210> 1549
   <211> 14
   <212> DNA
   <213> Artificial Sequence
<220>
   <223> Synthetic Oligonucleotide
<400> 1549
   at gct agt gc act a 14
<210> 1550
   <211> 14
   <212> DNA
   <213> Artificial Sequence
<220>
   <223> Synthetic Oligonucleotide
<400> 1550
   agct cgct ac ct ct 14
<210> 1551
   <211> 14
   <212> DNA
   <213> Artificial Sequence
<220>
   <223> Synthetic Oligonucleotide
<400> 1551
   gaggt at ccc at ct 14
<210> 1552
   <211> 14
   <212> DNA
   <213> Artificial Sequence
<220>
   <223> Synthetic Oligonucleotide
<400> 1552
   ggcaact t ca acct 14
<210> 1553
   <211> 20
   <212> DNA
   <213> Artificial Sequence
<220>
   <223> Synthetic Oligonucleotide
<400> 1553
   gaagtagcca ccaactgtgc 20
<210> 1554
   <211> 12
   <212> DNA
   <213> Artificial Sequence
<220>
   <223> Synthetic Oligonucleotide
<400> 1554
   t agccaccaa ct 12
<210> 1555
   <211> 4
   <212> DNA
   <213> Artificial Sequence
<220>
   <223> Synthetic Oligonucleotide
<400> 1555
   act g 4
<210> 1556
   <211> 4
   <212> DNA
   <213> Artificial Sequence
<220>
   <223> Synthetic Oligonucleotide
<400> 1556
   act g 4
<210> 1557
   <211> 10
   <212> DNA
   <213> Artificial Sequence
<220>
   <223> Synthetic Oligonucleotide
<400> 1557
   agccaccaac 10
<210> 1558
   <211> 8
   <212> DNA
   <213> Artificial Sequence
<220>
   <223> Synthetic Oligonucleotide
<400> 1558
   gccaccaa 8
<210> 1559
   <211> 13
   <212> DNA
   <213> Artificial Sequence
<220>
   <223> Synthetic Oligonucleotide
<400> 1559
   t agccaccaa ct g 13
<210> 1560
   <211> 12
   <212> DNA
   <213> Artificial Sequence
<220>
   <223> Synthetic Oligonucleotide
<400> 1560
   t accgaacac ct 12
<210> 1561
   <211> 20
   <212> DNA
   <213> Artificial Sequence
<220>
   <223> Synthetic Oligonucleotide
<400> 1561
   gt ccct gaag at gt caat gc 20
<210> 1562
   <211> 20
   <212> DNA
   <213> Artificial Sequence
<220>
   <223> Synthetic Oligonucleotide
<400> 1562
   t gcact t t gt ggt accaagg 20
<210> 1563
   <211> 14
   <212> DNA
   <213> Artificial Sequence
<220>
   <223> Synthetic Oligonucleotide
<400> 1563
   gct t ct ccat cat a 14
<210> 1564
   <211> 14
   <212> DNA
   <213> Artificial Sequence
<220>
   <223> Synthetic Oligonucleotide
<400> 1564
   t gt ct t gct g c t t t 14
<210> 1565 <211> 4
   <212> DNA
   <213> Artificial Sequence
<220>
   <223> Synthetic Oligonucleotide
<400> 1565
   act g 4
<210> 1566
   <211> 4
   <212> DNA
   <213> Artificial Sequence
<220>
   <223> Synthetic Oligonucleotide
<400> 1566
   act g 4
<210> 1567
   <211> 4
   <212> DNA
   <213> Artificial Sequence
<220>
   <223> Synthetic Oligonucleotide
<400> 1567
   act g 4
<210> 1568
   <211> 20
   <212> DNA
   <213> Artificial Sequence
<220>
   <223> Synthetic Oligonucleotide
<400> 1568
   at ggct t ct c cat cat at cc 20
<210> 1569
   <211> 20
   <212> DNA
   <213> Artificial Sequence
<220>
   <223> Synthetic Oligonucleotide
<400> 1569
   ct t gggcat g ct cgt cagt c 20
<210> 1570
   <211> 20
   <212> DNA
   <213> Artificial Sequence
<220>
   <223> Synthetic Oligonucleotide
<400> 1570
   cct t ccct ga aggt t cct cc 20
<210> 1571
   <211> 20
   <212> DNA
   <213> Artificial Sequence
<220>
   <223> Synthetic Oligonucleotide
<400> 1571
   ct gct agcct ct ggat t t ga 20
<210> 1572
   <211> 4
   <212> DNA
<213> Artificial Sequence
<220>
   <223> Synthetic Oligonucleotide
<400> 1572
   act g 4
<210> 1573
   <211> 14
   <212> DNA
   <213> Artificial Sequence

<220>
   <223> Synthetic Oligonucleotide
<400> 1573
   gaat at ct at aat g 14
<210> 1574
   <211> 12
   <212> DNA
   <213> Artificial Sequence
<220>
   <223> Synthetic Oligonucleotide
<400> 1574
   gt aagcaagg ct 12
<210> 1575
   <211> 2254
   <212> DNA
   <213> Rattus norvegicus
<400> 1575
<210> 1576
   <211> 3300
   <212> DNA
   <213> Mus muscul us
<400> 1576

## Description

### BACKGROUND

Targeting disease-causing gene sequences was first suggested nearly 40 years ago (Belikova et al., Tet. Lett., 1967, 37, 3557-3562), and antisense activity was demonstrated in cell culture a decade later (Zamecnik et al., Proc. Natl. Acad. Sci. U.S.A., 1978, 75, 280-284). One advantage of antisense technology in the treatment of a disease or condition that stems from a disease-causing gene is that it is a direct genetic approach that has the ability to modulate expression of specific disease-causing genes.

Generally, the principle behind antisense technology is that an antisense compound hybridizes to a target nucleic acid and effects modulation of gene expression activity or function, such as transcription, translation or splicing. The modulation of gene expression can be achieved by, for example, target degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi is a form of antisense-mediated gene silencing involving the introduction of dsRNA leading to the sequence-specific reduction of targeted endogenous mRNA levels. Sequence-specificity makes antisense compounds extremely attractive as tools for target validation and gene functionalization, as well as research tools for identifying and characterizing nucleases and as therapeutics to selectively modulate the expression of genes involved in the pathogenesis of any one of a variety of diseases.

Antisense technology is an effective means for reducing the expression of one or more specific gene products and can therefore prove to be uniquely useful in a number of therapeutic, diagnostic, and research applications. Chemically modified nucleosides are routinely used for incorporation into antisense compounds to enhance one or more properties, such as nuclease resistance, pharmacokinetics or affinity for a target RNA.

Despite the expansion of knowledge since the discovery of antisense technology, there remains an unmet need for antisense compounds with greater efficacy, reduced toxicity and lower cost. Until the present disclosure, high-affinity modifications have not been employed in the design of short antisense compounds for reducing target RNA *in vivo.* This is because of concerns regarding the degree of target specificity that a sequence is nucleotides or shorter would have when employed to reduce target in a living system. Previous studies have described that greater specificity, and therefore greater potential for potency, is achieved by antisense compounds between 16 and 20 nucleobases in Length.

WO 2005/038013 (ISIS PHARMACEUTICALS, INC.) describes antisense compounds and methods for modulating the expression of target genes expressed in the kidney.

WO 2005/042552 (ISIS PHARMACEUTICALS, INC.) describes compositions and methods for modulating the expression of SGLT2.

The present disclosure describes incorporation of chemically-modified high-affinity nucleotides into antisense compounds allows for short antisense compounds about 8-16 nucleobases in length useful in the reduction of target RNAs in animals with increased potency and improved therapeutic index. Thus, provided herein are short antisense compounds comprising high-affinity nucleotide modifications useful for reducing a target RNA *in vivo.* Such short antisense compounds are effective at lower doses than previously described antisense compounds, allowing for a reduction in toxicity and cost of treatment.

### SUMMARY OF THE INVENTION

The invention provides a short antisense compound 10 to 14 monomers in length, comprising a 2'-deoxyribonucleotide gap region flanked on each side by a wing, wherein each wing independently consists of 1 to 3 high-affinity modified monomers which are sugar-modified nucleotides that comprise a bridge between the 4' and the 2' position of the sugar, and wherein the short antisense compound is targeted to a nucleic acid encoding SGLT2.

The invention also provides a short antisense compound 10 to 14 monomers in length, comprising a 2'-deoxyribonucleotide gap region flanked on each side by a wing, wherein each wing independently consists of 1 to 3 high-affinity modified monomers which are sugar-modified nucleotides comprising the 2'-subsitutent group OCH₂CH₂OCH₃, and wherein the short antisense compound is targeted to a nucleic acid encoding SGLT2.

The invention also provides an *ex vivo* method of modulating expression of SGLT2 by contacting an SGLT2 nucleic acid with a short antisense compound as described above.

The invention also provides the short antisense compound as described above, for use in treating a metabolic disorder in an animal.

Other aspects of the invention are set out in the appended claims.

### SUMMARY OF THE DISCLOSURE

Disclosed herein are short antisense compounds and methods of using said compounds to reduce target RNA expression in cells or tissues. In certain embodiments, disclosed herein is a method of reducing expression of a target in an animal, comprising administering to the animal a short antisense compound targeted to a nucleic acid of such target. In certain embodiments, shorts antisense compounds are oligonucleotide compounds. In certain embodiments short antisense oligonucleotides are about 8 to 16, preferably 9 to 15, more preferably 9 to 14, more preferably 10 to 14 nucleotides in length and comprises a gap region flanked on each side by a wing, wherein each wing independently consists of 1 to 3 nucleotides. Preferred motifs include but are not limited to wing - deoxy gap -wing motifs selected from 3-10-3, 2-10-3, 2-10-2, 1-10-1, 2-8-2, 1-8-1, 3-6-3 or 1-6-1. In a preferred embodiment, the short antisense oligonucleotide comprise at least one high-affinity modification. In a further embodiment, the high-affinity modification includes chemically-modified high-affinity nucleotides. In a preferred embodiment, each wing independently consists of 1 to 3 high-affinity modified nucleotides. In one embodiment the high affinity modified nucleotides are sugar-modified nucleotides.

In certain embodiments short antisense compounds exhibit greater uptake in the gut as compared to antisense compounds of greater length. Thus, also disclosed herein are methods of reducing a target in an animal, comprising orally administering the short antisense compounds of the present disclosure.

Further disclosed are methods of treating a metabolic disorder in an animal, comprising administering to an animal in need of such therapy a short antisense compound targeted to a nucleic acid involved in regulating glucose metabolism or clearance, lipid metabolism, cholesterol metabolism, or insulin signaling.

Also disclosed are methods of increasing insulin sensitivity, decreasing blood glucose or decreasing HbA_{1c} in an animal, comprising administering to said animal a short antisense compound targeted to a nucleic acid encoding a target involved in regulating glucose metabolism or clearance, lipid metabolism, cholesterol metabolism, or insulin signaling.

Further disclosed are methods of decreasing total serum cholesterol, serum LDL, serum VLDL, serum HDL, serum triglycerides, serum apolipoprotein(a) or free fatty acids in an animal, comprising administering to said animal a short antisense compound targeted to a nucleic acid encoding a target that is involved in regulating glucose metabolism or clearance, lipid metabolism, cholesterol metabolism, or insulin signaling, wherein said short antisense compound is 8 to 16 nucleotides in length and comprises a gap region flanked on each side by a wing, wherein each wing independently consists of 1 to 3 high-affinity modified nucleotides.

In certain embodiments, short antisense compounds further comprise a conjugate group. Conjugate groups include, but are not limited to, C₁₆ and cholesterol.

In certain embodiments short antisense compounds comprise at least one modified nucleobase, internucleoside linkage or sugar moiety. In certain embodiments, such modified internucleoside linkage is a phosphorothioate internucleoside linkage. In certain embodiments, each internucleoside linkage is a phosphorothioate internucleoside linkage.

In certain embodiments, short antisense compounds comprise at least one high affinity modification. In certain such embodiments, the high-affinity modification is a chemically-modified high-affinity nucleotide. In certain embodiments, chemically-modified high affinity nucleotides are sugar-modified nucleotides. In certain embodiments, at least one of the sugar-modified nucleotides comprises a bridge between the 4' and the 2' position of the sugar. Each of the sugar-modified nucleotides is, independently, in the β-D or α-L sugar conformation. In certain embodiments, each of said high-affinity modified nucleotides confers a Tₘ of at least 1 to 4 degrees per nucleotide. In certain embodiments, each of said sugar-modified nucleotides comprises a 2'-substituent group that is other than H or OH. Such sugar-modified nucleotides include those having a 4' to 2' bridged bicyclic sugar moiety. In certain embodiments, each of the 2'-substituent groups is, independently, alkoxy, substituted alkoxy, or halogen. In certain embodiments, each of the 2'-substituent groups is OCH₂CH₂OCH₃ (2'-MOE).

In certain embodiments, short antisense compounds have one or more sugar-modified nucleotides comprising a bridge between the 4' and 2' position of the sugar, wherein each of said bridges independently comprises from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
wherein
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂ The-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one aspect, each of said bridges is, independently, -[C(R₁)(R₂)]ₙ-, -(C(R₁)(R₂)]ₙ-O-, -C(R₁R₂)-N(R₁)-O- or -C(R₁R₂)-O-N(R₁)-. In another aspect, each of said bridges is, independently, 4'-(CH₂)₃-2', 4'-(CH₂)₂-2', 4'-CH₂-O-2', 4'-(CH₂)₂-O-2', 4'-CH₂-O-N(R₁)-2' and 4-CH₂-N(R₁)-O-2'- wherein each R, is, independently, H, a protecting group or C₁-C₁₂ alkyl.

In certain embodiment disclosed herein are short antisense compounds useful in the reduction of targets and/or target RNAs associated with disease states in animals. In certain embodiments, disclosed are methods of using the short antisense compounds for reducing expression of a target RNA in an animal. In certain embodiments, disclosed herein is the use of a short antisense compound in the disclosed of a medicament for the treatment of a metabolic disorder in an animal. In certain embodiments, disclosed herein is the use of a short antisense compound in the preparation of a medicament for increasing insulin sensitivity, decreasing blood glucose or decreasing HbA_{1c} in an animal. Also disclosed is the use of a short antisense compound in the preparation of a medicament for decreasing total serum cholesterol, serum LDL, serum VLDL, serum HDL, serum triglycerides, serum apolipoprotein(a) or free fatty acids in an animal.

In certain embodiments, short antisense compounds disclosed herein exhibit equal or increased potency with regard to target RNA knockdown as compared to longer parent antisense oligonucleotide at least 20 nucleotides in length. In certain embodiments, short antisense compounds exhibit a faster onset of action (target RNA reduction) as compared to the parent antisense oligonucleotide. In certain embodiments, increased potency is in the kidney. In certain embodiments, target RNA is predominately expressed in the kidney. In certain embodiments, increased potency is in the liver. In certain embodiments, target RNA is predominately expressed in the liver.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### A. Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, chemical analysis, pharmaceutical preparation, formulation and delivery, and treatment of subjects. Certain such techniques and procedures may be found for example in "Carbohydrate Modifications in Antisense Research" Edited by Sangvi and Cook, American Chemical Society, Washington D.C., 1994; and "Remington's Pharmaceutical Sciences," Mack Publishing Co., Easton, Pa., 18th edition, 1990.

Unless otherwise indicated, the following terms have the following meanings:

As used herein, the term "nucleoside" means a glycosylamine comprising a nucleobase and a sugar. Nucleosides includes, but are not limited to, naturally occurring nucleosides, abasic nucleosides, modified nucleosides, and nucleosides having mimetic bases and/or sugar groups.

As used herein, the term "nucleotide" refers to a glycosomine comprising a nucleobase and a sugur having a phosphate group covalently linked to the sugar. Nucleotides may be modified with any of a variety of substituents.

As used herein, the term "nucleobase" refers to the base portion of a nucleoside or nucleotide. A nucleobase may comprise any atom or group of atoms capable of hydrogen bonding to a base of another nucleic acid.

As used herein, the term "heterocyclic base moiety," refers to a nucleobase comprising a heterocycle.

As used herein, the term "deoxyribonucleotide" means a nucleotide having a hydrogen at the 2' position of the sugar portion of the nucleotide. Deoxyribonucleotides may be modified with any of a variety of substituents.

As used herein, the term "ribonucleotide" means a nucleotide having a hydroxy at the 2' position of the sugar portion of the nucleotide. Ribonucleotides may be modified with any of a variety of substituents.

As used herein, the term "oligomeric compound" refers to a polymeric structure comprising two or more sub-structures and capable of hybridizing to a region of a nucleic acid molecule. In certain embodiments, oligomeric compounds are oligonucleosides. In certain embodiments, oligomeric compounds are oligonucleotides. In certain embodiments, oligomeric compounds are antisense compounds. In certain embodiments, oligomeric compounds are antisense oligonucleotides. In certain embodiments, oligomeric compounds are short antisense compounds. In certain embodiments, oligomeric compounds are short antisense oligonucleotides. In certain embodiments, oligomeric compounds are chimeric oligonucleotides.

As used herein, the term "monomer" refers to a single unit of an oligomer. Monomers include, but are not limited to, nucleosides and nucleotides, whether naturally occuring or modified.

As used herein "oligonucleoside" refers to an oligonucleotide in which the internucleoside linkages do not contain a phosphorus atom.

As used herein, the term "oligonucleotide" refers to an oligomeric compound comprising a plurality of linked nucleotides. In certain embodiment, one or more nucleotides of an oligonucleotide is modified. In certain embodiments, an oligonucleotide comprises ribonucleic acid (RNA) or deoxyribonucleic acid (DNA).

In certain embodiments, oligonucleotides are composed of naturally- and/or non-naturally-occurring nucleobases, sugars and covalent internucleotide linkages, and may further include non-nucleic acid conjugates.

As used herein "internucleotide linkage" refers to a covalent linkage between adjacent nucleotides.

As used herein, the term "monomeric linkage" refers to a covalent linkage between two monmers. Monomeric linkages include, but are not limited to internucleotide linkages and internucleoside linkages.

As used herein "naturally occuring internucleotide linkage" refers to a 3' to 5' phosphodiester linkage.

As used herein, the term "antisense compound" refers to an oligomeric compound that is at least partially complementary to a target nucleic acid molecule to which it hybridizes. In certain embodiments, an antisense compound modulates (increases or decreases) expression of a target nucleic acid. Antisense compounds include, but are not limited to, compounds that are oligonucleotides, oligonucleosides, oligonucleotide analogs, oligonucleotide mimetics, and chimeric combinations of these. Consequently, while all antisense compounds are oligomeric compounds, not all oligomeric compounds are antisense compounds.

As used herein, the term "antisense oligonucleotide" refers to an antisense compound that is an oligonucleotide.

As used herein, the term "parent antisense oligonucleotide" refers to an oligonucleotide 20 nucleotides in length having a deoxy gap region having ten 2'-deoxyribonucleotides, flanked by a first and a second wing region each having five 2'-O-(2-methoxyethyl) ribonucleotides (a 5-10-5 MOE gapmer) and comprising the sequence of the corresponding short antisense compound to which it is a parent.

As used herein, the term "short antisense compound" refers to an antisense compound about 8, 9, 10, 11, 12, 13, 14, 15 or 16 monomers in length. In certain embodiments, a short antisense compound has at least one high-affinity modification.

As used herein, the term "short antisense oligonucleotide" or refers to an antisense oligonucleotide about 8, 9, 10, 11, 12, 13, 14, 15 or 16 nucleotides in length. In certain embodiments, a short antisense oligonucleotide has at least one high-affinity modification.

As used herein, the term "short gapmer" refers to a short antisense oligonucleotide having a first and a second wing region each independently 1 to 3 nucleotides in length and a gap region 2 to 14 nucleobase in length.

As used herein, the term "motif" refers to the pattern of unmodified and modified nucleotides in a short antisense compound

As used herein, the term "chimeric antisense oligomer" refers to an antisense oligomeric compound, having at least one sugar, nucleobase or internucleoside linkage that is differentially modified as compared to at least on other sugar, nucleobase or internucleoside linkage within the same antisense oligomeric compound. The remainder of the sugars, nucleobases and internucleoside linkages can be independently modified or unmodified, the same or different.

As used herein, the term "chimeric antisense oligonucleotide" refers to an antisense oligonucleotide, having at least one sugar, nucleobase or internucleoside linkage that is differentially modified as compared to at least on other sugar, nucleobase or internucleoside linkage within the same antisense oligonucleotide. The remainder of the sugars, nucleobases and internucleoside linkages can be independently modified or unmodified, the same or different.

As used herein, the term "mixed-backbone antisense oligonucleotide" refers to an antisense oligonucleotide wherein at least one internucleoside linkage of the antisense oligonucleotide is different from at least one other internucleotide linkage of the antisense oligonucleotide.

As used herein, the term "target" refers to a protein, the modulation of which is desired.

As used herein, the term "target gene" refers to a gene encoding a target.

As used herein, the terms "target nucleic acid" and "nucleic acid molecule encoding a target" refer to any nucleic acid molecule the expression or activity of which is capable of being modulated by an antisense compound. Target nucleic acids include, but are not limited to, RNA (including, but not limited to pre-mRNA and mRNA or portions thereof) transcribed from DNA encoding a target, and also cDNA derived from such RNA, and miRNA. For example, the target nucleic acid can be a cellular gene (or mRNA transcribed from the gene) whose expression is associated with a particular disorder or disease state, or a nucleic acid molecule from an infectious agent.

As used herein, the term "targeting" or "targeted to" refers to the association of an antisense compound to a particular target nucleic acid molecule or a particular region of nucleotides within a target nucleic acid molecule.

As used herein, the term "5' target site" refers to the nucleotide of a target nucleic acid which is complementary to the 5'-most nucleotide of a particular antisense compound.

As used herein, the term "3' target site" refers to the nucleotide of a target nucleic acid which is complementary to the 3'-most nucleotide of a particular antisense compound.

As used herein, the term "target region," refers to a portion of a target nucleic acid to which one or more antisense compounds is complementary.

As used herein, the term "target segment" refers to a smaller or sub-portions of a region within a target nucleic acid.

As used herein, the term "nucleobase complementarity" refers to a nucleobase that is capable of base pairing with another nucleobase. For example, in DNA, adenine (A) is complementary to thymine (T).

For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

As used herein, the term "non-complementary nucleobase" refers to a pair of nucleobases that do not form hydrogen bonds with one another or otherwise support hybridization.

As used herein, the term "complementary" refers to the capacity of an oligomeric compound to hybridize to another oligomeric compound or nucleic acid through nucleobase complementarity. In certain embodiments, an antisense compound and its target are complementary to each other when a sufficient number of corresponding positions in each molecule are occupied by nucleobases that can bond with each other to allow stable association between the antisense compound and the target. One skilled in the art recognizes that the inclusion of mismatches is possible without eliminating the ability of the oligomeric compounds to remain in association. Therefore, described herein are antisense compounds that may comprise up to about 20% nucleotides that are mismatched (i.e., are not nucleobase complementary to the corresponding nucleotides of the target). Preferably the antisense compounds contain no more than about 15%, more preferably not more than about 10%, most preferably not more than 5% or no mismatches. The remaining nucleotides are nucleobase complementary or otherwise do not disrupt hybridization (e.g., universal bases). One of ordinary skill in the art would recognize the compounds provided herein are at least 80%, at least 85%, at least 90%, at least 95%, at least 96%, at least 97%, at least 98%, at least 99% or 100% complementary to a target nucleic acid.

As used herein, the term "mismatch" refers to a non-complementary nucleobase within a complementary oligomeric compound.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymidine and uracil which pair through the formation of hydrogen bonds. The natural base guanine is nucleobase complementary to the natural bases cytosine and 5-methyl cytosine. Hybridization can occur under varying circumstances.

As used herein, the term "specifically hybridizes" refers to the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "designing" or "designed to" refer to the process of designing an oligomeric compound that specifically hybridizes with a selected nucleic acid molecule.

As used herein, the term "modulation" refers to a perturbation of function or activity when compared to the level of the function or activity prior to modulation. For example, modulation includes the change, either an increase (stimulation or induction) or a decrease (inhibition or reduction) in gene expression. As further example, modulation of expression can include perturbing splice site selection of pre-mRNA processing.

As used herein, the term "expression" refers to all the functions and steps by which a gene's coded information is converted into structures present and operating in a cell. Such structures include, but are not limited to the products of transcription and translation.

As used herein, "variant" refers to an alternative RNA transcript that can be produced from the same genomic region of DNA. Variants include, but are not limited to "pre-mRNA variants" which are transcripts produced from the same genomic DNA that differ from other transcripts produced from the same genomic DNA in either their start or stop position and contain both intronic and exonic sequence. Variants also include, but are not limited to, those with alternate splice junctions, or alternate initiation and termination codons.

As used herein, "high-affinity modified monomer" refers to a monomer having at least one modified nucleobase, internucleoside linkage or sugar moiety, when compared to naturally occurring monomers, such that the modification increases the affinity of an antisense compound comprising the high-affinity modified monomer to its target nucleic acid. High-affinity modifications include, but are not limited to, monomers (e.g., nucleosides and nucleotides) comprising 2'-modifed sugars.

As used herein, the term "2'-modified" or "2'-substituted" means a sugar comprising substituent at the 2' position other than H or OH. 2'-modified monomers, include, but are not limited to, BNA's and monomers (e.g., nucleosides and nucleotides) with 2'- substituents, such as allyl, amino, azido, thio, O-allyl, O-C₁-C₁₀ alkyl, -OCF₃, O-(CH₂)₂O-CH₃, 2'-O(CH₂)₂SCH₃, O-(CH₂)₂-O-N(Rₘ)(Rₙ), or O-CH₂-C(=O)-N(Rm)(Rn), where each Rₘ and Rₙ is, independently, H or substituted or unsubstituted C₁-C₁₀ alkyl. In certain embodiments, short antisense compounds comprise a 2'modified monomer that does not have the formula 2'-O(CH₂)ₙH, wherein n is one to six. In certain embodiments, short antisense compounds comprise a 2'modified monomer that does not have the formula 2'-OCH₃. In certain embodiments, short antisense compounds comprise a 2'modified monomer that does not have the formula or, in the alternative, 2'-O(CH₂)₂OCH₃.

As used herein, the term "bicyclic nucleic acid" or "BNA" or "bicyclic nucleoside" or "bicyclic nucleotide" refers to a nucleoside or nucleotide wherein the furanose portion of the nucleoside includes a bridge connecting two carbon atoms on the furanose ring, thereby forming a bicyclic ring system.

As used herein, unless otherwise indicated, the term "methyleneoxy BNA" alone refers to β-D-methyleneoxy BNA.

As used herein, the term "MOE" refers to a 2'-methoxyethyl substituent.

As used herein, the term "gapmer" refers to a chimeric oligomeric compound comprising a central region (a "gap") and a region on either side of the central region (the "wings"), wherein the gap comprises at least one modification that is different from that of each wing. Such modifications include nucleobase, monomeric linkage, and sugar modifications as well as the absence of modification (unmodified). Thus, in certain embodiments, the nucleotide linkages in each of the wings are different than the nucleotide linkages in the gap. In certain embodiments, each wing comprises nucleotides with high affinity modifications and the gap comprises nucleotides that do not comprise that modification. In certain embodiments the nucleotides in the gap and the nucleotides in the wings all comprise high affinity modifications, but the high affinity modifications in the gap are different than the high affinity modifications in the wings. In certain embodiments, the modifications in the wings are the same as one another. In certain embodiments, the modifications in the wings are different from each other. In certain embodiments, nucleotides in the gap are unmodified and nucleotides in the wings are modified. In certain embodiments, the modification(s) in each wing are the same. In certain embodiments, the modification(s) in one wing are different from the modification(s) in the other wing. In certain embodiments, short antisense compounds are gapmers having 2'-deoxynucleotides in the gap and nucleotides with high-affinity modifications in the wing.

As used herein, the term "prodrug" refers to a therapeutic agent that is prepared in an inactive form that is converted to an active form (i.e., drug) within the body or cells thereof by the action of endogenous enzymes or other chemicals and/or conditions.

As used herein, the term "pharmaceutically acceptable salts" refers to salts of active compounds that retain the desired biological activity of the active compound and do not impart undesired toxicological effects thereto.

As used herein, the term "cap structure" or "terminal cap moiety" refers to chemical modifications, which have been incorporated at either terminus of an antisense compound.

As used herein, the term "prevention" refers to delaying or forestalling the onset or development of a condition or disease for a period of time from hours to days, preferably weeks to months.

As used herein, the term "amelioration" refers to a lessening of at least one indicator of the severity of a condition or disease. The severity of indicators may be determined by subjective or objective measures which are known to those skilled in the art.

As used herein, the term "treatment" refers to administering a composition of the invention to effect an alteration or improvement of the disease or condition. Prevention, amelioration, and/or treatment may require administration of multiple doses at regular intervals, or prior to onset of the disease or condition to alter the course of the disease or condition. Moreover, a single agent may be used in a single individual for each prevention, amelioration, and treatment of a condition or disease sequentially, or concurrently.

As used herein, the term "pharmaceutical agent" refers to a substance provides a therapeutic benefit when administered to a subject.

As used herein, the term "therapeutically effective amount" refers to an amount of a pharmaceutical agent that provides a therapeutic benefit to an animal.

As used herein, "administering" means providing a pharmaceutical agent to an animal, and includes, but is not limited to administering by a medical professional and self administering.

As used herein, the term "co-administration" refers to administration of two or more pharmaceutical agents to an animal. The two or more pharmaceutical agents may be in a single pharmaceutical composition, or may be in separate pharmaceutical compositions. Each of the two or more pharmaceutical agents may be administered through the same or different routes of administration. Co-administration encompasses administration in parallel or sequentially.

As used herein, the term "pharmaceutical composition" refers to a mixture of substances suitable for administering to an individual. For example, a pharmaceutical composition may comprise an antisense oligonucleotide and a sterile aqueous solution.

As used herein, the term "individual" refers to a human or non-human animal selected for treatment or therapy.

As used herein, the term "animal" refers to a human or non-human animal, including, but not limited to, mice, rats, rabbits, dogs, cats, pigs, and non-human primates, including, but not limited to, monkeys and chimpanzees.

As used herein, the term "subject" refers to an animal, including, but not limited to a human, to whom a pharmaceutical composition is administered.

As used herein, the term "duration" refers to the period of time during which an activity or event continues. In certain embodiments, the duration of treatment is the period of time during which doses of a pharmaceutical agent are administered.

As used herein, the term "parenteral administration," refers to administration through injection or infusion. Parenteral administration includes, but is not limited to, subcutaneous administration, intravenous administration, or intramuscular administration.

As used herein, the term "subcutaneous administration" refers to administration just below the skin. "Intravenous administration" means administration into a vein.

As used herein, the term "dose" refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual.

As used herein, the term "dosage unit" refers to a form in which a pharmaceutical agent is provided. In certain embodiments, a dosage unit is a vial comprising lyophilized antisense oligonucleotide. In certain embodiments, a dosage unit is a vial comprising reconstituted antisense oligonucleotide.

As used herein, the term "pharmaceutical agent" refers to a substance provides a therapeutic benefit when administered to an individual. For example, in certain embodiments, an antisense oligonucleotide is a pharmaceutical agent.

As used herein, the term "active pharmaceutical ingredient" refers to the substance in a pharmaceutical composition that provides a desired effect.

As used herein, the term "therapeutically effective amount" refers to an amount of a pharmaceutical agent that provides a therapeutic benefit to an individual. In certain embodiments, a therapeutically effective amount of an antisense compound is the amount that needs to be administered to result in an observable benefit.

As used herein, the term "hypercholesterolemia" refers to a condition characterized by elevated serum cholesterol.

As used herein, the term "hyperlipidemia" refers to a condition characterized by elevated serum lipids.

As used herein, the term "hypertriglyceridemia" refers to a condition characterized by elevated triglyceride levels.

As used herein, the term "non-familial hypercholesterolemia" refers to a condition characterized by elevated cholesterol that is not the result of a single inherited gene mutation.

As used herein, the term "polygenic hypercholesterolemia" refers to a condition characterized by elevated cholesterol that results from the influence of a variety of genetic factors. In certain embodiments, polygenic hypercholesterolemia may be exacerbated by dietary intake of lipids.

As used herein, the term "familial hypercholesterolemia (FH)" refers to an autosomal dominant metabolic disorder characterized by a mutation in the LDL-receptor (LDL-R) gene, markedly elevated LDL-C and premature onset of atherosclerosis. A diagnosis of familial hypercholesterolemia is made when a individual meets one or more of the following criteria: genetic testing confirming 2 mutated LDL-receptor genes; genetic testing confirming one mutated LDL-receptor gene; document history of untreated serum LDL-cholesterol greater than 500 mg/dL; tendinous and/or cutaneous xanthoma prior to age 10 years; or, both parents have documented elevated serum LDL-cholesterol prior to lipid-lowering therapy consistent with heterozygous familial hypercholesterolemia.

As used herein, the term "homozygous familial hypercholesterolemia" or "HoFH" refers to a condition characterized by a mutation in both maternal and paternal LDL-R genes.

As used herein, the term "heterozygous familial hypercholesterolemia" or "HeFH" refers to a condition characterized by a mutation in either the maternal or paternal LDL-R gene.

As used herein, the term "mixed dyslipidemia" refers to a condition characterized by elevated serum cholesterol and elevated serum triglycerides.

As used herein, the term "diabetic dyslipidemia" or "Type II diabetes with dyslipidemia" refers to a condition characterized by Type II diabetes, reduced HDL-C, elevated serum triglycerides, and elevated small, dense LDL particles.

As used herein, the term "CHD risk equivalents," refers to indicators of clinical atherosclerotic disease that confer a high risk for coronary heart disease. For example, in certain embodiments, CHD risk equivalents include, without limitation, clinical coronary heart disease, symptomatic carotid artery disease, peripheral arterial disease, and/or abdominal aortic aneurysm.

As used herein, the term "non-alcoholic fatty liver disease (NAFLD)" refers to a condition characterized by fatty inflammation of the liver that is not due to excessive alcohol use (for example, alcohol consumption of over 20 g/day). In certain embodiments, NAFLD is related to insulin resistance and the metabolic syndrome.

As used herein, the term "non-alcoholic steatohepatitis (NASH)" refers to a condition characterized by inflammation and the accumulation of fat and fibrous tissue in the liver, that is not due to excessive alcohol use. NASH is an extreme form of NAFLD.

As used herein, the term "major risk factors" refers to factors that contribute to a high risk for a particular disease or condition. In certain embodiments, major risk factors for coronary heart disease include, without limitation, cigarette smoking, hypertension, low HDL-C, family history of coronary heart disease, and age.

As used herein, the term "CHD risk factors" refers to CHD risk equivalents and major risk factors.

As used herein, the term "coronary heart disease (CHD)" refers to a narrowing of the small blood vessels that supply blood and oxygen to the heart, which is often a result of atherosclerosis.

As used herein, the term "reduced coronary heart disease risk" refers to a reduction in the likelihood that a individual will develop coronary heart disease. In certain embodiments, a reduction in coronary heart disease risk is measured by an improvement in one or more CHD risk factors, for example, a decrease in LDL-C levels.

As used herein, the term "atherosclerosis" refers to a hardening of the arteries affecting large and medium-sized arteries and is characterized by the presence of fatty deposits. The fatty deposits are called "atheromas" or "plaques," which consist mainly of cholesterol and other fats, calcium and scar tissue, and damage the lining of arteries.

As used herein, the term "history of coronary heart disease" refers to the occurrence of clinically evident coronary heart disease in the medical history of a individual or a individual's family member.

As used herein, the term "Early onset coronary heart disease" refers to a diagnosis of coronary heart disease prior to age 50.

As used herein, the term "statin intolerant individual" refers to a individual who as a result of statin therapy experiences one or more of creatine kinase increases, liver function test abnormalities, muscle aches, or central nervous system side effects.

As used herein, the term "efficacy" refers to the ability to produce a desired effect. For example, efficacy of a lipid-lowering therapy may be reduction in the concentration of one or more of LDL-C, VLDL-C, IDL-C, non-HDL-C, ApoB, lipoprotein(a), or triglycerides.

As used herein, the term "acceptable safety profile" refers to a pattern of side effects that is within clinically acceptable limits.

As used herein, the term "side effects" refers to physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies. For example, increased aminotransferase levels in serum may indicate liver toxicity or liver function abnormality. For example, increased bilirubin may indicate liver toxicity or liver function abnormality.

As used herein, the term "injection site reaction" refers to inflammation or abnormal redness of skin at a site of injection in an individual.

As used herein, the term "individual compliance" refers to adherence to a recommended or prescribed therapy by an individual.

As used herein, the term "lipid-lowering therapy" refers to a therapeutic regimen provided to a individual to reduce one or more lipids in a individual. In certain embodiments, a lipid-lowering therapy is provide to reduce one or more of ApoB, total cholesterol, LDL-C, VLDL-C, IDL-C, non-HDL-C, triglycerides, small dense LDL particles, and Lp(a) in an individual.

As used herein, the term "lipid-lowering agent" refers to a pharmaceutical agent provided to a individual to achieve a lowering of lipids in the individual. For example, in certain embodiments, a lipid-lowering agent is provided to an individual to reduce one or more of ApoB, LDL-C, total cholesterol, and triglycerides.

As used herein, the term "LDL-C target" refers to an LDL-C level that is desired following lipid-lowering therapy.

As used herein, the term "comply" refers to the adherence with a recommended therapy by an individual.

As used herein, the term "recommended therapy" refers to a therapeutic regimen recommended by a medical professional for the treatment, amelioration, or prevention of a disease.

As used herein, the term "low LDL-receptor activity" refers to LDL-receptor activity that is not sufficiently high to maintain clinically acceptable levels of LDL-C in the bloodstream.

As used herein, the term "cardiovascular outcome" refers to the occurrence of major adverse cardiovascular events.

As used herein, the term "improved cardiovascular outcome" refers to a reduction in the occurrence of major adverse cardiovascular events, or the risk thereof. Examples of major adverse cardiovascular events include, without limitation, death, reinfarction, stroke, cardiogenic shock, pulmonary edema, cardiac arrest, and atrial dysrhythmia.

As used herein, the term "surrogate markers of cardiovascular outcome" refers to indirect indicators of cardiovascular events, or the risk thereof. For example, surrogate markers of cardiovascular outcome include carotid intimal media thickness (CIMT). Another example of a surrogate marker of cardiovascular outcome includes atheroma size. Atheroma size may be determined by intravascular ultrasound (IVUS).

As used herein, the term "increased HDL-C" refers to an increase in serum HDL-C in an individual over time.

As used herein, the term "lipid-lowering" refers to a reduction in one or more serum lipids in an individual over time.

As used herein, the term "metabolic disorder" refers to a condition characterized by an alteration or disturbance in metabolic function. "Metabolic" and "metabolism" are terms well know in the art and generally include the whole range of biochemical processes that occur within a living organism. Metabolic disorders include, but are not limited to, hyperglycemia, prediabetes, diabetes (type I and type II), obesity, insulin resistance and metabolic syndrome.

As used herein, the term "metabolic syndrome" refers to a clustering of lipid and non-lipid cardiovascular risk factors of metabolic origin. It has been closely linked to the generalized metabolic disorder known as insulin resistance. The National Cholesterol Education Program (NCEP) Adult Treatment Panel III (ATPIII) established criteria for diagnosis of metabolic syndrome when three or more of five risk determinants are present. The five risk determinants are abdominal obesity defined as waist circumference of greater than 102 cm for men or greater than 88cm for women, triglyceride levels greater than or equal to 150 mg/dL, HDL cholesterol levels of less than 40 mg/dL for men and less than 50 mg/dL for women, blood pressure greater than or equal to 130/85 mm Hg and fasting glucose levels greater than or equal to 110 mg/dL. These determinants can be readily measured in clinical practice (JAMA, 2001, 285: 2486-2497).

The term "alkyl," as used herein, refers to a saturated straight or branched hydrocarbon radical containing up to twenty four carbon atoms. Examples of alkyl groups include, but are not limited to, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred. The term "lower alkyl" as used herein includes from 1 to about 6 carbon atoms. Alkyl groups as used herein may optionally include one or more further substituent groups.

The term "alkenyl," as used herein, refers to a straight or branched hydrocarbon chain radical containing up to twenty four carbon atoms and having at least one carbon-carbon double bond. Examples of alkenyl groups include, but are not limited to, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

The term "alkynyl," as used herein, refers to a straight or branched hydrocarbon radical containing up to twenty four carbon atoms and having at least one carbon-carbon triple bond. Examples of alkynyl groups include, but are not limited to, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substitutent groups.

The term "aminoalkyl" as used herein, refers to an amino substituted alkyl radical. This term is meant to include C₁-C₁₂ alkyl groups having an amino substituent at any position and wherein the alkyl group attaches the aminoalkyl group to the parent molecule. The alkyl and/or amino portions of the aminoalkyl group can be further substituted with substituent groups.

The term "aliphatic," as used herein, refers to a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation polyalkoxys, such as polyalkylene glycols, polyamines, and polyimines. Aliphatic groups as used herein may optionally include further substitutent groups.

The term "alicyclic" or "alicyclyl" refers to a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substitutent groups.

The term "alkoxy," as used herein, refers to a radical formed between an alkyl group and an oxygen atom wherein the oxygen atom is used to attach the alkoxy group to a parent molecule. Examples of alkoxy groups include, but are not limited to, methoxy, ethoxy, propoxy, isopropoxy, n-butoxy, sec-butoxy, *tert-*butoxy, n-pentoxy, neopentoxy, n-hexoxy and the like. Alkoxy groups as used herein may optionally include further substitutent groups.

The terms "halo" and "halogen," as used herein, refer to an atom selected from fluorine, chlorine, bromine and iodine.

The terms "aryl" and "aromatic," as used herein, refer to a mono- or polycyclic carbocyclic ring system radicals having one or more aromatic rings. Examples of aryl groups include, but are not limited to, phenyl, naphthyl, tetrahydronaphthyl, indanyl, idenyl and the like. Preferred aryl ring systems have from about 5 to about 20 carbon atoms in one or more rings. Aryl groups as used herein may optionally include further substitutent groups.

The terms "aralkyl" and "arylalkyl," as used herein, refer to a radical formed between an alkyl group and an aryl group wherein the alkyl group is used to attach the aralkyl group to a parent molecule. Examples include, but are not limited to, benzyl, phenethyl and the like. Aralkyl groups as used herein may optionally include further substitutent groups attached to the alkyl, the aryl or both groups that form the radical group.

The term "heterocyclic radical" as used herein, refers to a radical mono-, or poly-cyclic ring system that includes at least one heteroatom and is unsaturated, partially saturated or fully saturated, thereby including heteroaryl groups. Heterocyclic is also meant to include fused ring systems wherein one or more of the fused rings contain at least one heteroatom and the other rings can contain one or more heteroatoms or optionally contain no heteroatoms. A heterocyclic group typically includes at least one atom selected from sulfur, nitrogen or oxygen. Examples of heterocyclic groups include, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, quinoxalinyl, pyridazinonyl, tetrahydrofuryl and the like. Heterocyclic groups as used herein may optionally include further substitutent groups.

The terms "heteroaryl," and "heteroaromatic," as used herein, refer to a radical comprising a mono- or poly-cyclic aromatic ring, ring system or fused ring system wherein at least one of the rings is aromatic and includes one or more heteroatom. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include, but are not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like. Heteroaryl radicals can be attached to a parent molecule directly or through a linking moiety such as an aliphatic group or hetero atom. Heteroaryl groups as used herein may optionally include further substitutent groups.

The term "heteroarylalkyl," as used herein, refers to a heteroaryl group as previously defined having an alky radical that can attach the heteroarylalkyl group to a parent molecule. Examples include, but are not limited to, pyridinylmethyl, pyrimidinylethyl, napthyridinylpropyl and the like. Heteroarylalkyl groups as used herein may optionally include further substitutent groups on one or both of the heteroaryl or alkyl portions.

The term "mono or poly cyclic structure" as used in the present invention includes all ring systems that are single or polycyclic having rings that are fused or linked and is meant to be inclusive of single and mixed ring systems individually selected from aliphatic, alicyclic, aryl, heteroaryl, aralkyl, arylalkyl, heterocyclic, heteroaryl, heteroaromatic, heteroarylalkyl. Such mono and poly cyclic structures can contain rings that are uniform or have varying degrees of saturation including fully saturated, partially saturated or fully unsaturated. Each ring can comprise ring atoms selected from C, N, O and S to give rise to heterocyclic rings as well as rings comprising only C ring atoms which can be present in a mixed motif such as for example benzimidazole wherein one ring has only carbon ring atoms and the fused ring has two nitrogen atoms. The mono or poly cyclic structures can be further substituted with substituent groups such as for example phthalimide which has two =O groups attached to one of the rings. In another aspect, mono or poly cyclic structures can be attached to a parent molecule directly through a ring atom, through a substituent group or a bifunctional linking moiety.

The term "acyl," as used herein, refers to a radical formed by removal of a hydroxyl group from an organic acid an d has the general formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substitutent groups.

The term "hydrocarbyl" includes groups comprising C, O and H. Included are straight, branched and cyclic groups having any degree of saturation. Such hydrocarbyl groups can include one or more heteroatoms selected from N, O and S and can be further mono or poly substituted with one or more substituent groups.

The terms "substituent" and "substituent group," as used herein, include groups that are typically added to other groups or parent compounds to enhance desired properties or give desired effects. Substituent groups can be protected or unprotected and can be added to one available site or to many available sites in a parent compound. Substituent groups may also be further substituted with other substituent groups and may be attached directly or via a linking group such as an alkyl or hydrocarbyl group to a parent compound. Such groups include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxo (-O-Rₐₐ), aryl, aralkyl, heterocyclic, heteroaryl, heteroarylalkyl, amino (-NR_{bb}R_{cc}), imino(=NR_{bb}), amido (-C(O)NR_{bb}R_{cc} or -N(R_{bb})C(O)Rₐₐ), azido (-N₃), nitro (-NO₂), cyano (-CN), carbamido (-OC(O)NR_{bb}R_{cc} or -N(R_{bb})C(O)ORₐₐ), ureido (-N(R_{bb})C(O)NR_{bb}R_{cc}), thioureido (-N(R_{bb})C(S)NR_{bb}R_{cc}), guanidinyl (-N(R_{bb})C(=NR_{bb})NR_{bb}R_{cc}), amidinyl (-C(=NR_{bb})NR_{bb}R_{cc} or -N(R_{bb})C(NR_{bb})Rₐₐ), thiol (-SR_{bb}), sulfinyl (-S(O)R_{bb}), sulfonyl (-S(O)₂R_{bb}), sulfonamidyl (-S(O)₂NR_{bb}R_{cc} or -N(R_{bb})S(O)₂R_{bb}) and conjugate groups. Wherein each Rₐₐ, R_{bb} and R_{cc} is, independently, H, an optionally linked chemical functional group or a further substituent group with a preferred list including without limitation H, alkyl, alkenyl, alkynyl, aliphatic, alkoxy, acyl, aryl, aralkyl, heteroaryl, alicyclic, heterocyclic and heteroarylalkyl.

### B. Certain Oligomeric Compounds

In certain embodiments, it is desirable to chemically modify oligomeric compounds, compared to naturally occuring oligomers, such as DNA or RNA. Certain such modifications alter the activity of the oligomeric compound. Certain such chemical modifications can alter activity by, for example: increasing affinity of an antisense compound for its target nucleic acid, increasing its resistance to one or more nucleases, and/or altering the pharmacokinetics or tissue distribution of the oligomeric compound. In certain instances, the use of chemistries that increase the affinity of an oligomeric compound for its target can allow for the use of shorter oligomeric compounds.

### 1. Certain monomers

In certain embodiment, oligomeric compounds comprise one or more modified monomer. In certain such embodiments, oligomeric compounds comprise one or more high affinity monomer. In certain embodiments, such high-affinity monomer is selected from monomers (e.g., nucleosides and nucleotides) comprising 2'-modifed sugars, including, but not limited to: BNA's and monomers (e.g., nucleosides and nucleotides) with 2'- substituents such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl.

In certain embodiments, the oligomeric compounds including, but not limited to short antisense compounds of the present disclosure comprise one or more high affinity monomers provided that the oligomeric compound does not comprise a nucleotide comprising a 2'-O(CH₂)ₙH, wherein n is one to six.

In certain embodiments, the oligomeric compounds including, but not limited to short antisense compounds of the present disclosure comprise one or more high affinity monomer provided that the oligomeric compound does not comprise a nucleotide comprising a 2'-OCH₃ or a 2'-O(CH₂)₂OCH₃.

In certain embodiments, the oligomeric compounds including, but not limited to short antisense compounds of the present disclosure, comprise one or more high affinity monomer provided that the oligomeric compound does not comprise a α-L-Methyleneoxy (4'-CH₂-O-2') BNA.

In certain embodiments, the oligomeric compounds including, but not limited to short antisense compounds of the present disclosure, comprise one or more high affinity monomer provided that the oligomeric compound does not comprise a β-D-Methyleneoxy (4'-CH₂-O-2') BNA.

In certain embodiments, the oligomeric compounds including, but not limited to short antisense compounds of the present disclosure comprise one or more high affinity monomer provided that the oligomeric compound does not comprise a α-L-Methyleneoxy (4'-CH₂-O-2') BNA or a β-D-Methyleneoxy (4'-CH₂-O-2') BNA.

### a. Certain Nucleobases

The naturally occurring base portion of a nucleoside is typically a heterocyclic base. The two most common classes of such heterocyclic bases are the purines and the pyrimidines. For those nucleosides that include a pentofuranosyl sugar, a phosphate group can be linked to the 2', 3' or 5' hydroxyl moiety of the sugar. In forming oligonucleotides, those phosphate groups covalently link adjacent nucleosides to one another to form a linear polymeric compound. Within oligonucleotides, the phosphate groups are commonly referred to as forming the internucleotide backbone of the oligonucleotide. The naturally occurring linkage or backbone of RNA and of DNA is a 3' to 5' phosphodiester linkage.

In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable with the compounds described herein. In certain embodiments, a modified nucleobase is a nucleobase that is fairly similar in structure to the parent nucleobase, such as for example a 7-deaza purine, a 5-methyl cytosine, or a G-clamp.

In certain embodiments, nucleobase mimetic include more complicated structures, such as for example a tricyclic phenoxazine nucleobase mimetic. Methods for preparation of the above noted modified nucleobases are well known to those skilled in the art.

### b. Certain sugars

Oligomeric compounds described herein may comprise one or more monomer, including a nucleoside or nucleotide, having a modified sugar moiety. For example, the furanosyl sugar ring of a nucleoside can be modified in a number of ways including, but not limited to, addition of a substituent group, bridging of two non-geminal ring atoms to form a bicyclic nucleic acid (BNA).

In certain embodiments, oligomeric compounds comprise one or more monomers that is a BNA. In certain such embodiments, BNA s include, but are not limited to, (A) α-L-Methyleneoxy (4'-CH₂-O-2') BNA , (B) β-D-Methyleneoxy (4'-CH₂-O-2') BNA , (C) Ethyleneoxy (4'-(CH₂)₂-O-2') BNA , (D) Aminooxy (4'-CH-O-N(R)-2') BNA and (E) Oxyamino (4'-CH₂-N(R)-O-2') BNA, as depicted in Figure 1.

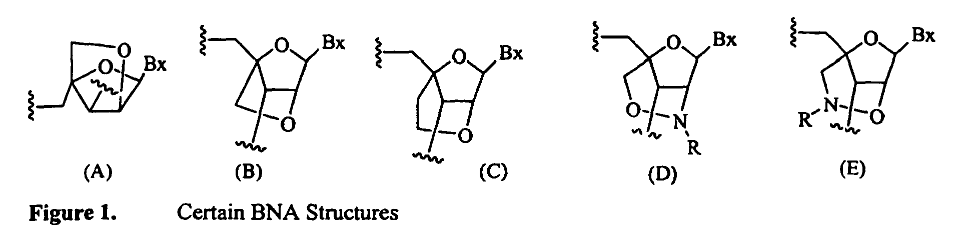

In certain embodimnents, BNA compounds include, but are not limited to, compounds having at least one bridge between the 4' and the 2' position of the sugar wherein each of the bridges independently comprises 1 or from 2 to 4 linked groups independently selected from -[C(R₁)(R₂)]ₙ-, -C(R)=C(R₂)-, -C(R₁)=N-, -C(=NR₁)-, -C(=O)-, -C(=S)-, -O-, -Si(R₁)₂-, -S(=O)ₓ- and -N(R₁)-;
wherein:
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one embodiment, each of the bridges of the BNA compounds is, independently, -[C(R₁)(R₂)]ₙ-, -[C(R₁)(R₂)]ₙ-O-, -C(R₁R₂)-N(R₁)-O- or -C(R₁R₂)-O-N(R₁)-. In another embodiment, each of said bridges is, independently, 4'-CH₂-2', 4'-(CH₂)₂-2', 4'-(CH₂)₃-2', 4'-CH₂-O-2', 4'-(CH₂)₂-O-2', 4'-CH₂-O-N(R₁)-2' and 4'-CH₂-N(R₁)-O-2'- wherein each R₁ is, independently, H, a protecting group or C₁-C₁₂ alkyl.

Certain BNA's have been prepared and disclosed in the patent literature as well as in scientific literature (Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; WO 94/14226; WO 2005/021570; Singh et al., J. Org. Chem., 1998, 63, 10035-10039; Examples of issued US patents and published applications that disclose BNA s include, for example, U.S. Patent Nos. 7,053,207; 6,268,490; 6,770,748; 6,794,499; 7,034,133; and 6,525,191; and U.S. Pre-Grant Publication Nos. 2004-0171570; 2004-0219565; 2004-0014959; 2003-0207841; 2004-0143114; and 20030082807.

Also provided herein are BNAs in which the 2'-hydroxyl group of the ribosyl sugar ring is linked to the 4' carbon atom of the sugar ring thereby forming a methyleneoxy (4'-CH₂-O-2') linkage to form the bicyclic sugar moiety (reviewed in Elayadi et al., Curr. Opinion Invens. Drugs, 2001, 2, 558-561; Braasch et al., Chem. Biol., 2001, 8 1-7; and Orum et al., Curr. Opinion Mol. Ther., 2001, 3, 239-243; see also U.S. Patents: 6,268,490 and 6,670,461). The linkage can be a methylene (-CH₂-) group bridging the 2' oxygen atom and the 4' carbon atom, for which the term methyleneoxy (4'-CH₂-O-2') BNA is used for the bicyclic moiety; in the case of an ethylene group in this position, the term ethyleneoxy (4'-CH₂CH₂-O-2') BNA is used (Singh et al., Chem. Commun., 1998, 4, 455-456: Morita et al., Bioorganic Medicinal Chemistry, 2003, 11, 2211-2226). Methyleneoxy (4'-CH₂-O-2') BNA and other bicyclic sugar analogs display very high duplex thermal stabilities with complementary DNA and RNA (Tm = +3 to +10° C), stability towards 3'-exonucleolytic degradation and good solubility properties. Potent and nontoxic antisense oligonucleotides compriseing BNAs have been described (Wahlestedt et al., Proc. Natl. Acad. Sci. U. S. A., 2000, 97, 5633-5638).

An isomer of methyleneoxy (4'-CH₂-O-2') BNA that has also been discussed is alpha-L-methyleneoxy (4'-CH₂-O-2') BNA which has been shown to have superior stability against a 3'-exonuclease. The alpha-L- methyleneoxy (4'-CH₂-O-2') BNA's were incorporated into antisense gapmers and chimeras that showed potent antisense activity (Frieden et al., Nucleic Acids Research, 2003, 21, 6365-6372).

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA, phosphorothioate- methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs compriseing oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-Amino- and 2'-methylamino-BNA's have been prepared and the thermal stability of their duplexes with complementary RNA and DNA strands has been previously reported.

Modified sugar moieties are well known and can be used to alter, typically increase, the affinity of the antisense compound for its target and/or increase nuclease resistance. A representative list of preferred modified sugars includes but is not limited to bicyclic modified sugars (BNA's), including methyleneoxy (4'-CH₂-O-2') BNA and ethyleneoxy (4'-(CH₂)2-O-2' bridge) BNA ; substituted sugars, especially 2'-substituted sugars having a 2'-F, 2'-OCH₃ or a 2'-O(CH₂)₂-OCH₃ substituent group; and 4'-thio modified sugars. Sugars can also be replaced with sugar mimetic groups among others. Methods for the preparations of modified sugars are well known to those skilled in the art. Some representative patents and publications that teach the preparation of such modified sugars include, but are not limited to, U.S. Patents: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; 5,700,920; 6,531,584; and 6,600,032; and WO 2005/121371.

In certain embodiments, BNA's include bicyclic nucleoside having the formula: 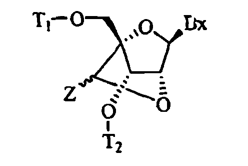 wherein:
Bx is a heterocyclic base moiety;
T₁ is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain such embodiments, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H, C₁-C₆ alkyl, or substituted C₁-C₆ alkyl and X is O or NJ₁.

In certain embodiments, the *Z* group is C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, the Z group is C₁-C₆ alkyl substituted with one or more X^{x}, wherein each X^{x} is independently halo (e.g., fluoro), hydroxyl, alkoxy (e.g., CH₃O-), substituted alkoxy or azido.

In certain embodiments, the *Z* group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, the *Z* group is -CH₂X^{x}, wherein X^{x} is halo (e.g., fluoro), hydroxyl, alkoxy (e.g., CH₃O-) or azido.

In certain such embodiments, the *Z* group is in the (R)-configuration:

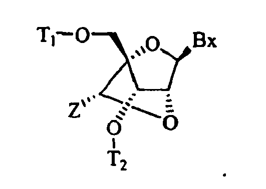

In certain such embodiments, the Z group is in the (S)-configuration:

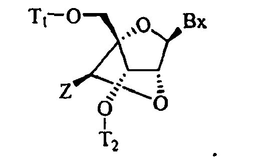

In certain embodiments, each T₁ and T₂ is a hydroxyl protecting group. A preferred list of hydroxyl protecting groups includes benzyl, benzoyl, 2,6-dichlorobenzyl, t-butyldimethylsilyl, t-butyldiphenylsilyl, mesylate, tosylate, dimethoxytrityl (DMT), 9-phenylxanthine-9-yl (Pixyl) and 9-(p-methoxyphenyl)xanthine-9-yl (MOX). In certain embodiments, T₁ is a hydroxyl protecting group selected from acetyl, benzyl, t-butyldimethylsilyl, t-butyldiphenylsilyl and dimethoxytrityl wherein a more preferred hydroxyl protecting group is T₁ is 4,4'-dimethoxytrityl.

In certain embodiments, T₂ is a reactive phosphorus group wherein preferred reactive phosphorus groups include diisopropylcyanoethoxy phosphoramidite and H-phosphonate. In certain embodiments T₁ is 4,4'-dimethoxytrityl and T₂ is diisopropylcyanoethoxy phosphoramidite.

In certain embodiments, oligomeric compounds have at least one monomer of the formula: 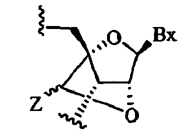 or of the formula: 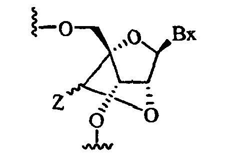 or of the formula: 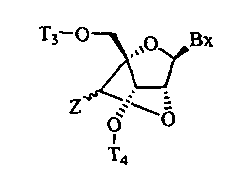 wherein
Bx is a heterocyclic base moiety;
T₃ is H, a hydroxyl protecting group, a linked conjugate group or an internucleoside linking group attached to a nucleoside, a nucleotide, an oligonucleoside, an oligonucleotide, a monomeric subunit or an oligomeric compound;
T₄ is H, a hydroxyl protecting group, a linked conjugate group or an internucleoside linking group attached to a nucleoside, a nucleotide, an oligonucleoside, an oligonucleotide, a monomeric subunit or an oligomeric compound;
wherein at least one of T₃ and T₄ is an internucleoside linking group attached to a nucleoside, a nucleotide, an oligonucleoside, an oligonucleotide, a monomeric subunit or an oligomeric compound; and
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In one embodiment, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H or C₁-C₆ alkyl, and X is O or NJ₁.

In certain such embodiments, at least one Z is C₁-C₆ alkyl or substituted C₁-C₆ alkyl. In certain embodiments, each Z is, independently, C₁-C₆ alkyl or substituted C₁-C₆ alkyl. In certain embodiments, at least one Z is C₁-C₆ alkyl. In certain embodiments, each Z is, independently, C₁-C₆ alkyl. In certain embodiments, at least one Z is methyl. In certain embodiments, each Z is methyl. In certain embodiments, at least one Z is ethyl. In certain embodiments, each Z is ethyl. In certain embodiments, at least one Z is substituted C₁-C₆ alkyl. In certain embodiments, each Z is, independently, substituted C₁-C₆ alkyl. In certain embodiments, at least one Z is substituted methyl. In certain embodiments, each Z is substituted methyl. In certain embodiments, at least one Z is substituted ethyl. In certain embodiments, each Z is substituted ethyl.

In certain embodiments, at least one substituent group is C₁-C₆ alkoxy (e.g., at least one Z is C₁-C₆ alkyl substituted with one or more C₁-C₆ alkoxy). In another embodiment, each substituent group is, independently, C₁-C₆ alkoxy (e.g., each Z is, independently, C₁-C₆ alkyl substituted with one or more C₁-C₆ alkoxy).

In certain embodiments, at least one C₁-C₆ alkoxy substituent group is CH₃O- (e.g., at least one Z is CH₃OCH₂-). In another embodiment, each C₁-C₆ alkoxy substituent group is CH₃O- (e.g., each Z is CH₃OCH₂-).

In certain embodiments, at least one substituent group is halogen (e.g., at least one Z is C₁-C₆ alkyl substituted with one or more halogen). In certain embodiments, each substituent group is, independently, halogen (e.g., each Z is, independently, C₁-C₆ alkyl substituted with one or more halogen). In certain embodiments, at least one halogen substituent group is fluoro (e.g., at least one Z is CH₂FCH₂-, CHF₂CH₂- or CF₃CH₂-). In certain embodiments, each halo substituent group is fluoro (e.g., each Z is, independently, CH₂FCH₂-, CHF₂CH₂- or CF₃CH₂-).

In certain embodiments, at least one substituent group is hydroxyl (e.g., at least one Z is C₁-C₆ alkyl substituted with one or more hydroxyl). In certain embodiments, each substituent group is, independently, hydroxyl (e.g., each Z is, independently, C₁-C₆ alkyl substituted with one or more hydroxyl). In certain embodiments, at least one Z is HOCH₂-. In another embodiment, each Z is HOCH₂-.

In certain embodiments, at least one Z is CH₃-, CH₃CH₂-, CH₂OCH₃-, CH₂F- or HOCH₂-. In certain embodiments, each Z is, independently, CH₃-, CH₃CH₂-, CH₂OCH₃-, CH₂F- or HOCH₂-.

In certain embodiments, at least one Z group is C₁-C₆ alkyl substituted with one or more X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, at least one Z group is C₁-C₆ alkyl substituted with one or more X^{x}, wherein each X^{x} is, independently, halo (e.g., fluoro), hydroxyl, alkoxy (e.g., CH₃O-) or azido.

In certain embodiments, each Z group is, independently, C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, C₁-C₆ alkyl substituted with one or more X^{x}, wherein each X^{x} is independently halo (e.g., fluoro), hydroxyl, alkoxy (e.g., CH₃O-) or azido.

In certain embodiments, at least one Z group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁ In certain embodiments, at least one Z group is -CH₂X^{x}, wherein X^{x} is halo (e.g., fluoro), hydroxyl, alkoxy (e.g., CH₃O-) or azido.

In certain embodiments, each Z group is, independently, -CH₂X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, -CH₂X^{x}, wherein each X^{x} is, independently, halo (e.g., fluoro), hydroxyl, alkoxy (e.g., CH₃O-) or azido.

In certain embodiments, at least one Z is CH₃-. In another embodiment, each Z is, CH₃-.

In certain embodiments, the Z group of at least one monomer is in the (*R*)- configuration represented by the formula: 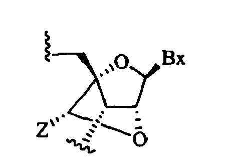 or the formula: 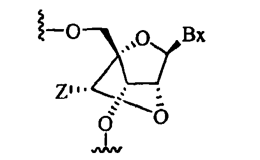 or the formula:

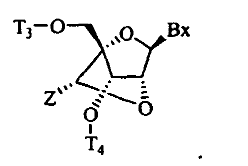

In certain embodiments, the *Z* group of each monomer of the formula is in the (*R*)- configuration.

In certain embodiments, the *Z* group of at least one monomer is in the (*S*)- configuration represented by the formula: 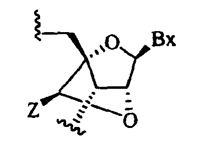 or the formula: 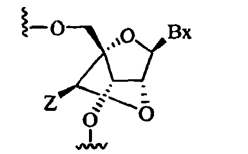 or the formula:

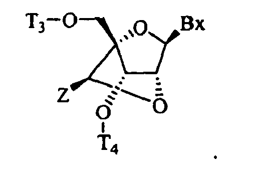

In certain embodiments, the *Z* group of each monomer of the formula is in the (*S*)- configuration.

In certain embodiments, T₃ is H or a hydroxyl protecting group. In certain embodiments, T₄ is H or a hydroxyl protecting group. In a further embodiment T₃ is an internucleoside linking group attached to a nucleoside, a nucleotide or a monomeric subunit. In certain embodiments, T₄ is an internucleoside linking group attached to a nucleoside, a nucleotide or a monomeric subunit. In certain embodiments,T₃ is an internucleoside linking group attached to an oligonucleoside or an oligonucleotide. In certain embodiments, T₄ is an internucleoside linking group attached to an oligonucleoside or an oligonucleotide. In certain embodiments, T₃ is an internucleoside linking group attached to an oligomeric compound. In certain embodiments, T₄ is an internucleoside linking group attached to an oligomeric compound. In In certain embodiments, at least one of T₃ and T₄ comprises an internucleoside linking group selected from phosphodiester or phosphorothioate.

In certain embodiments, oligomeric compounds have at least one region of at least two contiguous monomers of the formula: 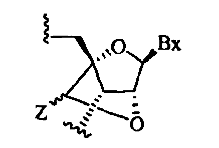 or of the formula: 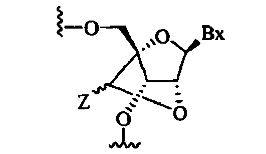 or of the formula: to

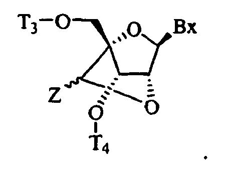

In certain embodiments, the oligomeric compound comprises at least two regions of at least two contiguous monomers of the above formula. In certain embodiments, the oligomeric compound comprises a gapped oligomeric compound. In certain embodiments, the oligmeric compound comprises at least one region of from about 8 to about 14 contiguous β-D-2'-deoxyribofuranosyl nucleosides. In certain embodiments, the oligomeric compound comprises at least one region of from about 9 to about 12 contiguous β-D-2'-deoxyribofuranosyl nucleosides.

In certain embodiments, monmers include sugar mimetics. In certain such embodiments, a mimetic is used in place of the sugar or sugar-internucleoside linkage combination, and the nucleobase is maintained for hybridization to a selected target. Representative examples of a sugar mimetics include, but are not limited to, cyclohexenyl or morpholino. Representative examples of a mimetic for a sugar-internucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14. Methods of synthesis of sugar, nucleoside and nucleobase mimetics are well known to those skilled in the art.

### 3. Monomeric Linkages

Described herein are linking groups that link monomers (including, but not limited to, modified and unmodified nucleosides and nucleotides) together, thereby forming an oligomeric compound. The two main classes of linking groups are defined by the presence or absence of a phosphorus atom. Representative phosphorus containing linkages include, but are not limited to, phosphodiesters (P=O), phosphotriesters, methylphosphonates, phosphoramidate, and phosphorothioates (P=S). Representative non-phosphorus containing linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)2-O-); and N,N'-dimethylhydrazine (-CH₂N(CH₃)-N(CH₃)-). Oligomeric compounds having non-phosphorus linking groups are referred to as oligonucleosides. Modified linkages, compared to natural phosphodiester linkages, can be used to alter, typically increase, nuclease resistance of the oligomeric compound. In certain embodiments, linkages having a chiral atom can be prepared a racemic mixtures, as separate enantomers. Representative chiral linkages include, but are not limited to, alkylphosphonates and phosphorothioates. Methods of preparation of phosphorous-containing and non-phosphorous-containing linkages are well known to those skilled in the art.

The oligomeric compounds described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids et al. Included in the antisense compounds provided herein are all such possible isomers, as well as their racemic and optically pure forms.

### 4. Oligomeric Compounds

In certain embodiments, provided herein are oligomeric compounds having reactive phosphorus groups useful for forming linkages including for example phosphodiester and phosphorothioate internucleoside linkages. Methods of preparation and/or purification of precursors or oligomeric compounds are not a limitation of the compositions or methods provided herein. Methods for synthesis and purification of oligomeric compounds including DNA, RNA, oligonucleotides, oligonucleosides, and antisense compounds are well known to those skilled in the art.

Generally, oligomeric compounds comprise a plurality of monomeric subunits linked together by linking groups. Nonlimiting examples of oligomeric compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound.

In certain embodiments, the present invention provides chimeric oligomeric compounds. In certain such embodiments, chimeric oligomeric compounds are chimeric oligonucleotides. In certain such embodiments, the chimeric oligonucleotides comprise differently modified nucleotides. In certain embodiments, chimeric oligonucleotides are mixed-backbone antisense oligonucleotides.

In general a chimeric oligomeric compound will have modified nucleosides that can be in isolated positions or grouped together in regions that will define a particular motif. Any combination of modifications and/or mimetic groups can comprise a chimeric oligomeric compound as described herein.

In certain embodiments, chimeric oligomeric compounds typically comprise at least one region modified so as to confer increased resistance to nuclease degradation, increased cellular uptake, and/or increased binding affinity for the target nucleic acid. In certain embodiments, an additional region of the oligomeric compound may serve as a substrate for enzymes capable of cleaving RNA:DNA or RNA:RNA hybrids. By way of example, RNase H is a cellular endonuclease that cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter oligomeric compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

In certain embodiments, chimeric oligomeric compounds are gapmers. In certain embodiments, chimeric compounds are short antisense compounds. In certain embodiments, short antisense compounds are gapmers. In certain such embodiments, a mixed-backbone antisense oligomer has one type of internucleotide linkages in one or both wings and a different type of internucleotide linkages in the gap. In certain such embodiments, the mixed-backbone antisense oligonucleotide has phosphodiester linkages in the wings and phosphorothioate linkages in the gap. In certain embodiments in which the internucleotide linkages in a wing is different from the internucleotide linkages in the gap, the internucleotide linkage bridging that wing and the gap is the same as the internucleotide linkage in the wing. In certain embodiments in which the internucleotide linkages in a wing is different from the internucleotide linkages in the gap, the internucleotide linkage bridging that wing and the gap is the same as the internucleotide linkage in the gap.

### C. Certain Short Antisense Compounds

Disclosed herein are short antisense compounds 8 to 16, preferably 9 to 15, more preferably 9 to 14, more preferably 10 to 14 nucleotides in length. In certain embodiments, short antisense compounds are 9 to 14 nucleotides in length. In certain embodiments, short antisense compounds are 10 to 14 nucleotides in length. In certain embodiments, such short antisense compounds are short antisense oligonucleotides.

In certain embodiments, short antisense compounds comprise one or more chemical modifications. In certain such embodiments, short antisense compounds comprise at least one modified nucleotide. In certain embodiments short antisense compounds comprise at least two or more modified nucleotides. In certain embodiments, short antisense compounds comprise at least one modified internucleotide linkage. In certain embodiments, short antisense compounds are mixed-backbone oligonucleotides. In certain embodiments, short antisense compounds are chimeric oligonucleotides. In certain embodiments, short antisense oligonucleotides are uniformly modified. In certain embodiments, short antisense oligonucleotides comprise modifications independently selected at each nucleobase and at each linkage.

In certain embodiments, short antisense compounds are short gapmers. In certain such embodiments, short gapmers comprise at least one high affinity modification in one or more wings of the compound. In certain embodiments, short antisense compounds comprise 1 to 3 high-affinity modifications in each wing. In certain embodiments, high affinity modifications of the short antisense compounds allow for a target affinity similar to, or even greater than, the target affinity of longer antisense compounds. In certain embodiments, the high-affinity modified nucleotides are sugar modified nucleotides. Such sugar modified nucleotides include those comprising a bridge between the 4' and 2' position of the sugar. Exemplary high affinity sugar modifications include, but are not limited to, BNA s and other 2'-modifications such as 2'-MOE. In an alternate embodiment of the invention, the high affinity modification is not a 2'-O-(CH₂)ₙH (n=1-6) sugar-modified nucleotide. In an additional alternate embodiment, the high affinity modified nucleotide is not a 2'-OCH₃ or a 2'-OCH₂CH₂OCH₃ nucleotide. In certain embodiments, the high-affinity modified nucleotides confer a Tₘ of at least 1, at least 1.5, at least 2, at least 2.5, at least 3.0, at least 3.5 or at least 4.0 degrees per nucleotide. Some high-affinity nucleotide modifications are known in the art to increase toxicity. As shown herein, short antisense compounds having a limited number (generally 2 to 6) of high affinity modifications exhibit little to no increase in toxicity but retain or increase affinity for the target RNA, while also significantly reducing expression of the RNA target. Short antisense compounds of the invention may optionally comprise a conjugate group, such as, for example, cholesterol or C₁₆.

### 1. Certain Wings

In certain embodiments, the short antisense compounds comprise a 5' wing and/or a 3' wing. In such embodiments, the features of the 3' wing and the features of the 5' wing are selected independently. Thus, in such embodiments, the number of monomers in the 5' wing and the number of monomers (length) in the 3' wing may be the same or may be different; the modifications, if any, in the 5' wing may be the same as the modifications, if any, in the 3' wing or such modifications, if any, may be different; and the monomeric linkages in the 5' wing and the monomeric linkages in the 3' wing may be the same or they may be different.

In certain embodiments a wing comprises one, two or three monomers (i.e. has a length of 1, 2, or 3). In certain embodiments, the monomers of a wing are modified. In certain such embodiments, the monomers of the wing are modified to increase affinity of the antisense compound for its target nucleic acid. In certain embodiments, the monomers of a wing are nucleosides or nucleotides. In certain such embodiments, the nucleosides or nucleotides of the wing comprise a 2' modification. In certain such embodiments, the monomers (nucleosides or nucleotides) of the wing are BNA's. In certain such embodiments, the monomers of the wing are selected from α-L-Methyleneoxy (4'-CH₂-O-2') BNA , β-D-Methyleneoxy (4'-CH₂-O-2') BNA , Ethyleneoxy (4'-(CH₂)₂-O-2') BNA , Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, the monomeric linkages in a wing are naturally occurring internucleotide linkages. In certain embodiments, the monomeric linkages in a wing are non-naturally occurring internucleotide or internucleoside linkages. In certain such embodiments, the monomeric linkages in the wing are more resistant to one or more nucleases than naturally occurring internucleotide linkages. In certain such embodiments, the monomeric linkages in the wing are phosphorothioate linkages (P=S). In certain embodiments where a wing has more than one monomeric linkage, the monomeric linkages are the same as one another. In certain embodiments where a wing has more than one monomers linkage, the monomers linkages are different from each other.

One of ordinary skill in the art will recognize that the features and modifications discussed above may be used in any combination to prepare a wing. The table below provides non-limiting examples showing how one might prepare a wing by selecting a certain number of monomers, monomeric modifications (if any), and monomeric linkages both within the wing.

| Length | Monomer type/ modifications | monomeric linkages within wing |
|---|---|---|
| 1 | 2' MOE | None |
| 1 | BNA | None |
| 1 | Methyleneoxy BNA | None |
| 1 | ENA | None |
| 2 | 2' MOE | P=S |
| 2 | BNA | P=S |
| 2 | Methyleneoxy BNA | P=S |
| 2 | ENA | P=S |
| 2 | 2' MOE | P=O |
| 2 | BNA | P=O |
| 2 | Methyleneoxy BNA | P=O |
| 2 | ENA | P=O |
| 3 | 2' MOE | P=S |
| 3 | BNA | P=S |
| 3 | Methyleneoxy BNA | P=S |
| 3 | ENA | P=S |
| 3 | 2' MOE | P=O |
| 3 | BNA | P=O |
| 3 | Methyleneoxy BNA | P=O |
| 3 | ENA | P=O |

In certain embodiments in which a wing comprises two, three or four monomers, those two, three or four monomers all comprise the same modifications, if any. In certain embodiments in which a wing comprises two, three or four monomers, one or more of those two, three or four nucleobases comprises one or more modifications that is different from one or more of the modifications of one or more of the remaining monomers.

### 2. Certain Gaps

In certain embodiments, the short antisense compounds comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, the monomeric linkages in the gap are naturally occurring internucleotide linkages. In certain embodiments, the monomeric linkages in the gap are non-naturally occurring linkages. In certain such embodiments, the monomeric linkages in the gap are more resistant to one or more nuclease than naturally occurring internucleotide linkages. In certain such embodiments, the monomeric linkages in the gap are phosphorothioate linkages (P=S). In certain embodiments, the monomeric linkages in the gap are all the same as one another. In certain embodiments, the monomeric linkages within the gap are not all the same.

One of ordinary skill in the art will recognize that the features and modifications discussed above may be used in any combination to prepare a gap. The table below provides non-limiting examples showing how one might prepare a gap by selecting a certain number of monomers, monomeric modifications (if any), and monomeric linkages within the gap region.

| Length | Monomer type/ modifications | Monomeric linkages within gap |
|---|---|---|
| 5 | DNA | P=S |
| 6 | DNA | P=S |
| 7 | DNA | P=S |
| 8 | DNA | P=S |
| 9 | DNA | P=S |
| 10 | DNA | P=S |
| 11 | DNA | P=S |
| 12 | DNA | P=S |
| 13 | DNA | P=S |
| 14 | DNA | P=S |
| 6 | DNA | P=O |
| 7 | DNA | P=O |
| 8 | DNA | P=O |
| 9 | DNA | P=O |
| 10 | DNA | P=O |
| 11 | DNA | P=O |
| 12 | DNA | P=O |
| 8 | RNA | P=S |
| 9 | RNA | P=S |
| 10 | RNA | P=S |
| 11 | RNA | P=S |
| 12 | RNA | P=S |

### 3. Certain Gapped Antisense Oligomeric Compounds

One of ordinary skill in the art will recognize that the wings and the gaps discussed above may be selected and then combined in a variety of combinations to generate gapped oligomeric compounds, including, but not limited to, gapped antisense oligomeric compounds, and gapped antisense oligonucleotides. The features (length, modifications, linkages) of the 5' wing and the 3' wing may be selected independently of one another. The features of the gap include at least one difference in modification compared to the features of the 5' wing and at least one difference compared to the features of the 3' wing (i.e., there must be at least one difference in modification between neighboring regions to distinguish those neighboring regions from one another). The features of the gap may otherwise be selected independently.

In certain embodiments, the monomeric linkages within a wing and the monomeric linkages within the gap are the same. In certain embodiments, the monomeric linkages within a wing and the monomeric linkages within the gap are different. In certain such embodiments, the monomeric linkage bridging the wing and the gap are the same as the monomeric linkages in the wing. In certain embodiments, the monomeric linkage bridging the wing and the gap are the same as the monomeric linkages in the gap. In certain embodiments, short antisense compounds have uniform linkages throughout the compound. In certain such embodiments, all of the linkages are phosphorothioate (P=S) linkages.

One of ordinary skill in the art will recognize that the 3' wings, 5' wings, gaps, and linkages discussed above may be used in any combination to prepare a gapmer. The table below provides non-limiting examples showing how one might prepare a gapmer by selecting a certain 5' wing, a gap, a 3' wing and certain linkages bridging the gap and each wing.

| 5' Wing | | | 5'Bridge | Gap | | | 3' Bridge | 3' Wing | | |
|---|---|---|---|---|---|---|---|---|---|---|
| Length | Monomer | Link | Link | Length | Monomer | Link | Link | Length | Monomer | Link |
| 2 | MOE | P=S | P=S | 6 | DNA | P=S | P=S | 2 | MOE | P=S |
| 2 | BNA | P=S | P=O | 8 | DNA | P=O | P=S | 3 | BNA | P=S |
| 1 | MOE | None | P=S | 10 | DNA | P=S | P=S | 1 | MOE | P=S |
| 2 | MOE | P=S | P=S | 8 | RNA | P=S | P=S | 2 | MOE | P=S |
| 3 | Methyleneoxy BNA | P=S | P=S | 8 | RNA | P=S | P=S | 3 | MOE | P=S |
| 3 | DNA | P=O | P=O | 10 | RNA | P=S | P=O | 3 | 2'OH | P=O |
| 2 | 2-F | P=S | P=S | 5 | RNA | P=S | P=S | 2 | 2'-F | P=S |
| 1 | MOE | P=O | P=S | 5 | DNA | P=O | P=S | 4 | MOE | P=S |

In certain embodiments, the oligomeric compounds disclosed herein may comprise from about 8 to about 16, preferably 9 to 15, more preferably 9 to 14, more preferably 10 to 14 monomers (i.e. from about 8 to about 16 linked monomers). One of ordinary skill in the art will appreciate that this comprehends antisense compounds of 8, 9, 10, 11, 12, 13, 14, 15 or 16 nucleobases. In certain embodiments, oligomeric compounds are antisense compounds.

In certain embodiments, short antisense compounds are 8 nucleobases in length.

In certain embodiments, short antisense compounds are 9 nucleobases in length.

In certain embodiments, short antisense compounds are 10 nucleobases in length.

In certain embodiments, short antisense compounds are 11 nucleobases in length.

In certain embodiments, short antisense compounds are 12 nucleobases in length.

In certain embodiments, short antisense compounds are 13 nucleobases in length.

In certain embodiments, short antisense compounds are 14 nucleobases in length.

In certain embodiments, short antisense compounds are 15 nucleobases in length.

In certain embodiments, short antisense compounds are 16 nucleobases in length.

In certain embodiments, short antisense compounds are 8 monomers in length. In certain embodiments, short antisense compounds are 9 monomers in length. In certain embodiments, short antisense compounds are 10 monomers in length. In certain embodiments, short antisense compounds are 11 monomers in length. In certain embodiments, short antisense compounds are monomers in length. In certain embodiments, short antisense compounds are 13 monomers in length. In certain embodiments, short antisense compounds are 14 monomers in length. In certain embodiments, short antisense compounds are 15 monomers in length. In certain embodiments, short antisense compounds are 16 monomers in length. In certain embodiments, short antisense compounds comprise 9 to 15 monomers. In certain embodiments, short antisense compounds comprise 10 to 15 monomers. In certain embodiments, short antisense compounds comprise 12 to 14 monomers. In certain embodiments, short antisense compounds comprise 12 to 14 nucleotides or nucleosides.

One having skill in the art and informed by the short antisense compounds illustrated herein will be able, without undue experimentation, to identify further short antisense compounds.

In certain embodiments, short antisense compounds comprise a gap flanked by more than one wing on either or both sides. Thus, in certain embodiments, a short antisense compound comprises two or more 5' wings and two or more 3' wings. In certain embodiments, a short antisense compound comprises one 5' wing and two or more 3' wings. In certain embodiments, a short antisense compound comprises one 3' wing and two or more 5' wings. Certain such embodiments comprise, for example, the following regions: a first 5' wing - a bridge - a second 5' wing - a bridge - a gap - a bridge - a second 3' wing - a bridge - a first 3'wing. In such embodiments, each region has at least one difference in modification when compared to its neighboring region. Thus, in such embodiments, the second 5' wing and the second 3' wing each independently comprises one or more differences in modification compared to the gap and compared to the first 5' wing and the first 3' wing. In such embodiments, the modifications of the first 3' wing and first 5' wing may either or both be the same or different from the modifications of the gap, if any.

### 4. Certain Conjugate Groups

In one aspect, oligomeric compounds are modified by covalent attachment of one or more conjugate groups. In general, conjugate groups modify one or more properties of the attached oligomeric compound including but not limited to pharmacodynamic, pharmacokinetic, binding, absorption, cellular distribution, cellular uptake, charge and clearance. Conjugate groups are routinely used in the chemical arts and are linked directly or via an optional linking moiety or linking group to a parent compound such as an oligomeric compound. A preferred list of conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes.

Preferred conjugate groups amenable to the present invention include lipid moieties such as a cholesterol moiety (Letsinger et al., Proc. Natl. Acad. Sci. USA, 1989, 86, 6553); cholic acid (Manoharan et al., Bioorg. Med. Chem. Lett., 1994, 4, 1053); a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765); a thiocholesterol (Oberhauser et al., Nucl. Acids Res., 1992, 20, 533); an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49); a phospholipid, e.g., di-hexadecyl-rac-glycerol or triethylammonium-1,2-di-O-hexadecyl-rac-glycero-3-H-phosphonate (Manoharan et al., Tetrahedron Lett., 1995, 36, 3651; Shea et al., Nucl. Acids Res., 1990, 18, 3777); a polyamine or a polyethylene glycol chain (Manoharan et al., Nucleosides & Nucleotides, 1995, 14, 969); adamantane acetic acid (Manoharan et al., Tetrahedron Lett., 1995, 36, 3651); a palmityl moiety (Mishra et al., Biochim. Biophys. Acta, 1995, 1264, 229); or an octadecylamine or hexylamino-carbonyl-oxycholesterol moiety (Crooke et al., J. Pharmacol. Exp. Ther., 1996,277,923).

Linking groups or bifunctional linking moieties such as those known in the art are amenable to the compounds provided herein. Linking groups are useful for attachment of chemical functional groups, conjugate groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

### 5. Synthesis, Purification and Analysis

Oligomerization of modified and unmodified nucleosides and nucleotides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57,5707-5713).

Oligomeric compounds provided herein can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

Methods of purification and analysis of oligomeric compounds are known to those skilled in the art. Analysis methods include capillary electrophoresis (CE) and electrospray-mass spectroscopy. Such synthesis and analysis methods can be performed in multi-well plates. The method of the invention is not limited by the method of oligomer purification.

### D. Antisense

Antisense mechanisms are all those involving the hybridization of a compound with target nucleic acid, wherein the outcome or effect of the hybridization is either target degradation or target occupancy with concomitant stalling of the cellular machinery involving, for example, transcription or splicing.

One type of antisense mechanism involving target degradation includes an RNase H. RNase H is a cellular endonuclease which cleaves the RNA strand of an RNA:DNA duplex. It is known in the art that single-stranded antisense compounds which are "DNA-like" elicit RNAse H activity in mammalian cells. Activation of RNase H, therefore, results in cleavage of the RNA target, thereby greatly enhancing the efficiency of DNA-like oligonucleotide-mediated inhibition of gene expression.

In certain embodiments, chemically-modified antisense compounds have a higher affinity for target RNAs than does non-modified DNA. In certain such embodiments, that higher affinity in turn provides increased potency allowing for the administration of lower doses of such compounds, reduced potential for toxicity and improvement in therapeutic index and decreased overall cost of therapy.

The present disclosure demonstrates that the incorporation of chemically-modified high-affinity nucleotides and nucleosides into antisense compounds allows for the design of short antisense compounds 8-16 nucleobases in length useful for the reduction of target RNAs and/or target proteins in cells, tissues, and animals, including but not limited to, humans with increased potency and improved therapeutic index. Thus, in certain embodiments, disclosed herein are short antisense compounds comprising high-affinity nucleotide modifications useful for reducing a target RNA *in vivo*. Certain such short antisense compounds are effective at lower doses than previously described antisense compounds, allowing for a reduction in toxicity and cost of treatment. In addition, certain short antisense compounds have greater potential for oral dosing.

To address the need for more potent antisense compounds, disclosed herein are short antisense compounds (8-16, preferably 9 to 15, more preferably 9 to 14, more preferably 10 to 14 nucleotides in length) with increased activity *in vivo* relative to longer compounds. Certain short antisense compounds are gapmer compounds comprising high-affinity chemically-modified nucleotides on the 3' and 5' ends (wings) of the compound. In certain embodiments, the addition of high-affinity modified nucleotides allows antisense compounds to be active against, and specific for, their intended target RNA *in vivo* despite being shorter in length. Contemplated herein are short antisense compounds wherein each of the wings independently comprises 1 to 3 high-affinity modified nucleotides. In certain embodiments, the high-affinity modifications are sugar modifications. High-affinity modified nucleotides include, but are not limited to, BNA s or other 2'-modified nucleotides, such as 2'-MOE nucleotides. Also contemplated are short antisense compounds having at least one modified internucleotide linkage, such as a phosphorothioate internucleotide linkage. In certain embodiments, the short antisense compounds of the present invention can have all phosphorothioate internucleoside linkages. The short antisense compounds optionally comprise a conjugate group. As shown herein, short antisense compounds have greater affinity for target RNA than they have for DNA and are significantly more potent *in vivo* as shown by reduction of target mRNA as well as by amelioration of a variety of disease indications.

As used herein, an RNA which is involved in regulating glucose metabolism or clearance, lipid metabolism, cholesterol metabolism or insulin metabolism is any RNA involved in the biochemical pathways that regulate these processes. Such RNAs are well known in the art.

### 1. Modulation of Target Expression

In certain embodiments, a target is identified and antisense oligonucleotides are designed to modulate that target or its expression. In certain embodiments, designing an oligomeric compound to a target nucleic acid molecule can be a multistep process. Typically the process begins with the identification of a target protein, the activity of which is to be modulated, and then identifying the nucleic acid the expression of which yields the target protein. In certain embodiments, designing of an antisense compound results in an antisense compound that is hybridizable to the targeted nucleic acid molecule. In certain embodiments, the antisense compound is an antisense oligonucleotide or antisense oligonucleoside. In certain embodiments, an antisense compound and a target nucleic acid are complementary to one another. In certain such embodiments, an antisense compound is perfectly complementary to a target nucleic acid. In certain embodiments, an antisense compound includes one mismatch. In certain embodiments, an antisense compound includes two mismatches. In certain embodiments, an antisense compound includes three or more mismatches.

Modulation of expression of a target nucleic acid can be achieved through alteration of any number of nucleic acid functions. In certain embodiments, the functions of RNA to be modulated include, but are not limited to, translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. Thus, in one embodiment modulation of expression can mean increase or decrease in target RNA or protein levels. In another embodiment modulation of expression can mean an increase or decrease of one or more RNA splice products, or a change in the ratio of two or more splice products.

In certain embodiments, expression of a target gene is modulated using an oligomeric compound comprising from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of a target gene using one or more antisense compounds of 8, 9, 10, 11, 12, 13, 14, 15 or 16 nucleobases.

In certain embodiments, methods of modulating a target gene comprises use of a short antisense compound that is 8 nucleobases in length. In certain embodiments, methods of modulating a target gene comprises use of a short antisense compound that is 9 nucleobases in length In certain embodiments, methods of modulating a target gene comprises use of a short antisense compound that is 8 nucleobases in length. In certain embodiments, methods of modulating a target gene comprises use of a short antisense compound that is 10 nucleobases in length. In certain embodiments, methods of modulating a target gene comprises use of a short antisense compound that is 10 nucleobases in length. In certain embodiments, methods of modulating a target gene comprises use of a short antisense compound that is 11 nucleobases in length. In certain embodiments, methods of modulating a target gene comprises use of a short antisense compound that is 12 nucleobases in length. In certain embodiments, methods of modulating a target gene comprises use of a short antisense compound that is 13 nucleobases in length. In certain embodiments, methods of modulating a target gene comprises use of a short antisense compound that is 14 nucleobases in length. In certain embodiments, methods of modulating a target gene comprises use of a short antisense compound that is 15 nucleobases in length. In certain embodiments, methods of modulating a target gene comprises use of a short antisense compound that is 16 nucleobases in length.

In certain embodiments, methods of modulating expression of a target gene comprises use of a short antisense compound comprising 9 to 15 monomers. In certain embodiments, methods of modulating expression of a target gene comprises use of a short antisense compound comprising 10 to 15 monomers. In certain embodiments, methods of modulating expression of a target gene comprises use of a short antisense compound comprising 12 to 14 monomers. In certain embodiments, methods of modulating expression of a target gene comprises use of a short antisense compound comprising 12 or 14 nucleotides or nucleosides.

### 2. Hybridization

In certain embodiments, antisense compounds specifically hybridize when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

As used herein, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

### 3. Complementarity

It is understood in the art that incorporation of nucleotide affinity modifications may allow for a greater number of mismatches compared to an unmodified compound. Similarly, certain oligonucleotide sequences may be more tolerant to mismatches than other oligonucleotide sequences. One of ordinary skill in the art is capable of determining an appropriate number of mismatches between oligonucleotides, or between an oligonucleotide and a target nucleic acid, such as by determining melting temperature (Tₘ). Tₘ or Tₘ can be calculated by techniques that are familiar to one of ordinary skill in the art. For example, techniques described in Freier et al. (Nucleic Acids Research, 1997, 25, 22: 4429-4443) allow one of ordinary skill in the art to evaluate nucleotide modifications for their ability to increase the melting temperature of an RNA:DNA duplex.

### 4. Identity

Antisense compounds, or a portion thereof, may have a defined percent identity to a SEQ ID NO, or a compound having a specific Isis number. As used herein, a sequence is identical to the sequence disclosed herein if it has the same nucleobase pairing ability. For example, an RNA which contains uracil in place of thymidine in the disclosed sequences of the compounds described herein would be considered identical as they both pair with adenine. This identity may be over the entire length of the oligomeric compound, or in a portion of the antisense compound (e.g., nucleobases 1-20 of a 27-mer may be compared to a 20-mer to determine percent identity of the oligomeric compound to the SEQ ID NO. It is understood by those skilled in the art that an antisense compound need not have an identical sequence to those described herein to function similarly to the antisense compound described herein. Shortened versions of antisense compounds taught herein, or non-identical versions of the antisense compounds taught herein, are also provided herein. Non-identical versions are those wherein each base does not have the same pairing activity as the antisense compounds disclosed herein. Bases do not have the same pairing activity by being shorter or having at least one abasic site. Alternatively, a non-identical version can include at least one base replaced with a different base with different pairing activity (e.g., G can be replaced by C, A, or T). Percent identity is calculated according to the number of bases that have identical base pairing corresponding to the SEQ ID NO or antisense compound to which it is being compared. The non-identical bases may be adjacent to each other, dispersed through out the oligonucleotide, or both.

For example, a 16-mer having the same sequence as nucleobases 2-17 of a 20-mer is 80% identical to the 20-mer. Alternatively, a 20-mer containing four nucleobases not identical to the 20-mer is also 80% identical to the 20-mer. A 14-mer having the same sequence as nucleobases 1-14 of an 18-mer is 78% identical to the 18-mer. Such calculations are well within the ability of those skilled in the art.

The percent identity is based on the percent of nucleobases in the original sequence present in a portion of the modified sequence. Therefore, a 30 nucleobase antisense compound comprising the full sequence of the complement of a 20 nucleobase active target segment would have a portion of 100% identity with the complement of the 20 nucleobase active target segment, while further comprising an additional 10 nucleobase portion. In the context of the instant description, the complement of an active target segment may constitute a single portion. In preferred embodiments, the oligonucleotides provided herein are at least 80%, at least 85%, at least 90%, at least 95%, at least 96%, at least 97%, at least 98%, at least 99% or 100% identical to at least a portion of the complement of the active target segments presented herein.

### E. Target Nucleic Acids, Regions and Segments

The target nucleic acid is a nucleic acid molecule encoding SGLT2. Nucleic acid molecules that encode SGLT2 include, without limitation, SEQ ID NO: 3 encoding human SGLT2 (NM_003 041.1).

The targeting process usually includes determination of at least one target region, segment, or site within the target nucleic acid for the antisense interaction to occur such that the desired effect"will result.

In certain embodiments, the 5'-most nucleotide of a target region is the 5' target site of a short antisense compound and the 3'-most nucleotide of a target region is the 3' target site of the same short antisense compound. In certain embodiments, the 5'-most nucleotide of a target region is the 5' target site of a short antisense compound and the 3'-most nucleotide of a target region is the 3' target site of a different short antisense compound. In certain embodiments, a target region comprises a nucleotide sequence within 10, 15, or 20 nucleotides of a 5' target site or a 3' target site.

In certain embodiments, a target region is a structurally defined region of the nucleic acid. For example, in certain such embodiments, a target region may encompass a 3' UTR, a 5' UTR, an exon, an intron, a coding region, a translation initiation region, translation termination region, or other defined nucleic acid region.

The locations on the target nucleic acid defined by having one or more active short antisense compounds targeted thereto are referred to as "active target segments." In certain embodiments, the target nucleic acid having one or more active short antisense compounds targeted thereto is a target RNA. When an active target segment is defined by multiple short antisense compounds, the compounds are preferably separated by no more than about 10 nucleotides on the target sequence, more preferably no more than about 5 nucleotides on the target sequence, even more preferably the short antisense compounds are contiguous, most preferably the short antisense compounds are overlapping. There may be substantial variation in activity (e.g., as defined by percent inhibition) of the short antisense compounds within an active target segment. Active short antisense compounds are those that modulate the expression of their target nucleic acid, including but not limited to a target RNA. Active short antisense compounds inhibit expression of their target RNA at least 10%, preferably 20%. In a preferred embodiment, at least about 50%, preferably about 70% of the short antisense compounds targeted to the active target segment modulate expression of their target RNA at least 40%. In a more preferred embodiment, the level of inhibition required to define an active short antisense compound is defined based on the results from the screen used to define the active target segments.

A suitable target segment is at least about an 8-nucleobase portion of a target region to which an active short antisense compound is targeted. Target segments can include DNA or RNA sequences that comprise at least the 8 consecutive nucleobases from the 5'-terminus of one of the illustrative target segments (the remaining nucleobases being a consecutive stretch of the same DNA or RNA beginning immediately upstream of the 5'-terminus of the target segment and continuing until the DNA or RNA comprises about 8 to about 16 nucleobases). Target segments are also represented by DNA or RNA sequences that comprise at least the 8 consecutive nucleobases from the 3'-terminus of one of the illustrative target segments (the remaining nucleobases being a consecutive stretch of the same DNA or RNA beginning immediately downstream of the 3'-terminus of the target segment and continuing until the DNA or RNA comprises about 8 to about 16 nucleobases). It is also understood that antisense target segments may be represented by DNA or RNA sequences that comprise at least 8 consecutive nucleobases from an internal portion of the sequence of an illustrative target segment, and may extend in either or both directions until the short antisense compound comprises about 8 to about 16 nucleobases. One having skill in the art armed with the target segments illustrated herein will be able, without undue experimentation, to identify further target segments.

Once one or more target regions, segments or sites have been identified, short antisense compounds are chosen which are sufficiently complementary to the target, i.e., hybridize sufficiently well and with sufficient specificity, to give the desired effect.

The short antisense compounds may also be targeted to regions of the target nucleobase sequence comprising any consecutive nucleobases 8 to 16 nucleobases in length along the target nucleic acid molecule.

Target segments 8-16 nucleobases in length comprising a stretch of at least eight (8) consecutive nucleobases selected from within the illustrative target segments are considered to be suitable for targeting as well. Thus, the short antisense compounds may also encompass 8-16 nucleobases within those segments identified herein as beginning at a particular 5' target site. Any segment of 8, 9, 10, 11, or more preferably 12, 13, 14, 15 or 16 contiguous nucleobases in a 50, preferably 25, more preferably 16 nucleobase perimeter around these regions are also considered to be suitable for targeting.

In a further embodiment, the "suitable target segments" identified herein may be employed in a screen for additional short antisense compounds that modulate the expression of a target nucleic acid. "Modulators" are those compounds that decrease or increase the expression of a target nucleic acid and which comprise at least an 8-nucleobase portion which is complementary to a target segment. The screening method comprises the steps of contacting a target segment of a nucleic acid with one or more candidate modulators, and selecting for one or more candidate modulators which decrease or increase the expression of a target nucleic acid. Once it is shown that the candidate modulator or modulators are capable of modulating (e.g. either decreasing or increasing) the expression of a target nucleic acid, the modulator may then be employed in further investigative studies of the function of the target, or for use as a research, diagnostic, or therapeutic agent in accordance with the present disclosure.

For all short antisense compounds discussed herein, sequence, monomer, monomeric modification, and monomeric linkage may each be selected independently. In certain embodiments, short antisense compounds are described by a motif. In such embodiments, any motif may be used with any sequence, whether or not the sequence and/or the motif is specifically disclosed herein. In certain embodiments, short antisense compounds comprise modifications that are not amenable to description by motif (for example, short antisense compounds comprising several different modifications and/or linkages at various positions throughout the compound). Such combinations may be incorporated for any sequence, whether or not it is disclosed herein. The sequence listing accompanying this filing provides certain nucleic acid sequences independent of chemical modification. Though that listing identifies each sequence as either "RNA" or "DNA" as required, in reality, those sequences may be modified with any combination of chemical modifications and/or motifs.

In certain embodiments, short antisense compounds comprise at least one high-affinity modified monomer. In certain embodiments, provided are short antisense compounds targeted to nucleic acid molecules encoding.

### F. Certain Targets

In certain embodiments, short antisense compounds may be designed to modulate any target. In certain embodiments, the target is clinically relevant. In such embodiments, modulation of the target results in clinical benefit. Certain targets are preferentially expressed in the kidney. Certain targets are preferentially expressed in the liver. Certain targets are associated with a metabolic disorder. Certain targets are associated to a cardiovascular disorder. In certain embodiments, the target is SGLT2.

In certain embodiments, short antisense compounds exhibit liver and kidney-specific target RNA reduction *in vivo*. Such property renders those short antisense compounds particularly useful for inhibition of many target RNAs involved in metabolic and cardiovascular diseases. Thus, disclosed herein are methods of treating cardiovascular or metabolic disorders by contacting said kidney or liver tissues with short antisense compounds targeted to RNAS associated with said disorders. Thus, also disclosed are methods for ameliorating any of a variety of metabolic or cardiovascular disease indications with the short antisense compounds of the present disclosed.

### 2. SGLT-2

Sodium dependent glucose transporter 2 (SGLT-2) is expressed in the kidney proximal tubule epithelial cells, and functions to reabsorb glucose preventing glucose loss in the urine. For the human genome SGLT-2 is a member of an 11-membered family of sodium substrate co-transporters. Many of these family members share sequence homology, for example SGLT-1 shares about 59% sequence identity with SGLT-2 and about 70% sequence identity with SGLT-3. SGLT-1 is a glucose transporter found in the heart and the CNS. SGLT-3 is a glucose sensing sodium channel in the small intestine. The separate localization patterns for these SGLTs is one point of distinction between the homologous family members. (Handlon, A.L., Expert Opin. Ther. Patents (2005) 15(11):1532-1540; Kanai et al., J. Clin. Invest., 1994, 93, 397-404; Wells et al., Am. J. Physiol. Endocrinol. Metab., 1992, 263, F459-465).

Studies of human SGLT2 injected into Xenopus oocytes demonstrated that this protein mediates sodium-dependent transport of D-glucose and .alpha.-methyl-D-glucopyranoside (.alpha.-MeGlc; a glucose analog) with a Km value of 1.6 mM for .alpha.-MeGlc and a sodium to glucose coupling ratio of 1:1 (Kanai et al., J. Clin. Invest., 1994, 93, 397-404; You et al., J. Biol. Chem., 1995, 270, 29365-29371). This transport activity was suppressed by phlorizin, a plant glycoside that binds to the glucose site of the SGLTs but is not transported and thus inhibits SGLT action (You et al., J. Biol. Chem., 1995, 270, 29365-29371).

Diabetes is a disorder characterized by hyperglycemia due to deficient insulin action. Chronic hyperglycemia is a major risk factor for diabetes-associated complications, including heart disease, retinopathy, nephropathy and neuropathy. As the kidneys play a major role in the regulation of plasma glucose levels, renal glucose transporters are becoming attractive drug targets (Wright, Am. J. Physiol. Renal Physiol., 2001, 280, F10-18). Diabetic nephropathy is the most common cause of end-stage renal disease that develops in many patients with diabetes. Glucotoxicity, which results from long-term hyperglycemia, induces tissue-dependent insulin resistance in diabetic patients (Nawano et al., Am. J. Physiol. Endocrinol. Metab., 2000, 278, E535-543).

### Definitions

"Sodium dependent glucose transporter 2" is the gene product or protein of which expression is to be modulated by administration of a short antisense compound. Sodium dependent glucose transporter 2 is generally referred to as SGLT2 but may also be referred to as SLC5A2; sodium-glucose transporter 2; sodium-glucose cotransporter, kidney low affinity; sodium-glucose cotransporter, renal; solute carrier family 5 (sodium/glucose cotransporter), member 2; SL52.

"SGLT2 nucleic acid" means any nucleic acid encoding SGLT2. For example, in certain embodiments, a SGLT2 nucleic acid includes, without limitation, a DNA sequence encoding SGLT2, an RNA sequence transcribed from DNA encoding SGLT2, and an mRNA sequence encoding SGLT2. "SGLT2 mRNA" means an mRNA encoding a SGLT2 protein.

### Therapeutic indications

In certain embodiments, short antisense compounds are used to modulate expression of SGLT-2 and related proteins. In certain embodiments, such modulation is accomplished by providing short antisense compounds that hybridize with one or more target nucleic acid molecules encoding SGLT-2, including, but is not limited to, SGLT2, SL52, SLCSA2, Sodium-Glucose Co-Transporter, Kidney Low Affinity Sodium-Glucose Co-Transporter, Renal Sodium-Glucose Co-Tmpsporter 2 and Solute Carrier Family 5 Sodium/Glucose Co-Transporter Member 2. Also disclosed are methods of treating metabolic and/or cardiovascular disease and disorders as described herein. In particular embodiments, short antisense compounds that inhibit the expression of SGLT2 are used in methods of lowering blood glucose levels in an animal and methods of delaying or preventing the onset of type 2 diabetes. Such methods comprise administering a therapeutically or prophylactically effective amount of one or more of the compounds of the disclosed to the animal, which may be in need of treatment. The one or more compounds can be a short antisense compound targeting a nucleic acid encoding SGLT2. disclosed herein are methods of enhancing inhibition of expression of SGLT2 in kidney cells or kidney tissues, comprising contacting the cells or tissues with one or more of the compounds of the disclosed, such as short antisense compounds targeting a nucleic acid encoding SGLT2.

While certain compounds, compositions and methods have been described with specificity in accordance with certain embodiments, the following examples serve only to illustrate the compounds of the disclosed and are not intended to limit the same.

In certain embodiments, short antisense compounds are chimeric oligomeric compounds having mixed phosphorothioate and phosphodiester backbones. Certain mixed backbone short antisense compounds have a central gap comprising at least 5 contiguous 2'-deoxy nucleosides flanked by two wings each of which comprises at least one 2'-O-methoxyethyl nucleoside. In certain embodiments, the internucleoside linkages of the mixed backbone compounds are phosphorothioate linkages in the gap and phosphodiester linkages in the two wings. In certain embodiments, mixed backbone compounds have phosphorothioate linkages in the wings, except for one phosphodiester linkage at one or both of the extreme 5' and 3' ends of the oligonucleotide. In certain embodiments short antisense compounds targeted to SGLT2 have a motif (wing - deoxy gap -wing) selected from 3-10-3, 2-10-3, 2-10-2, 1-10-1,1-10-2, 2-8-2, 1-9-2, 1-8-1, 3-6-3 or 1-6-1. In certain embodiments short antisense compounds targeted to SGLT2 have a motif (wing - deoxy gap - wing) selected from 1-10-1, 1-10-2, 2-8-2, 1-9-2,1-8-1, 3-6-3 or 1-6-1.

In certain embodiments, short antisense compounds targeted to an SGLT2 nucleic acid and having a mixed backbone are efficiently delivered to the kidney. In certain embodiments, administration of short antisense compounds targeted to an SGLT2 nucleic acid and having a mixed backbone results in modulation of target gene expression in the kidney. In certain such embodiments, there is little or no liver or kidney toxicity. In certain embodiments, short antisense compounds targeted to an SGLT2 nucleic acid and having a mixed backbone are more potent for reducing SGLT-2 mRNA and have a faster onset compared with a short antisense compound that does not have a mixed back-bone, but is otherwise identical. In certain such embodiments, such increase potency and/or reduced toxicity is in mouse and/or rat. In certain such embodiments, such increase potency and/or reduced toxicity is in a human.

By way of example, and only for illustrative purposes, ISIS 145733, which comprises uniform phosphorothioate linkages and ISIS 257016 which comprises phosphodiester linkage in the wings and phosphorothioate linkages in the gap, are otherwise identical. Both comprise the sequence GAAGTAGCCACCAACTGTGC (SEQ ID NO. 1572). Both of the oligonucleotides further comprise a gap consisting of ten 2'-deoxynucleotides, flanked on each side by five-nucleotide "2'-methoxyethyl (2'-MOE) nucleotides. All cytidine residues are 5-methylcytidines. The mixed back-bone compound, ISIS 257016, was about 50 times more potent for reducing SGLT-2 mRNA compared to the non-mixed parent compound, ISIS 145733 (see EXAMPLE 3).

Pharmacokinetic studies of certain mixed backbone compound ISIS 257016 indicate that in certain embodiments, the compound acts as a prodrug that is metabolized to a 12 nucleobase pharmacophore. Studies with ISIS 370717, a 12 nucleobase short antisense compound corresponding to ISIS 257016, show that the compound has a similar pharmacological profile to ISIS 257016 but with a faster onset of action. ISIS 370717 is a 12 nucleobase antisense oligonucleotide targeted to SGLT-2 comprising the sequence TAGCCACCAACT (SEQ ID NO. 1554), further comprising a gap consisting of ten 2'-deoxynucleotides, flanked on both sides by one-nucleotide wings. The wings are composed of 2'-methoxyethyl (2'-MOE) nucleotides. All cytidine residues are 5-methylcytidines. The internucleoside linkages are phosphorothioate (P=S) throughout the oligonucleotide. The similarity in pharmacological activity of ISIS 257016 and ISIS 370717 supports the pharmacokinetic studies indicating ISIS 257016 was a prodrug having a 12 nucleotide pharmacophore (see EXAMPLE 4). Further, studies with stabilized (end-capped) versions of ISIS 257016 show dramatic loss of activity.

In certain embodiments, short antisense compounds comprising 2' MOE monomers in the wings are efficiently delivered to the kidney and treatment with such compounds results in efficient modulation of target gene expression in the kidney without liver or kidney toxicity. It is further shown herein that in certain embodiments, short antisense compounds are more potent for reducing SGLT-2 mRNA and have a faster onset compared with parent oligonucleotides targeted to SGLT-2 mRNA in mouse and rat. 2' MOE gap shortmers are shown herein to improve potency and bioavailability over parent compounds.

By way of example, and only for illustrative purposes studies with ISIS 370717 reveal significantly higher accumulation of the short antisense compound in the kidney tissue (approximately 500 micro grams per gram of tissue) compared to the longer parent. Moreover, SGLT-2 mRNA was reduced by more than 80% over the controls (see EXAMPLE 5). ISIS 370717 1-10-1 gapmer was used as a template to make sequence related oligos with varying motifs. Studies evaluating wing, gap and total length variations around the ISIS 370717 12 mer oligonucleotide can be seen in EXAMPLE 6. Certain motifs evaluated included 1-10-1, 2-8-2, 1-8-1, 3-6-3, and 1-6-1 (see Table 4 in EXAMPLE 6). The compounds were analyzed for their effect on SGLT2 mRNA levels. All the motifs inhibited the expression of SGLT2 in vivo in a dose-dependent manner. The 1-10-1, 2-8-2 and 1-8-1 gapmers were found to be particularly potent SGLT-2 mRNA was reduced by more than 80% over the controls using these motifs.

In certain embodiments, the invention provides short antisense compounds targeted to an SGLT2 nucleic acid and having a motif selected from: 1-10-1 and 1-10-2 MOE gapmer. (see Table 6 in EXAMPLE 7). Certain such compounds were analyzed for their effect on rat SGLT2 mRNA. Results in Table 7 illustrate that both the 1-10-1 and 1-10-2 MOE gapmers inhibit the expression of SGLT2 in vivo in a dose-dependent manner and over 80% reduction of SGLT-2 mRNA could be achieved.

Certain additional 1-10-1 and 2-8-2 MOE gapmers were evaluated in both mouse and rat in vivo models (see, e.g., EXAMPLE 8 and 9). Greater than 80% reduction in SGLT-2 mRNA was achieved with many of the 1-10-1 and 2-8-2 MOE gapmers at relatively low concentrations of oligo and in the absence of any toxicity effects.

In another non-limiting example, the effect of ISIS 388625 on dog SGLT2 mRNA levels was also analyzed. Dog studies illustrate that greater than 80% inhibition of the expression of SGLT2 can be achieved at a I mg/kg/wk dose. Even greater inhibition can be achieved at slightly higher doses. Administration of ISIS 388625 in dog was also shown to improved glucose tolerance. Peak plasma glucose levels were decreased by over 50% on average and the subsequent drop in glucose was lessened compared to saline controls in a standard glucose tolerance test (See EXAMPLE 11). Also, in a rat model of diabetes, short antisense compounds were shown to significantly decrease plasma glucose levels and HbA1C over time compared to PBS and control treated animals (See Example 10).

The animals in all studies were further evaluated for toxicity. For example, total body weight, liver, spleen and kidney weight were evaluated. Significant changes in spleen, liver or body weight can indicate that a particular compound causes toxic effects. All changes were found to be within the margin of error. No significant changes in body weight were observed during the treatment or at study termination. No significant changes in liver or spleen weights were observed.

### Certain Short Antisense Compounds Targeted to an SGLT2 nucleic acid

In certain embodiments, short antisense compounds are targeted to an SGLT2 nucleic acid having the sequence of GENBANK® Accession No. NM_003041.1, incorporated herein as SEQ ID NO: 2. In certain such embodiments, a short antisense compound targeted to SEQ ID NO: 3 is at least 90% complementary to SEQ ID NO: 3. In certain such embodiments, a short antisense compound targeted to SEQ ID NO: 3 is at least 95% complementary to SEQ ID NO: 3. In certain such embodiments, a short antisense compound targeted to SEQ ED NO: 3 is 100% complementary to SEQ ID NO: 1. In certain embodiments, a short antisense compound targeted to SEQ ID NO: 3 comprises a nucleotide sequence selected from the nucleotide sequences set forth in Table 1 and 2.

The nucleotide sequence set forth in each SEQ ID NO set forth in Tables 1 and 2 is independent of any modification to a sugar moiety, a monomeric linkage, or a nucleobase. As such, short antisense compounds defined by a SEQ ID NO may comprise, independently, one or more modifications to a sugar moiety, an internucleoside linkage, or a nucleobase. Antisense compounds described by Isis Number (Isis NO.) indicate a combination of nucleobase sequence and one or more modifications to a sugar moiety, an internucleoside linkage, or a nucleobase.

Tables 1 and 2 illustrate examples of short antisense compounds targeted to SEQ ID NO: 3. Table 1 illustrates short antisense compounds that are 100% complementary to SEQ ID NO: 3. Table 2 illustrates short antisense compounds that have one or two mismatches with respect to SEQ ID NO: 3. The column labeled 'gapmer motif' indicates the wing-gap-wing motif of each short antisense compounds. The gap segment comprises 2'-deoxynucleotides and each nucleotide of each wing segment comprises a 2'-modified sugar. The particular 2'-modified sugar is also indicated in the 'gapmer motif column. For example, '2-10-2 MOE' means a 2-10-2 gapmer motif, where a gap segment of ten 2'-deoxynucleotides is flanked by wing segments of two nucleotides, where the nucleotides of the wing segments are 2'-MOE nucleotides. Internucleoside linkages are phosphorothioate. The short antisense compounds comprise 5-methylcytidine in place of unmodified cytosine, unless unmodified cytosine" is listed in the gapmer motif column, in which case the indicated cytosines are unmodified cytosines. For example, "5-mC in gap only' indicates that the gap segment has 5-methyleytosines, while the wing segments have unmodified cytosines.

**Table 1. Short Antisense Compounds Targeted to SEQ ID NO: 3**

| **ISIS No** | **5' Target Site** | **3' Target Site** | **Sequence (5'-3')** | **Gapmer Motif** | **SEQ ID NO** |
|---|---|---|---|---|---|
| 379684 | 84 | 95 | TGTCAGCAGGAT | 1-10-1 MOE | 214 |
| 405193 | 113 | 124 | CAGCAGGAAATA | 2-8-2 MOE | 215 |
| 405194 | 114 | 125 | CCAGCAGGAAAT | 2-8-2 MOE | 216 |
| 405195 | 115 | 126 | ACCAGCAGGAAA | 2-8-2 MOE | 217 |
| 405196 | 116 | 127 | GACCAGCAGGAA | 2-8-2 MOE | 218 |
| 405197 | 117 | 128 | TGACCAGCAGGA | 2-8-2 MOE | 219 |
| 379685 | 117 | 128 | TGACCAGCAGGA | 1-10-1 MOE | 219 |
| 405198 | 118 | 129 | ATGACCAGCAGG | 2-8-2 MOE | 221 |
| 405199 | 119 | 130 | AATGACCAGCAG | 2-8-2 MOE | 222 |
| 405200 | 120 | 131 | CAATGACCAGCA | 2-8-2 MOE | 223 |
| 405201 | 121 | 132 | CCAATGACCAGC | 2-8-2 MOE | 224 |
| 379686 | 135 | 146 | ACCACAAGCCAA | 1-10-1 MOE | 225 |
| 379711 | 172 | 183 | TAGCCGCCCACA | 1-10-1 MOE | 226 |
| 388628 | 172 | 183 | TAGCCGCCCACA | 2-8-2 MOE | 226 |
| 405202 | 207 | 218 | CCGGCCACCACA | 2-8-2 MOE | 228 |
| 405203 | 208 | 219 | ACCGGCCACCAC | 2-8-2 MOE | 229 |
| 405204 | 236 | 247 | GATGTTGCTGGC | 2-8-2 MOE | 230 |
| 379687 | 236 | 247 | GATGTTGCTGGC | 1-10-1 MOE | 230 |
| 405205 | 237 | 248 | CGATGTTGCTGG | 2-8-2 MOE | 232 |
| 405206 | 238 | 249 | CCGATGTTGCTG | 2-8-2 MOE | 233 |
| 405207 | 239 | 250 | GCCGATGTTGCT | 2-8-2 MOE | 234 |
| 405208 | 240 | 251 | TGCCGATGTTGC | 2-8-2 MOE | 235 |
| 405209 | 241 | 252 | CTGCCGATGTTG | 2-8-2 MOE | 236 |
| 405210 | 260 | 271 | CAGGCCCACAAA | 2-8-2 MOE | 237 |
| 405211 | 261 | 272 | CCAGGCCCACAA | 2-8-2 MOE | 238 |
| 405212 | 262 | 273 | GCCAGGCCCACA | 2-8-2 MOE | 239 |
| 379688 | 288 | 299 | CCAAGCCACTTG | 1-10-1 MOE | 240 |
| 379689 | 318 | 329 | AGAGCGCATTCC | 1-10-1 MOE | 241 |
| 379690 | 435 | 446 | ACAGGTAGAGGC | 1-10-1 MOE | 242 |
| 405248 | 474 | 485 | AGATCTTGGTGA | 2-8-2 MOE | 243 |
| 379691 | 474 | 485 | AGATCTTGGTGA | 1-10-1 MOE | 243 |
| 382676 | 527 | 539 | TGTTCCAGCCCAG | 1-10-2 MOE | 245 |
| 388625 | 528 | 539 | TGTTCCAGCCCA | 2-8-2 MOE | 246 |
| 389780 | 528 | 539 | TGTTCCAGCCCA | 1-9-2 MOE | 246 |
| 379692 | 528 | 539 | TGTTCCAGCCCA | 1-10-1 MOE | 246 |
| 392170 | 528 | 539 | TGTTCCAGCCCA | 1-10-1 Methyleneoxy BNA | 246 |
| 392173 | 528 | 539 | TGTTCCAGCCCA | 2-8-2 Methyleneoxy BNA | 246 |
| 405213 | 529 | 540 | ATGTTCCAGCCC | 2-8-2 MOE | 251 |
| 405214 | 564 | 575 | TGGTGATGCCCA | 2-8-2 MOE | 252 |
| 405215 | 565 | 576 | ATGGTGATGCCC | 2-8-2 MOE | 253 |
| 405216 | 566 | 577 | CATGGTGATGCC | 2-8-2 MOE | 254 |
| 379693 | 566 | 577 | CATGGTGATGCC | 1-10-1 MOE | 254 |
| 405217 | 567 | 578 | TCATGGTGATGC | 2-8-2 MOE | 256 |
| 405218 | 568 | 579 | ATCATGGTGATG | 2-8-2 MOE | 257 |
| 405219 | 587 | 598 | CCCTCCTGTCAC | 2-8-2 MOE | 258 |
| 405220 | 588 | 599 | GCCCTCCTGTCA | 2-8-2 MOE | 259 |
| 405221 | 589 | 600 | AGCCCTCCTGTC | 2-8-2 MOE | 260 |
| 405222 | 590 | 601 | CAGCCCTCCTGT | 2-8-2 MOE | 261 |
| 405223 | 591 | 602 | CCAGCCCTCCTG | 2-8-2 MOE | 262 |
| 405224 | 592 | 603 | GCCAGCCCTCCT | 2-8-2 MOE | 263 |
| 379694 | 629 | 640 | GACGAAGGTCTG | 1-10-1 MOE | 264 |
| 405225 | 707 | 718 | GTATTTGTCGAA | 2-8-2 MOE | 265 |
| 379695 | 737 | 748 | GGACACCGTCAG | 1-10-1 MOE | 266 |
| 379696 | 974 | 985 | CAGCTTCAGGTA | 1-10-1 MOE | 267 |
| 405226 | 998 | 1009 | CATGACCATGAG | 2-8-2 MOE | 268 |
| 405227 | 999 | 1010 | GCATGACCATGA | 2-8-2 MOE | 269 |
| 405228 | 1000 | 1011 | GGCATGACCATG | 2-8-2 MOE | 270 |
| 405229 | 1001 | 1012 | TGGCATGACCAT | 2-8-2 MOE | 271 |
| 405230 | 1002 | 1013 | CTGGCATGACCA | 2-8-2 MOE | 272 |
| 379697 | 1002 | 1013 | CTGGCATGACCA | 1-10-1 MOE | 272 |
| 405231 | 1003 | 1014 | CCTGGCATGACC | 2-8-2 MOE | 274 |
| 379698 | 1091 | 1102 | GCAGCCCACCTC | 1-10-1 MOE | 275 |
| 405232 | 1092 | 1103 | AGCAGCCCACCT | 2-8-2 MOE | 276 |
| 405233 | 1093 | 1104 | GAGCAGCCCACC | 2-8-2 MOE | 277 |
| 405234 | 1130 | 1141 | CATGAGCTTCAC | 2-8-2 MOE | 278 |
| 405235 | 1131 | 1142 | GCATGAGCTTCA | 2-8-2 MOE | 279 |
| 382677 | 1131 | 1143 | GGCATGAGCTTCA | 1-10-2 MOE | 280 |
| 388626 | 1132 | 1143 | GGCATGAGCTTC | 2-8-2 MOE | 281 |
| 379699 | 1132 | 1143 | GGCATGAGCTTC | 1-10-1 MOE | 281 |
| 405236 | 1133 | 1144 | GGGCATGAGCTT | 2-8-2 MOE | 283 |
| 405237 | 1157 | 1168 | CAGCATGAGTCC | 2-8-2 MOE | 284 |
| 405238 | 1158 | 1169 | CCAGCATGAGTC | 2-8-2 MOE | 285 |
| 379700 | 1158 | 1169 | CCAGCATGAGTC | 1-10-1 MOE | 285 |
| 405239 | 1159 | 1170 | GCCAGCATGAGT | 2-8-2 MOE | 287 |
| 379701 | 1230 | 1241 | CCATGGTGAAGA | 1-10-1 MOE | 288 |
| 405240 | 1542 | 1553 | CACAGCTGCCCG | 2-8-2 MOE | 289 |
| 405241 | 1543 | 1554 | ACACAGCTGCCC | 2-8-2 MOE | 290 |
| 405242 | 1544 | 1555 | CACACAGCTGCC | 2-8-2 MOE | 291 |
| 382678 | 1544 | 1556 | GCACACAGCTGCC | 1-10-2 MOE | 292 |
| 388627 | 1545 | 1556 | GCACACAGCTGC | 2-8-2 MOE | 293 |
| 379702 | 1545 | 1556 | GCACACAGCTGC | 1-10-1 MOE | 293 |
| 379703 | 1701 | 1712 | GCCGGAGACTGA | 1-10-1 MOE | 295 |
| 405243 | 1976 | 1987 | ATTGAGGTTGAC | 2-8-2 MOE | 296 |
| 405244 | 1977 | 1988 | CATTGAGGTTGA | 2-8-2 MOE | 297 |
| 405245 | 1978 | 1989 | GCATTGAGGTTG | 2-8-2 MOE | 298 |
| 405246 | 1979 | 1990 | GGCATTGAGGTT | 2-8-2 MOE | 299 |
| 405247 | 1980 | 1991 | GGGCATTGAGGT | 2-8-2 MOE | 300 |

**Table 2: Short antisense compounds targeted to SEQ ID NO: 3 and having 1 or 2 mismatches**

| **ISIS No** | **5' Target Site** | **3' Target Site** | **Sequence (5'-3')** | **Gapmer Motif** | **SEQ ID NO** |
|---|---|---|---|---|---|
| 405200 | 96 | 107 | CAATGACCAGCA | 2-8-2 MOE | 223 |
| 405215 | 382 | 393 | ATGGTGATGCCC | 2-8-2 MOE | 253 |
| 405216 | 383 | 394 | CATGGTGATGCC | 2-8-2 MOE | 254 |
| 379693 | 383 | 394 | CATGGTGATGCC | 1-10-1 MOE | 254 |
| 379701 | 471 | 482 | CCATGGTGAAGA | 1-10-1 MOE | 288 |
| 405218 | 472 | 483 | ATCATGGTGATG | 2-8-2 MOE | 257 |
| 405246 | 536 | 547 | GGCATTGAGGTT | 2-8-2 MOE | 299 |
| 405248 | 570 | 581 | AGATCTTGGTGA | 2-8-2 MOE | 243 |
| 379691 | 570 | 581 | AGATCTTGGTGA | 1-10-1 MOE | 243 |
| 379698 | 683 | 694 | GCAGCCCACCTC | 1-10-1 MOE | 275 |
| 405232 | 684 | 695 | AGCAGCCCACCT | 2-8-2 MOE | 276 |
| 379711 | 685 | 696 | TAGCCGCCCACA | 1-10-1 MOE | 226 |
| 388628 | 685 | 696 | TAGCCGCCCACA | 2-8-2 MOE | 226 |
| 379698 | 950 | 961 | GCAGCCCACCTC | 1-10-1 MOE | 275 |
| 405232 | 951 | 962 | AGCAGCCCACCT | 2-8-2 MOE | 276 |
| 405235 | 978 | 989 | GCATGAGCTTCA | 2-8-2 MOE | 279 |
| 382677 | 978 | 990 | GGCATGAGCTTCA | 1-10-2 MOE | 280 |
| 388626 | 979 | 990 | GGCATGAGCTTC | 2-8-2 MOE | 281 |
| 379699 | 979 | 990 | GGCATGAGCTTC | 1-10-1 MOE | 281 |
| 405236 | 980 | 991 | GGGCATGAGCTT | 2-8-2 MOE | 283 |
| 379698 | 1043 | 1054 | GCAGCCCACCTC | 1-10-1 MOE | 275 |
| 405239 | 1171 | 1182 | GCCAGCATGAGT | 2-8-2 MOE | 287 |
| 405209 | 1213 | 1224 | CTGCCGATGTTG | 2-8-2 MOE | 236 |
| 405233 | 1364 | 1375 | GAGCAGCCCACC | 2-8-2 MOE | 277 |
| 405240 | 1366 | 1377 | CACAGCTGCCCG | 2-8-2 MOE | 289 |
| 405211 | 1500 | 1511 | CCAGGCCCACAA | 2-8-2 MOE | 238 |
| 405212 | 1501 | 1512 | GCCAGGCCCACA | 2-8-2 MOE | 239 |
| 379695 | 1643 | 1654 | GGACACCGTCAG | 1-10-1 MOE | 266 |
| 379698 | 1875 | 1886 | GCAGCCCACCTC | 1-10-1 MOE | 275 |
| 405239 | 1993 | 2004 | GCCAGCATGAGT | 2-8-2 MOE | 287 |
| 405211 | 2210 | 2221 | CCAGGCCCACAA | 2-8-2 MOE | 238 |
| 405212 | 2211 1 | 2222 | GCCAGGCCCACA | 2-8-2 MOE | 239 |

In certain embodiments, a target region is nucleotides 85-184 of SEQ ID NO: 3. In certain embodiments, a short antisense compound is targeted to nucleotides 85-184 of SEQ ID NO: 3. In certain such embodiments, a short antisense compound targeted to nucleotides 85-184 comprises a nucleotide sequence selected from SEQ ID NO 214, 215, 216, 217, 218, 219, 221, 222, 223, 224, 225, or 227. In certain such embodiments, a short antisense compound targeted to nucleotides 85-184 of SEQ ID NO: 3 is selected from Isis No 379684, 405193, 405194, 405195, 405196, 405197, 379685, 405198, 405199, 405200, 405201, 379686, 379711 or 388628.

In certain embodiments, a target region is nucleotides 113-132 of SEQ ID NO: 3. In certain embodiments, a short antisense compound is targeted to nucleotides 113-132 of SEQ ID NO: 3. In certain such embodiments, a short antisense compound targeted to nucleotides 113-132 comprises a nucleotide sequence selected from SEQ ID NO 215, 216, 217, 218, 219, 221, 222, 223, or 224. In certain such embodiments, a short antisense compound targeted to nucleotides 113-132 of SEQ ID NO: 3 is selected from Isis No 405193, 405194, 405195, 405196, 405197, 379685, 405198, 405199, 405200, or 405201.

In certain embodiments, a target region is nucleotides 207-329 of SEQ ID NO: 3. In certain embodiments, a short antisense compound is targeted to nucleotides 207-329 of SEQ ID NO: 3. In certain such embodiments, a short antisense compound targeted to nucleotides 207-329 comprises a nucleotide sequence selected from SEQ ID NO 228, 229, 230, 232, 233, 234, 235, 236, 237, 238, 239, 240, or 241. In certain such embodiments, a short antisense compound targeted to nucleotides 207-329 of SEQ ID NO: 3 is selected from Isis No 405202, 405203, 405204, 379687, 405205, 405206, 405207, 405208, 405209, 405210, 405211, 405212, 379688, or 379689.

In certain embodiments, a target region is nucleotides 207-273 of SEQ ID NO: 3. In certain embodiments, a short antisense compound is targeted to nucleotides 207-273 of SEQ ID NO: 3. In certain such embodiments, a short antisense compound targeted to nucleotides 207-273 comprises a nucleotide sequence selected from SEQ ID NO 228, 229, 230, 232, 233, 234, 235, 236, 237, 238, or 239. In certain such embodiments, a short antisense compound targeted to nucleotides 207-273 of SEQ ID NO: 3 is selected from Isis No 405202, 405203, 405204, 379687, 405205, 405206, 405207, 405208, 405209, 405210, 405211, or 405212.

In certain embodiments, a target region is nucleotides 207-219 of SEQ ID NO: 3. In certain embodiments, a short antisense compound is targeted to nucleotides 207-219 of SEQ ID NO: 3. In certain such embodiments, a short antisense compound targeted to nucleotides 207-219 comprises a nucleotide sequence selected from SEQ ID NO 228 or 229. In certain such embodiments, a short antisense compound targeted to nucleotides 207-219 of SEQ ID NO: 3 is selected from Isis NO.. 405202 or 405203.

In certain embodiments, a target region is nucleotides 236-252 of SEQ ID NO: 3. In certain embodiments, a short antisense compound is targeted to nucleotides 236-252 of SEQ ID NO: 3. In certain such embodiments, a short antisense compound targeted to nucleotides 236-252 comprises a nucleotide sequence selected from SEQ ID NO 230, 232, 233, 234, 235, or 236. In certain such embodiments, a short antisense compound targeted to nucleotides 236-252 of SEQ ID NO: 3 is selected from Isis NO. 405204, 379687, 405205, 405206, 405207, 405208, or 405209.

In certain embodiments, a target region is nucleotides 260-273 of SEQ ID NO: 3. In certain embodiments, a short antisense compound is targeted to nucleotides 260-273 of SEQ ID NO: 3. In certain such embodiments, a short antisense compound targeted to nucleotides 260-273 comprises a nucleotide sequence selected from SEQ ID NO 237, 238, or 239. In certain such embodiments, a short antisense compound targeted to nucleotides 260-273 of SEQ ID NO: 3 is selected from Isis NO. 405210, 405211, or 405212.

In certain embodiments, a target region is nucleotides 435-640 of SEQ ID NO: 3. In certain embodiments, a short antisense compound is targeted to nucleotides 435-640 of SEQ ID NO: 3. In certain such embodiments, a short antisense compound targeted to nucleotides 435-640 comprises a nucleotide sequence selected from SEQ ID NO 242, 243, 245, 246, 251, 252, 253, 254, 256, 257, 258, 259, 260, 261, 262, 263, or 264. In certain such embodiments, a short antisense compound targeted to nucleotides 435-640 of SEQ ID NO: 3 is selected from Isis NO. 379690, 405248, 379691, 389780, 379692, 382676, 388625, 392170, 392173, 405213, 405214, 405215, 405216, 379693, 405217, 405218, 405219, 405220, 405221, 405222, 405223, 405224, or 379694.

In certain embodiments, a target region is nucleotides 527-540 of SEQ ID NO: 3. In certain embodiments, a short antisense compound is targeted to nucleotides 527-540 of SEQ ID NO: 3. In certain such embodiments, a short antisense compound targeted to nucleotides 527-540 comprises a nucleotide sequence selected from SEQ ID NO 245, 246, or 251. In certain such embodiments, a short antisense compound targeted to nucleotides 527-540 of SEQ ID NO: 3 is selected from Isis NO. 389780, 379692, 382676, 388626, 392170, 392173, or 405213.

In certain embodiments, a target region is nucleotides 564-603 of SEQ ID NO: 3. In certain embodiments, a short antisense compound is targeted to nucleotides 564-603 of SEQ ID NO: 3. In certain such embodiments, a short antisense compound targeted to nucleotides 564-603 comprises a nucleotide sequence selected from SEQ ID NO 252, 253, 254, 256, 257, 258, 259, 260, 261, 262, or 263. In certain such embodiments, a short antisense compound targeted to nucleotides 564-603 of SEQ ID NO: 3 is selected from Isis NO. 405214, 405215, 405216, 379693, 405217, 405218, 405219, 405220, 405221, 405222, 405223, or 405224.

In certain embodiments, a target region is nucleotides 564-579 of SEQ ID NO: 3. In certain embodiments, a short antisense compound is targeted to nucleotides 564-579 of SEQ ID NO: 3. In certain such embodiments, a short antisense compound targeted to nucleotides 564-579 comprises a nucleotide sequence selected from SEQ ID NO 252, 253, 254, 256, or 257. In certain such embodiments, a short antisense compound targeted to nucleotides 564-579 of SEQ ID NO: 3 is selected from Isis NO. 405214, 405215, 405216, 379693, 405217, or 405218.

In certain embodiments, a target region is nucleotides 587-603 of SEQ ID NO: 3. In certain embodiments, a short antisense compound is targeted to nucleotides 587-603 of SEQ ID NO: 3. In certain such embodiments, a short antisense compound targeted to nucleotides 587-603 comprises a nucleotide sequence selected from SEQ ID NO 258, 259, 260, 261, 262, or 263. In certain such embodiments, a short antisense compound targeted to nucleotides 587-603 of SEQ ID NO: 3 is selected from Isis NO. 405219, 405220, 405221, 405222, 405223, or 405224.

In certain embodiments, a target region is nucleotides 974-1014 of SEQ ID NO: 3. In certain embodiments, a short antisense compound is targeted to nucleotides 974-1014 of SEQ ID NO: 3. In certain such embodiments, a short antisense compound targeted to nucleotides 974-1014 comprises a nucleotide sequence selected from SEQ ID NO 267, 268, 269, 270, 271, 272, or 274. In certain such embodiments, a short antisense compound targeted to nucleotides 974-1014 of SEQ ID NO: 3 is selected from Isis NO. 379696, 405226, 405227, 405228, 405229, 405230, 379697, or 405231.

In certain embodiments, a target region is nucleotides 998-1014 of SEQ ID NO: 3. In certain embodiments, a short antisense compound is targeted to nucleotides 998-1014 of SEQ ID NO: 3. In certain such embodiments, a short antisense compound targeted to nucleotides 998-1014 comprises a nucleotide sequence selected from SEQ ID NO 268, 269, 270, 271, 272, or 274. In certain such embodiments, a short antisense compound targeted to nucleotides 998-1014 of SEQ ID NO: 3 is selected from Isis NO. 405226, 405227, 405228, 405229, 405230, 379697, or 405231.

In certain embodiments, a target region is nucleotides 1091-1170 of SEQ ID NO: 3. In certain embodiments, a short antisense compound is targeted to nucleotides 1091-1170 of SEQ ID NO: 3. In certain such embodiments, a short antisense compound targeted to nucleotides 1091-1170 comprises a nucleotide sequence selected from SEQ ID NO 275, 276, 277, 278, 279, 280, 281, 283, 284, 285, 286, or 287. In certain such embodiments, a short antisense compound targeted to nucleotides 1091-1170 of SEQ ID NO: 3 is selected from Isis NO. 379698, 405232, 405233, 405234, 405235, 388626, 379699, 382677, 405236, 405237, 405238, 379700, or 405239.

In certain embodiments, a target region is nucleotides 1091-1104 of SEQ ID NO: 3. In certain embodiments, a short antisense compound is targeted to nucleotides 1091-1104 of SEQ ID NO: 3. In certain such embodiments, a short antisense compound targeted to nucleotides 1091-1104 comprises a nucleotide sequence selected from SEQ ID NO 275, 276, or 277. In certain such embodiments, an short antisense compound targeted to nucleotides 1091-1104 of SEQ ID NO: 3 is selected from Isis NO. 379698, 405232, or 405233.

In certain embodiments, a target region is nucleotides 1130-1144 of SEQ ID NO: 3. In certain embodiments, a short antisense compound is targeted to nucleotides 1130-1144 of SEQ ID NO: 3. In certain such embodiments, a short antisense compound targeted to nucleotides 1130-1144 comprises a nucleotide sequence selected from SEQ ID NO 278, 279, 280, 281, or 283. In certain such embodiments, a short antisense compound targeted to nucleotides 1130-1144 of SEQ ID NO: 3 is selected from Isis NO. 405234, 405235, 388626, 379699, 382677, or 405236.

In certain embodiments, a target region is nucleotides 1157-1170 of SEQ ID NO: 3. In certain embodiments, a short antisense compound is targeted to nucleotides 1157-1170 of SEQ ID NO: 3. In certain such embodiments, a short antisense compound targeted to nucleotides 1157-1170 comprises a nucleotide sequence selected from SEQ ID NO 284, 285, or 287. In certain such embodiments, a short antisense compound targeted to nucleotides 1157-1170 of SEQ ID NO: 3 is selected from Isis NO. 405237, 405238, 379700, or 405239.

In certain embodiments, a target region is nucleotides 1542-1556 of SEQ ID NO: 3. In certain embodiments, a short antisense compound is targeted to nucleotides 1542-1556 of SEQ ID NO: 3. In certain such embodiments, a short antisense compound targeted to nucleotides 1542-1556 comprises a nucleotide sequence selected from SEQ ID NO 289, 290, 291, 292, or 293. In certain such embodiments, a short antisense compound targeted to nucleotides 1542-1556 of SEQ ID NO: 3 is selected from Isis NO. 405240, 405241, 405242, 388629, 379702, or 382678.

In certain embodiments, a target region is nucleotides 1976-1991 of SEQ ID NO: 3. In certain embodiments, a short antisense compound is targeted to nucleotides 1976-1991 of SEQ ID NO: 3. In certain such embodiments, a short antisense compound targeted to nucleotides 1976-1991 comprises a nucleotide sequence selected from SEQ ID NO 296, 297, 298, 299, or 300. In certain such embodiments, a short antisense compound targeted to nucleotides 1976-1991 of SEQ ID NO: 3 is selected from Isis NO. 405243, 405244, 405245, 405246, or 405247.

In certain embodiments, short antisense compounds targeted to an SGLT2 nucleic acid are 8 to 16, preferably 9 to 15, more preferably 9 to 14, more preferably 10 to 14 nucleotides in length. In certain embodiments, short antisense compounds targeted to an SGLT2 nucleic acid are 9 to 14 nucleotides in length. In certain embodiments, short antisense compounds targeted to an SGLT2 nucleic acid are 10 to 14 nucleotides in length. In certain embodiments, such short antisense compounds are short antisense oligonucleotides.

In certain embodiments, short antisense compounds targeted to an SGLT2 nucleic acid are short gapmers. In certain such embodiments, short gapmers targeted to an SGLT2 nucleic acid comprise at least one high affinity modification in one or more wings of the compound. In certain embodiments, short antisense compounds targeted to an SGLT2 nucleic acid comprise 1 to 3 high-affinity modifications in each wing. In certain such embodiments, the nucleosides or nucleotides of the wing comprise a 2' modification. In certain such embodiments, the monomers of the wing are BNA's. In certain such embodiments, the monomers of the wing are selected from α-L-Methyleneoxy (4'-CH₂-O-2') BNA , β-D-Methyleneoxy (4'-CH₂-O-2') BNA , Ethyleneoxy (4'-(CH₂)₂-O-2') BNA, Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, short antisense compounds targeted to an SGLT2 nucleic acid comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, short antisense compounds targeted to an SGLT2 nucleic acid have uniform monomeric linkages. In certain such embodiments, those linkages are all phosphorothioate linkages. In certain embodiments, the linkages are all phosphodiester linkages. In certain embodiments, short antisense compounds targeted to an SGLT2 nucleic acid have mixed backbones.

In certain embodiments, short antisense compounds targeted to an SGLT2 nucleic acid are 8 monomers in length. In certain embodiments, short antisense compounds targeted to an SGLT2 nucleic acid are 9 monomers in length. In certain embodiments, short antisense compounds targeted to an SGLT2 nucleic acid are 10 monomers in length. In certain embodiments, short antisense compounds targeted to an SGLT2 nucleic acid are 11 monomers in length. In certain embodiments, short antisense compounds targeted to an SGLT2 nucleic acid are monomers in length. In certain embodiments, short antisense compounds targeted to an SGLT2 nucleic acid are 13 monomers in length. In certain embodiments, short antisense compounds targeted to an SGLT2 nucleic acid are 14 monomers in length. In certain embodiments, short antisense compounds targeted to an SGLT2 nucleic acid are 15 monomers in length. In certain embodiments, short antisense compounds targeted to an SGLT2 nucleic acid are 16 monomers in length. In certain embodiments, short antisense compounds targeted to an SGLT2 nucleic acid comprise 9 to 15 monomers. In certain embodiments, short antisense compounds targeted to an SGLT2 nucleic acid comprise 10 to 15 monomers. In certain embodiments, short antisense compounds targeted to an SGLT2 nucleic acid comprise 12 to 14 monomers. In certain embodiments, short antisense compounds targeted to an SGLT2 nucleic acid comprise 12 to 14 nucleotides or nucleosides.

In certain embodiments, disclosed herein are methods of modulating expression of SGLT2. In certain embodiments, such methods comprise use of one or more short antisense compound targeted to an SGLT2 nucleic acid wherein the short antisense compound targeted to an SGLT2 nucleic acid is from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of SGLT2 using one or more short antisense compounds targeted to an SGLT2 nucleic acid of 8, 9, 10, 11, 12, 13, 14, 15 or 16 monomers.

In certain embodiments, methods of modulating SGLT2 comprise use of a short antisense compound targeted to an SGLT2 nucleic acid that is 8 monomers in length. In certain embodiments, methods of modulating SGLT2 comprise use of a short antisense compound targeted to an SGLT2 nucleic acid that is 9 monomers in length. In certain embodiments, methods of modulating SGLT2 comprise use of a short antisense compound targeted to an SGLT2 nucleic acid that is 10 monomers in length. In certain embodiments, methods of modulating SGLT2 comprise use of a short antisense compound targeted to an SGLT2 nucleic acid that is 11 monomers in length. In certain embodiments, methods of modulating SGLT2 comprise use of a short antisense compound targeted to an SGLT2 nucleic acid that is 12 monomers in length. In certain embodiments, methods of modulating SGLT2 comprise use of a short antisense compound targeted to an SGLT2 nucleic acid that is 13 monomers in length In certain embodiments, methods of modulating SGLT2 comprise use of a short antisense compound targeted to an SGLT2 nucleic acid that is 14 monomers in length. In certain embodiments, methods of modulating SGLT2 comprise use of a short antisense compound targeted to an SGLT2 nucleic acid that is 15 monomers in length. In certain embodiments, methods of modulating SGLT2 comprise use of a short antisense compound targeted to an SGLT2 nucleic acid that is 16 monomers in length.

In certain embodiments, methods of modulating expression of SGLT2 comprise use of a short antisense compound targeted to an SGLT2 nucleic acid comprising 9 to 15 monomers. In certain embodiments, methods of modulating expression of SGLT2 comprise use of a short antisense compound targeted to an SGLT2 nucleic acid comprising 10 to 15 monomers. In certain embodiments, methods of modulating expression of SGLT2 comprise use of a short antisense compound targeted to an SGLT2 nucleic acid comprising 12 to 14 monomers. In certain embodiments, methods of modulating expression of SGLT2 comprise use of a short antisense compound targeted to an SGLT2 nucleic acid comprising 12 or 14 nucleotides or nucleosides.

### Salts, prodrugs and bioequivalents

The antisense compounds provided herein comprise any pharmaceutically acceptable salts, esters, or salts of such esters, or any other functional chemical equivalent which, upon administration to an animal including a human, is capable of providing (directly or indirectly) the biologically active metabolite or residue thereof. Accordingly, for example, the disclosure is also drawn to prodrugs and pharmaceutically acceptable salts of the antisense compounds, pharmaceutically acceptable salts of such prodrugs, and other bioequivalents.

The term "prodrug" indicates a therapeutic agent that is prepared in an inactive or less active form that is converted to an active form (i.e., drug) within the body or cells thereof by the action of endogenous enzymes, chemicals, and/or conditions. In particular, prodrugs versions of the oligonucleotides are prepared as SATE ((S-acetyl-2-thioethyl) phosphate) derivatives according to the methods disclosed in WO 93/24510 or WO 94/26764. Prodrugs can also include antisense compounds wherein one or both ends comprise nucleobases that are cleaved (e.g., by incorporating phosphodiester backbone linkages at the ends) to produce the active compound. In certain embodiments, one or more non-drug moieties is cleaved from a prodrug to yield the active form. In certain such embodiments, such non-drug moieties is not a nucleotide or oligonucleotide.

The term "pharmaceutically acceptable salts" refers to physiologically and pharmaceutically acceptable salts of the compounds described herein: i.e., salts that retain the desired biological activity of the parent compound and do not impart undesired toxicological effects thereto. Sodium salts of antisense oligonucleotides are useful and are well accepted for therapeutic administration to humans.

In certain embodiments, salts, including, but not limited to sodium salts, of double stranded nucleic acids (including but not limited to dsRNA compounds) are also provided.

### G. Certain Pharmaceutical Compositions

In certain embodiments, pharmaceutical compositions of the present invention comprise one or more short antisense compound and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition of the present invention is prepared using known techniques, including, but not limited to mixing, dissolving, granulating, dragee-making, levigating, emulsifying, encapsulating, entrapping or tabletting processes.

In certain embodiments, a pharmaceutical composition of the present invention is a liquid (e.g., a suspension, elixir and/or solution). In certain of such embodiments, a liquid pharmaceutical composition is prepared using ingredients known in the art, including, but not limited to, water, glycols, oils, alcohols, flavoring agents, preservatives, and coloring agents.

In certain embodiments, a pharmaceutical composition of the present invention is a solid (e.g., a powder, tablet, and/or capsule). In certain of such embodiments, a solid pharmaceutical composition comprising one or more oligonucleotides is prepared using ingredients known in the art, including, but not limited to, starches, sugars, diluents, granulating agents, lubricants, binders, and disintegrating agents.

In certain embodiments, a pharmaceutical composition of the present invention is formulated as a depot preparation. Certain such depot preparations are typically longer acting than non-depot preparations. In certain embodiments, such preparations are administered by implantation (for example subcutaneously or intramuscularly) or by intramuscular injection. In certain embodiments, depot preparations are prepared using suitable polymeric or hydrophobic materials (for example an emulsion in an acceptable oil) or ion exchange resins, or as sparingly soluble derivatives, for example, as a sparingly soluble salt.

In certain embodiments, a pharmaceutical composition of the present invention comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition of the present invention comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition of the present invention comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80.TM., and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80.TM.; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition of the present invention comprises a sustained-release system. A non-limiting example of such a sustained-release system is a semi-permeable matrix of solid hydrophobic polymers. In certain embodiments, sustained-release systems may, depending on their chemical nature, release pharmaceutical agents over a period of hours, days, weeks or months.

In certain embodiments, a pharmaceutical composition of the present invention is prepared for oral administration. In certain of such embodiments, a pharmaceutical composition is formulated by combining one or more oligonucleotides with one or more pharmaceutically acceptable carriers. Certain of such carriers enable pharmaceutical compositions to be formulated as tablets, pills, dragees, capsules, liquids, gels, syrups, slurries, suspensions and the like, for oral ingestion by a subject. In certain embodiments, pharmaceutical compositions for oral use are obtained by mixing oligonucleotide and one or more solid excipient. Suitable excipients include, but are not limited to, fillers, such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). In certain embodiments, such a mixture is optionally ground and auxiliaries are optionally added. In certain embodiments, pharmaceutical compositions are formed to obtain tablets or dragee cores. In certain embodiments, disintegrating agents (e.g., cross-linked polyvinyl pyrrolidone, agar, or alginic acid or a salt thereof, such as sodium alginate) are added.

In certain embodiments, dragee cores are provided with coatings. In certain such embodiments, concentrated sugar solutions may be used, which may optionally comprise gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to tablets or dragee coatings.

In certain embodiments, pharmaceutical compositions for oral administration are push-fit capsules made of gelatin. Certain of such push-fit capsules comprise one or more pharmaceutical agents of the present invention in admixture with one or more filler such as lactose, binders such as starches, and/or lubricants such as talc or magnesium stearate and, optionally, stabilizers. In certain embodiments, pharmaceutical compositions for oral administration are soft, sealed capsules made of gelatin and a plasticizer, such as glycerol or sorbitol. In certain soft capsules, one or more pharmaceutical agents of the present invention are be dissolved or suspended in suitable liquids, such as fatty oils, liquid paraffin, or liquid polyethylene glycols. In addition, stabilizers may be added.

In certain embodiments, pharmaceutical compositions are prepared for buccal administration. Certain of such pharmaceutical compositions are tablets or lozenges formulated in conventional manner.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may comprise formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may comprise substances that increase the viscosity of the suspension, such as sodium carboxymethyl cellulose, sorbitol, or dextran. Optionally, such suspensions may also comprise suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition is prepared for administration by inhalation. Certain of such pharmaceutical compositions for inhalation are prepared in the form of an aerosol spray in a pressurized pack or a nebulizer. Certain of such pharmaceutical compositions comprise a propellant, e.g., dichlorodifluoromethane, trichlorofluoromethane, dichlorotetrafluoroethane, carbon dioxide or other suitable gas. In certain embodiments using a pressurized aerosol, the dosage unit may be determined with a valve that delivers a metered amount. In certain embodiments, capsules and cartridges for use in an inhaler or insufflator may be formulated. Certain of such formulations comprise a powder mixture of a pharmaceutical agent of the invention and a suitable powder base such as lactose or starch.

In certain embodiments, a pharmaceutical composition is prepared for rectal administration, such as a suppositories or retention enema. Certain of such pharmaceutical compositions comprise known ingredients, such as cocoa butter and/or other glycerides.

In certain embodiments, a pharmaceutical composition is prepared for topical administration. Certain of such pharmaceutical compositions comprise bland moisturizing bases, such as ointments or creams. Exemplary suitable ointment bases include, but are not limited to, petrolatum, petrolatum plus volatile silicones, lanolin and water in oil emulsions such as Eucerin.TM., available from Beiersdorf (Cincinnati, Ohio). Exemplary suitable cream bases include, but are not limited to, Nivea.TM. Cream, available from Beiersdorf (Cincinnati, Ohio), cold cream (USP), Purpose Cream.TM., available from Johnson & Johnson (New Brunswick, N.J.), hydrophilic ointment (USP) and Lubriderm.TM., available from Pfizer (Morris Plains, N.J.).

In certain embodiments, a pharmaceutical composition of the present invention comprises an oligonucleotide in a therapeutically effective amount. In certain embodiments, the therapeutically effective amount is sufficient to prevent, alleviate or ameliorate symptoms of a disease or to prolong the survival of the subject being treated. Determination of a therapeutically effective amount is well within the capability of those skilled in the art.

In certain embodiments, one or more short antisense compound of the present invention is formulated as a prodrug. In certain embodiments, upon in vivo administration, a prodrug is chemically converted to the biologically, pharmaceutically or therapeutically more active form of the short antisense compound. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, a prodrug is produced by modifying a pharmaceutically active compound such that the active compound will be regenerated upon in vivo administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see, e.g., Nogrady (1985) Medicinal Chemistry A Biochemical Approach, Oxford University Press, New York, pages 388-392).

In certain embodiments, a pharmaceutical composition comprising one or more pharmaceutical agents of the present invention is useful for treating a conditions or disorders in a mammalian, and particularly in a human, subject. Suitable administration routes include, but are not limited to, oral, rectal, transmucosal, intestinal, enteral, topical, suppository, through inhalation, intrathecal, intraventricular, intraperitoneal, intranasal, intraocular and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., in the renal or cardiac area).

In certain embodiments, short antisense compounds, compared to their parent oligonucleotides, make them particularly suited to oral administration. In certain embodiments, short antisense compounds are better suited for oral administration than their parent oligonucleotides because they have increased potency compared to those parent oligonucleotides. In certain embodiments, short antisense compounds are better suited for oral administration than their parent oligonucleotides because they have better stability, availability or solubility properties compared to those parent oligonucleotides.

In a further aspect, a pharmaceutical agent is sterile lyophilized oligonucleotide that is reconstituted with a suitable diluent, e.g., sterile water for injection. The reconstituted product is administered as a subcutaneous injection or as an intravenous infusion after dilution into saline. The lyophilized drug product consists of the oligonucleotide which has been prepared in water for injection, adjusted to pH 7.0-9.0 with acid or base during preparation, and then lyophilized. The lyophilized oligonucleotide may be 25-800 mg of the oligonucleotide. It is understood that this encompasses 25, 50, 75, 100, 125, 150, 175, 200, 225, 250, 275, 300, 325, 350, 375, 425, 450, 475, 500, 525, 550, 575, 600, 625, 650, 675, 700, 725, 750, 775, and 800 mg of lyophilized oligonucleotide. The lyophilized drug product may be packaged in a 2 mL Type I, clear glass vial (ammonium sulfate-treated), stoppered with a bromobutyl rubber closure and sealed with an aluminum FLIP-OFF® overseal.

The compositions of the present invention may additionally comprise other adjunct components conventionally found in pharmaceutical compositions, at their art-established usage levels. Thus, for example, the compositions may comprise additional, compatible, pharmaceutically-active materials such as, for example, antipruritics, astringents, local anesthetics or anti-inflammatory agents, or may comprise additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation.

The antisense compounds provided herein may also be admixed, encapsulated, conjugated or otherwise associated with other molecules, molecule structures or mixtures of compounds.

Also described herein are pharmaceutical compositions and formulations which include the antisense compounds provided herein. The pharmaceutical compositions may be administered in a number of ways depending upon whether local or systemic treatment is desired and upon the area to be treated. In a preferred embodiment, administration is topical to the surface of the respiratory tract, particularly pulmonary, e.g., by nebulization, inhalation, or insufflation of powders or aerosols, by mouth and/or nose.

The pharmaceutical formulations described herein, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers, finely divided solid carriers, or both, and then, if necessary, shaping the product (e.g., into a specific particle size for delivery). In a preferred embodiment, the pharmaceutical formulations are prepared for pulmonary administration in an appropriate solvent, e.g., water or normal saline, possibly in a sterile formulation, with carriers or other agents to allow for the formation of droplets of the desired diameter for delivery using inhalers, nasal delivery devices, nebulizers, and other devices for pulmonary delivery. Alternatively, the pharmaceutical formulations may be formulated as dry powders for use in dry powder inhalers.

A "pharmaceutical carrier" or "excipient" can be a pharmaceutically acceptable solvent, suspending agent or any other pharmacologically inert vehicle for delivering one or more nucleic acids to an individual and are known in the art. The excipient may be liquid or solid and is selected, with the planned manner of administration in mind, so as to provide for the desired bulk, consistency, etc., when combined with a nucleic acid and the other components of a given pharmaceutical composition.

### H. Certain Therapeutic Uses

In certain embodiments, antisense compounds are used to modulate the expression of a target gene in an animal, such as a human. In certain embodiments, such compounds can be used to treat metabolic disorders or modulate one or more disease indications. For example, the methods comprise the step of administering to said animal in need of therapy for a disease or condition associated with a target gene an effective amount of an antisense compound that modulates expression of the target gene. Antisense compounds provided herein which effectively modulate expression of a target RNA or protein products of expression are considered active antisense compounds. Active antisense compounds also include compounds which effectively modulate one or more of a number of disease indications, including metabolic and cardiovascular disease indications, examples of which are described below.

Modulation of expression of a target gene can be measured in a bodily fluid, which may or may not contain cells; tissue; or organ of the animal. Methods of obtaining samples for analysis, such as body fluids (e.g., sputum, serum, urine), tissues (e.g., biopsy), or organs, and methods of preparation of the samples to allow for analysis are well known to those skilled in the art. Methods for analysis of RNA and protein levels are discussed above and are well known to those skilled in the art. The effects of treatment can be assessed by measuring biomarkers, or disease indications, associated with the target gene expression in the aforementioned fluids, tissues or organs, collected from an animal contacted with one or more compounds described herein, by routine clinical methods known in the art. These biomarkers include but are not limited to: liver transaminases, bilirubin, albumin, blood urea nitrogen, creatine and other markers of kidney and liver function; interleukins, tumor necrosis factors, intracellular adhesion molecules, C-reactive protein, chemokines, cytokines, and other markers of inflammation.

The antisense compounds provided herein can be utilized in pharmaceutical compositions by adding an effective amount of a compound to a suitable pharmaceutically acceptable diluent or carrier. Acceptable carriers and diluents are well known to those skilled in the art. Selection of a diluent or carrier is based on a number of factors, including, but not limited to, the solubility of the compound and the route of administration. Such considerations are well understood by those skilled in the art. In one aspect, the antisense compounds described herein inhibit expression of a target gene. The compounds can also be used in the manufacture of a medicament for the treatment of diseases and disorders related to a target gene.

Methods whereby bodily fluids, organs or tissues are contacted with an effective amount of one or more of the antisense compounds or compositions provided herein are also contemplated. Bodily fluids, organs or tissues can be contacted with one or more of the compounds resulting in modulation of target gene expression in the cells of bodily fluids, organs or tissues. An effective amount can be determined by monitoring the modulatory effect of the antisense compound or compounds or compositions on target nucleic acids or their products by methods routine to the skilled artisan.

### Co-administration

In certain embodiments, two or more antisense compounds are co-administered. In certain embodiments, pharmaceutical compositions include one or more antisense compounds, particularly oligonucleotides, targeted to a first nucleic acid and one or more antisense compounds targeted to a second nucleic acid target. One or more of those antisense compounds may be a short antisense compound. In certain embodiments, pharmaceutical compositions include two or more antisense compounds targeted to different regions of the same nucleic acid target. One or more of such antisense compounds may be a short antisense compound. Two or more combined compounds may be used together or sequentially.

In certain embodiments, one or more pharmaceutical compositions are co-administered with one or more other pharmaceutical agents. In certain embodiments, such one or more other pharmaceutical agents are designed to treat the same disease or condition as the one or more pharmaceutical compositions of the present invention. In certain embodiments, such one or more other pharmaceutical agents are designed to treat a different disease or condition as the one or more pharmaceutical compositions of the present invention. In certain embodiments, such one or more other pharmaceutical agents are designed to treat an undesired effect of one or more pharmaceutical compositions of the present invention. In certain embodiments, one or more pharmaceutical compositions of the present invention are co-administered with another pharmaceutical agent to treat an undesired effect of that other pharmaceutical agent. In certain embodiments, one or more pharmaceutical compositions of the present invention and one or more other pharmaceutical agents are administered at the same time. In certain embodiments, one or more pharmaceutical compositions of the present invention and one or more other pharmaceutical agents are administered at different times. In certain embodiments, one or more pharmaceutical compositions of the present invention and one or more other pharmaceutical agents are prepared together in a single formulation. In certain embodiments, one or more pharmaceutical compositions of the present invention and one or more other pharmaceutical agents are prepared separately.

In certain embodiments, pharmaceutical agents that may be co-administered with a pharmaceutical composition of the present invention include lipid-lowering agents. In certain such embodiments, pharmaceutical agents that may be co-administered with a pharmaceutical composition of the present invention include, but are not limited to atorvastatin, simvastatin, rosuvastatin, and ezetimibe. In certain such embodiments, the lipid-lowering agent is administered prior to administration of a pharmaceutical composition of the present invention. In certain such embodiments, the lipid-lowering agent is administered following administration of a pharmaceutical composition of the present invention. In certain such embodiments the lipid-lowering agent is administered at the same time as a pharmaceutical composition of the present invention. In certain such embodiments the dose of a co-administered lipid-lowering agent is the same as the dose that would be administered if the lipid-lowering agent was administered alone. In certain such embodiments the dose of a co-administered lipid-lowering agent is lower than the dose that would be administered if the lipid-lowering agent was administered alone. In certain such embodiments the dose of a co-administered lipid-lowering agent is greater than the dose that would be administered if the lipid-lowering agent was administered alone.

In certain embodiments, a co-administered lipid-lowering agent is a HMG-CoA reductase inhibitor. In certain such embodiments the HMG-CoA reductase inhibitor is a statin. In certain such embodiments the statin is selected from atorvastatin, simvastatin, pravastatin, fluvastatin, and rosuvastatin. In certain embodiments, a co-administered lipid-lowering agent is a cholesterol absorption inhibitor. In certain such embodiments, cholesterol absorption inhibitor is ezetimibe. In certain embodiments, a co-administered lipid-lowering agent is a co-formulated HMG-CoA reductase inhibitor and cholesterol absorption inhibitor. In certain such embodiments the co-formulated lipid-lowering agent is ezetimibe/simvastatin. In certain embodiments, a co-administered lipid-lowering agent is a microsomal triglyceride transfer protein inhibitor.

In certain embodiments, a co-administered pharmaceutical agent is a bile acid sequestrant. In certain such embodiments, the bile acid sequestrant is selected from cholestyramine, colestipol, and colesevelam.

In certain embodiments, a co-administered pharmaceutical agent is a nicotinic acid. In certain such embodiments, the nicotinic acid is selected from immediate release nicotinic acid, extended release nicotinic acid, and sustained release nicotinic acid.

In certain embodiments, a co-administered pharmaceutical agent is a fibric acid. In certain such embodiments, a fibric acid is selected from gemfibrozil, fenofibrate, clofibrate, bezafibrate, and ciprofibrate.

Further examples of pharmaceutical agents that may be co-administered with a pharmaceutical composition of the present invention include, but are not limited to, corticosteroids, including but not limited to prednisone; immunoglobulins, including, but not limited to intravenous immunoglobulin (IVIg); analgesics (e.g., acetaminophen); anti-inflammatory agents, including, but not limited to non-steroidal anti-inflammatory drugs (e.g., ibuprofen, COX-1 inhibitors, and COX-2, inhibitors); salicylates; antibiotics; antivirals; antifungal agents; antidiabetic agents (e.g., biguanides, glucosidase inhibitors, insulins, sulfonylureas, and thiazolidenediones); adrenergic modifiers; diuretics; hormones (e.g., anabolic steroids, androgen, estrogen, calcitonin, progestin, somatostan, and thyroid hormones); immunomodulators; muscle relaxants; antihistamines; osteoporosis agents (e.g., biphosphonates, calcitonin, and estrogens); prostaglandins, antineoplastic agents; psychotherapeutic agents; sedatives; poison oak or poison sumac products; antibodies; and vaccines.

In certain embodiments, the pharmaceutical compositions of the present invention may be administered in conjuction with a lipid-lowering therapy. In certain such embodiments, a lipid-lowering therapy is therapeutic lifestyle change. In certain such embodiments, a lipid-lowering therapy is LDL apheresis.

### I. Kits, Research Reagents and Diagnostics

The antisense compounds provided herein can be utilized for diagnostics, and as research reagents and kits. Furthermore, antisense compounds, which are able to inhibit gene expression or modulate gene expression with specificity, are often used by those of ordinary skill to elucidate the function of particular genes or to distinguish between functions of various members of a biological pathway.

For use in kits and diagnostics, the antisense compounds described herein, either alone or in combination with other compounds or therapeutics, can be used as tools in differential and/or combinatorial analyses to elucidate expression patterns of a portion or the entire complement of genes expressed within cells and tissues. Methods of gene expression analysis are well known to those skilled in the art.

### J. Certain Advantages of Short Antisense Compounds

In certain embodiments, short antisense compounds have advantages when compared to their parent oligonucleotides. For example, in certain embodiments, short antisense compounds have greater affinity for a target nucleic acid than their parent oligonucleotide. In certain embodiments, short antisense compounds have greater potency in vitro than their parent oligonucleotide. In certain such embodiments, that increased in vitro potency is not entirely explained by increased affinity. In certain embodiments, such increased in vitro potency may be attributable to increased ability of short antisense compounds to penetrate cells and/or increased ability to access target nucleic acids in a cell. In certain embodiments, short antisense compounds have greater potency in vivo than their parent oligonucleotides. In certain embodiments, such greater in vivo potency is not attributable to increased in vitro potency or increased affinity. In certain embodiments, short antisense compounds have even greater in vivo potency compared to their parent oligonucleotides than would be predicted based on in vitro potencies or on affinities. In certain embodiments, such increased in vivo potency may be attributable to increased bioavailability, better penetration into the cell, better access to target nucleic acid once in the cell, or other factors.

In certain embodiments, one would expect short antisense compounds to be less specific for their target nucleic acid compared to their parent oligonucleotides. In certain such embodiments, one would expect increased side-effects, including potential for toxic effects, from short antisense compounds. In certain embodiments, such additional side-effects are not observed. In certain embodiments, non-target nucleic acids to which a particular short antisense compound may bind are not available to the short antisense compound. In such embodiments, side-effects, including toxicity, are less problematic than would be predicated

In certain embodiments, because they are smaller, short antisense compounds are less likely to bind proteins. In certain such embodiments, such less binding of proteins results in lower toxicity, since protein binding may have undesired consequences. In certain embodiments, such less binding of proteins results in greater potency, since it leaves more antisense compounds available for therapeutic effect. In certain embodiments, less binding of proteins results in decreased drug-drug interaction toxicity.

### Example 1

### Cell culture and treatment with short antisense compounds

The effect of short antisense compounds on target nucleic acid expression can be tested in any one of a number of cultured or primary cell lines. Cells lines can be obtained from publicly available sources, such as the American Type Culture Collection (Manassas, VA). Cells are cultured according to methods well known to those of ordinary skill in the art.

When cells reached appropriate confluency, they were treated with oligonucleotide using LIPOFECTIN® as described. When cells reached 65-75% confluency, they were treated with oligonucleotide. Oligonucleotide was mixed with LIPOFECTIN® Invitrogen Life Technologies, Carlsbad, CA) in Opti-MEM®-1 reduced serum medium (Invitrogen Life Technologies, Carlsbad, CA) to achieve the desired concentration of oligonucleotide and a LIPOFECTIN® concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM®-1 and then treated with 130 µL of the transfection mixture. Cells grown in 24-well plates or other standard tissue culture plates were treated similarly, using appropriate volumes of medium and oligonucleotide. Cells were treated and data were obtained in duplicate or triplicate. After approximately 4-7 hours of treatment at 37°C, the medium containing the transfection mixture was replaced with fresh culture medium. Cells were harvested 16-24 hours after oligonucleotide treatment.

Control oligonucleotides are used to determine the optimal oligomeric compound concentration for a particular cell line. Furthermore, when oligomeric compounds are tested in oligomeric compound screening experiments or phenotypic assays, control oligonucleotides are tested in parallel.

The concentration of oligonucleotide used varies from cell line to cell line. To determine the optimal oligonucleotide concentration for a particular cell line, the cells are treated with a positive control oligonucleotide at a range of concentrations. The concentration of positive control oligonucleotide that results in 80% inhibition of the target mRNA is then utilized as the screening concentration for new oligonucleotides in subsequent experiments for that cell line. If 80% inhibition is not achieved, the lowest concentration of positive control oligonucleotide that results in 60% inhibition of the target mRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 nM to 40 nM when the antisense oligonucleotide is transfected by electroporation.

### Example 2: Real-time Quantitative PCR Analysis of Target mRNA Levels

Quantitation of target mRNA levels was accomplished by real-time quantitative PCR using the ABI PRISM® 7600, 7700, or 7900 Sequence Detection System (PE-Applied Biosystems, Foster City, CA) according to manufacturer's instructions.

Prior to quantitative PCR analysis, primer-probe sets specific to the target gene being measured were evaluated for their ability to be "multiplexed" with a GAPDH amplification reaction. After isolation the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 toll total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR were normalized using either the expression level of GAPDH, a gene whose expression is constant, or by quantifying total RNA using RiboGreen® (Molecular Probes, Inc. Eugene, OR). GAPDH expression was quantified by RT, real-time PCR, by being run simultaneously with the target, multiplexing, or separately. Total RNA was quantified using RiboGreen® RNA quantification reagent (Molecular Probes, Inc. Eugene, OR).

170 µL of RiboGreen® working reagent (RiboGreen® reagent diluted 1:350 in 10mM Tris-HCl, I mM EDTA, pH 7.5) was pipetted into a 96-well plate containing 30 µL purified cellular RNA. The plate was read in a CytoFluor® 4000 (PE Applied Biosystems) with excitation at 485nm and emission at 530nm.

The GAPDH PCR probes have JOE covalently linked to the 5' end and TAMRA or MGB covalently linked to the 3' end, where JOE is the fluorescent reporter dye and TAMRA or MGB is the quencher dye. In some cell types, primers and probe designed to a GAPDH sequence from a different species are used to measure GAPDH expression. For example, a human GAPDH primer and probe set is used to measure GAPDH expression in monkey-derived cells and cell lines.

Probes and primers for use in real-time PCR were designed to hybridize to target nucleic acids using routine methods. For example, PrimerExpress® (Applied Biosystems, Foster City, CA) software is routinely used to design probes and primers for use in real-time PCR.

### EXAMPLE 3: Administration of a Parent and Parent Mixed Backbone Antisense Compound Targeting SGLT-2 mRNA.

ISIS 257016 was administered to *db*/*db* mice (Charles River Laboratories, Wilmington, MA) intraperitoneally at a dose of 1, 7.5, 14 or 17 mg/kg twice a week. Control groups included a group receiving saline on the same dosing schedule and a group receiving ISIS 145733. ISIS 257016 and ISIS 145733 both comprise the sequence GAAGTAGCCACCAACTGTGC (SEQ ID NO: 1572) further comprising a central "gap" region consisting of ten 2'-deoxynucleotides, which is flanked on both sides (5' and 3' directions) by five-nucleotide "wings". The wings are composed of 2'-methoxyethyl (2'-MOE) nucleotides. All cytidine residues are 5-methylcytidines. The internucleoside (backbone) linkages are phosphorothioate (P=S) throughout the oligonucleotide for ISIS 145733; however ISIS 257016 has a mixed backbone. The internucleoside linkages for ISIS 257016 are phosphodiester (P=O) in the wings and phosphorothioate in the gap. Forty-eight hours following administration of the last dose the mice were sacrificed and kidney tissue was analyzed for SGLT-2 mRNA levels. The results are shown below in Table 3.

**Table 3: Antisense inhibition of SGLT2 mRNA expression in vivo by 5-10-5 MOE gapmers**

| | **% change in SGLT2 expression relative to saline** | |
|---|---|---|
| **Dose of oligonucleotide nmol/kg** | **ISIS 145733** | **ISIS 257016** |
| 17 | -37.5 | -76 |
| 14 | -31.25 | -74 |
| 7.5 | -12.5 | -62.5 |
| 1 | +3 | -44 |

Both ISIS 257016 and ISIS 145733 markedly reduced SGLT-2 levels compared to saline control. (mRNA levels determined using RT, real-time PCR as described above) However, ISIS 257016 has been shown to be about 20-50 times more potent for reducing SGLT-2 mRNA compared to ISIS 145733. An associated reduction in plasma glucose levels was seen for the treatment groups (661 ±14 for the saline group compared to 470 ±23 for the group receiving ISIS 257016). Accumulation of ISIS 257016 and ISIS 145733 in the kidney was similar over the dose range, however little of the full length 257016 antisense was detected in the kidney which supports the theory that a degradation product is responsible for the increased activity. Also the onset of action following a single dose of 25 mg/kg correlated to a time pint were little intact 257016 antisense compound was left.

Similar studies were performed in lean mice, *ob*/*ob* mice and in ZDF rats (Charles Rivers Laboratories) using ISIS 257016, ISIS 145733 or saline in a similar same dosing schedule as described above. The sequence of the binding site for ISIS 145733 and ISIS 257016 is conserved between mouse and rat (see Table 4). Reduction of SGLT-2 mRNA in the kidney was similar to that seen above. In a study utilizing rats, at a dose of 10 mg/kg given two times a week for two weeks, ISIS 145733 was shown to reduce SGLT-2 mRNA levels by about 40% whereas the reduction achieved with ISIS 257016 was greater than 80%. ISIS 257016 reduces SGLT2 expression maximally at a low dose of 12.5 mg/kg. Additional studies at lower dosing ranges show significant reduction of SGLT2 mRNA levels with the mixed backbone antisense compound at doses less than 1 mg/kg/wk.

### EXAMPLE 4: Administration of a Parent and Short Andsense Compound Targeting SGLT-2 mRNA

Pharmacokinetic studies indicated that ISIS 257016 was acting as a prodrug that was metabolized to a 12 nucleobase pharmacophore. In a next study, ZDF rats were dosed intraperitoneally twice per week with 1.5 mg/kg of either ISIS 257016 or ISIS 370717, or with saline at a similar dosing schedule. ISIS 370717 is a 12 nucleobase antisense compound targeted to SGLT-2 nucleic acid comprising the sequence TAGCCACCAACT (SEQ ID NO: 154) and further comprising central "gap" region consisting of ten 2'-deoxynucleotides, which is flanked on both sides (5' and 3' directions) by one-nucleotide "wings". The wings are composed of 2'-methoxyethyl (2'-MOE) nucleotides. All cytidine residues are 5-methylcytidines. The internucleoside (backbone) linkages are phosphorothioate (P=S) throughout the oligonucleotide.

Following five weeks of dosing the animals were sacrificed and kidney tissue was analyzed for SGLT-2 mRNA levels. The pharmacological activity of ISIS 257016 and ISIS 370717 were similar, however, the 12 nucleotide antisense compound displayed a faster onset of action. ISIS 370717 displayed nearly 80% inhibition of SGLT2 expression in kidney on day two after a single dose of 2.8 umoles/kg whereas ISIS 257016 displayed only about 25% inhibition on day 2 after the same single dose administration. The date support that ISIS 257016 is a prodrug having a 12 nucleotide pharmacophore.

### EXAMPLE 5: Potency and Bioavailability of a Short Antisense Compound

The improved potency displayed by ISIS 370717 and the improved oral bioavailability for these short antisense compounds makes these compounds useful for oral administration. Normal rats received ISIS 370717, ISIS 145733 or saline at 100 mg/kg twice per week via intrajejunal administration. About 48 hours following the last dose, the animals were sacrificed and kidney tissue was analyzed for antisense compound concentration and SGLT-2 mRNA levels. There was a significantly higher accumulation of ISIS 370717 in the kidney tissue (approximately 500 micro grams per gram of tissue) compared to the controls. Moreover, SGLT-2 mRNA was reduced by more than 80% over the controls.

### EXAMPLE 6: Wing, Gap and Total Length Variations Around a 12 nucleotide short antisense compound

ISIS 370717 1-10-1 MOE gapmer was used as a template to make sequence related oligos with varying motifs. These variations are provided in Table 4. The antisense compounds were designed to target different regions of the mouse or rat SGLT2 nucleic acid, using published sequences (GenBank accession number U29881.1, incorporated herein as SEQ ID NO: 1575, and GenBank accession number AJ292928.1, incorporated herein as SEQ ID NO: 1576, respectively).

**Table 4: Short Antisense compounds targeting SGLT2 nucleic acids**

| **ISIS NO** | **5' Target Site on mouse SEQ ID NO:** 1575 | **5' Target Site on rat SEQ ID NO:** 1576 | **Gapmer Motif** | **Sequence (5'-3') Sequence (5'-3')** | **SEQ ID** NO |
|---|---|---|---|---|---|
| 257016 | 2680 | 148 | 5-10-5 MOE | GAAGTAGCCACCAACTGTGC | 1553 |
| 370717 | 2684 | 152 | 1-10-1 MOE | TAGCCACCAACT | 1554 |
| 386169 | 2684 | 152 | 2-8-2 MOE | TAGCCACCAACT | 1555 |
| 386176 | 2685 | 153 | 1-8-1 MOE | AGCCACCAAC | 1556 |
| 386196 | 2684 | 152 | 3-6-3 MOE | TAGCCACCAACT | 1557 |

The antisense compounds were analyzed for their effect on mouse SGLT2 mRNA levels. Data are ranges taken from three experiments in which mice were dosed twice per week for three weeks with 2.5, 0.5 or 0.1 umol/kg of the above MOE gapmers given by intraperitoneal injection. Mice were sacrificed 48 hours following last administration and evaluated for SGLT2 levels in kidney. SGLT2 mRNA levels were determined by RT, real-time PCR as described by other examples herein. PCR results were normalized to an internal ISIS control. The results are shown below in Table 5.

**Table 5: Antisense inhibition of SGLT2 in vivo by 1-10-1 and 1-10-2 MOE gapmers**

| **% change in SGLT2 expression relative to saline** | | | | | |
|---|---|---|---|---|---|
| **Dose of oligonucleotide umol/kg** | **ISIS 370717** | **ISIS 386169** | **ISIS 386176** | **ISIS 386196** | **ISIS 386197** |
| 2.5 | -82 | -85 | -80 | -50 | -20 |
| 0.5 | -70 | -80 | -68 | -30 | -15 |
| 0.1 | -55 | -70 | -65 | -35 | -20 |

These results illustrate that all the various motifs tested inhibit the expression of SGLT2 *in vivo* in a dose-dependent manner. The 1-10-1, 2-**8-2** and 1-8-1 gapmers were found to be particularly potent.

### EXAMPLE 7: Antisense Inhibition of Rat SGLT-2 by 1-10-1 and 1-10-2 MOE Gapmers

1-10-1 and 1-10-2 MOE gapmer antisense compounds, provided in Table 6, were designed to target different regions of the mouse or rat SGLT2 RNA. All short antisense compounds in Table 6 are chimeric oligonucleotides ("gapmers") either 12 or 13 nucleotides in length, composed of a central "gap" segment consisting of ten 2'-deoxynucleotides, which are flanked on the 5' side by a one-nucleoside "wing" and on the 3' side by a two or one-nucleotide "wing". The wings are composed of 2'-methoxyethyl (2'-MOE) nucleotides. The internucleoside (backbone) linkages are phosphorothioate (P=S) throughout the oligonucleotide. All cytidine residues are 5-methylcytidines.

**Table 6: Antisense compounds targeting SGLT2 nucleic acid**

| **ISIS NO** | **5' Target Site on SEQ ID NO: XXX** (mouse) | **5' Target Site on SEQ ID NO: XXX** (rat) | **Gapmer Motif** | **Sequence (5'-3')** | **SEQ ID NO** |
|---|---|---|---|---|---|
| 370717 | 2684 | 152 | 1-10-1 MOE | TAGCCACCAACT | 1554 |
| 382675 | 2683 | 151 | 1-10-1 MOE | TAGCCACCAACTG | 1559 |
| 379692 | | 508 | 1-10-1 MOE | TGTTCCAGCCCA | 246 |
| 382676 | | 507 | 1-10-2 MOE | TGTTCCAGCCCAG | 246 |
| 379699 | | 1112 | 1-10-2 MOE | GGCATGAGCTTC | 281 |
| 382677 | | 1111 | 1-10-2 MOE | GGCATGAGCTTCA | 281 |
| 382677 | | 958 | 1-10-2 MOE | GGCATGAGCTTCA | 281 |

The short antisense compounds were analyzed for their effect on rat SGLT2 mRNA levels. Data are ranges taken from three experiments in which Male Sprague-Dawley rats (170-200g) were dosed twice per week for three weeks with 450, 150 or 50 nmol/kg of either a 1-10-1 or 1-10-2 MOE gapmer given by intraperitoneal injection. Rats were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 7.

**Table 7: Antisense inhibition of SGLT2 mRNA in vivo by 1-I0-1 and 1-10-2 MOE gapmers**

| | **% change in SGLT2 expression relative to saline** | | | | | |
|---|---|---|---|---|---|---|
| **Dose of oligonucleotide** | **ISIS** | **ISIS** | **ISIS** | **ISIS** | **ISIS** | **ISIS** |
| **nmol/kg** | **370717 1-10-1** | **382675 1-10-2** | **379692 1-10-1** | **382676 1-10-2** | **379699 1-10-1** | **382677 1-10-2** |
| 450 | -70 | -80 | -90 | -85 | -83 | -75 |
| 150 | -70 | -65 | -85 | -80 | -75 | -60 |
| 50 | -55 | -50 | -80 | -65 | -60 | -40 |

These results illustrate that both the 1-10-1 and 1-10-2 MOE gapmers reduce SGLT2 mRNA *in vivo* in a dose-dependent manner.

Rats were further evaluated for total body weight, liver, spleen and kidney weight. Significant changes in spleen, liver or body weight can indicate that a particular compound causes toxic effects. All changes were within the margin of error of the experiment. No significant changes in body weight were observed during the treatment or at study termination. No significant changes in liver or spleen weights were observed.

Toxic effects of short antisense compounds administered *in vivo* can also be assessed by measuring the levels of enzymes and proteins associated with disease or injury of the liver or kidney. Elevations in the levels of the serum transaminases aspartate aminotransferase (AST) and alanine aminotransferase (ALT) are often indicators of liver disease or injury. Serum total bilirubin is an indicator of liver and biliary function, and albumin and blood urea nitrogen (BUN) are indicators of renal function. Glucose and triglyceride levels are sometimes altered due to toxicity of a treatment. Serum glucose also depends in part upon the activity of SGLT2. The levels of ALT, AST, total bilirubin, albumin, BUN, glucose and triglyceride were measured in rats treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function. Triglyceride and glucose levels were not significantly elevated relative to saline-treated animals.

### EXAMPLE 8: Antisense Inhibition of Mouse and Rat SGLT2 by 1-10-1 MOE Gapmers

1-10-1 MOE gapmer antisense compounds designed to target different regions of mouse SGLT2 mRNA are shown in Table 8.

**Table 8: Composition of Antisense Compounds Targeting SGLT2 mRNA**

| **ISIS NO** | **5' Target Site on SEQ ID NO: XXX (mouse)** | **5' Target Site on SEQ ID NO: XXX (rat)** | **Motif** | **Sequence (5'-3')** | **SEQ ID NO** |
|---|---|---|---|---|---|
| 370717 | 2684 | 152 | 1-10-1 MOE | TAGCCACCAACT | 1554 |
| 379692 | | 508 | 1-10-1 MOE | TGTTCCAGCCCA | 246 |
| 379699 | | 1112 | 1-10-1 MOE | GGCATGAGCTTC | 281 |
| 379702 | | 1525 | 1-10-1 MOE | GCACACAGCTGC | 293 |
| 381408 | 3034** | | 1-10-1 MOE | TACCGAACACCT | 1560 |

| | | | | | |
|---|---|---|---|---|---|
| ** indicates 3 mismatches to a target sequence | | | | | |

The short antisense compounds were analyzed for their effect on mouse SGLT2 mRNA levels. Data was taken from three experiments in which Male 6-week old Balb/c mice were dosed twice per week for two weeks with 450, 150, or 50 nmol/kg of one of the above 1-10-1 MOE gapmers given by intraperitoneal injection. Mice were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 9.

**Table 9: Antisense inhibition of SGLT2 mRNA in vivo by 1-10-1 MOE gapmers**

| | **% change in SGLT2 expression relative to saline** | | | | |
|---|---|---|---|---|---|
| **Dose of oligonucleotide nmol/kg** | **ISIS 370717** | **ISIS 379692** | **ISIS 379699** | **ISIS 379702** | **ISIS 381408** |
| 450 | -65 | -80 | -80 | -75 | - |
| 150 | -55 | -70 | -62.5 | -72.5 | - |
| 50 | -47.5 | -52.5 | -42.5 | -52.5 | - |

These results illustrate that all the 1-10-1 MOE gapmers except, ISIS 381408, inhibit the expression of SGLT2 in vivo in a dose-dependent manner in mouse. Activity of ISIS 381408 has been shown in Rat studies (See Table 9).

### Evaluation of 1-10-1 Gapmers in Rat

The effect of the above 1-10-1 gapmers (see Table 8 above) on rat SGLT2 mRNA levels. Data are taken from four experiments in which male Sprague-Dawley rats (170-200g) were dosed twice per week for three weeks with 250 nmol/kg given by intraperitoneal injection. Rats were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 10.

**Table 10: Antisense inhibition of SGLT2 mRNA in vivo by 1-10-1 MOE gapmers**

| | **% change in SGLT2 expression relative to saline** | | | | |
|---|---|---|---|---|---|
| **Dose of oligonucleotide nmol/kg** | **ISIS 370717** | **ISIS 379692** | **ISIS 379699** | **ISIS 379702** | **ISIS 381408** |
| 250 | -70 | -85 | -75 | -25 | -5 |

These results illustrate that all the 1-10-1 MOE gapmers inhibit the expression of SGLT2 in *in vivo* rat studies.

### EXAMPLE 9: Antisense Inhibition of Mouse and Rat SGLT2 Expression by Additional 1-10-1 and 2-8-2 MOE Gapmers

1-10-1 and 2-8-2 MOE gapmer short antisense compounds were designed to target different regions of the mouse SGLT2 RNA but have complementarity across species. The short antisense compounds are shown in Table 11. All short antisense compounds in Table 11 are gapmers 12 nucleotides in length, composed of a central "gap" segment consisting of 2'-deoxynucleotides, which are flanked on both sides (5' and 3' directions) by wing segments having 2'-modifications. The wings are composed of 2'-methoxyethyl (2'-MOE) nucleotides. The internucleoside (backbone) linkages are phosphorothioate (P=S) throughout the oligonucleotide. All cytidine residues are 5-methyleytidines.

**Table 11. Short Antisense Compounds Targeting SGLT2 nucleic add**

| **ISIS NO** | **5' Target Site (rat)** | **Target SEQ ID (rat)** | **Gapmer Motif** | **Sequence (5'-3')** | **SEQ ID NO** |
|---|---|---|---|---|---|
| 379692 | 508 | | 1-10-1 MOE | TGTTCCAGCCCA | 246 |
| 388625 | 508 | | 1-10-1 MOE | TGTRCCAGCCCA | 246 |
| 379699 | 1112 | | 1-10-1 MOE | GGCATGAGCTTC | 281 |
| 388626 | 1112 | | 2-8-2 MOE | GGCATGAGCTTC | 281 |
| 379702 | 1525 | | 2-8-2 MOE | GCACACAGCTGC | 293 |
| 388627 | 1525 | | 2-8-2 MOE | GCACACAGCTGC | 293 |

The short antisense compounds were analyzed for their effect on mouse SGLT2 mRNA levels *in vivo*. Data was taken from three experiments in which male 6-week old Balb/c mice were dosed twice per week for three weeks with 0.5, 0.1, or 0.02 umol/kg of either a 1-10-1 or 2-8-2 MOE gapmer given by intraperitoneal injection. Mice were sacrificed 48 hours following last administration and evaluated for SGLT2 levels in kidney. Target levels were determined by RT, real-time PCR as described by other examples herein. PCR results were normalized to an internal ISIS control. The results are shown below in Table 12.

**Table 12: Antisense inhibition of SGLT2 mRNA in vivo by 1-10-1 and 2-8-2 MOE gapmers**

| | **% change in SGLT2 expression relative to saline** | | | | | |
|---|---|---|---|---|---|---|
| **Dose of oligonucleotide umol/kg** | **ISIS 379692 1-10-1** | **ISIS 388625 2-8-2** | **ISIS 379699 1-10-1** | **ISIS 388626 2-8-2** | **ISIS 379702 1-10-1** | **ISIS 388627 2-8-2** |
| 0.5 | -85 | -90 | -75 | -80 | -70 | -65 |
| 0.1 | -75 | -88 | -60 | -60 | -65 | -50 |
| 0.02 | -55 | -65 | -30 | -45 | -40 | -38 |

These results illustrate that both the 1-10-1 and 2-8-2 MOE gapmers inhibit the expression of SGLT2 in vivo in a dose-dependent manner.

Mice were further evaluated for total body weight, liver, spleen and kidney weight. All changes were within the margin of error of the experiment. No significant changes in body weight were observed during the treatment or at study termination. No significant changes in liver or spleen weights were observed.

The levels of ALT, AST, BUN, transaminases, plasma creatinine, glucose and triglyceride were measured in mice treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function. Triglyceride and glucose levels were not significantly elevated relative to saline-treated animals.

### Evaluation of ISIS 3796921-10-1 MOE Gapmer, ISIS 392170 1-10-1 Methyleneoxy BNA Gapmer, ISIS 388625 2-8-2 MOE Gapmer and ISIS 392173 2-8-2 Methyleneoxy BNA Gapmer in Mice

The effect of ISIS 379692 1-10-1 MOE gapmer and ISIS 388625 2-8-2 MOE gapmer are compared with the effect of ISIS 392170 1-10-1 Methyleneoxy BNA Gapmer and ISIS 392173 2-8-2 Ethyleneoxy BNA Gapmer (see Table 13 on mouse SGLT2 mRNA levels *in vivo.* Data are taken from three experiments in which male 6-week old Balb/c mice were dosed twice per week for three weeks with 5, 25 and 125 nmol/kg of either the ISIS 379692 1-10-1 MOE gapmer or the ISIS 388625 2-8-2 MOE gapmer given by intraperitoneal injection. Mice were sacrificed 48 hours following last administration and evaluated for SGLT2 mRNA levels in kidney. Target levels were determined by RT, real-time PCR as described by other examples herein. PCR results were normalized to an internal ISIS control. The data are expressed as percent change ("+" indicates an increase, "-" indicates a decrease) relative to saline treated animals and are illustrated in Table 13.

**Table 13. Antisense inhibition of SGLT2 mRNA in vivo by a 1-10-1 and a 2-8-2 MOE gapmer**

| **Dose of oligonucleotide nmol/kg** | **ISIS 379692 1-10-1 MOE** | **ISIS 392170 1-10-1 Methyleneo xy BNA** | **ISIS 388625 2-8-2 MOE** | **ISIS 392173 2-8-2 Methyleneo xy BNA** |
|---|---|---|---|---|
| 125 | -58 | -69 | -70 | -75 |
| 25 | -46 | -54 | -47 | -57 |
| 5 | -7 | -23 | -18 | -44 |

These results illustrate that both the 1-10-1 and 2-8-2 MOE gapmer inhibit the expression of SGLT2 *in vivo* at the highest three dosing ranges in a dose-dependent manner. The results also illustrate that the Methyleneoxy BNA constructs are more potent then the MOE constructs. No significant changes in body weight were observed during the treatment or at study termination. No significant changes in liver or spleen weights were observed. The toxicity parameters including levels of ALT, AST, BUN, and creatinine were within normal ranges and are not significantly changed relative to saline-treated animals, demonstrating that the compounds do not significantly affect renal or hepatic function.

### Evaluation of ISIS 379692 1-10-1 MOE Gapmer and ISIS 388625 2-8-2 MOE Gapmer in Rat

The effect of ISIS 379692 1-10-1 MOE gapmer and ISIS 388625 MOE 2-8-2 gapmer (see Table 14) on rat SGLT2 mRNA levels *in vivo*. Data are taken from four experiments in which male Sprague-Dawley rats (170-200g) were dosed twice per week for three weeks with 200, 50, 12.5, or 3.125 nmol/kg of either the ISIS 379692 1-10-1 MOE gapmer or the ISIS 388625 2-8-2 MOE gapmer given by intraperitoneal injection. Rats were sacrificed 48 hours following last administration and evaluated for SGLT2 levels in kidney. Target levels were determined by RT, real-time PCR as described by other examples herein. PCR results were normalized to an internal ISIS control. The results are shown below in Table 14.

**Table 14: Antisense inhibition of SGLT2 mRNA in vivo by a 1-10-1 and a 2-8-2 MOE gapmer**

| | | |
|---|---|---|
| **% change in SGLT2 expression relative to saline** | | |

| **Dose of oligonucleotide umol/kg** | **ISIS 379692 1-10-1** | **ISIS 388625 2-8-2** |
|---|---|---|
| 200 | -80 | -80 |
| 50 | -65 | -65 |
| 12.5 | -15 | -15 |
| 3.125 | +30 | +25 |

These results illustrate that both the 1-10-1 and 2-8-2 MOE gapmer inhibit the expression of SGLT2 in vivo at the highest three dosing ranges in a dose-dependent manner.

Rats were further evaluated for total body weight, liver, spleen and kidney weight. All changes were within the margin of error of the experiment. No significant changes in body weight were observed during the treatment or at study termination. No significant changes in liver or spleen weights were observed.

The levels of ALT, AST, BUN, cholesterol, plasma creatinine and triglycerides were measured in rats treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function.

### EXAMPLE 10: Antisense Inhibition of SGLT2 Expression in ZDF rat

ISIS 388625, 388626 and control oligo ISIS 388628 were analyzed for their effect on ZDF rat plasma glucose levels and HbA1c. The leptin receptor deficient Zucker diabetic fatty (ZDF) rat is a useful model for the investigation of type 2 diabetes. Diabetes develops spontaneously in these male rats at ages 8-10 weeks, and is associated with hyperphagia, polyuria, polydipsia, and impaired weight gain, symptoms which parallel the clinical symptoms of diabetes (Phillips MS, et al., 1996, Nat Genet 13, 18-19). Six week old ZDF rats were injected intraperitoneally with short antisense compound at a dose of 40 0nM/kg once a week for twelve weeks. Data are illustrated in Tables 15 and 16.

**Table 15: Plasma glucose**

| ISIS NO. | Seq ID NO | Sequence (5'-3') | Motif | Plasma glucose levels recorded on specific dates (mg/dl) | | | |
|---|---|---|---|---|---|---|---|
| | | | | Day 10 | Day 40 | Day 55 | Day 66 |
| PBS | | n/a | n/a | 450.7 | 478.5 | 392.8 | 526.2 |
| 388625 | 246 | TGTTCCAGCCCA | 2-8-2 MOE | 435.5 | 278.7 | 213.8 | 325.5 |
| 388626 | 281 | GGCATGAGCTTC | 2-8-2 MOE | 434.7 | 300.5 | 219.8 | 379.8 |
| 388628 | 226 | TAGCCGCCCACA | 2-8-2 MOE | 436.0 | 502.0 | 411.2 | 668.8 |

**Table 16: HbA1c Status**

| ISIS NO. | Seq ID NO | Sequence (5'-3') | Motif | Percentage HbA1c on specific dates (%) p < 0.001 | | |
|---|---|---|---|---|---|---|
| | | | | Day 40 | Day 55 | Day 68 |
| PBS | | n/a | n/a | 8.0 | 8.9 | 10.0 |
| 388625 | 246 | TGTTCCAGCCCA | 2-8-2 MOE | 6.5 | 5.8 | 4.3 |
| 388626 | 281 | GGCATGAGCTTC | 2-8-2 MOE | 6.6 | 5.9 | 4.0 |
| 388628 | 226 | TAGCCGCCCACA | 2-8-2 MOE | 8.0 | 9.1 | 7.8 |

ISIS 388625 and 388626 significantly reduced plasma glucose levels and HbA1C compared to PBS and control treated animals.

### EXAMPLE 11: Antisense Inhibition of SGLT2 Expression in Dog Kidney (ISIS 388625)

ISIS 388625 is a 2-8-2 MOE Gapmer with sequence TGTTCCAGCCCA (SEQ ID NO: 246) (e.g. see Table 15). The effect of ISIS 388625 on dog SGLT2 mRNA levels. Data are taken from two dosing groups in which a total of nine male beagle dogs were dosed with either one or ten mg/kg/week of ISIS 388625 or saline given by subcutaneous injection twice weekly. On day 46 of the study all dogs were sacrificed and evaluated for SGLT2 levels in kidney. Target levels were determined by quantitative RT, real-time PCR as described by other examples herein. PCR results were normalized to an internal ISIS control. The results are shown below in Table 17.

**Table 17: Antisense inhibition of SGLT2 mRNA in vivo by ISIS 388625**

| | **% change in SGLT2 expression Relative to saline** |
|---|---|
| **Dose of oligonucleotide mg/kg/wk** | **ISIS 388625** |
| 1 | -85 |
| 10 | -95 |

These results illustrate that greater than 80% reduction of SGLT2 mRNA can be achieved at a 1 mg/kg/wk dose of ISIS 388625. Even greater reduction can be achieved at slightly higher doses. Administration of ISIS 388625 in dog was also shown to improve glucose tolerance. Peak plasma glucose levels were decreased by over 50% on average and the subsequent drop in glucose was lessened compared to saline controls in a standard glucose tolerance test. Urinary glucose excretion was also increased.

### EXAMPLE 12: In vivo testing of short antisense compounds targeted to SGLT2 nucleic acid

Twenty 1-10-1 MOE gapmers that are complementary to human/monkey/mouse/rat SGLT2 were designed, synthesized and tested *in vivo* for suppression of SGLT2 mRNA levels in kidney. Target sites for mouse and rat are indicated in Table 18. Target sites for human are indicated in Tables 1 and 2. Data are averages from two experiments in which male 6-week old Balb/c mice were administered intraperitoneal injections of 350 nmol/kg of oligonucleotide, twice per week, over a period of two weeks (a total of four injections). Mice were sacrificed 48 hours following the last administration and evaluated for SGLT2 mRNA levels in kidney. SGLT2 mRNA levels were determined by quantitative real-time PCR analysis according to standard procedures, using two different PCR primer probe sets, primer probe set (PPS) 534 and PPS 553. SGLT2 mRNA levels were normalized to cyclophilin mRNA levels, which were also measured by quantitative real-time PCR. The results are shown below in Table 18.

**Table 18: Antisense inhibition of SGLT2 in vivo**

| **ISIS NO** | **5' Target Site on SEQ NO:XXX (mouse)** | **5' Target Site on SEQ ID NO:XXX (rat)** | **Sequence (5'-3')** | **Motif** | **PPS 534 % Saline** | **PPS 553 % Saline** | **SEQ ID NO** |
|---|---|---|---|---|---|---|---|
| PBS | | | N/A | | --- | --- | |
| 370717 | 2684 | 152 | TAGCCACCAACT | 1-10-1 MOE | -84.4 | -84.3 | 1554 |
| 379684 | 2070 | 64 | TGTCAGCAGGAT | 1-10-1 MOE | -45.0 | -43.2 | 214 |
| 379685 | 2103 | 97 | TGACCAGCAGGA | 1-10-1 MOE | -10.3 | -20.5 | 219 |
| 379686 | 2121* | 115 | ACCACAAGCCAA | 1-10-1 MOE | -71.9 | -75.1 | 225 |
| 379687 | 2824 | 216 | GATGTTGCTGGC | 1-10-1 MOE | -47.1 | -52.1 | 230 |
| 379688 | 2876 | 268 | CCAAGCCACTTG | 1-10-1 MOE | -62.6 | -70.4 | 240 |
| 379689 | | 298 | AGAGCGCATTCC | 1-10-1 MOE | -17.5 | -30.4 | 241 |
| 379690 | | 415 | ACAGGTAGAGGC | 1-10-1 MOE | -18.9 | -22.5 | 242 |
| 379691 | | 454 | AGATCTTGGTGA | 1-10-1 MOE | -35.0 | -48.6 | 243 |
| 379692 | | 508 | TGTTCCAGCCCA | 1-10-1 | -88.1 | -88.5 | 246 |
| 379693 | | 546 | CATGGTGATGCC | 1-10-1 | -51.6 | -59.9 | 254 |
| 379694 | | 609 | GACGAAGGTCTG | 1-10-1 | -42.1 | -54.4 | 264 |
| 379695 | | 717 | GGACACCGTCAG | 1-10-1 | -52.5 | -64.1 | 266 |
| 379696 | | 954 | CAGCTTCAGGTA | 1-10-1 | -24.6 | -36.2 | 267 |
| 379697 | | 982 | CTGGCATGACCA | 1-10-1 | -32.0 | -46.3 | 272 |
| 379698 | | 1071 | GCAGCCCACCTC | 1-10-1 | -11.8 | -27.0 | 275 |
| 379699 | | 1112 | GGCATGAGCTTC | 1-10-1 | -83.5 | -85.8 | 281 |
| 379700 | | 1138 | CCAGCATGAGTC | 1-10-1 | -2.8 | -16.4 | 285 |
| 379701 | | 1210 | CCATGGTGAAGA | 1-10-1 | -0.3 | -11.9 | 288 |
| 379702 | | 1525 | GCACACAGCTGC | 1-10-1 | -87.8 | -89.5 | 293 |
| 379703 | | 1681 | GCCGGAGACTGA | 1-10-1 | -44.2 | -45.9 | 295 |

| | | | | | | | |
|---|---|---|---|---|---|---|---|
| * indicates 1 or 2 mismatches to a target sequence | | | | | | | |

## Claims

1. A short antisense compound 10 to 14 monomers in length, comprising a 2'-deoxyribonucleotide gap region flanked on each side by a wing, wherein each wing independently consists of 1 to 3 high-affinity modified monomers which are sugar-modified nucleotides that comprise a bridge between the 4' and the 2' position of the sugar, and wherein the short antisense compound is targeted to a nucleic acid encoding SGLT2.

2. A short antisense compound 10 to 14 monomers in length, comprising a 2'-deoxyribonucleotide gap region flanked on each side by a wing, wherein each wing independently consists of 1 to 3 high-affinity modified monomers which are sugar-modified nucleotides comprising the 2'-subsitutent group OCH₂CH₂OCH₃, and wherein the short antisense compound is targeted to a nucleic acid encoding SGLT2.

3. The short antisense compound of claim 1, wherein the conformation of each of said sugar-modified nucleotides is, independently, β-D or α-L.

4. The short antisense compound of claim 1, wherein each of said bridges independently comprises 1 or from 2 to 4 linked groups independently selected from -[C(R₁)R₂)]ₙ-, -C(R₁)=C(R₂)-, -C(R₁)=N-, -C(=NR₁)-, -C(-O)-, -C(=S)-, -O-, -Si(R₁)₂-, -S(-O)ₓ- and -N(R₁)-;
wherein
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J_{2,} SJ₁, N₃, COOJ₁, acyl (C(=O)-H), substituted acyl, CN, sulfonyl (S(-O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

5. The short antisense compound of claim 4, wherein each of said bridges is, independently, 4'-CH₂-2', 4'-(CH₂)₂-2', 4'-CH₂-O-2', 4'-(CH₂)₂-O-2', 4'CH₂-O-N(R₁)-2' and 4'-CH₂-N(R₁)-O-2'- wherein each R₁ is, independently, H, a protecting group or C₁-C₁₂ alkyl.

6. The short antisense compound of any preceding claim, wherein at least one monomeric linkage is a modified monomeric linkage, such as a phosphorothioate linkage.

7. The short antisense compound of any preceding claim, wherein each monomeric linkage is a phosphorothioate internucleoside linkage.

8. The short antisense compound of any preceding claim that is 10-13 monomers in length, 10-12 monomers in length, 10-11 monomers in length, 10 monomers in length, 11 monomers in length, 12 monomers in length, 13 monomers in length, or 14 monomers in length.

9. The short antisense compound of any preceding claim, which comprises a nucleotide sequence selected from SEQ ID NOs: 214-219, 221-226, 228-230, 232-243, 245-246, 251-254, 256-272, 274-281, 283-285, 287-293 and 295-300.

10. The short antisense compound of any preceding claim, that is 12 monomers in length.

11. The short antisense compound of claim 10, having a motif selected from 1-10-1 and 2-8-2 wherein the first number represents the number of monomers in the 5'-wing, the second number represents the number of monomers in the gap, and the third number represents the number of monomers in the 3' wing.

12. The short antisense compound of any preceding claim, wherein the compound is composed of a central gap segment consisting of 2'-deoxynucleotides, which is flanked on both sides by wing segments composed of 2'-methoxyethyl (2'-MOE) nucleotides, wherein the internucleoside linkages are phosphorothioate (P=S) throughout the oligonucleotide, and all cytidine residues are 5-methylcytidines.

13. The short antisense compound of claim 12, which comprises a nucleotide sequence selected from SEQ ID NOs: 275, 276, 277, 278, 279, 280, 281, 283, 284, 285 and 287.

14. The short antisense compound of any preceding claim, wherein said compound comprises the nucleotide sequence of SEQ ID NO:281, is 12 monomers in length, has a 2-8-2 motif wherein the first number represents the number of monomers in the 5'-wing, the second number represents the number of monomers in the gap, and the third number represents the number of monomers in the 3' wing, and wherein the compound is composed of a central gap segment consisting of 2'-deoxynucleotides, which is flanked on both sides by wing segments composed of 2'-methoxyethyl (2'-MOE) nucleotides, wherein the internucleoside linkages are phosphorothioate (P=S) throughout the oligonucleotide, and all cytidine residues are 5-methylcytidines.

15. The short antisense compound of any preceding claim, which is a pharmaceutically acceptable salt.

16. An *ex vivo* method of modulating expression of SGLT2 by contacting an SGLT2 nucleic acid with a short antisense compound of any of claims 1-15.

17. The short antisense compound of any of claims 1-15, for use in treating a metabolic disorder in an animal.

## Patentansprüche

1. Kurze Antisense-Verbindung mit einer Länge von 10 bis 14 Monomeren, umfassend einen auf jeder Seite von einem Flügel flankierten 2'-Desoxyribonukleotid-Lückenbereich, wobei die Flügel jeweils unabhängig aus 1 bis 3 hochaffinen modifizierten Monomeren, bei denen es sich um zuckermodifizierte Nukleotide, die eine Brücke zwischen der 4'- und der 2'-Position des Zuckers enthalten, handelt, bestehen und wobei die kurze Antisense-Verbindung auf eine für SGLT2 codierende Nukleinsäure als Ziel gerichtet ist.

2. Kurze Antisense-Verbindung mit einer Länge von 10 bis 14 Monomeren, umfassend einen auf jeder Seite von einem Flügel flankierten 2'-Desoxyribonukleotid-Lückenbereich, wobei die Flügel jeweils unabhängig aus 1 bis 3 hochaffinen modifizierten Monomeren, bei denen es sich um zuckermodifizierte Nukleotide, die die 2'-Substituentengruppe OCH₂CH₂OCH₃ enthalten, handelt, bestehen und wobei die kurze Antisense-Verbindung auf eine für SGLT2 codierende Nukleinsäure als Ziel gerichtet ist.

3. Kurze Antisense-Verbindung nach Anspruch 1, wobei die zuckermodifizierten Nukleotide jeweils unabhängig in β-D- oder α-L-Konformation vorliegen.

4. Kurze Antisense-Verbindung nach Anspruch 1, wobei die Brücken jeweils unabhängig 1 oder 2 bis 4 verknüpfte Gruppen, unabhängig ausgewählt aus -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- und - N(R₁)-, umfassen;
wobei
x gleich 0, 1 oder 2 ist;
n gleich 1, 2, 3 oder 4 ist;
R₁ und R₂ jeweils unabhängig für H, eine Schutzgruppe, Hydroxyl, C₁-C₁₂-Alkyl, substituiertes C₁-C₁₂-Alkyl, C₂-C₁₂-Alkenyl, substituiertes C₂-C₁₂-Alkenyl, C₂-C₁₂-Alkinyl, substituiertes C₂-C₁₂-Alkinyl, C₅-C₂₀-Aryl, substituiertes C₅-C₂₀-Aryl, einen Heterocyclusrest, einen substituierten Heterocyclusrest, Heteroaryl, substituiertes Heteroaryl, einen alicyclischen C₅-C₇-Rest, einen substituierten alicyclischen C₅-C₇-Rest, Halogen, OJ₁, NJ₁J₂, SJ₁, N₃, COOJ₁, Acyl (C(=O)-H), substituiertes Acyl, CN, Sulfonyl (S(=O)₂-J₁) oder Sulfoxyl (S(=O)-J₁) stehen; und
J₁ und J₂ jeweils unabhängig für H, C₁-C₁₂-Alkyl, substituiertes C₁-C₁₂-Alkyl, C₂-C₁₂-Alkenyl, substituiertes C₂-C₁₂-Alkenyl, C₂-C₁₂-Alkinyl, substituiertes C₂-C₁₂-Alkinyl, C₅-C₂₀-Aryl, substituiertes C₅-C₂₀-Aryl, Acyl (C(=O)-H), substituiertes Acyl, einen Heterocyclusrest, einen substituierten Heterocyclusrest, C₁-C₁₂-Aminoalkyl, substituiertes C₁-C₁₂-Aminoalkyl oder eine Schutzgruppe stehen.

5. Kurze Antisense-Verbindung nach Anspruch 4, wobei die Brücken jeweils unabhängig für 4'-CH₂-2', 4'-(CH₂)₂-2', 4'-CH₂-O-2', 4'-(CH₂)₂-O-2', 4'-CH₂-O-N(R₁)-2' und 4'-CH₂-N(R₁)-O-2' stehen, wobei R₁ jeweils unabhängig für H, eine Schutzgruppe oder C₁-C₁₂-Alkyl steht.

6. Kurze Antisense-Verbindung nach einem vorhergehenden Anspruch, wobei es sich bei wenigstens einer monomeren Verknüpfung um eine modifizierte monomere Verknüpfung, wie etwa eine Phosphorothioat-Verknüpfung, handelt.

7. Kurze Antisense-Verbindung nach einem vorhergehenden Anspruch, wobei es sich bei der monomeren Verknüpfung jeweils um eine Phosphorothioat-Internukleosidverknüpfung handelt.

8. Kurze Antisense-Verbindung nach einem vorhergehenden Anspruch, die eine Länge von 10-13 Monomeren, 10-12 Monomeren, 10-11 Monomeren, 10 Monomeren, 11 Monomeren, 12 Monomeren, 13 Monomeren oder 14 Monomeren aufweist.

9. Kurze Antisense-Verbindung nach einem vorhergehenden Anspruch, die eine Nukleotidsequenz, ausgewählt aus SEQ ID NOs: 214-219, 221-226, 228-230, 232-243, 245-246, 251-254, 256-272, 274-281, 283-285, 287-293 und 295-300, umfasst.

10. Kurze Antisense-Verbindung nach einem vorhergehenden Anspruch, die eine Länge von 12 Monomeren aufweist.

11. kurze Antisense-Verbindung nach Anspruch 10, mit einem aus 1-10-1 und 2-8-2 ausgewählten Motiv, wobei die erste Zahl die Anzahl an Monomeren im 5'-Flügel, die zweite Zahl die Anzahl an Monomeren in der Lücke und die dritte Zahl die Anzahl an Monomeren im 3'-Flügel repräsentiert.

12. kurze Antisense-Verbindung nach einem vorhergehenden Anspruch, wobei sich die Verbindung aus einem aus 2'-Desoxynukleotiden bestehenden zentralen Lückensegment, das auf beiden Seiten von sich aus 2'-Methoxyethyl-(2'-MOE-)Nukleotiden zusammensetzenden Flügelsegmenten flankiert ist, zusammensetzt, wobei es sich bei den Internukleosidverknüpfungen über das gesamte Oligonukleotid um Phosphorothioat (P=S) und bei allen Cytidinresten um 5-Methylcytidine handelt.

13. kurze Antisense-Verbindung nach Anspruch 12, die eine Nukleotidsequenz, ausgewählt aus SEQ ID NOs: 275, 276, 277, 278, 279, 280, 281, 283, 284, 285 und 287, umfasst.

14. Kurze Antisense-Verbindung nach einem vorhergehenden Anspruch, wobei die Verbindung die Nukleotidsequenz der SEQ ID NO: 281 umfasst, eine Länge von 12 Monomeren aufweist, ein 2-8-2-Motiv besitzt, wobei die erste Zahl die Anzahl an Monomeren im 5'-Flügel, die zweite Zahl die Anzahl an Monomeren in der Lücke und die dritte Zahl die Anzahl an Monomeren im 3'-Flügel repräsentiert, und wobei sich die Verbindung aus einem aus 2'-Desoxynukleotiden bestehenden zentralen Lückensegment, das auf beiden Seiten von sich aus 2'-Methoxyethyl-(2'-MOE-)Nukleotiden zusammensetzenden Flügelsegmenten flankiert ist, zusammensetzt, wobei es sich bei den Internukleosidverknüpfungen über das gesamte Oligonukleotid um Phosphorothioat (P=S) und bei allen Cytidinresten um 5-Methylcytidine handelt.

15. Kurze Antisense-Verbindung nach einem vorhergehenden Anspruch, bei der es sich um ein pharmazeutisch unbedenkliches Salz handelt.

16. Ex-vivo-Verfahren zur Modulation der Expression von SGLT2 durch Inkontaktbringen einer SGLT2-Nukleinsäure mit einer kurzen Antisense-Verbindung nach einem der Ansprüche 1-15.

17. Kurze Antisense-Verbindung nach einem der Ansprüche 1-15 zur Verwendung bei der Behandlung einer Stoffwechselstörung bei einem Tier.

## Revendications

1. Composé antisens court de 10 à 14 monomères de longueur, comprenant une région de brèche de 2'-désoxyribonucléotide flanquée sur chaque côté par une aile, où chaque aile est constituée indépendamment de 1 à 3 monomères modifiés à affinité élevée qui sont des nucléotides modifiés par un glucide qui comprennent un pont entre la position 4' et 2' du glucide, et où le composé antisens court est ciblé vers un acide nucléique codant pour SGLT2.

2. Composé antisens court de 10 à 14 monomères de longueur, comprenant une région de brèche de 2'-désoxyribonucléotide flanquée sur chaque côté par une aile, où chaque aile est constituée indépendamment de 1 à 3 monomères modifiés à affinité élevée qui sont des nucléotides modifiés par un glucide comprenant le groupe 2'-substituant OCH₂CH₂OCH₃, et où le composé antisens court est ciblé vers un acide nucléique codant pour SGLT2.

3. Composé antisens court de la revendication 1, dans lequel la conformation de chacun desdits nucléotides modifiés par un glucide est, indépendamment, β-D ou α-L.

4. Composé antisens court de la revendication 1, dans lequel chacun desdits ponts comprend indépendamment 1 ou de 2 à 4 groupes liés indépendamment choisis parmi -[C(R₁) (R₂)]ₙ-, -C(R₁)=C(R₂)-, -C(R₁)=N-, -C(=NR₁)-, -C(=O)-, -C(=S)-, -O-, -Si(R₁)2-, -S(=O)ₓ- et -N(R₁)- ;
dans lequel
x est 0, 1 ou 2 ;
n est 1, 2, 3 ou 4 ;
chaque R₁ et R₂ est, indépendamment, H, un groupe protecteur, un hydroxyle, un alkyle en C₁-C₁₂, un alkyle en C₁-C₁₂ substitué, un alcényle en C₂-C₁₂, un alcényle en C₂-C₁₂ substitué, un alcynyle en C₂-C₁₂, un alcynyle en C₂-C₁₂ substitué, un aryle en C₅-C₂₀, un aryle en C₅-C₂₀ substitué, un radical hétérocycle, un radical hétérocycle substitué, un hétéroaryle, un hétéroaryle substitué, un radical alicyclique en C₅-C₇, un radical alicyclique en C₅-C₇ substitué, un halogène, OJ₁, NJ₁J₂, SJ₁, N₃, COOJ₁, un acyl(C(=O)-H), un acyle substitué, CN, un sulfonyl(S(=O)₂-J₁), ou un sulfoxyl(S(=O)-J₁) ; et
chaque J₁ et J₂ est, indépendamment, H, un alkyle en C₁-C₁₂, un alkyle en C₁-C₁₂ substitué, un alcényle en C₂-C₁₂, un alcényle en C₂-C₁₂ substitué, un alcynyle en C₂-C₁₂, un alcynyle en C₂-C₁₂ substitué, un aryle en C₅-C₂₀, un aryle en C₅-C₂₀ substitué, un acyl(C(=O)-H), un acyle substitué, un radical hétérocycle, un radical hétérocycle substitué, un amino(alkyle en C₁-C₁₂), un amino (alkyle en C₁-C₁₂) substitué ou un groupe protecteur.

5. Composé antisens court de la revendication 4, où chacun desdits ponts est, indépendamment, 4'-CH₂-2', 4'-(CH₂)₂-2', 4'-CH₂-O-2', 4'-(CH₂)₂-O-2', 4'-CH₂-O-N(R₁)-2', 4'-CH₂-N(R₁)-O-2', où chaque R₁ est, indépendamment, H, un groupe protecteur ou un alkyle en C₁-C₁₂,

6. Composé antisens court de l'une quelconque des revendications précédentes, où au moins un fragment de liaison monomérique est un fragment de liaison monomérique, tel qu'un fragment de liaison phosphorothioate.

7. Composé antisens court de l'une quelconque des revendications précédentes, où chaque fragment de liaison monomère est un fragment de liaison internucléoside phosphorothioate.

8. Composé antisens court de l'une quelconque des revendications précédentes qui fait de 10 à 13 monomères de longueur, de 10 à 12 monomères de longueur, de 10 à 11 monomères de longueur, de 10 monomères de longueur, de 11 monomères de longueur, de 12 monomères de longueur, de 13 monomères de longueur, ou de 14 monomères de longueur.

9. Composé antisens court de l'une quelconque des revendications précédentes, qui comprend une séquence nucléotidique choisie parmi SEQ ID NO: 214-219, 221-226, 228-230, 232-243, 245-246, 251-254, 256-272, 274-281, 283-285, 287-293 et 295-300.

10. Composé antisens court de l'une quelconque des revendications précédentes, qui fait 12 monomères de longueur.

11. Composé antisens court de la revendication 10, ayant un motif choisi parmi 1-10-1 et 2-8-2 dans lequel le premier nombre représente le nombre de monomères dans l'aile 5', le deuxième nombre représente le nombre de monomères dans la brèche, et le troisième nombre représente le nombre de monomères dans l'aile 3'.

12. Composé antisens court de l'une quelconque des revendications précédentes, dans lequel le composé est constitué d'un segment de brèche central constitué de 2'-désoxynucléotides, qui est flanqué sur les deux côtés par des segments d'aile composés de 2'-méthoxyéthyle (2'-MOE)-nucléotides, où les fragments de liaison internucléoside sont un phosphorothioate (P=S) dans l'ensemble de l'oligonucléotide, et tous les résidus cytidine sont des 5-méthylcytidines.

13. Composé antisens court de la revendication 12, qui comprend une séquence nucléotidique choisie parmi SEQ ID NO: 275, 276, 277, 278, 279, 280, 281, 283, 284, 285 et 287.

14. Composé antisens court de l'une quelconque des revendications précédentes, dans lequel ledit composé comprend la séquence nucléotidique de SEQ ID NO: 281, fait 12 monomères de longueur, a un motif 2-8-2 dans lequel le premier nombre représente le nombre de monomères dans l'aile 5', le deuxième nombre représente le nombre de monomères dans la brèche, et le troisième nombre représente le nombre de monomères dans l'aile 3', et où le composé est constitué d'un segment de brèche central constitué de 2'-désoxynucléotides, qui est flanqué sur les deux côtés par des segments d'aile composés de 2'-méthoxyéthyle (2'-MOE)-nucléotides, où les fragments de liaison internucléoside sont un phosphorothioate (P=S) dans l'ensemble de l'oligonucléotide, et tous les résidus cytidine sont des 5-méthylcytidines.

15. Composé antisens court de l'une quelconque des revendications précédentes, qui est un sel pharmaceutiquement acceptable.

16. Procédé *ex vivo* de modulation de l'expression de SGLT2 par mise en contact d'un acide nucléique SGLT2 avec un composé antisens court de l'une quelconque des revendications 1 à 15.

17. Composé antisens court de l'une quelconque des revendications 1 à 15, pour utilisation dans le traitement d'un trouble métabolique chez un animal.
